# EUROPEAN PATENT APPLICATION

(11) **EP 1 852 440 A1**
(43) Date of publication of application: **07.11.2007**
(21) Application number: 07102532.4
(22) Date of filing: 28.01.2000
(51) Int. Cl.: C07K 14/00, C07K 14/435, C07K 7/08, A61K 38/10, A61K 38/17

(54) **Alpha-conotoxin peptides**

(30) Priority: 29.01.1999 US 118381 P
(62) Divisional of application: 00908382.5
(71) Applicant: University of Utah Research Foundation, Salt Lake City, Utah 84108 (US); Cognetix, Inc., Salt Lake City, UT 84108 (US)
(72) Inventor: Watkins, Maren, Salt Lake City, UT 84105 (US); Hillyard, David, R., Salt Lake City, UT 84108 (US); McIntosh, Michael J., Salt Lake City, UT 84108 (US); Jones, Robert M., San Diego, CA 92122 (US); Olivera, Baldomero M., Salt Lake City, UT 84105 (US)
(74) Representative: Comoglio, Elena

(57) **Abstract**

The invention relates to relatively short peptides (termed α-conotoxins herein), about 10-30 residues in length, which are naturally available in minute amounts in the venom of the cone snails or analogous to the naturally available peptides, and which preferably include two disulfide bonds.

## Description

This invention was made with Government support under Grant No. PO 1 GM48677 awarded by the National Institute of General Medical Sciences, National Institutes of Health, Bethesda, Maryland. The United States Government has certain rights in the invention.

### BACKGROUND OF THE INVENTION

The invention relates to relatively short peptides (termed α-conotoxins herein), about 10-30 residues in length, which are naturally available in minute amounts in the venom of the cone snails or analogous to the naturally available peptides, and which preferably include two disulfide bonds.

The publications and other materials used herein to illuminate the background of the invention, and in particular, cases to provide additional details respecting the practice, for convenience are referenced in the following text by author and date and are listed alphabetically by author in the appended bibliography.

The predatory cone snails *(Conus)* have developed a unique biological strategy. Their venom contains relatively small peptides that are targeted to various neuromuscular receptors and may be equivalent in their pharmacological diversity to the alkaloids of plants or secondary metabolites of microorganisms. Many of these peptides are among the smallest nucleic acid-encoded translation products having defined conformations, and as such, they are somewhat unusual. Peptides in this size range normally equilibrate among many conformations. Proteins having a fixed conformation are generally much larger.

The cone snails that produce these peptides are a large genus of venomous gastropods comprising approximately 500 species. All cone snail species are predators that inject venom to capture prey, and the spectrum of animals that the genus as a whole can envenomate is broad. A wide variety of hunting strategies are used, however, every *Conus* species uses fundamentally the same basic pattern of envenomation.

Several peptides isolated from *Conus* venoms have been characterized. These include the α-, µ- and ω-conotoxins which target nicotinic acetylcholine receptors, muscle sodium channels, and neuronal calcium channels, respectively (Olivera et al., 1985). Conopressins, which are vasopressin analogs, have also been identified (Cruz et al.. 1987). In addition, peptides named conantokins have been isolated from *Conus geographus* and *Conus tulipa* (Mena et al., 1990; Haack et al., 1990).

The α-conotoxins are small peptides highly specific for neuromuscular junction nicotinic acetylcholine receptors (Gray et al.,1981; Marshall and Harvey, 1990; Blount et al.,1992; Jacobsen et al.,1997) or highly specific for neuronal nicotinic acetylcholine receptors (Fainzilber et al., 1994; Johnson et al., 1995; Cartier et al., 1996; Luo et al., 1998). The α-conotoxins with specificity for neuromuscular junction nicotinic acetylcholine receptors are used as neuromuscular blocking agents for use in conjunction with surgery, as disclosed in U.S. Patent No. 6,268,473 and international publication WO00/43409. Additional α-conotoxins and uses for them have been described in U.S. Patent Nos. 4,447,356 (Olivera et al., 1984); 5,432,155; 5,514,774.

Additional uses for α-conotoxins are described in U.S. Patent No. 6,265,541. In this application, α-conotoxins with specificity for neuronal nicotinic acetylcholine receptors are used for treating disorders regulated at neuronal nicotinic acetylcholine receptors. Such disorders include, but are not limited to, cardiovascular disorders, gastric motility disorders, urinary incontinence, nicotine addiction, mood disorders (such as bipolar disorder, unipolar depression, dysthymia and seasonal effective disorder) and small cell lung carcinoma, as well as the localization of small cell lung carcinoma.

It is desired to provide additional α-conotoxins peptides having uses as described herein.

### SUMMARY OF THE INVENTION

The invention relates to relatively short peptides (termed α-conotoxins herein), about 10-30 residues in length, which are naturally available in minute amounts in the venom of the cone snails or analogous to the naturally available peptides, and which preferably include two disulfide bonds.

More specifically, the present invention is directed to α-conotoxin peptides having the general formula I:
Xaa₁-Xaa₂-Xaa₃-Xaa₄-Xaa₅-Cys-Cys-Xaa₆-Xaa₇-Xaa₈-Xaa₉-Cys-Xaa₁₀-Xaa₁₁-Xaa₁₂-Cys-Xaa₁₃ (SEQ ID NO1:), wherein Xaa₁ is des-Xaa₁, Ile, Leu or Val; Xaa₂ is des-Xaa₂, Ala or Gly; Xaa₃ is des-Xaa₃, Gly, Trp (D or L), neo-Trp, halo-Trp or any unnatural aromatic amino acid; Xaa₄ is des-Xaa₄, Asp, Phe, Gly, Ala, Glu, γ-carboxy-Glu (Gla) or any unnatural aromatic amino acid; Xaa₅ is Glu, Gla, Asp, Ala, Thr, Ser, Gly, Ile, Tyr, nor-Tyr, mono-halo-Tyr, di-halo-Tyr, O-sulpho-Tyr, O-phospho-Tyr, nitro-Tyr or any unnatural hydroxy containing amino acid; Xaa₆ is Ser, Thr, Arg, ornithine, homoarginine, Lys, N-methyl-Lys, N,N-dimethyl-Lys, N,N,N-trimethyl-Lys or any unnatural basic amino acid; Xaa₇ is Asp, Glu, Gla, Arg, ornithine, homoarginine, Lys, N-methyl-Lys, N,N-dimethyl-Lys, N,N,N-trimethyl-Lys or any unnatural basic amino acid; Xaa₈ is Ser, Thr, Asn, Ala, Gly, His, halo-His, Pro or hydroxy-Pro; Xaa₉ is Thr, Ser, Ala, Asp, Asn, Pro, hydroxy-Pro, Arg, ornithine, homoarginine, Lys, N-methyl-Lys, N,N-dimethyl-Lys, N,N,N-trimethyl-Lys or any unnatural basic amino acid; Xaa₁₀ is Gly, Ser, Thr, Ala, Asn, Arg, ornithine, homoarginine, Lys, N-methyl-Lys, N,N-dimethyl-Lys, N,N,N-trimethyl-Lys or any unnatural basic amino acid; Xaa₁₁ is Gln, Leu, His, halo-His, Trp (D or L), halo-Trp, neo-Trp, Tyr, nor-Tyr, mono-halo-Tyr, di-halo-Tyr, O-sulpho-Tyr, O-phospho-Tyr, nitro-Tyr, Arg, ornithine, homoarginine, Lys, N-methyl-Lys, N,N-dimethyl-Lys, N,N,N-trimethyl-Lys, any unnatural basic amino acid or any unnatural aromatic amino acid; Xaa₁₂ is Asn, His, halo-His, Ile, Leu, Val, Gln, Arg, ornithine, homoarginine, Lys, N-methyl-Lys, N,N-dimethyl-Lys, N,N,N-trimethyl-Lys or any unnatural basic amino acid; Xaa₁₃ is des-Xaa₁₃, Val, Ile, Leu, Arg, ornithine, homoarginine, Lys, N-methyl-Lys, N,N-dimethyl-Lys, N,N,N-trimethyl-Lys or any unnatural basic amino acid. The C-terminus may contain a free carboxyl group or an amide group. The halo is chlorine, bromine or iodine, preferably iodine for Tyr and His and preferably bromine for Trp. The Cys residues may be in D or L configuration and may optionally be substituted with homocysteine (D or L). The Tyr residues may be substituted with the 3-hydroxyl or 2-hydroxyl isomers and corresponding O-sulpho- and O-phospho-derivatives. The acidic amino acid residues may be substituted with any synthetic acidic bioisoteric amino acid surrogate, e.g., tetrazolyl derivatives of Gly and Ala.

More specifically, the present invention is directed to α-conotoxin peptides having the general formula II:
Xaa₁-Xaa₂-Xaa₃-Xaa₄-Cys-Cys-Xaa₅-Xaa₆-Xaa₇-Xaa₈-Cys-Xaa₉-Xaa₁₀-Xaa₁₁-Xaa₁₂-Xaa₁₃-Xaa₁₄-Cys-Xaa₁₅-Xaa₁₆-Xaa₁₇ (SEQ ID NO:2), wherein Xaa₁ is des-Xaa₁, Asp, Glu or γ-carboxy-Glu (Gla); Xaa₂ is des-Xaa₂, Gln, Ala, Asp, Glu, Gla; Xaa₃ is des-Xaa₃, Gly, Ala, Asp, Glu, Gla, Pro or hydroxy-Pro; Xaa₄ is des-Xaa₄, Gly, Glu, Gla, Gln, Asp, Asn, Pro or hydroxy-Pro; Xaa₅ is Ser, Thr, Gly, Glu, Gla, Asn, Trp (D or L), neo-Trp, halo-Trp, Arg, omithine, homoarginine, Lys, N-methyl-Lys, N,N-dimethyl-Lys, N,N,N-trimethyl-Lys, any unnatural basic amino acid, Tyr, nor-Tyr, mono-halo-Tyr, di-halo-Tyr, O-sulpho-Tyr, O-phospho-Tyr, nitro-Tyr or any unnatural hydroxy containing amino acid; Xaa₆ is Asp, Asn, His, halo-His, Thr, Ser, Tyr, nor-Tyr, mono-halo-Tyr, di-halo-Tyr, O-sulpho-Tyr, O-phospho-Tyr, nitro-Tyr or any unnatural hydroxy containing amino acid; Xaa₇ is Pro or hydroxy-Pro; Xaa₈ is Ala, Ser, Thr, Asp, Val, Ile, Pro, hydroxy-Pro, Tyr, nor-Tyr, mono-halo-Tyr, di-halo-Tyr, O-sulpho-Tyr, O-phospho-Tyr, nitro-Tyr or any unnatural hydroxy containing amino acid; Xaa₉ is Gly, Ile, Leu, Val, Ala, Thr, Ser, Pro, hydroxy-Pro, Phe, Trp (D or L), neo-Trp, halo-Trp, Arg, ornithine, homoarginine, Lys, N-methyl-Lys, N,N-dimethyl-Lys, N,N,N-trimethyl-Lys, any unnatural basic amino acid or any unnatural aromatic amino acid; Xaa₁₀ is Ala, Asn, Phe, Pro, hydroxy-Pro, Glu, Gla, Gln, His, halo-His, Val, Ser, Thr, Arg, ornithine, homoarginine, Lys, N-methyl-Lys, N,N-dimethyl-Lys, N,N,N-trimethyl-Lys or any unnatural basic amino acid; Xaa₁₁ is Thr, Ser, His, halo-His, Leu, Ile, Val, Asn, Met, Pro, hydroxy-Pro, Arg, ornithine, homoarginine, Lys, N-methyl-Lys, N,N-dimethyl-Lys, N,N,N-trimethyl-Lys, any-unnatural basic amino acid, Tyr, nor-Tyr, mono-halo-Tyr, di-halo-Tyr, O-sulpho-Tyr, O-phospho-Tyr, nitro-Tyr or any unnatural hydroxy containing amino acid; Xaa₁₂ is Asn, Pro, hydroxy-Pro, Gln, Ser, Thr, Arg, ornithine, homoarginine, Lys, N-methyl-Lys, N,N-dimethyl-Lys N,N,N-trimethyl-Lys, any unnatural basic amino acid, Tyr, nor-Tyr, mono-halo-Tyr, di-halo-Tyr, O-sulpho-Tyr, O-phospho-Tyr, nitro-Tyr or any unnatural hydroxy containing amino acid; Xaa₁₃ is des-Xaa_{13,} Gly, Thr, Ser, Pro, hydroxy-Pro, Tyr, nor-Tyr, mono-halo-Tyr, di-halo-Tyr, O-sulpho-Tyr, O-phospho-Tyr, nitro-Tyr or any unnatural hydroxy containing amino acid; Xaa₁₄ is des-Xaa₁₄, Ile, Val, Asp, Leu, Phe, Arg, ornithine, homoarginine, Lys, N-methyl-Lys, N,N-dimethyl-Lys, N,N,N-trimethyl-Lys, any unnatural basic amino acid, Tyr, nor-Tyr, mono-halo-Tyr, di-halo-Tyr, O-sulpho-Tyr, O-phospho-Tyr, nitro-Tyr or any unnatural hydroxy containing amino acid; and Xaa₁₅ is des-Xaa₁₅, Gly, Ala, Met, Ser, Thr, Trp (D or L), neo-Trp, halo-Trp, any unnatural aromatic amino acid, Arg, ornithine, homoarginine, Lys, N-methyl-Lys, N,N-dimethyl-Lys, N,N,N-trimethyl-Lys or any unnatural basic amino acid; Xaa₁₆ is des-Xaa₁₆, Trp (D or L), neo-Trp, halo-Trp, any unnatural aromatic amino acid, Arg, ornithine, homoarginine, Lys, N-methyl-Lys, N,N-dimethyl-Lys, N,N,N-trimethyl-Lys or any unnatural basic amino acid; Xaa₁₇ is des-Xaa₁₇, Arg, ornithine, homoarginine, Lys, N-methyl-Lys, N,N-dimethyl-Lys, N,N,N-trimethyl-Lys or any unnatural basic amino acid. The C-terminus may contain a free carboxyl group or an amide group. The halo is preferably bromine, chlorine or iodine, more preferably iodine for His or Tyr and bromine for Trp. The Cys residues may be in D or L configuration and may optionally be substituted with homocysteine (D or L). The Tyr residues may be substituted with the 3-hydroxyl or 2-hydroxyl isomers and corresponding O-sulpho- and O-phospho-derivatives. The acidic amino acid residues may be substituted with any synthetic acidic bioisoteric amino acid surrogate, e.g., tetrazolyl derivatives of Gly and Ala.

More specifically, the present invention is directed to α-conotoxin peptides having the general formula III:
Xaa₁-Xaa₂-Xaa₃-Xaa₄-Xaa₅-Cys-Cys-Xaa₆-Xaa₇-Xaa₈-Xaa₉-Cys-Xaa₁₀-Xaa₁₁-Xaa₁₂-Xaa₁₃-Xaa₁₄-Xaa₁₅-Xaa₁₆-Cys-Xaa₁₇-Xaa₁₈-Xaa₁₉-Xaa₂₀-Xaa₂₁-Xaa₂₂-Xaa₂₃-Xaa₂₄ (SEQ ID NO:3), wherein Xaa₁ is des-Xaa₁, Ser or Thr; Xaa₂ is des-Xaa₂, Asp, Glu, γ-carboxy-Glu (Gla), Asn, Ser or Thr; Xaa₃ is des-Xaa₃, Ala, Gly, Asn, Ser, Thr, Pro, hydroxy-Pro, Arg, ornithine, homoarginine, Lys, N-methyl-Lys, N,N-dimethyl-Lys, N,N,N-trimethyl-Lys or any unnatural basic amino acid; Xaa₄ is des-Xaa₄, Ala, Val, Leu, Ile, Gly, Glu, Gla, Gln, Asp, Asn, Phe, Pro, hydroxy-Pro or any unnatural aromatic amino acid; Xaa₅ is des-Xaa₅, Thr, Ser, Asp, Glu, Gla, Gln, Gly, Val, Asp, Asn, Ala, Pro, hydroxy-Pro, Arg, omithine, homoarginine, Lys, N-methyl-Lys, N,N-dimethyl-Lys, N,N,N-trimethyl-Lys or any unnatural basic amino acid; Xaa₆ is Thr, Ser, Asp, Asn, Met, Val, Ala, Gly, Leu, Ile, Phe, any unnatural aromatic amino acid, Pro, hydroxy-Pro, Tyr, nor-Tyr, mono-halo-Tyr, di-halo-Tyr, O-sulpho-Tyr, O-phospho-Tyr, nitro-Tyr or any unnatural hydroxy containing amino acid; Xaa₇ is Ile, Leu, Val, Ser, Thr, Gln, Asn, Asp, Arg, His, halo-His, Phe, any unnatural aromatic amino acid, homoarginine, ornithine, Lys, N-methyl-Lys, N,N-dimethyl-Lys, N,N,N-trimethyl-Lys, any unnatural basic amino acid, Tyr, nor-Tyr, mono-halo-Tyr, di-halo-Tyr, O-sulpho-Tyr, O-phospho-Tyr, nitro-Tyr or any unnatural hydroxy containing amino acid; Xaa₈ is Pro, hyroxy-Pro, Ser, Thr, Ile, Asp, Leu, Val, Gly, Ala, Phe, any unnatural aromatic amino acid, Arg, omithine, homoarginine, Lys, N-methyl-Lys, N,N-dimethyl-Lys, N,N,N-trimethyl-Lys or any unnatural basic amino acid; Xaa₉ is Val, Ala, Gly, Ile, Leu, Asp, Ser, Thr, Pro, hydroxy-Pro, Arg, ornithine, homoarginine, Lys, N-methyl-Lys, N,N-dimethyl-Lys, N,N,N-trimethyl-Lys or any unnatural basic amino acid; Xaa₁₀ is His, halo-His, Arg, homoarginine, ornithine, Lys, N-methyl-Lys, N,N-dimethyl-Lys, N,N,N-trimethyl-Lys, any unnatural basic amino acid, Asn, Ala, Ser, Thr, Phe, Ile, Leu, Gly, Trp (D or L), neo-Trp, halo-Trp, any unnatural aromatic amino acid, Tyr, nor-Tyr, mono-halo-Tyr, di-halo-Tyr, O-sulpho-Tyr, O-phospho-Tyr, nitro-Tyr or any unnatural hydroxy containing amino acid; Xaa₁₁ is Leu, Gln, Val, Ile, Gly, Met, Ala, Lys, N-methyl-Lys, N,N-dimethyl-Lys, N,N,N-trimethyl-Lys, Ser, Thr, Arg, homoarginine, ornithine, any unnatural basic amino acid, Asn, Glu, Gla, Gln, Phe, Trp (D or L), neo-Trp, halo-Trp or any unnatural aromatic amino acid; Xaa₁₂ is Glu, Gla, Gln, Asn, Asp, Pro, hydroxy-Pro, Ser, Gly, Thr, Lys, N-methyl-Lys, N,N-dimethyl-Lys, N,N,N-trimethyl-Lys, Arg, homoarginine, ornithine, any unnatural basic amino acid, Phe, His, halo-His, any unnatural aromatic amino acid, Leu, Met, Gly; Ala, Tyr, nor-Tyr, mono-halo-Tyr, di-halo-Tyr, 0-sulpho-Tyr, O-phospho-Tyr, nitro-Tyr or any unnatural hydroxy containing amino acid; Xaa₁₃ is His, halo-His, Asn, Thr, Ser, Ile, Val, Leu, Phe, any unnatural aromatic amino acid, Arg, homoarginine, ornithine, Lys, N-methyl-Lys, N.N-dimethyl-Lys, N,N,N-trimethyl-Lys, any unnatural basic amino acid, Tyr, nor-Try, mono-halo-Tyr, di-halo-Tyr, O-sulpho-Tyr, O-phospho-Tyr, nitro-Tyr or any unnatural hydroxy containing amino acid; Xaa₁₄ is Ser, Thr, Ala, Gln, Pro, hydroxy-Pro, Gly, Ile, Leu, Arg, ornithine, homoarginine, Lys, N-methyl-Lys, N,N-dimethyl-Lys, N,N,N-trimethyl-Lys or any unnatural basic amino acid; Xaa₁₅ is Asn, Glu, Gla, Asp, Gly, His, halo-His, Ala, Leu, Gln, Arg, ornithine, homoarginine, Lys, N-methyl-Lys, N,N-dimethyl-Lys, N,N,N-trimethyl-Lys, any unnatural basic amino acid, Tyr, nor-Tyr, mono-halo-Tyr, di-halo-Tyr. O-sulpho-Tyr, O-phospho-Tyr, nitro-Tyr or any unnatural hydroxy containing amino acid; Xaa₁₆ is Met, Ile, Thr, Ser, Val, Leu, Pro, hydroxy-Pro, Phe, any unnatural aromatic amino acid, Tyr, nor-Tyr, mono-halo-Tyr, di-halo-Tyr, O-sulpho-Tyr, O-phospho-Tyr, nitro-Tyr, any unnatural hydroxy containing amino acid, Glu, Gla, Ala, His, halo-His, Arg, ornithine, homoarginine, Lys, N-methyl-Lys. N.N-dimethyl-Lys, N,N,N-trimethyl-Lys or any unnatural basic amino acid; Xaa₁₇ is des-Xaa₁₇, Gly, Asp. Asn, Ala, Ile, Leu, Ser, Thr, His, halo-His, Arg, ornithine, homoarginine, Lys, N-methyl-Lys, N,N-dimethyl-Lys, N,N,N-trimethyl-Lys or any unnatural basic amino acid; Xaa₁₈ is des-Xaa₁₈, Gly, Glu, Gla, Gln, Trp (D or L), neo-Trp, halo-Trp, any unnatural aromatic amino acid, Arg, ornithine, homoarginine, Lys, N-methyl-Lys, N,N-dimethyl-Lys, N,N,N-trimethyl-Lys or any unnatural basic amino acid; Xaa₁₉ is des-Xaa₁₉, Ser, Thr, Val, Ile, Ala, Arg, ornithine, homoarginine, Lys, N-methyl-Lys, N,N-dimethyl-Lys, N,N,N-trimethyl-Lys or any unnatural basic amino acid; Xaa₂₀ is des-Xaa₂₀, Val, Asp, His, halo-His, Arg, ornithine, homoarginine, Lys, N-methyl-Lys, N,N-dimethyl-Lys, N,N,N-trimethyl-Lys or any unnatural basic amino acid; Xaa₂₁ is des-Xaa₂₁, Asn, Pro or hydroxy-Pro; Xaa₂₂ is des-Xaa₂₂, Arg, ornithine, homoarginine, Lys, N-methyl-Lys, N,N-dimethyl-Lys, N,N,N-trimethyl-Lys or any unnatural basic amino acid; Xaa₂₃ is des-Xaa₂₃, Ser or Thr; Xaa₂₄ is des-Xaa₂₄, Leu. Ile or Val; with the proviso that (a) Xaa₅ is not Gly, when Xaa₁ is des-Xaa₁, Xaa₂ is des-Xaa₂, Xaa₃ is des-Xaa₃, Xaa₄ is des-Xaa₄ Xaa₆ is Ser, Xaa₇ is His, Xaa₈ is Pro, Xaa₉ is Ala, Xaa₁₀ is Ser, Xaa₁₁ is Val, Xaa₁₂ is Asn, Xaa₁₃ is Asn, Xaa₁₄ is Pro, Xaa₁₅ is Asp, Xaa₁₆ is Ile, Xaa₁₇ is des-Xaa₁₇, Xaa₁₈ is des-Xaa₁₈, Xaa₁₉ is des-Xaa₁₉, Xaa₂₀ is des-Xaa₂₀, Xaa₂₁ is des-Xaa₂₁, Xaa₂₂ is des-Xaa₂₂, Xaa₂₃ is des-Xaa₂₃, and Xaa₂₄ is des-Xaa₂₄. The C-terminus may contain a free carboxyl group or an amide group. The halo is preferably bromine, chlorine or iodine, more preferably iodine for His and Tyr and bromine for Trp. The Cys residues may be in D or L configuration and may optionally be substituted with homocysteine (D or L). The Tyr residues may be substituted with the 3-hydroxyl or 2-hydroxyl isomers and corresponding O-sulpho- and O-phospho-derivatives. The acidic amino acid residues may be substituted with any synthetic acidic bioisoteric amino acid surrogate, e.g., tetrazolyl derivatives of Gly and Ala.

The present invention is also directed to novel specific α-conotoxin peptides of general formula I having the formulas:
Asp-Xaa₁-Cys-Cys-Ser-Asp-Ser-Arg-Cys-Gly-Xaa₂-Asn-Cys-Leu (SEQ ID NO:4);
Ala-Cys-Cys-Ser-Asp-Arg-Arg-Cys-Arg-Xaa₃-Arg-Cys (SEQ ID NO:5);
Phe-Thr-Cys-Cys-Arg-Arg-Gly-Thr-Cys-Ser-Gln-His-Cys (SEQ ID NO:6);
Asp-Xaa₄-Cys-Cys-Arg-Arg-His-Ala-Cys-Thr-Leu-Ile-Cys (SEQ ID NO:7);
Asp-Xaa₄-Cys-Cys-Arg-Arg-Xaas-Xaas-Cys-Thr-Leu-Ile-Cys (SEQ ID NO:8);
Gly-Cys-Cys-Ser-Asp-Xaa₅-Arg-Cys-Arg-Xaa₄-Arg-Cys-Arg (SEQ ID NO:9);
Gly-Gly-Cys-Cys-Ser-Asp-Xaa₅-Arg-Cys-Ala-Xaa₃-Arg-Cys (SEQ ID NO:10);
Ile-Ala-Xaa₃-Asp-Ile-Cys-Cys-Ser-Xaa₁-Xaa₅-Asp-Cys-Asn-His-Xaa₂-Cys-Val (SEQ ID NO:11); and
Gly-Cys-Cys-Ser-Asp-Xaa₅-Arg-Cys-Xaa₂-His-Gln-Cys (SEQ ID NO:12),
wherein Xaa₁ is Glu or γ-carboxy-Glu (Gla); Xaa₂ is Lys, N-methyl-Lys, N,N-dimethyl-Lys or N,N,N-trimethyl-Lys; Xaa₃ is Trp (D or L), halo-Trp or neo-Trp; Xaa₄ is Tyr, nor-Tyr, mono-halo-Tyr, di-halo-Tyr, O-sulpho-Tyr, O-phospho-Tyr or nitro-Tyr; and Xaa₅ is Pro or hydroxy-Pro; and the C-terminus contains a carboxyl or amide group. The halo is preferably bromine, chlorine or iodine, more preferably iodine for Tyr and bromine for Trp. In addition, the His residues may be substituted with halo-His; the Arg residues may be substituted by Lys, ornithine, homoargine, N-methyl-Lys, N,N-dimethyl-Lys, N,N,N-trimethyl-Lys or any unnatural basic amino acid; the Lys residues may be substituted by Arg, ornithine, homoargine, N-methyl-Lys, N,N-dimethyl-Lys, N,N,N-trimethyl-Lys or any unnatural basic amino acid; the Tyr residues may be substituted with any unnatural hydroxy containing amino acid; the Ser residues may be substituted with Thr; the Thr residues may be substituted with Ser; and the Phe and Trp residues may be substituted with any unnatural aromatic amino acid. The Cys residues may be in D or L configuration and may optionally be substituted with homocysteine (D or L). The Tyr residues may be substituted with the 3-hydroxyl or 2-hydroxyl isomers and corresponding O-sulpho- and O-phospho-derivatives. The acidic amino acid residues may be substituted with any synthetic acidic bioisoteric amino acid surrogate, e.g., tetrazolyl derivatives of Gly and Ala. 8

More specifically, the present invention is directed to the following α-conotoxin peptides of general formula I:
- Im1.1:: SEQ ID NO:4, wherein Xaa₁ is Glu and Xaa₂ is Lys;
- Im1.2:: SEQ ID NO:5, wherein Xaa₃ is Trp;
- Rg1.2:: SEQ ID NO:6;
- Rg 1.6:: SEQ ID NO:7, wherein Xaa₄ is Tyr;
- Rg1.6A:: SEQ ID NO : 8, wherein Xaa₄ is Tyr and Xaa₅ is Pro;
- Rg 1.7:: SEQ ID NO:9, wherein Xaa₄ is Tyr and Xaa₅ is Pro;
- Rg1.9:: SEQ ID NO:10, wherein Xaa₃ is Trp and Xaa₅ is Pro;
- Rg1.10:: SEQ ID NO:11, wherein Xaa₁ is Glu, Xaa₂ is Lys, Xaa₃ is Trp and Xaa₅ is Pro; and
- Rg1.11:: SEQ ID NO:12, wherein Xaa₂ is Lys and Xaa₅ is Pro.
The C-terminus of Im1.1, Rg1.7 an Rg1.10 preferably contains a free carboxyl group. The C-terminus of Im1.2, Rg1.2, Rg1.6, Rg1.6A, Rg1.9 and Rg1.11 preferably contains an amide group.

The present invention is further directed to novel specific α-conotoxin peptides of general formula II having the formulas:
Cys-Cys-Ser-Asp-Xaa₅-Ala-Cys-Xaa₂-Gln-Thr-Xaa₅-Gly-Cys-Arg (SEQ ID NO:13);
Cys-Cys-Xaa₁-Asn-Xaa₅-Ala-Cys-Arg-His-Thr-Gln-Gly-Cys (SEQ ID NO:14);
Gly-Cys-Cys-Xaa₃-His-Xaa₅-Ala-Cys-Gly-Arg-His-Xaa₄-Cys (SEQ ID NO:15);
Ala-Xaa₅-Cys-Cys-Asn-Asn-Xaa₅-Ala-Cys-Val-Xaa₂-His-Arg-Cys (SEQ ID NO:16);
Ala-Xaa₅-Gly-Cys-Cys-Asn-Asn-Xaa₅-Ala-Cys-Val-Xaa₂-His-Arg-Cys (SEQ ID NO:17);
Xaa₅-Xaa₅-Cys-Cys-Asn-Asn-Xaa₅-Ala-Cys-Val-Xaa₂-His-Arg-Cys (SEQ ID NO:18);
Asp-Xaa₁-Asn-Cys-Cys-Xaa₃-Asn-Xaa₅-Ser-Cys-Xaa₅-Arg-Xaa₅-Arg-Cys-Thr (SEQ ID NO:19);
Gly-Cys-Cys-Ser-Thr-Xaa₅-Xaa₅-Cys-Ala-Val-Leu-Xaa₄-Cys (SEQ ID NO:20);
Gly-Cys-Cys-Gly-Asn-Xaa₅-Asp-Cys-Thr-Ser-His-Ser-Cys (SEQ ID NO:21);
Gly-Cys-Cys-Ser-Asn-Xaa₅-Xaa₅-Cys-Ala-His-Asn-Asn,Xaa₅-Asp-Cys-Arg (SEQ ID NO:42);
Gly-Cys-Cys-Xaa₄-Asn-Xaa₅-Val-Cys-Xaa₂-Xaa₂-Xaa₄-Xaa₄-Cys-Xaa₃-Xaa₂ (SEQ ID NO:154);
Xaa₆-Xaa₁-Xaa₅-Gly-Cys-Cys-Arg-His-Xaa₅-Ala-Cys-Gly-Xaa₂-Asn-Arg-Cys (SEQ ID NO:155);
Cys-Cys-Ala-Asp-Xaa₅-Asp-Cys-Arg-Phe-Arg-Xaa₅-Gly-Cys (SEQ ID NO: 156);
Gly-Cys-Cys-Xaa₄-Asn-Xaa₅-Ser-Cys-Xaa₃-Xaa₅-Xaa₂-Thr-Xaa₄-Cys-Ser-Xaa₃-Xaa₂ (SEQ ID NO:157);
Cys-Cys-Ser-Asn-Xaa₅-Thr-Cys-Xaa₂-Xaa₁-Thr-Xaa₄-Gly-Cys (SEQ ID NO: 158);
Cys-Cys-Ala-Asn-Xaa₅-Ile-Cys-Xaa₂-Asn-Thr-Xaa₅-Gly-Cys (SEQ ID NO:159);
Cys-Cys-Asn-Asn-Xaa₅-Thr-Cys-Xaa₂-Xaa₁-Thr-Xaa₄-Gly-Cys (SEQ ID N0:160);
Cys-Cys-Ser-Asn-Xaa₅-Val-Cys-Xaa₂-Xaa₁-Thr-Xaa₄-Gly-Cys (SEQ ID NO: 161);
Gly-Gly-Cys-Cys-Ser-Xaa₄-Xaa₅-Xaa₅-Cys-Ile-Ala-Ser-Asn-Xaa₅-Xaa₂-Cys-Gly (SEQ ID NO: 162);
Gly-Cys-Cys-Ser-His-Xaa₅-Val-Cys-Ser-Ala-Met-Ser-Xaa₅-Ile-Cys (SEQ ID NO: 163);
Gly-Cys-Cys-Xaa₂-Asn-Xaa₅-Xaa₄-Cys-Gly-Ala-Ser-Xaa₂-Thr-Xaa₄-Cys(SEQ ID NO: 164);
Gly-Cys-Cys-Ser-Xaa₄-Xaa₅-Xaa₅-Cys-Phe-Ala-Thr-Asn-Xaa₅-Asp-Cys (SEQ ID NO: 165);
Gly-Gly-Cys-Cys-Ser-Xaa₄-Xaa₅-Xaa₅-Cys-Ile-Ala-Asn-Asn-Xaa₅-Leu-Cys-Ala (SEQ ID NO: 166);
Gly-Gly-Cys-Cys-Ser-Xaa₄-Xaa₅-Xaa₅-Cys-Ile-Ala-Asn-Asn-Xaa₅-Phe-Cys-Ala (SEQ ID NO:167);
Asp-Cys-Cys-Ser-Asn-Xaa₅-Xaa₅-Cys-Ser-Gln-Asn-Asn-Xaa₅-Asp-Cys-Met (SEQ ID NO: 168); and
Asp-Cys-Cys-Ser-Asn-Xaa₅-Xaa₅-Cys-Ala-His-Asn-Asn-Xaa₅-Asp-Cys-Arg (SEQ ID NO: 169),
wherein Xaa₁ is Glu or γ-carboxy-Glu (Gla); Xaa₂ is Lys, N-methyl-Lys, N,N-dimethyl-Lys or N,N,N-trimethyl-Lys; Xaa₃ is Trp (D or L), halo-Trp or neo-Trp; Xaa₄ is Tyr, nor-Tyr, mono-halo-Tyr, di-halo-Tyr, O-sulpho-Tyr, O-phospho-Tyr or nitro-Tyr; and Xaa₅ is Pro or hydroxy-Pro; and the C-terminus contains a carboxyl or amide group. The halo is preferably bromine, chlorine or iodine, more preferably iodine for Tyr and bromine for Trp. In addition, the His residues may be substituted with halo-His; the Arg residues may be substituted by Lys, ornithine, homoargine, N-methyl-Lys, N,N-dimethyl-Lys, N,N,N-trimethyl-Lys or any unnatural basic amino acid; the Lys residues may be substituted by Arg, ornithine, homoargine, N-methyl-Lys, N,N-dimethyl-Lys, N,N,N-trimethyl-Lys or any unnatural basic amino acid; the Tyr residues may be substituted with any unnatural hydroxy containing amino acid; the Ser residues may be substituted with Thr; the Thr residues may be substituted with Ser; and the Phe and Trp residues may be substituted with any unnatural aromatic amino acid. The Cys residues may be in D or L configuration and may optionally be substituted with homocysteine (D or L). The Tyr residues may be substituted with the 3-hydroxyl or 2-hydroxyl isomers and corresponding O-sulpho- and O-phospho-derivatives. The acidic amino acid residues may be substituted with any synthetic acidic bioisoteric amino acid surrogate, e.g., tetrazolyl derivatives of Gly and Ala.

More specifically, the present invention is directed to the following α-conotoxin peptides of general formula II:
- Sn1.1:: SEQ ID NO: 13, wherein Xaa₂ is Lys and Xaa₅ is Pro;
- Sn1.2:: SEQ ID NO: 14, wherein Xaa₁ is Glu and Xaa₅ is Pro;
- S11.3:: SEQ ID NO: 15, wherein Xaa₃ is Trp, Xaa₄ is Tyr and Xaa₅ is Pro;
- A1.2:: SEQ ID NO: 16, wherein Xaa₂ is Lys and Xaa₅ is Pro;
- Bu1.1:: SEQ ID NO: 17, wherein Xaa₂ is Lys and Xaa₅ is Pro;
- Bu1.2:: SEQ ID NO: 18, wherein Xaa₂ is Lys and Xaa₅ is Pro;
- Bu 1.3:: SEQ ID NO: 19, wherein Xaa₁ is Glu, Xaa₃ is Trp and Xaa₅ is Pro;
- Bu1.4:: SEQ ID NO:20, wherein Xaa₄ is Tyr and Xaa₅ is Pro ;
- Cr1.3:: SEQ ID NO:21, wherein Xaa₅ is Pro;
- Di1.1:: SEQ ID NO:42 wherein Xaa₅ is Pro;
- Ms1.7:: SEQ ID NO: 154, wherein Xaa₂ is Lys, Xaa₃ is Trp, Xaa₄ is Tyr and Xaa₅ is Pro;
- P1.7:: SEQ ID N0:155, wherein Xaa₁ is Glu, Xaa₂ is Lys, Xaa₅ is Pro and Xaa₆ is GIn;
- Ms1.2:: SEQ ID NO:156, wherein Xaa₅ is Pro;
- Ms1.3:: SEQ ID NO: 157, wherein Xaa₂ is Lys, Xaa₃ is Trp, Xaa₄ is Tyr and Xaa₅ is Pro;
- Ms1.4:: SEQ ID NO:158, wherein Xaa₁ is Glu, Xaa₂ is Lys, Xaa₄ is Tyr and Xaa₅ is Pro;
- Ms1.5:: SEQ ID NO:159, wherein Xaa₂ is Lys and Xaa₅ is Pro;
- Ms1.8:: SEQ ID NO:160, wherein Xaa₁ is Glu, Xaa₂ is Lys, Xaa₄ is Tyr and Xaa₅ is Pro;
- Ms1.9:: SEQ ID NO:161, wherein Xaa₁ is Glu, Xaa₂ is Lys, Xaa₄ is Tyr and Xaa₅ is Pro;
- Bt1.7:: SEQ ID NO:162, wherein Xaa₂ is Lys, Xaa₄ is Tyr and Xaa₅ is Pro;
- Lv1.5:: SEQ ID NO:163, wherein Xaa₅ is Pro;
- Ms1.10:: SEQ ID NO:164, wherein Xaa₂ is Lys, Xaa₄ is Tyr and Xaa₅ is Pro;
- Om1.1:: SEQ ID NO:165, wherein Xaa₄ is Tyr and Xaa₅ is Pro;
- R1.6:: SEQ ID NO:166, wherein Xaa₄ is Tyr and Xaa₅ is Pro;
- R1.7:: SEQ ID NO:167, wherein Xaa₄ is Tyr and Xaa₅ is Pro;
- Vr1.1:: SEQ ID NO:168, wherein Xaa₅ is Pro; and
- Vr1.2:: SEQ ID NO:169, wherein Xaa₅ is Pro.
The C-terminus preferably contains a carboxyl group for the peptides Sn1.1, Sn1.2, Cr1.3, Di1.1, Ms1.2, Ms1.4, Ms1.5, Ms1.8, Ms1.9, Vr1.1 and Vr1.2. The C-terminus of the other peptides preferably contains an amide group.

The present invention is also directed to novel specific α-conotoxin peptides of general formula III having the formulas:
Gly-Cys-Cys-Ser-Asn-Xaa₅-Val-Cys-His-Leu-Xaa₁-His-Ser-Asn-Met-Cys (SEQ ID NO:22);
Gly-Cys-Cys-Ser-Asn-Xaa₅-Val-Cys-Arg-Gln-Asn-Asn-Ala-Xaa₁-Xaa₄-Cys-Arg (SEQ ID NO:23);
Xaa₅-Gln-Cys-Cys-Ser-His-Xaa₅-Ala-Cys-Asn-Val-Asp-His-Xaa₁-Xaa₁-Ile-Cys-Arg (SEQ ID NO:24);
Xaa₅-Xaa₁-Cys-Cys-Ser-His-Xaa₅-Ala-Cys-Asn-Val-Asp-His-Xaa₅-Xaa₁-Ile-Cys-Arg (SEQ ID NO:25);
Xaa₅-Gln-Cys-Cys-Ser-His-Xaa₅-Ala-Cys-Asn-Val-Asp-His-Xaa₅-Xaa₁,-Ile-Cys-Asp (SEQ ID NO:26);
Xaa₅-Arg-Cys-Cys-Ser-His-Xaa₅-Ala-Cys-Asn-Val-Asp-His-Xaa₅-Xaa₁-Ile-Cys-Arg (SEQ ID NO:27);
Xaa₅-Gln-Cys-Cys-Ser-His-Xaa₅-Ala-Cys-Asn-Val-Asp-His-Xaa₅-Gly-Ile-Cys-Arg (SEQ ID NO:28);
Xaa₅-Gln-Cys-Cys-Ser-His-Xaa₅-Ala-Cys-Asn-Val-Asp-His-Xaa₅-Xaa₁-Thr-Cys-Arg (SEQ ID NO:29);
Xaa₅-Gln-Cys-Cys-Ser-His-Xaa₅-Ala-Cys-Asn-Val-Asp-His-Xaa₅-Xaa₁-Val-Cys-Arg (SEQ ID NO:30);
Xaa₅-Gln-Cys-Cys-Ser-His-Xaa₅-Ala-Cys-Asn-Ile-Asp-His-Xaa₅-Xaa₁-Ile-Cys-Arg (SEQ ID NO:31); 
Xaa5-Gln-Cys-Cys-Ser-His-Xaa5-Ala-Cys-Asn-Val-Asp-His-Xaa5-Xaa1-Ile-Cys-Arg-Arg-
Arg-Arg (SEQ ID NO:32);
Gly-Gly-Cys-Cys-Ser-His-Xaa₅-Ala-Cys-Ala-Val-Asn-His-Xaa₅-Xaa₁-Leu-Cys (SEQ ID NO:33);
Gly-Cys-Cys-Ser-His-Xaa₅-Ala-Cys-Ser-Val-Asn-His-Xaa₅-Xaa₁-Leu-Cys (SEQ ID NO:34);
Gly-Cys-Cys-Ser-His-Xaa₅-Ala-Cys-Asn-Val-Asp-His-Xaa₅-Xaa₁-lie-Cys(SEQ ID NO:35);
Gly-Cys-Cys-Ser-His-Xaa₅-Ala-Cys-Ser-Gly-Xaa₂-Thr-Gln-Xaa₁-Xaa₅-Cys-Arg-Xaa₁-Ser (SEQ ID NO:36);
Xaa₅-Cys-Cys-Ser-His-Xaa₅-Ala-Cys-Ser-Gly-Asn-Asn-Xaa₅-Xaa₁-Phe-Cys-Arg-Gln (SEQ ID NO:37);
Gly-Cys-Cys-Ser-His-Xaa₅-Ala-Cys-Ser-Gly-Asn-Asn-Xaa₅-Xaa₁-Phe-Cys-Arg-Gln (SEQ ID NO:38);
Gly-Cys-Cys-Ser-His-Xaa₅-Xaa₅-Cys-Ala-Met-Asn-Asn-Xaa₅-Asp-Xaa₄-Cys (SEQ ID NO:39);
Gly-Cys-Cys-Ser-His-Xaa₅-Xaa₅-Cys-Phe-Leu-Asn-Asn-Xaa₅-Asp-Xaa₄-Cys (SEQ ID NO:40);
Gly-Cys-Cys-Ser-Asn-Xaa₅-Xaa₅-Cys-He-Ala-Xaa₂-Asn-Xaa₅-His-Met-Cys-Gly (SEQ ID NO:41);
Gly-Cys-Cys-Ser-Asn-Xaa₅-Ala-Cys-Ala-Gly-Asn-Asn-Xaa₅-His-Val-Cys-Arg-Gln (SEQ ID NO:43);
Gly-Cys-Cys-Ser-Arg-Xaa₅-Ala-Cys-Ile-Ala-Asn-Asn-Xaa₅-Asp-Leu-Cys (SEQIDNO:44); 
Gly-Cys-Cys-Ser-Asn-Xaa5-Val-Cys-His-Val-Xaa1-His-Xaa5-Xaa1-Leu-Cys-Arg-Arg-Arg-
Arg (SEQ ID NO:45);
Gly-Gly-Cys-Cys-Ser-Phe-Xaa₅-Ala-Cys-Arg-Xaa₂-Xaa₅-Arg-Xaa₅-Xaa₁-Met-Cys-Gly (SEQ ID NO:46);
Xaa₅-Xaa₁-cys-cys-Ser-Asp-Xaa₅-Arg-cys-Asn-Ser-Ser-His-Xaa₅-Xaa₁-Leu-cys-Gly(SEQ ID NO:47);
Xaa₅-Gln-Cys-Cys-Ser-Asp-Xaa₅-Arg-Cys-Asn-Val-Gly-His-Xaa₅-Xaa₁,-Leu-Cys-Gly(SEQ ID NO:48);
Xaa₆-Val-Cys-Cys-Ser-Asp-Xaa₅-Arg-Cys-Asn-Val-Gly-His-Xaa₅-Xaa₁-Ile-Cys-Gly (SEQ ID NO:49);
Gly-Cys-Cys-Ser-Arg-Xaa₅-Xaa₅-Cys-Ile-Ala-Asn-Asn-Xaa₅-Asp-Leu-Cys (SEQ ID NO:50);
Xaa₅-Gln-Cys-Cys-Ser-His-Leu-Ala-Cys-Asn-Val-Asp-His-Xaa₅-Xaa₁-Ile-Cys-Arg (SEQ ID NO:51);
Gly-Cys-Cys-Ser-Xaa₄-Phe-Asp-Cys-Arg-Met-Met-Phe-Xaa₅-Xaa₁-Met-Cys-Gly-Xaa₃-Arg (SEQ ID NO:52);
Gly-Gly-Cys-Cys-Ser-Phe-Ala-Ala-Cys-Arg-Xaa₂-Xaa₄-Arg-Xaa₅-Xaa₁-Met-Cys-Gly(SEQ ID NO:53); 
Gly-Gly-Cys-Cys-Phe-His-Xaa5-Val-Cys-Xaa4-Ile-Asn-Leu-Leu-Xaa1-Met-Cys-Arg-Gln-
Arg (SEQ ID NO:54);
 
Ser-Ala-Thr-Cys-Cys-Asn-Xaa4-Xaa5-Xaa5-Cys-Xaa4-Xaa1-Thr-Xaa4-Xaa5-Xaa1-Ser-Cys-
Leu (SEQ ID NO:55);
Ala-Cys-Cys-Ala-Xaa₄-Xaa₅-Xaa₅-Cys-Phe-Xaa₁-Ala-Xaa₄-Xaa₅-Xaa₁-Arg-Cys-Leu (SEQ ID NO:56); 
Asn-Ala-Xaa1-Cys-Cys-Xaa4-Xaa4-Xaa5-Xaa5-Cys-Xaa4-Xaa1-Ala-Xaa4-Xaa5-Xaa1-Ile-Cys-
Leu (SEQ ID NO:57);
 
Xaa1-Cys-Cys-Thr-Asn-Xaa5-Val-Cys-His-Ala-Xaa1-His-Gln-Xaa1-Leu-Cys-Ala-Arg-Arg-
Arg (SEQ ID NO:170);
Gly-Cys-Cys-Ser-Asn-Xaa₅-Val-Cys-His-Leu-Xaa₁-His-Ser-Asn-Leu-Cys (SEQ ID NO:171); 
Xaa1-Cys-Cys-Thr-Asn-Xaa5-Val-Cys-His-Val-Xaa1-His-Gln-Xaa1-Leu-Cys-Ala-Arg-Arg-
Arg (SEQ ID NO:172);
Xaa₆-Xaa₁-Cys-Cys-Ser-Xaa₄-Xaa₅-Ala-Cys-Asn-Leu-Asp-His-Xaa₅-Xaa₁-Leu-Cys (SEQ ID NO:173);
Xaa₅-Xaa₁-Cys-Cys-Ser-Asp-Xaa₅-Arg-Cys-Asn-Ser-Thr-His-Xaa₅-Xaa₁-Leu-Cys-Gly(SEQ ID NO:174); 
Leu-Asn-Cys-Cys-Met-Ile-Xaa5-Xaa5-Cys-Xaa3-Xaa2-Xaa2-Xaa4-Gly-Asp-Arg-Cys-Ser-
Xaa1-Val-Arg (SEQ ID NO:175);
 
Ala-Phe-Gly-Cys-Cys-Asp-Leu-Ile-Xaa5-Cys-Leu-Xaa1-Arg-Xaa4-Gly-Asn-Arg-Cys-Asn-
Xaa1-Val-His (SEQ ID NO:176);
 
Leu-Gly-Cys-Cys-Asn-Val-Thr-Xaa5-Cys-Xaa3-Xaa1-Xaa2-Xaa4-Gly-Asp-Xaa2-Cys-Asn-
Xaa1-Val-Arg (SEQ ID NO:177);
 
Asp-Xaa1-Cys-Cys-Ser-Asn-Xaa5-Ala-Cys-Arg-Val-Asn-Asn-Xaa5-His-Val-Cys-Arg-Arg-
Arg (SEQ ID NO:178);
 
Leu-Asn-Cys-Cys-Ser-Ile-Xaa5-Gly-Cys-Xaa3-Asn-Xaa1-Xaa4-Xaa2-Asp-Arg-Cys-Ser-Xaa2-
Val-Arg (SEQ ID NO:179);
Gly-Gly-Cys-Cys-Ser-His-Xaa₅-Val-Cys-Xaa₄-Phe-Asn-Asn-Xaa₅-Gln-Met-Cys-Arg (SEQ ID NO: 180)
Gly-Gly-Cys-Cys-Ser-His-Xaa₅-Val-Cys-Asn-Leu-Asn-Asn-Xaa₅-Gln-Met-Cys-Arg (SEQ ID NO: 181)
Gly-Cys-Cys-Ser-His-Xaa₅-Xaa₅-Cys-Xaa₄-Ala--Asn-Asn-Gln-Ala-Xaa₄-Cys-Asn (SEQ ID NO: 182)
Gly-Gly-Cys-Cys-Ser-His-Xaa₅-Ala-Cys-Ser-Val-Thr-His-Xaa₅-Xaa₁--Leu-Cys (SEQ ID NO: 183)
Gly-Gly-Cys-Cys-Ser-His-Xaa₄-Xaa₅-Ala-Cys-Ser-Val-Xaa₁-His-Gln-Asp-Leu-Cys-Asp (SEQ ID NO: 184) 
Val-Ser-Cys-Cys-Val-Val-Arg-Xaa5-Cys-Xaa3-Ile-Arg-Xaa4-Gln-Xaa1-Xaa1-Cys-Leu-Xaa1-
Ala-Asp-Xaa5-Arg-Thr-Leu (SEQ ID NO:185);
 
Xaa6-Asn-Cys-Cys-Ser-Ile-Xaa5-Gly-Cys-Xaa3-Xaa1-Xaa2-Xaa4-Gly-Asp-Xaa2-Cys-Ser-
Xaa1-Val-Arg (SEQ ID NO:186);
Gly-Cys-Cys-Ser-Asn-Xaa₅-Val-Cys-His-Leu-Xaa₁-His-Xaa₅-Asn-Ala-Cys (SEQ ID NO:187);
Gly-Cys-Cys-Ser-Asn-Xaa₅-Ile-Cys-Xaa₄-Phe-Asn-Asn-Xaa₅-Arg-Ile-Cys-Arg (SEQ ID NO:188);
Xaa₁-Cys-Cys-Ser-Gln-Xaa₅-Xaa₅-Cys-Arg-Xaa₃-Xaa₂-His-Xaa₅-Xaa₁-Leu-Cys-Ser (SEQ ID NO:189);
Gly-Cys-Cys-Ser-His-Xaa₅-Ala-Cys-Ala-Gly-Asn-Asn-Gln-His-Ile-Cys (SEQ ID NO:190);
Gly-Cys-Cys-Ala-Val-Xaa₅-Ser-Cys-Arg-Leu-Arg-Asn-Xaa₅-Asp-Leu-Cys-Gly-Gly (SEQ ID NO:191);
Gly-Cys-Cys-Ser-His-Xaa₅-Ala-Cys-Asn-Val-Asn-Asn-Xaa₅-His-Ile-Cys(SEQIDNO:192); 
Thr-Xaa5-Xaa1-Xaa1-Cys-Cys-Xaa5-Asn-Xaa5-Xaa5-Cys-Phe-Ala-Thr-Asn-Ser-Asp-Ile-Cys-
Gly (SEQ ID NO:193);
Asp-Ala-Cys-Cys-Ser-Asp-Xaa₅-Arg-Cys-Ser-Gly-Xaa₂-His-Gln-Asp-Leu-Cys (SEQ ID NO:194);
Xaa₁-Asp-Cys-Cys-Ser-Asp-Xaa₅-Arg-Cys-Ser-Val-Gly-His-Gln-Asp-Leu-Cys (SEQ ID NO:195);
Gly-Cys-Cys-Ser-His-Xaa₅-Ala-Cys-Ala-Gly-Ser-Asn-Ala-His-Ile-Cys (SEQ ID NO:196);
Xaa₁-Asp-Cys-Cys-Ser-Asp-Xaa₅-Arg-Cys-Ser-Val-Gly-His-Gln-Asp-Met-Cys (SEQ ID NO:197);
Gly-Cys-Cys-Ser-His-Xaa₅-Ala-Cys-Ala-Gly-Asn-Asn-Xaa₅-His-Ile-Cys (SEQ ID NO:198);
Gly-Cys-Cys-Gly-Asn-Xaa₅-Ser-Cys-Ser-Ile-His-Ile-Xaa₅-Xaa₄-Val-Cys-Asn (SEQ ID NO:199); 
Thr-Asp-Ser-Xaa1-Xaa1-Cys-Cys-Leu-Asp-Ser-Arg-Cys-Ala-Gly-Gln-His-Gln-Asp-Leu-
Cys-Gly (SEQ ID NO:200);
Gly-Cys-Cys-Ser-Asn-Xaa₅-Xaa₅-Cys-Xaa₄-Ala-Asn-Asn-Gln-Ala-Xaa₄-Cys-Asn (SEQ ID NO:201);
Gly-Cys-Cys-Ser-His-Xaa₅-Ala-Cys-Ser-Val-Asn-Asn-Xaa₅-Asp-Ile-Cys(SEQIDNO:202);
Gly-Xaa₂-Cys-Cys-Ile-Asn-Asp-Ala-Cys-Arg-Ser-Xaa₂-His-Xaa₂-Gln-Xaa₄-Cys-Ser (SEQ ID NO:203);
Gly-Cys-Cys-Xaa₄-Asn-Ile-Ala-Cys-Arg-Ile-Asn-Asn-Xaa₅-Arg-Xaa₄-Cys-Arg (SEQ ID NO:204);
Gly-Cys-Cys-Ser-His-Xaa₅-Val-Cys-Arg-Phe-Asn-Xaa₄-Xaa₅-Xaa₂-Xaa₄-Cys-Gly (SEQ ID NO:205);
Asp-Xaa₁-Cys-Cys-Ala-Ser-Xaa₅-Xaa₅-Cys-Arg-Leu-Asn-Asn-Xaa₅-Xaa₄-Val-Cys-His (SEQ ID NO:206);
Gly-Cys-Cys-Ser-Asn-Xaa₅-Val-Cys-Xaa₃-Gln-Asn-Asn-Ala-Xaa₁-Xaa₄-Cys-Arg-Xaa₁-Ser (SEQ ID NO:207);
Gly-Cys-Cys-Ser-His-Xaa₅-Xaa₅-Cys-Ala-Gln-Asn-Asn-Gln-Asp-Xaa₄-Cys (SEQ ID NO:208);
Gly-Cys-Cys-Ser-His-Xaa₅-Ala-Cys-Ser-Gly-Asn-Asn-Arg-Xaa₁-Xaa₄-Cys-Arg-Xaa₁-Ser (SEQ ID NO:209);
Asp-Xaa₅-Cys-Cys-Ser-Xaa₄-Xaa₅-Asp-Cys-Gly-Ala-Asn-His-Xaa₅-Xaa₁-Ile-Cys-Gly(SEQ ID NO:210);
Xaa₁-Cys-Cys-Ser-Gln-Xaa₅-Xaa₅-Cys-Arg-Xaa₃-Xaa₂-His-Xaa₅-Xaa₁-Leu-Cys-Ser (SEQ ID NO:211);
Gly-Cys-Cys-Ser-His-Xaa₅-Ala-Cys-Ala-Gly-Asn-Asn-Xaa₅-His-lie-Cys(SEQ ID NO:212);
Gly-Cys-Cys-Ser-Asp-Xaa₅-Ser-Cys-Asn-Val-Asn-Asn-Xaa₅-Asp-Xaa₄-Cys (SEQ ID NO:213);
Xaa₁-Xaa₁-Cys-Cys-Ser-Asp-Xaa₅-Arg-Cys-Ser-Val-Gly-His-Gln-Asp-Met-Cys-Arg (SEQ ID NO:214);
Gly-Gly-Cys-Cys-Ser-Asn-Xaa₅-Ala-Cys-Leu-Val-Asn-His-Leu-Xaa₁-Met-Cys (SEQ ID NO:215);
Arg-Asp-Xaa₅-Cys-Cys-Phe-Asn-Xaa₅-Ala-Cys-Asn-Val-Asn-Asn-Xaa₅-Gln-Ile-Cys (SEQ ID NO:216); 
Cys-Cys-Ser-Asp-Xaa5-Ser-Cys-Xaa3-Arg-Leu-His-Ser-Leu-Ala-Cys-Thr-Gly-Ile-Val-Asn-
Arg (SEQ ID NO:217);
Cys-Cys-Thr-Asn-Xaa₅-Ala-Cys-Leu-Val-Asn-Asn-Ile-Arg-Phe-Cys-Gly(SEQIDNO:218);
Asp-Xaa₁-Cys-Cys-Ser-Asp-Xaa₅-Arg-Cys-His-Gly-Asn-Asn-Arg-Asp-His-Cys-Ala (SEQ ID NO:219);
Asp-Cys-Cys-Ser-His-Xaa₅-Leu-Cys-Arg-Leu-Phe-Val-Xaa₅-Gly-Leu-Cys-Ile (SEQ ID NO:220);
Gly-Cys-Cys-Ser-His-Xaa₅-Val-Cys-Xaa₂-Val-Arg-Xaa₄-Xaa₅-Asp-Leu-Cys-Arg (SEQ ID NO:221);
Gly-Cys-Cys-Ser-His-Xaa₅-Ala-Cys-Asn-Val-Asn-Asn-Xaa₅-His-Ile-Cys(SEQIDNO:222);
Gly-Cys-Cys-Ser-His-Xaa₅-Val-Cys-Xaa₂-Val-Arg-Xaa₄-Ser-Asp-Met-Cys (SEQ ID NO:223);
Gly-Gly-Cys-Cys-Ser-His-Xaa₅-Ala-Cys-Xaa₂-Val-His-Phe-Xaa₅-His-Ser-Cys (SEQ ID NO:224); 
Val-Cys-Cys-Ser-Asn-Xaa5-Val-Cys-His-Val-Asp-His-Xaa5-Xaa1-Leu-Cys-Arg-Arg-Arg-
Arg (SEQ ID NO:225);
Gly-Cys-Cys-Ser-His-Xaa₅-Val-Cys-Asn-Leu-Ser-Asn-Xaa₅-Gln-Ile-Cys-Arg (SEQ ID NO:226);
Xaa₆-Xaa₁-Cys-Cys-Ser-His-Xaa₅-Ala-Cys-Asn-Val-Asp-His-Xaa₅-Xaa₁-Ile-Cys-Arg (SEQ ID NO:227);
Gly-Cys-Cys-Ser-Asn-Xaa₅-Ala-Cys-Leu-Val-Asn-His-Ile-Arg-Phe-Cys-Gly (SEQ ID NO:228);
Asp-Cys-Cys-Asp-Asp-Xaa₅-Ala-Cys-Thr-Val-Asn-Asn-Xaa₅-Gly-Leu-Cys-Thr (SEQ ID NO:229); and 
Gly-Cys-Cys-Ser-Asn-Xaa5-Xaa5-Cys-Ile-Ala-Xaa2-Asn-Xaa5-His-Met-Cys-Gly-Gly-Arg-
Arg (SEQ ID NO:230),
wherein Xaa₁ is Glu or γ-carboxy-Glu (Gla); Xaa₂ is Lys, N-methyl-Lys, N,N-dimethyl-Lys or N,N,N-trimethyl-Lys; Xaa₃ is Trp (D or L), halo-Trp or neo-Trp; Xaa₄ is Tyr, nor-Tyr, mono-halo-Tyr, di-halo-Tyr, O-sulpho-Tyr, O-phospho-Tyr or nitro-Tyr; and Xaa₅ is Pro or hydroxy-Pro; Xaa₆ is Gln or pyro-Glu; and the C-terminus contains a carboxyl or amide group. The halo is preferably bromine, chlorine or iodine, more preferably iodine for Tyr and bromine for Trp. In addition, the His residues may be substituted with halo-His; the Arg residues may be substituted by Lys, ornithine, homoargine, N-methyl-Lys, N,N-dimethyl-Lys, N,N,N-trimethyl-Lys or any unnatural basic amino acid; the Lys residues may be substituted by Arg, ornithine, homoargine, N-methyl-Lys, N,N-dimethyl-Lys, N,N,N-trimethyl-Lys or any unnatural basic amino acid; the Tyr residues may be substituted with any unnatural hydroxy containing amino acid; the Ser residues may be substituted with Thr; the Thr residues may be substituted with Ser; and the Phe and Trp residues may be substituted with any unnatural aromatic amino acid. The Cys residues may be in D or L configuration and may optionally be substituted with homocysteine (D or L). The Tyr residues may be substituted with the 3-hydroxyl or 2-hydroxyl isomers and corresponding O-sulpho- and O-phospho-derivatives. The acidic amino acid residues may be substituted with any synthetic acidic bioisoteric amino acid surrogate, e.g., tetrazolyl derivatives of Gly and Ala.

More specifically, the present invention is directed to the following α-conotoxin peptides of general formula III:
- SmI:: SEQ ID NO:22, wherein Xaa₁ is Glu and Xaa₅ is Pro;
- OB-29:: SEQ ID NO:23, wherein Xaa₁ is Glu, Xaa₃ is Tyr and Xaa₅ is Pro;
- Tx 1.1:: SEQ ID NO:24, wherein Xaa₁ is Glu and Xaa₅ is Pro;
- R1.1A:: SEQ ID NO:25, wherein Xaa₁ is Glu and Xaa₅ is Pro;
- R1.1B:: SEQ ID NO:26, wherein Xaa₁ is Glu and Xaa₅ is Pro;
- Om-9:: SEQ ID NO:27, wherein Xaa₁ is Glu and Xaa₅ is Pro;
- Om-10:: SEQ ID NO:28, wherein Xaa₅ is Pro;
- Om-21:: SEQ ID NO:29, wherein Xaa₁ is Glu and Xaa₅ is Pro;
- Om-25:: SEQ ID NO:30, wherein Xaa₁ is Glu and Xaa₅ is Pro;
- Om-27:: SEQ ID NO:31, wherein Xaa₁ is Glu and Xaa₅ is Pro;
- Om-28:: SEQ ID NO:32, wherein Xaa₁ is Glu and Xaa₅ is Pro;
- Bt1.2:: SEQ ID NO:33, wherein Xaa₁ is Glu and Xaa₅ is Pro;
- Bt1.4:: SEQ ID NO:34, wherein Xaa₁ is Glu and Xaa₅ is Pro;
- Da1.1:: SEQ ID NO:35, wherein Xaa₁ is Glu and Xaa₅ is Pro;
- OB-20:: SEQ ID NO:36, wherein Xaa₁ is Glu, Xaa₂ is Lys and Xaa₅ is Pro;
- TI:: SEQ ID NO:37, wherein Xaa₁ is Glu and Xaa₅ is Pro;
- TIB:: SEQ ID NO:38, wherein Xaa₁ is Glu and Xaa₅ is Pro;
- Pn1.1:: SEQ ID NO:39, wherein Xaa₅ is Pro;
- Pn1.2:: SEQ ID NO:40, wherein Xaa₁ is Glu and Xaa₅ is Pro;
- T1:: SEQ ID NO:41, wherein Xaa₂ is Lys and Xaa₅ is Pro;
- TIA:: SEQ ID NO:43, wherein Xaa₅ is Pro;
- Da1.2:: SEQ ID NO:44, wherein Xaa₅ is Pro;
- Cr1.2:: SEQ ID NO:45, wherein Xaa₁ is Glu and Xaa₅ is Pro;
- SI1.2:: SEQ ID NO:46, wherein Xaa₁ is Glu, Xaa₂ is Lys and Xaa₅ is Pro;
- Tx 1.3 :: SEQ ID NO:47, wherein Xaa₁ is Glu and Xaa₅ is Pro;
- Da1.3:: SEQ ID NO:48, wherein Xaa₁ is Glu and Xaa₅ is Pro;
- Da1.4:: SEQ ID NO:49, wherein Xaa₁ is Glu, Xaa₅ is Pro and Xaa₆ is Gln;
- Tx1.2:: SEQ ID NO:50, wherein Xaa₅ is Pro;
- Om-35:: SEQ ID NO:51, wherein Xaa₁ is Glu and Xaa₅ is Pro;
- Sl1.1:: SEQ ID NO:52, wherein Xaa₁ is Glu, Xaa₃ is Trp, Xaa₄ is Tyr and Xaa₅ is Pro;
- S11.6:: SEQ ID NO:53, wherein Xaa₁ is Glu, Xaa₂ is Lys, Xaa₄ is Tyr and Xaa₅ is Pro;
- S11.7:: SEQ ID NO:54, wherein Xaa₁ is Glu Xaa₄ is Tyr and Xaa₅ is Pro;
- Bt1.1:: SEQ ID NO:55, wherein Xaa₁ is Glu Xaa₄ is Tyr and Xaa₅ is Pro;
- Bt:1.3:: SEQ ID NO:56, wherein Xaa₁ is Glu Xaa₄ is Tyr and Xaa₅ is Pro;
- Bt1.5:: SEQ ID NO:57, wherein Xaa₁ is Glu Xaa₄ is Tyr and Xaa₅ is Pro;
- A1.4:: SEQ ID NO:170, wherein Xaa₁ is Glu and Xaa₅ is Pro;
- A1.5:: SEQ ID NO:171, wherein Xaa₁ is Glu and Xaa₅ is Pro;
- A1.6:: SEQ ID NO:172, wherein Xaa₁ is Glu and Xaa₅ is Pro;
- Af1.1:: SEQ ID NO:173, wherein Xaa₁ is Glu Xaa₄ is Tyr, Xaa₅ is Pro and Xaa₆ is Gln;
- Af1.2:: SEQ ID NO:174, wherein Xaa₁ is Glu and Xaa₅ is Pro;
- Ar1.2:: SEQ ID NO:175, wherein Xaa₁ is Glu, Xaa₂ is Lys, Xaa₃ is Trp, Xaa₄ is Try and Xaa₅ is Pro;
- Ar1.3:: SEQ ID NO:176, wherein Xaa₁ is Glu, Xaa₄ is Tyr and Xaa₅ is Pro;
- Ar1.4:: SEQ ID NO: 177, wherein Xaa₁ is Glu, Xaa₂ is Lys, Xaa₃ is Trp, Xaa₄ is Try and Xaa₅ is Pro;
- Ar1.5:: SEQ ID NO:178, wherein Xaa₁ is Glu and Xaa₅ is Pro;
- Ar1.6:: SEQ ID NO: 179, wherein Xaa₁ is Glu, Xaa₂ is Lys, Xaa₃ is Trp, Xaa₄ is Try and Xaa₅ is Pro;
- Ay1.2:: SEQ ID NO: 180, wherein Xaa₄ is Tyr and Xaa₅ is Pro;
- Ay1.3:: SEQ ID NO: 181, wherein Xaa₅ is Pro;
- Bn1.4:: SEQ ID NO: 182, wherein Xaa₄ is Tyr and Xaa₅ is Pro;
- Bt1.8:: SEQ ID NO:183, wherein Xaa₁ is Glu and Xaa₅ is Pro;
- Bt1.9:: SEQ ID NO: 184, wherein Xaa₁ is Glu, Xaa₄ is Tyr and Xaa₅, is Pro;
- Ca1.3:: SEQ ID NO:185, wherein Xaa₁ is Glu, Xaa₃ is Trp, Xaa₄ is Try and Xaa₅ is Pro;
- Ca1.4:: SEQ ID NO: 186, wherein Xaa₁ is Glu, Xaa₂ is Lys, Xaa₃ is Trp, Xaa₄ is Try, Xaa₅ is Pro and Xaa₆ is Gln;
- C1.2:: SEQ ID NO:187, wherein Xaa₁ is Glu and Xaa₅ is Pro;
- C1.3:: SEQ ID NO:188, wherein Xaa₄ is Tyr and Xaa₅ is Pro;
- Ep1.2:: SEQ ID NO:189, wherein Xaa₁ is Glu, Xaa₂ is Lys, Xaa₃ is Trp and Xaa₅ is Pro;
- G1.1:: SEQ ID NO: 190, wherein Xaa₅ is Pro;
- G1.3:: SEQ ID NO: 191, wherein Xaa₅ is Pro;
- Im1.3:: SEQ ID NO: 192, wherein Xaa₅ is Pro;
- Lv1.2:: SEQ ID NO: 193, wherein Xaa₁ is Glu and Xaa₅ is Pro;
- Lv1.3:: SEQ ID NO:194, wherein Xaa₂ is Lys and Xaa₅ is Pro;
- Lv1.4:: SEQ ID NO: 195, wherein Xaa₁ is Glu and Xaa₅ is Pro;
- Lv1.6:: SEQ ID NO: 196, wherein Xaa₅ is Pro;
- Lv1.7:: SEQ ID NO: 197, wherein Xaa₁ is Glu and Xaa₅ is Pro;
- Lv1.8:: SEQ ID NO:198, wherein Xaa₅ is Pro;
- Lv1.9:: SEQ ID NO:199, wherein Xaa₄ is Tyr and Xaa₅ is Pro;
- Lv1.10:: SEQ ID NO:200, wherein Xaa₁ is Glu;
- Mr1.3:: SEQ ID NO:201, wherein Xaa₄ is Tyr and Xaa₅ is Pro;
- Mr1.4:: SEQ ID NO:202, wherein Xaa₅ is Pro;
- Ms1.1 :: SEQ ID NO:203, wherein Xaa₂ is Lys, Xaa₄ is Tyr and Xaa₅ is Pro;

- Ms1.6:: SEQ ID NO:204, wherein Xaa₄ is Tyr and Xaa₅ is Pro;
- O1.1:: SEQ ID NO:205, wherein Xaa₂ is Lys, Xaa₄ is Tyr and Xaa₅ is Pro;
- O1.2:: SEQ ID NO:206, wherein Xaa₁ is Glu, Xaa₄ is Tyr and Xaa₅ is Pro;
- O1.4:: SEQ ID NO:207, wherein Xaa₁ is Glu, Xaa₃ is Trp, Xaa₄ is Tyr and Xaa₅ is Pro;
- O1.7:: SEQ ID NO:208, wherein Xaa₄ is Tyr and Xaa₅ is Pro;
- O1.8:: SEQ ID NO:209, wherein Xaa₁ is Glu, Xaa₄ is Tyr and Xaa₅ is Pro;
- Om1.2:: SEQ ID NO:210, wherein Xaa₁ is Glu, Xaa₄ is Tyr and Xaa₅ is Pro;
- Om1.3:: SEQ ID NO:211, wherein Xaa₁ is Glu, Xaa₂ is Lys, Xaa₃ is Trp and Xaa₅ is Pro;
- Om1.4:: SEQ ID NO:212, wherein Xaa₅ is Pro;
- Om1.5:: SEQ ID NO:213, wherein Xaa₄ is Tyr and Xaa₅ is Pro;
- Om1.6:: SEQ ID NO:214, wherein Xaa₁ is Glu and Xaa₅ is Pro;
- P1.4:: SEQ ID NO:215, wherein Xaa₁ is Glu and Xaa₅ is Pro;
- P1.5:: SEQ ID NO:216, wherein Xaa₅ is Pro;
- P1.6:: SEQ ID NO:217, wherein Xaa₃ is Trp and Xaa₅ is Pro;
- P1.8:: SEQ ID NO:218, wherein Xaa₅ is Pro;
- Rg1.1:: SEQ ID NO:219, wherein Xaa₁ is Glu and Xaa₅ is Pro;
- Rg1.3:: SEQ ID NO:220, wherein Xaa₅ is Pro;
- Rg1.4:: SEQ ID NO:221, wherein Xaa₂ is Lys, Xaa₄ is Tyr and Xaa₅ is Pro;
- Rg1.5:: SEQ ID NO:222, wherein Xaa₅ is Pro;
- Rg1.8:: SEQ ID NO:223, wherein Xaa₂ is Lys, Xaa₄ is Tyr and Xaa₅ is Pro;
- Sm1.4:: SEQ ID NO:224, wherein Xaa₂ is Lys and Xaa₅ is Pro;
- Sm1.5:: SEQ ID NO:225, wherein Xaa₁ is Glu and Xaa₅ is Pro;
- S1.5:: SEQ ID NO:226, wherein Xaa₅ is Pro;
- Tx1.5:: SEQ ID NO:227, wherein Xaa₁ is Glu, Xaa₅ is Pro and Xaa₆ is Gln;
- T1.1:: SEQ ID NO:228, wherein Xaa₅ is Pro;
- Vr1.3:: SEQ ID NO:229, wherein Xaa₅ is Pro; and
- Tb:: SEQ ID NO:230, wherein Xaa₂ is Lys and Xaa₅ is Pro.
The C-terminus preferably contains a carboxyl group for the peptides OB-29, Tx1.1, R1.1A, R1.1B, Om-9, Om-10, Om-21, Om-25, Om-27, Om-28, Cr1.2, Om-35, Bt1.1, Bt1.3, Bt1.5, A1.4, A1.6, Ar1.2, Ar1.3, Ar1.4, Ar1.5, Ar1.6, Ca1.3, Ca1.4, Ep1.2, Lv1.9, 01.2, Oml.3, Om1.6, P1.6, Rg1.1, Rg1.3, Rg1.4, Sm1.5, Tx1.5 and Vr1.3. The C-terminus of the other peptides preferably contains an amide group.

The present invention is also directed to the novel specific α-contoxin peptides having the formulas:
Cys-Cys-Thr-Ile-Xaa₅-Ser-Cys-Xaa₄-Xaa₁-Xaa₂-Xaa₂-Xaa₂-Ile-Xaa₂-Ala-Cys-Val-Phe(SEQ ID NO:231) and
Gly-Cys-Cys-Gly-Asn-Xaa₅-Ala-Cys-Ser-Gly-Ser-Ser-Xaa₂-Asp-Ala-Xaa₅-Ser-Cys (SEQ ID NO:232),
wherein Xaa₁ is Glu or γ-carboxy-Glu (Gla); Xaa₂ is Lys, N-methyl-Lys, N,N-dimethyl-Lys or N,N,N-trimethyl-Lys; Xaa₄ is Tyr, nor-Tyr, mono-halo-Tyr, di-halo-Tyr, 0-sulpho-Tyr, O-phospho-Tyr or nitro-Tyr; and Xaa₅ is Pro or hydroxy-Pro; and the C-terminus contains a carboxyl or amide group. The halo is preferably bromine, chlorine or iodine, more preferably iodine for Tyr. In addition, the His residues may be substituted with halo-His; the Arg residues may be substituted by Lys, ornithine, homoargine, N-methyl-Lys, N,N-dimethyl-Lys, N,N,N-trimethyl-Lys or any unnatural basic amino acid; the Lys residues may be substituted by Arg, omithine, homoargine, N-methyl-Lys, N,N-dimethyl-Lys, N,N,N-trimethyl-Lys or any unnatural basic amino acid; the Tyr residues may be substituted with any unnatural hydroxy containing amino acid; the Ser residues may be substituted with Thr; the Thr residues may be substituted with Ser; and the Phe residues may be substituted with any unnatural aromatic amino acid. The Cys residues may be in D or L configuration and may optionally be substituted with homocysteine (D or L). The Tyr residues may be substituted with the 3-hydroxyl or 2-hydroxyl isomers and corresponding O-sulpho- and O-phospho-derivatives. The acidic amino acid residues may be substituted with any synthetic acidic bioisoteric amino acid surrogate, e.g., tetrazolyl derivatives of Gly and Ala.

More specifically, the present invention is directed to the following α-conotoxin peptides:
- G1.2:: SEQ ID NO:231, wherein Xaa₁ is Glu, Xaa₂ is Lys, Xaa₄ is Tyr and Xaa₅ is Pro; and
- Rg1.12:: SEQ ID NO:232, wherein Xaa₂ is Lys and Xaa₅ is Pro.
The C-terminus of G1.2 preferably contains a carboxyl group, and the C-terminus of Rg1.12 preferably contains an amide group.

Examples of unnatural aromatic amino acid include, but are not limited to, such as nitro-Phe, 4-substituted-Phe wherein the substituent is C₁-C₃ alkyl, carboxyl, hyrdroxymethyl, sulphomethyl, halo, phenyl, -CHO, -CN, -SO₃H and -NHAc. Examples of unnatural hydroxy containing amino acid, include, but are not limited to, such as 4-hydroxymethyl-Phe, 4-hydroxyphenyl-Gly, 2,6-dimethyl-Tyr and 5-amino-Tyr. Examples of unnatural basic amino acids include, but are not limited to, N-1-(2-pyrazolinyl)-Arg, 2-(4-piperinyl)-Gly, 2-(4-piperinyl)-Ala, 2-[3-(2S)pyrrolininyl)-Gly and 2-[3-(2S)pyrrolininyl)-Ala. These and other unnatural basic amino acids, unnatural hydroxy containing amino acids or unnatural aromatic amino acids are described in Building Block Index, Version 3.0 (1999 Catalog, pages 4-47 for hydroxy containing amino acids and aromatic amino acids and pages 66-87 for basic amino acids; see also http://www.amino-acids.com), incorporated herein by reference, by and available from RSP Amino Acid Analogues, Inc., Worcester, MA.

Optionally, in the peptides of general formulas I, II and III and the specific peptides described above, the Asn residues may be modified to contain an N-glycan and the Ser and Thr residues may be modified to contain an O-glycan. In accordance with the present invention, a glycan shall mean any N-, S- or O-linked mono-, di-, tri-, poly- or oligosaccharide that can be attached to any hydroxy, amino or thiol group of natural or modified amino acids by synthetic or enzymatic methodologies known in the art. The monosaccharides making up the glycan can include D-allose, D-altrose, D-glucose, D-mannose, D-gulose, D-idose, D-galactose, D-talose, D-galactosamine, D-glucosamine, D-N-acetyl-glucosamine (GlcNAc), D-N-acetyl-galactosamine (GalNAc), D-fucose or D-arabinose. These saccharides may be structurally modified, e.g., with one or more O-sulfate, O-phosphate, O-acetyl or acidic groups, such as sialic acid, including combinations thereof. The gylcan may also include similar polyhydroxy groups, such as D-penicillamine 2,5 and halogenated derivatives thereof or polypropylene glycol derivatives. The glycosidic linkage is beta and 1-4 or 1-3, preferably 1-3. The linkage between the glycan and the amino acid may be alpha or beta, preferably alpha and is 1-.

Core O-glycans have been described by Van de Steen et al. (1998). Mucin type O-linked oligosaccharides are attached to Ser or Thr (or other hydroxylated residues of the present peptides) by a GalNAc residue. The monosaccharide building blocks and the linkage attached to this first GalNAc residue define the "core glycans", of which eight have been identified. The type of glysosidic linkage (orientation and connectivities) are defined for each core glycan. Suitable glycans and glycan analogs are described further in U.S. Patent No. 6,369,193 and in international publication No. WO00/23092. A preferred glycan is Gal(β1→3)GalNAc(α1→).

Optionally, in the peptides of general formulas I and II and the specific peptides described above, pairs of Cys residues may be replaced pairwise with Ser/(Glu or Asp) or Lys/(Glu or Asp) combinations. Sequential coupling by known methods (Bamay et al., 2000; Hruby et al., 1994; Bitan et al., 1997) allows replacement of native Cys bridges with lactam bridges.

The present invention is further directed to propeptides and nucleic acid sequences encoding the propeptides or peptides as described in further detail herein.

### DETAILED DESCRIPTION OF THE INVENTION

The invention relates to relatively short peptides (termed α-conotoxins herein), about 10-30 residues in length, which are naturally available in minute amounts in the venom of the cone snails or analogous to the naturally available peptides, and which preferably include two disulfide bonds.

The present invention, in another aspect, relates to a pharmaceutical composition comprising an effective amount of an α-conotoxin peptide. Such a pharmaceutical composition has the capability of acting as antagonists for nicotinic acetylcholine receptors. In one aspect, the α-conotoxins with specificity for neuromuscular junction nicotinic acetylcholine receptors are used as neuromuscular blocking agents for use in conjunction with surgery, as disclosed in U.S. patent No. 6,268,473 and international publication WO00/43409. In a second aspect, additional α-conotoxins and uses for them have been described in U.S. Patent Nos. 4,447,356 (Olivera et al., 1984); 5,432,155; 5,514,774.

In a third aspect additional uses for α-conotoxins are described in U.S. Patent No. 6,265,541. In this application, α-conotoxins with specificity for neuronal nicotinic acetylcholine receptors are used for treating disorders regulated at neuronal nicotinic acetylcholine receptors. Such disorders include, but are not limited to, cardiovascular disorders, gastric motility disorders, urinary incontinence, nicotine addiction, mood disorders (such as bipolar disorder, unipolar depression, dysthymia and seasonal effective disorder) and small cell lung carcinoma, as well as the localization of small cell lung carcinoma.

The α-conotoxin peptides described herein are sufficiently small to be chemically synthesized. General chemical syntheses for preparing the foregoing α-conotoxin peptides are described hereinafter. Various ones of the α-conotoxin peptides can also be obtained by isolation and purification from specific *Conus* species using the technique described in U.S. Patent No. 4,447,356 (Olivera et al., 1984).

Although the α-conotoxin peptides of the present invention can be obtained by purification from cone snails, because the amounts of α-conotoxin peptides obtainable from individual snails are very small, the desired α-conotoxin peptides are best practically obtained in commercially valuable amounts by chemical synthesis using solid-phase strategy. For example, the yield from a single cone snail may be about 10 micrograms or less of α-conotoxin peptide. The d-conotoxin is preferably present in an amount of at least about 85% purity and preferably at least about 95% purity. Chemical synthesis of biologically active α-conotoxin peptides depends of course upon correct determination of the amino acid sequence.

The α-conotoxin peptides can also be produced by recombinant DNA techniques well known in the art. Such techniques are described by Sambrook et al. (1989). The peptides produced in this manner are isolated, reduced if necessary, and oxidized to form the correct disulfide bonds.

One method of forming disulfide bonds in the conantokin peptides of the present invention is the air oxidation of the linear peptides for prolonged periods under cold room temperatures or at room temperature. This procedure results in the creation of a substantial amount of the bioactive, disulfide-linked peptides. The oxidized peptides are fractionated using reverse-phase high performance liquid chromatography (HPLC) or the like, to separate peptides having different linked configurations. Thereafter, either by comparing these fractions with the elution of the native material or by using a simple assay, the particular fraction having the correct linkage for maximum biological potency is easily determined. However, because of the dilution resulting from the presence of other fractions of less biopotency, a somewhat higher dosage may be required.

The peptides are synthesized by a suitable method, such as by exclusively solid-phase techniques, by partial solid-phase techniques, by fragment condensation or by classical solution couplings.

In conventional solution phase peptide synthesis, the peptide chain can be prepared by a series of coupling reactions in which constituent amino acids are added to the growing peptide chain in the desired sequence. Use of various coupling reagents, e.g., dicyclohexylcarbodiimide or diisopropylcarbonyldimidazole, various active esters, e.g., esters of N-hydroxyphthalimide or N-hydroxy-succinimide, and the various cleavage reagents, to carry out reaction in solution, with subsequent isolation and purification of intermediates, is well known classical peptide methodology. Classical solution synthesis is described in detail in the treatise, "Methoden der Organischen Chemie (Houben-Weyl): Synthese von Peptiden," (1974). Techniques of exclusively solid-phase synthesis are set forth in the textbook, "Solid-Phase Peptide Synthesis," (Stewart and Young, 1969), and are exemplified by the disclosure of U.S. Patent 4,105,603 (Vale et al., 1978). The fragment condensation method of synthesis is exemplified in U.S. Patent 3,972,859 (1976). Other available syntheses are exemplified by U.S. Patents No. 3,842,067 (1974) and 3,862,925 (1975). The synthesis of peptides containing γ-carboxyglutamic acid residues is exemplified by Rivier et al. (1987), Nishiuchi et al. (1993) and Zhou et al. (1996).

Common to such chemical syntheses is the protection of the labile side chain groups of the various amino acid moieties with suitable protecting groups which will prevent a chemical reaction from occurring at that site until the group is ultimately removed. Usually also common is the protection of an α-amino group on an amino acid or a fragment while that entity reacts at the carboxyl group, followed by the selective removal of the α-amino protecting group to allow subsequent reaction to take place at that location. Accordingly, it is common that, as a step in such a synthesis, an intermediate compound is produced which includes each of the amino acid residues located in its desired sequence in the peptide chain with appropriate side-chain protecting groups linked to various ones of the residues having labile side chains..

As far as the selection of a side chain amino protecting group is concerned, generally one is chosen which is not removed during deprotection of the α-amino groups during the synthesis. However, for some amino acids, e.g., His, protection is not generally necessary. In selecting a particular side chain protecting group to be used in the synthesis of the peptides, the following general rules are followed: (a) the protecting group preferably retains its protecting properties and is not split off under coupling conditions, (b) the protecting group should be stable under the reaction conditions selected for removing the α-amino protecting group at each step of the synthesis, and (c) the side chain protecting group must be removable, upon the completion of the synthesis containing the desired amino acid sequence, under reaction conditions that will not undesirably alter the peptide chain.

It should be possible to prepare many, or even all, of these peptides using recombinant DNA technology. However, when peptides are not so prepared, they are preferably prepared using the Merrifield solid-phase synthesis, although other equivalent chemical syntheses known in the art can also be used as previously mentioned. Solid-phase synthesis is commenced from the C-terminus of the peptide by coupling a protected α-amino acid to a suitable resin. Such a starting material can be prepared by attaching an α-amino-protected amino acid by an ester linkage to a chloromethylated resin or a hydroxymethyl resin, or by an amide bond to a benzhydrylamine (BHA) resin or paramethylbenzhydrylamine (MBHA) resin. Preparation of the hydroxymethyl resin is described by Bodansky et al. (1966). Chloromethylated resins are commercially available from Bio Rad Laboratories (Richmond, CA) and from Lab. Systems, Inc. The preparation of such a resin is described by Stewart and Young (1969). BHA and MBHA resin supports are commercially available, and are generally used when the desired polypeptide being synthesized has an unsubstituted amide at the C-terminus. Thus, solid resin supports may be any of those known in the art, such as one having the formulae -O-CH₂-resin support, -NH BHA resin support, or -NH-MBHA resin support. When the unsubstituted amide is desired, use of a BHA or MBHA resin is preferred, because cleavage directly gives the amide. In case the N-methyl amide is desired, it can be generated from an N-methyl BHA resin. Should other substituted amides be desired, the teaching of U.S. Patent No. 4,569,967 (Kornreich et al., 1986) can be used, or should still other groups than the free acid be desired at the C-terminus, it may be preferable to synthesize the peptide using classical methods as set forth in the Houben-Weyl text (1974).

The C-terminal amino acid, protected by Boc or Fmoc and by a side-chain protecting group, if appropriate, can be first coupled to a chloromethylated resin according to the procedure set forth in K. Horiki et al. (1978), using KF in DMF at about 60°C for 24 hours with stirring, when a peptide having free acid at the C-terminus is to be synthesized. Following the coupling of the BOC-protected amino acid to the resin support, the α-amino protecting group is removed, as by using trifluoroacetic acid (TFA) in methylene chloride or TFA alone. The deprotection is carried out at a temperature between about 0°C and room temperature. Other standard cleaving reagents, such as HCl in dioxane, and conditions for removal of specific α-amino protecting groups may be used as described in Schroder & Lubke (1965).

After removal of the α-amino-protecting group, the remaining α-amino- and side chain-protected amino acids are coupled step-wise in the desired order to obtain the intermediate compound defined hereinbefore, or as an alternative to adding each amino acid separately in the synthesis, some of them may be coupled to one another prior to addition to the solid phase reactor. Selection of an appropriate coupling reagent is within the skill of the art. Particularly suitable as a coupling reagent is N,N'-dicyclohexylcarbodiimide (DCC, DIC, HBTU, HATU, TBTU in the presence of HoBt or HoAt).

The activating reagents used in the solid phase synthesis of the peptides are well known in the peptide art. Examples of suitable activating reagents are carbodiimides, such as N,N'-diisopropylcarbodiimide and N-ethyl-N'-(3-dimethylaminopropyl)carbodiimide. Other activating reagents and their use in peptide coupling are described by Schroder & Lubke (1965) and Kapoor (1970).

Each protected amino acid or amino acid sequence is introduced into the solid-phase reactor in about a twofold or more excess, and the coupling may be carried out in a medium of dimethylformamide (DMF):CH₂Cl₂ (1:1) or in DMF or CH₂Cl₂ alone. In cases where intermediate coupling occurs, the coupling procedure is repeated before removal of the α-amino protecting group prior to the coupling of the next amino acid. The success of the coupling reaction at each stage of the synthesis, if performed manually, is preferably monitored by the ninhydrin reaction, as described by Kaiser et al. (1970). Coupling reactions can be performed automatically, as on a Beckman 990 automatic synthesizer, using a program such as that reported in Rivier et al. (1978).

After the desired amino acid sequence has been completed, the intermediate peptide can be removed from the resin support by treatment with a reagent, such as liquid hydrogen fluoride or TFA (if using Fmoc chemistry), which not only cleaves the peptide from the resin but also cleaves all remaining side chain protecting groups and also the α-amino protecting group at the N-terminus if it was not previously removed to obtain the peptide in the form of the free acid. If Met is present in the sequence, the Boc protecting group is preferably first removed using trifluoroacetic acid (TFA)/ethanedithiol prior to cleaving the peptide from the resin with HF to eliminate potential S-alkylation. When using hydrogen fluoride or TFA for cleaving, one or more scavengers such as anisole, cresol, dimethyl sulfide and methylethyl sulfide are included in the reaction vessel.

Cyclization of the linear peptide is preferably affected, as opposed to cyclizing the peptide while a part of the peptido-resin, to create bonds between Cys residues. To effect such a disulfide cyclizing linkage, fully protected peptide can be cleaved from a hydroxymethylated resin or a chloromethylated resin support by ammonolysis, as is well known in the art, to yield the fully protected amide intermediate, which is thereafter suitably cyclized and deprotected. Alternatively, deprotection, as well as cleavage of the peptide from the above resins or a benzhydrylamine (BHA) resin or a methylbenzhydrylamine (MBHA), can take place at 0°C with hydrofluoric acid (HF) or TFA, followed by oxidation as described above.

The peptides are also synthesized using an automatic synthesizer. Amino acids are sequentially coupled to an MBHA Rink resin (typically 100 mg of resin) beginning at the C-terminus using an Advanced Chemtech 357 Automatic Peptide Synthesizer. Couplings are carried out using 1,3-diisopropylcarbodimide in N-methylpyrrolidinone (NMP) or by 2-(1H-benzotriazole-1-yl)-1,1,3,3-tetramethyluronium hexafluorophosphate (HBTU) and diethylisopro- pylethylamine (DIEA). The FMOC protecting group is removed by treatment with a 20% solution of piperidine in dimethylformamide(DMF). Resins are subsequently washed with DMF (twice), followed by methanol and NMP.

Pharmaceutical compositions containing a compound of the present invention or its pharmaceutically acceptable salts as the active ingredient can be prepared according to conventional pharmaceutical compounding techniques. See, for example, Remington's Pharmaceutical Sciences, 18th Ed. (1990, Mack Publishing Co., Easton, PA). Typically, an antagonistic amount of the active ingredient will be admixed with a pharmaceutically acceptable carrier. The carrier may take a wide variety of forms depending on the form of preparation desired for administration, e.g., intravenous, oral or parenteral. The compositions may further contain antioxidizing agents, stabilizing agents, preservatives and the like.

For oral administration, the compounds can be formulated into solid or liquid preparations such as capsules, pills, tablets, lozenges, melts, powders, suspensions or emulsions. In preparing the compositions in oral dosage form, any of the usual pharmaceutical media may be employed, such as, for example, water, glycols, oils, alcohols, flavoring agents, preservatives, coloring agents, suspending agents, and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form, in which case solid pharmaceutical carriers are obviously employed. If desired, tablets may be sugar-coated or enteric-coated by standard techniques. The active agent can be encapsulated to make it stable to passage through the gastrointestinal tract while at the same time allowing for passage across the blood brain barrier. See for example, WO 96/11698. For parenteral administration, the compound may be dissolved in a pharmaceutical carrier and administered as either a solution or a suspension. Illustrative of suitable carriers are water, saline, dextrose solutions, fructose solutions, ethanol, or oils of animal, vegetative or synthetic origin. The carrier may also contain other ingredients, for example, preservatives, suspending agents, solubilizing agents, buffers and the like. When the compounds are being administered intrathecally, they may also be dissolved in cerebrospinal fluid.

The active agent is preferably administered in an therapeutically effective amount. The actual amount administered, and the rate and time-course of administration, will depend on the nature and severity of the condition being treated. Prescription of treatment, e.g. decisions on dosage, timing, etc., is within the responsibility of general practitioners or spealists, and typically takes account of the disorder to be treated, the condition of the individual patient, the site of delivery, the method of administration and other factors known to practitioners. Examples of techniques and protocols can be found in *Remington's Parmaceutical Sciences.* Typically the conopeptides of the present invention exhibit their effect at a dosage range from about 0.001 mg/kg to about 250 mg/kg, preferably from about 0.05 mg/kg to about 100 mg/kg of the active ingredient, more preferably from a bout 0.1mg/kg to about 75 mg/kg. A suitable dose can be administered in multiple sub-doses per day. Typically, a dose or sub-dose may contain from about 0.1 mg to about 500 mg of the active ingredient per unit dosage form. A more preferred dosage will contain from about 0.5 mg to about 100 mg of active ingredient per unit dosage form. Dosages are generally initiated at lower levels and increased until desired effects are achieved.

Alternatively, targeting therapies may be used to deliver the active agent more specifically to certain types of cell, by the use of targeting systems such as antibodies or cell specific ligands. Targeting may be desirable for a variety of reasons, e.g. if the agent is unacceptably toxic, or if it would otherwise require too high a dosage, or if it would not otherwise be able to enter the target cells.

The active agents, which are peptides, can also be administered in a cell based delivery system in which a DNA sequence encoding an active agent is introduced into cells designed for implantation in the body of the patient, especially in the spinal cord region. Suitable delivery systems are described in U.S. Patent No. 5,550,050 and published PCT Application Nos. WO 92/19195, WO 94/25503, WO 95/01203, WO 95/05452, WO 96/02286, WO 96/02646, WO 96/40871, WO 96/40959 and WO 97/12635. Suitable DNA sequences can be prepared synthetically for each active agent on the basis of the developed sequences and the known genetic code.

### EXAMPLES

The present invention is described by reference to the following Examples, which are offered by way of illustration and are not intended to limit the invention in any manner. Standard techniques well known in the art or the techniques specifically described below were utilized.

### EXAMPLE 1

### Isolation of α-Conotoxins

Crude venom was extracted from venom ducts (Cruz et al., 1976), and the components were purified as previously described (Cartier et al., 1996a). The crude extract from venom ducts was purified by reverse phase liquid chromatography (RPLC) using a Vydac C₁₈ semi-preparative column (10 x 250 mm) and elution with a linear gradient of acetonitrile in 0.1% TFA. Further purification of bioactive peaks was done on a Vydac C₁₈ analytical column (4.6 x 220 mm) eluted with a gradient of acetonitrile in 0.1 % TFA. The effluents were monitored at 220 nm. Peaks were collected, and aliquots were assayed for activity. Activity was monitored by assessing block of α3β4 nAChRs expressed in *Xenopus* oocytes.

The amino acid sequence of the purified peptides were determined by standard methods. The purified peptides were reduced and alkylated prior to sequencing by automated Edman degradation on an Applied Biosystems 477A Protein Sequencer with a 120A Analyzer (DNA/Peptide Facility, University of Utah) (Martinez et al., 1995; Shon et al., 1994).

In accordance with this method, peptides MII, AuIA, AuIB, AuIC, MAR-1, MAR-2, TI, OB-29, EpI, S1.1, Bn1.1, Bn1.2, Ca1.1, Ca1.2, Cn1.1, Cn1.2 and Sm1.3 were obtained.

### EXAMPLE 2

### Synthesis of Conopeptides

The synthesis of conopeptides, either the mature toxins or the precursor peptides, was separately performed using conventional protection chemistry as described by Cartier et al. (1996). Briefly, the linear chains were built on Rink amide resin by Fmoc procedures with 2-(1 H-benzotriol-1-yl)-1,1,3,3,-tetramethyluronium tetrafluoroborated coupling using an ABI model 430A peptide sythesizer with amino acid derivatives purchased from Bachem (Torrence CA). Orthogonal protection was used on cysteines: Cys³ and Cys¹⁶ were protected as the stable Cys(S-acetamidomethyl), while Cys² and Cys⁸ were protected as the acid-labile Cys(S-trityl). After removal of the terminal Fmoc protecting group and cleavage of the peptides from the resins, the released peptides were precipitated by filtering the reaction mixture into -10°C methyl t-butyl ether, which removed the protecting groups except on Cys³ and Cys¹⁶. The peptides were dissolved in 0.1 % TFA and 60% acetonitrile and purified by RPLC on a Vydac C₁₈ preparative column (22 x 250 mm) and eluted at a flow rate of 20 mL/min with a gradient of acetonitrile in 0.1 % TFA.

The disulfide bridges in the three conopeptides were formed as described in Cartier et al. (1996). Briefly, the disulfide bridges between Cys² and Cys⁸ were formed by air oxidation which was judged to be complete by analytical RPLC. The monocyclic peptides were purified by RPLC on a Vydac C₁₈ prepartive column (22 x 250 mm) and eluted with a gradient of acetonitrile in 0.1 % TFA. Removal of S-acetamidomethyl groups and closure of the disulfide bridge between Cys³ and Cys¹⁶ was carried out simultaneously be iodine oxidation. The cyclic peptides were purified by RPLC on a Vydac C₁₈ prepartive column (22 x 250 mm) and eluted with a gradient of acetonitrile in 0.1% TFA.

### EXAMPLE 3

### Isolation of DNA Encoding α-Conotoxins

DNA coding for α-conotoxins was isolated and cloned in accordance with conventional techniques using general procedures well known in the art, such as described in Olivera et al. (1996). Alternatively, cDNA libraries was prepared from *Conus* venom duct using conventional techniques. DNA from single clones was amplified by conventional techniques using primers which correspond approximately to the M 13 universal priming site and the M 13 reverse universal priming site. Clones having a size of approximately 300 nucleotides were sequenced and screened for similarity in sequence to known α-conotoxins. The DNA sequences and encoded propeptide or peptide sequences are set forth in Tables 1-134.

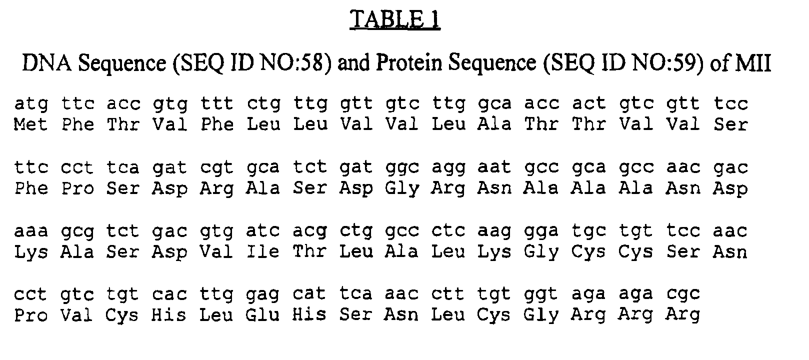

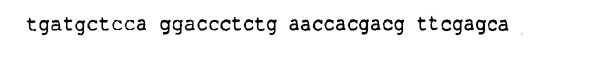

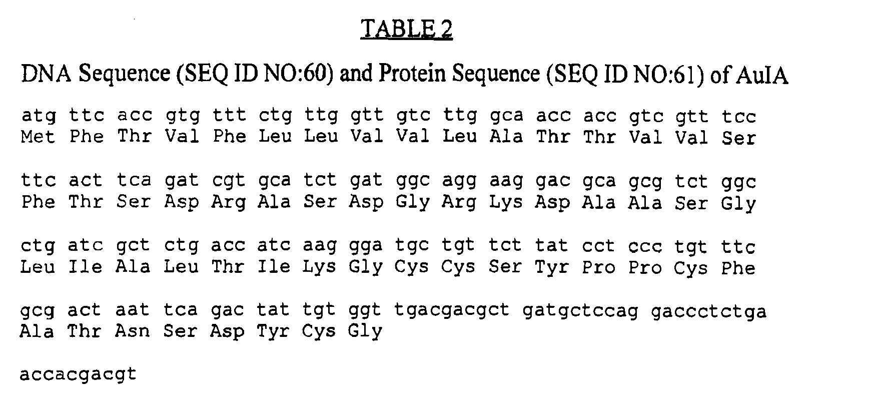

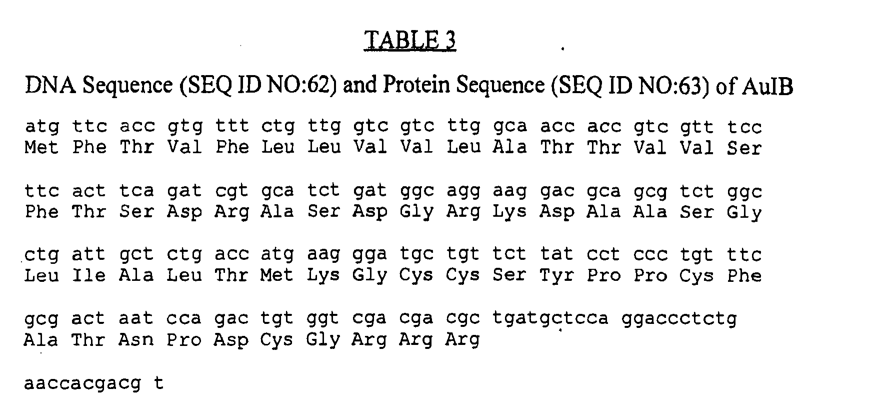

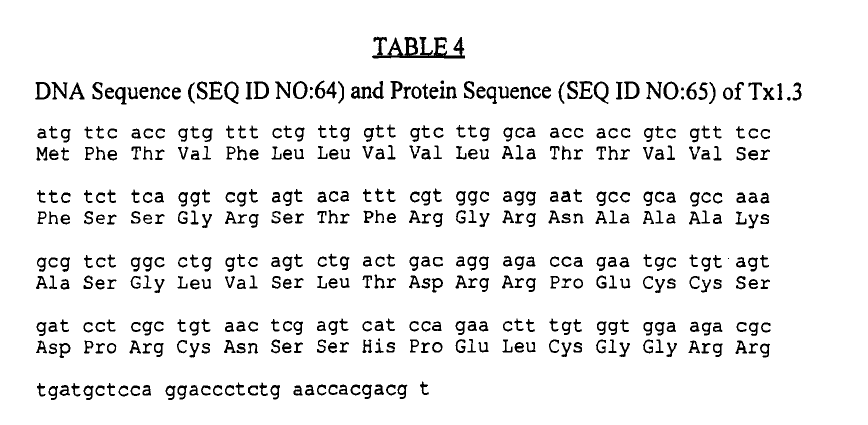

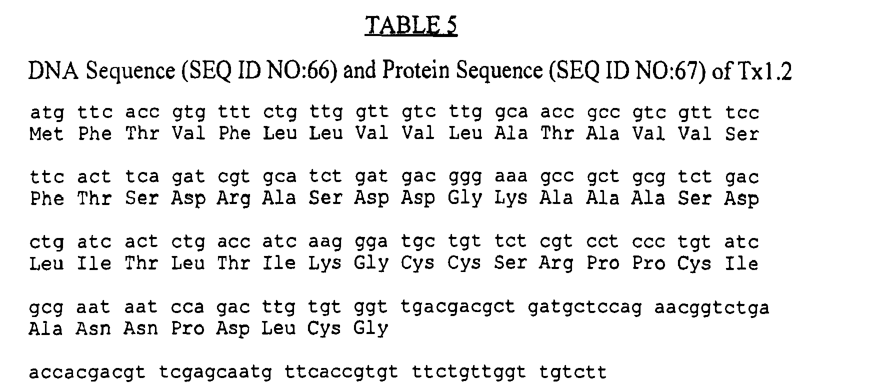

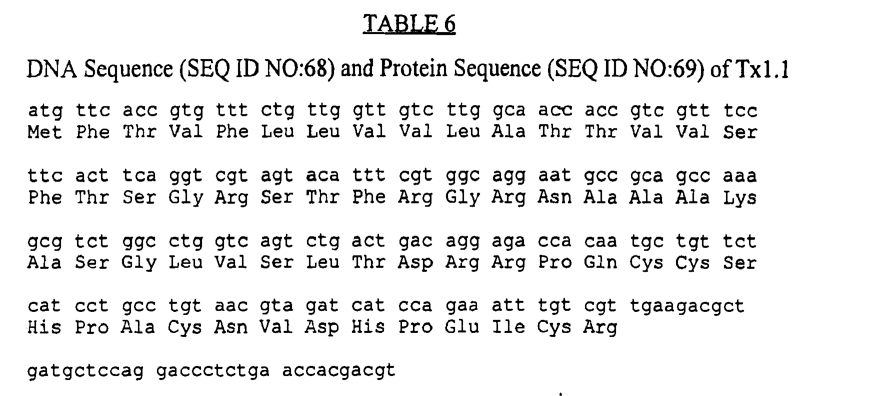

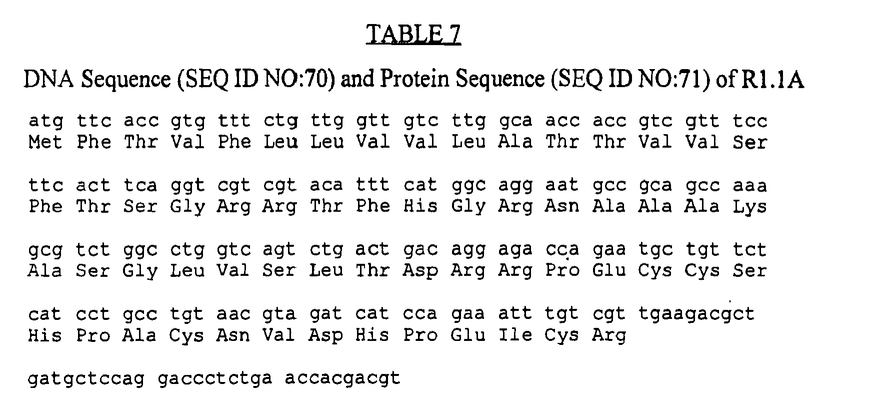

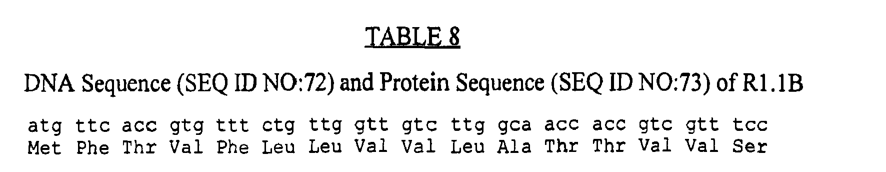

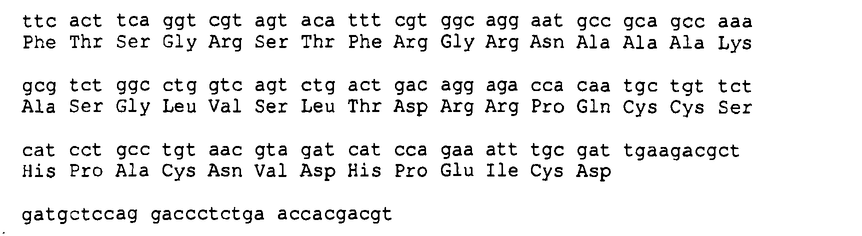

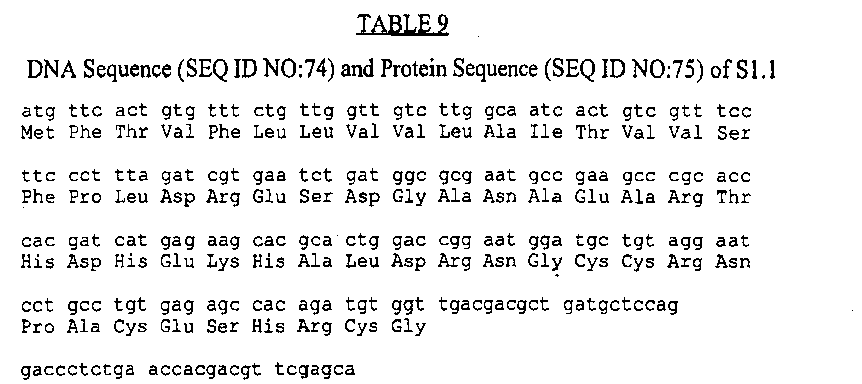

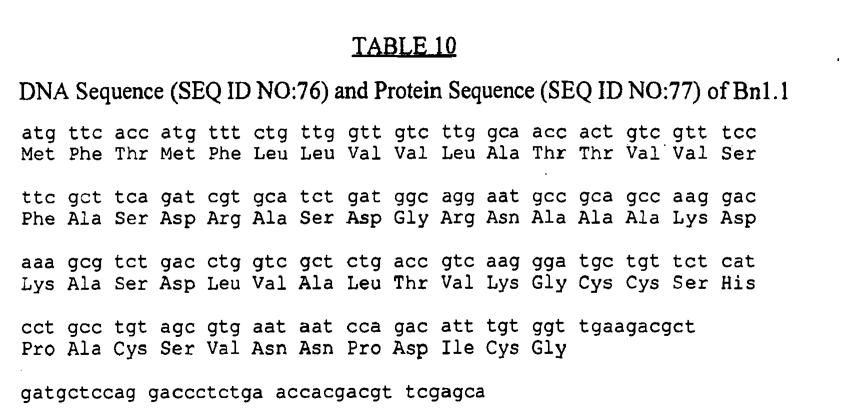

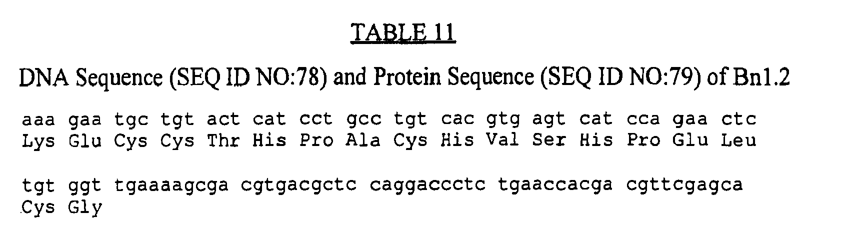

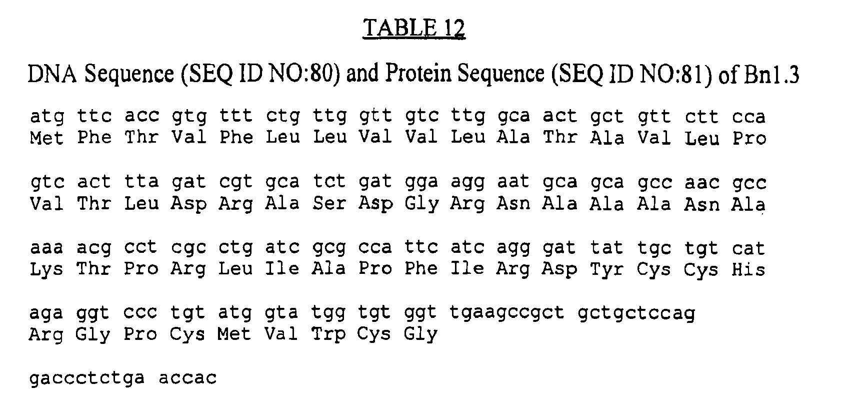

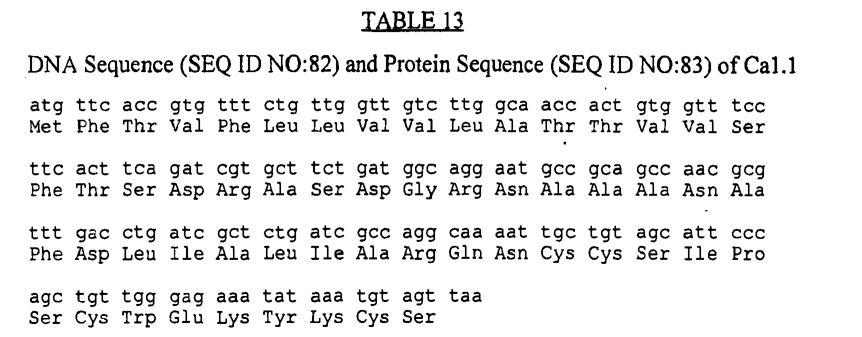

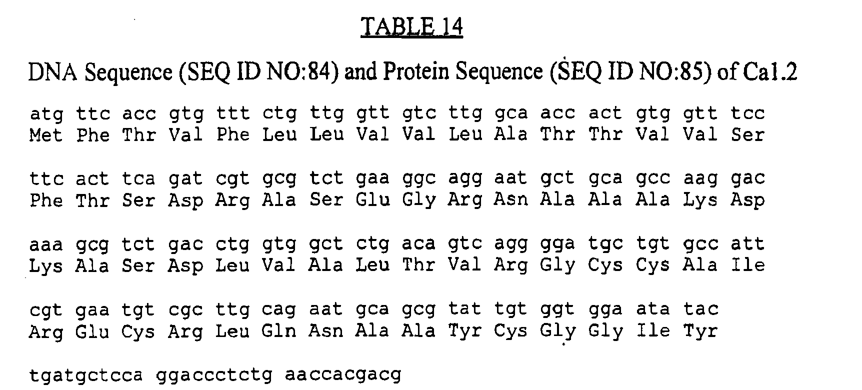

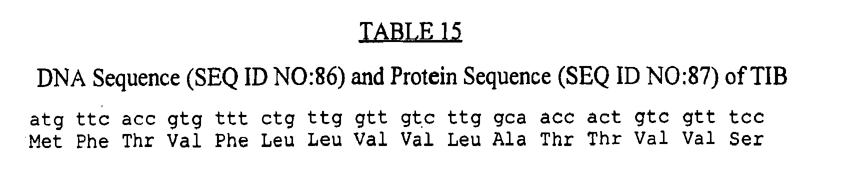

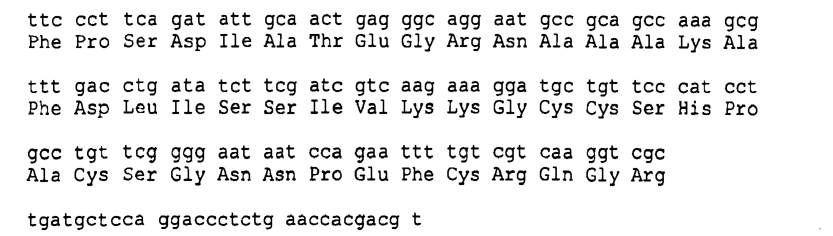

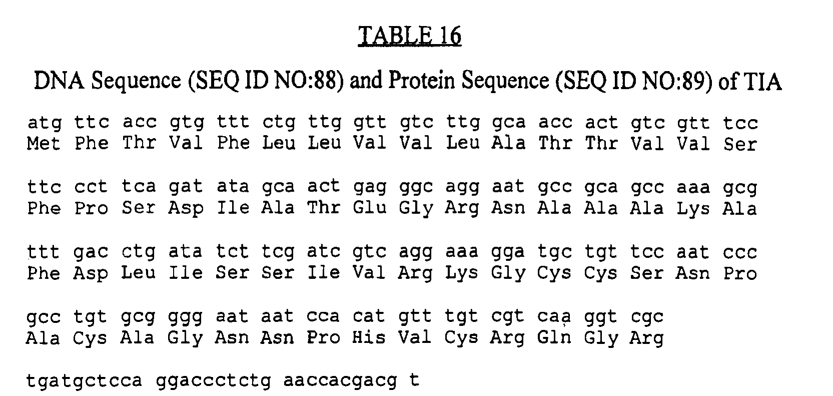

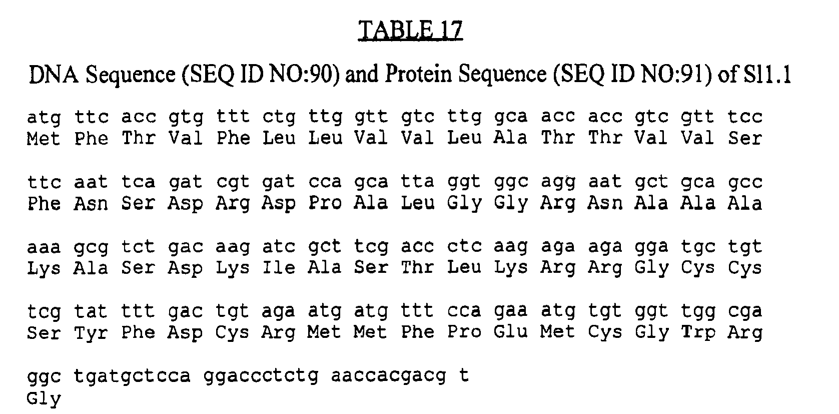

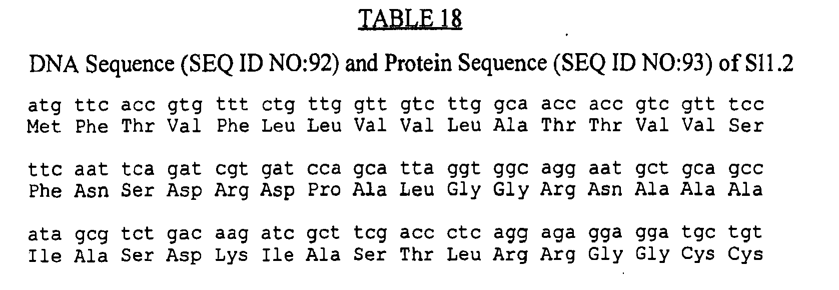

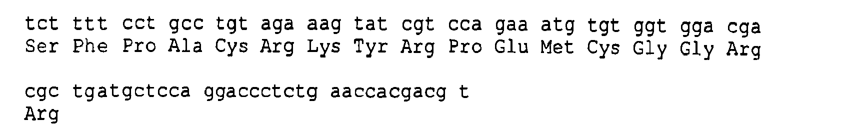

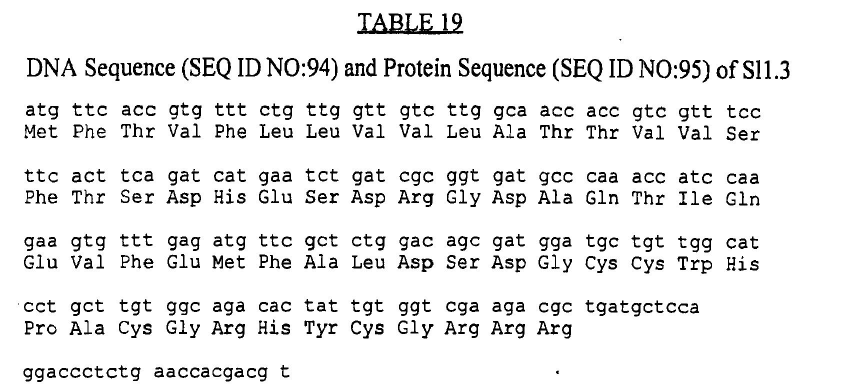

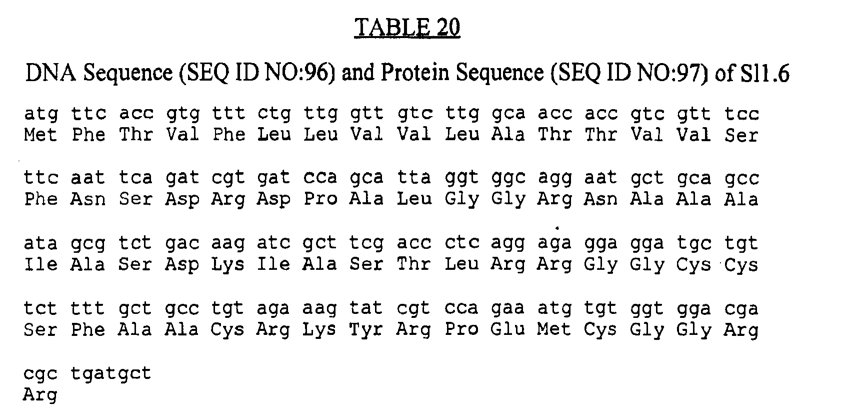

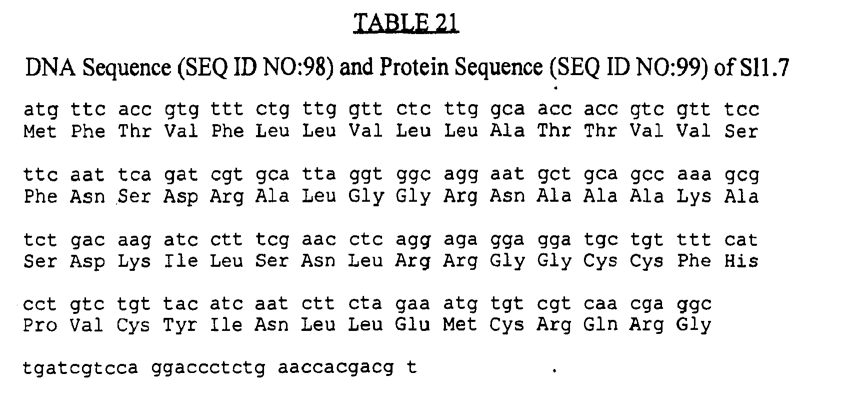

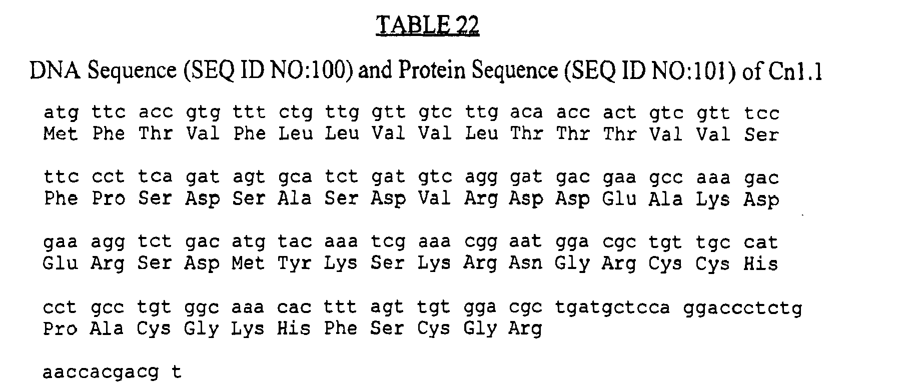

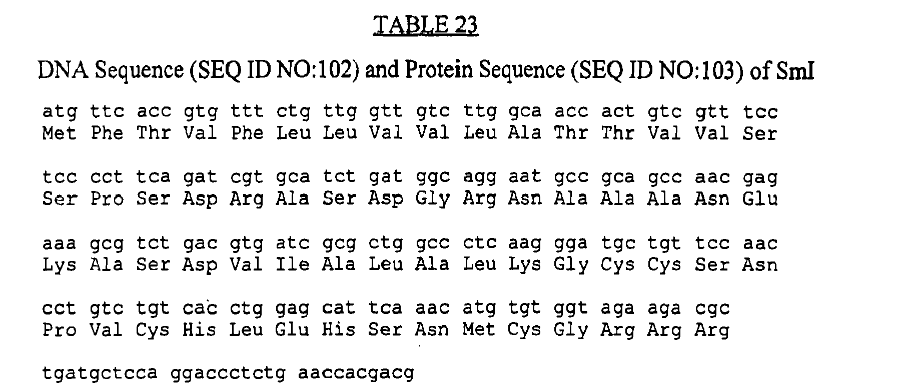

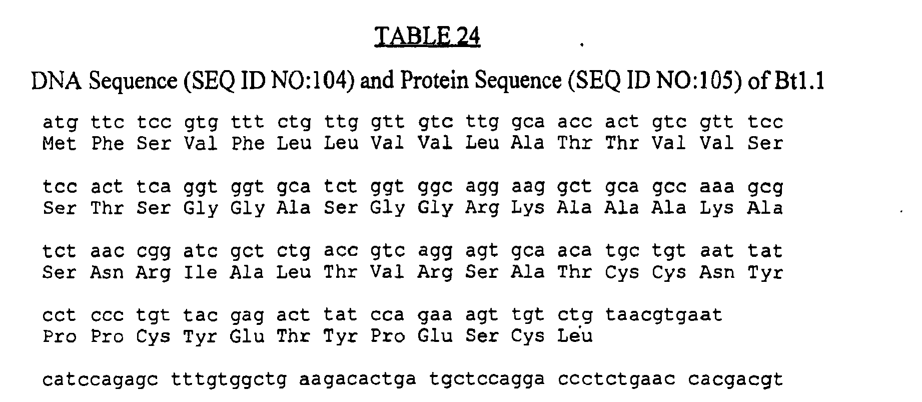

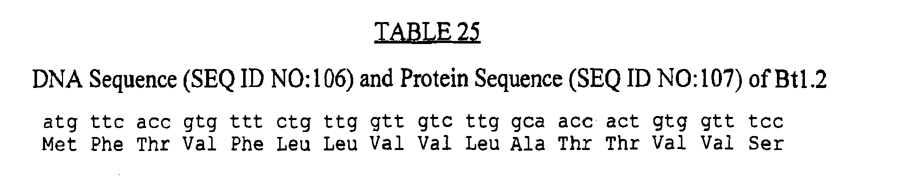

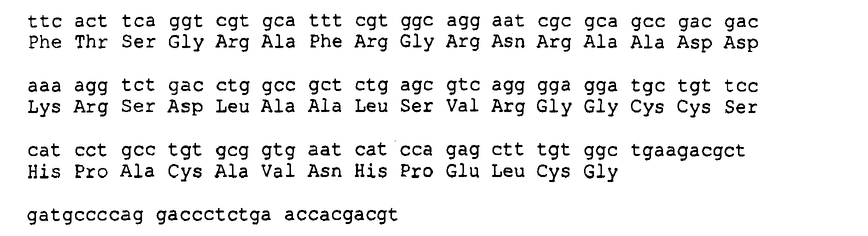

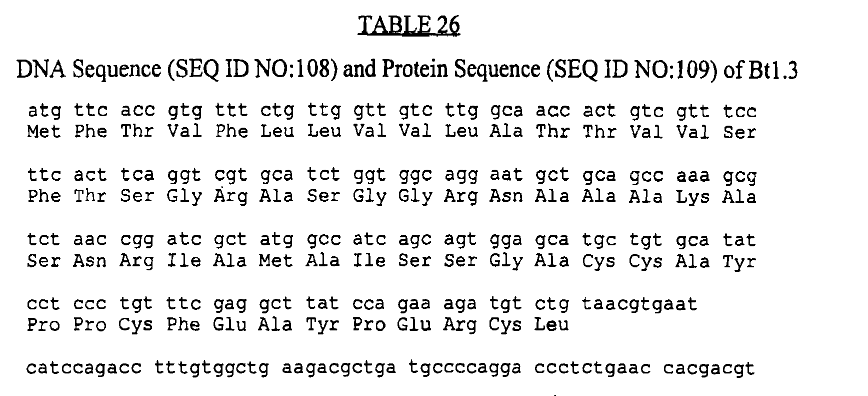

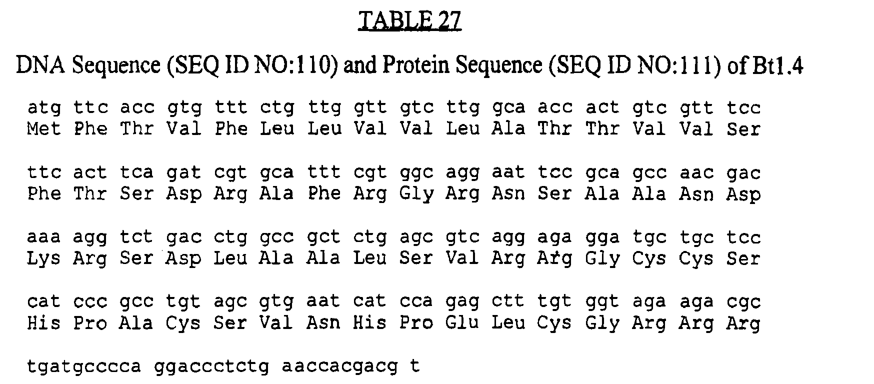

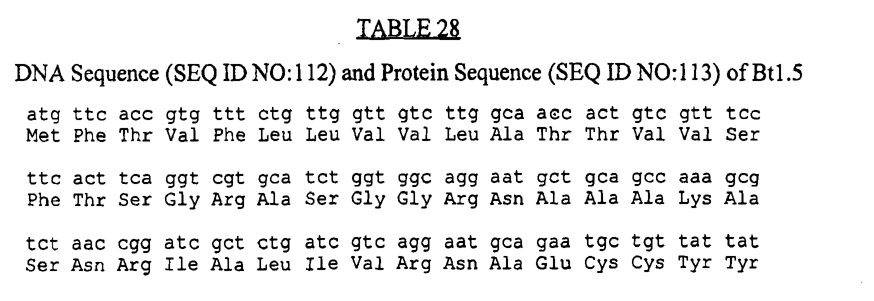

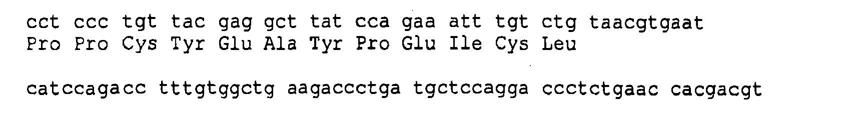

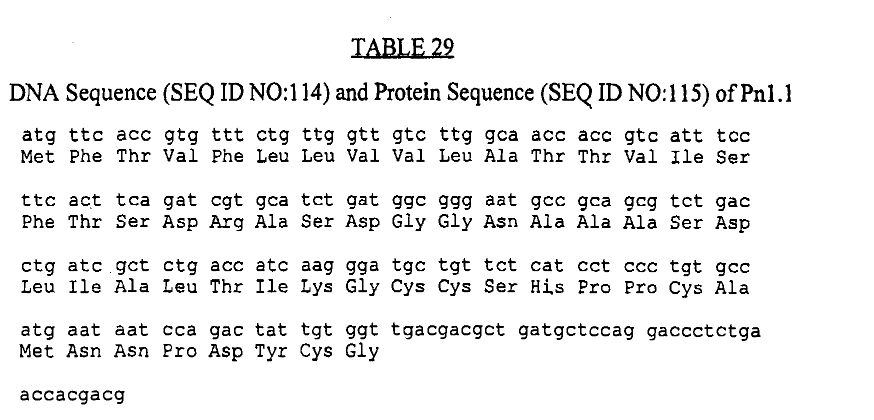

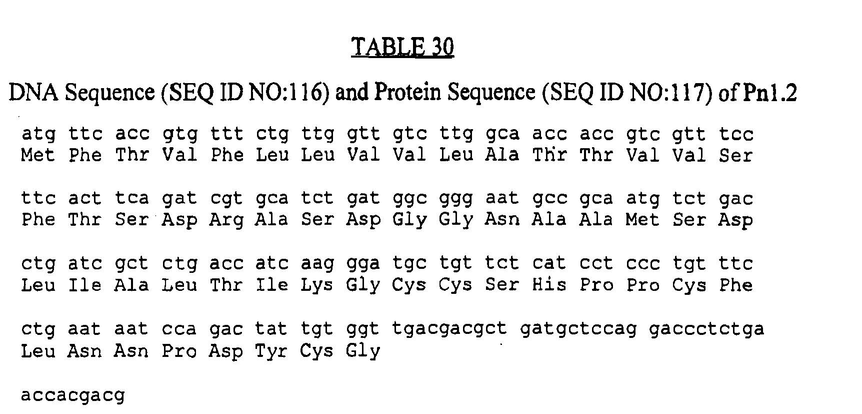

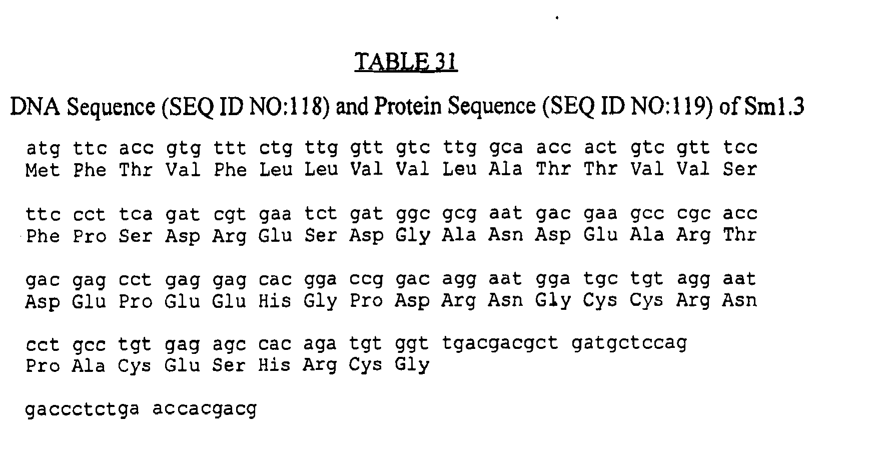

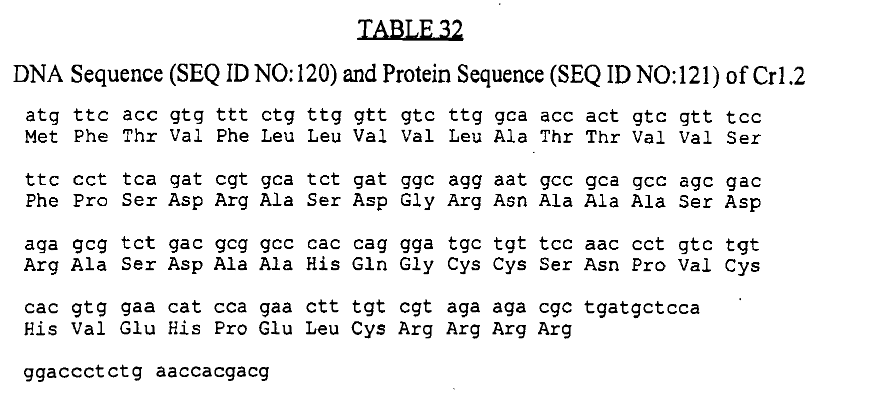

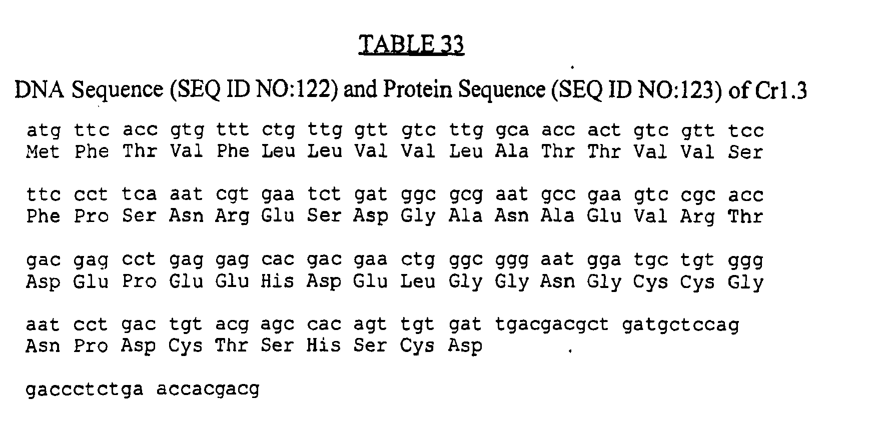

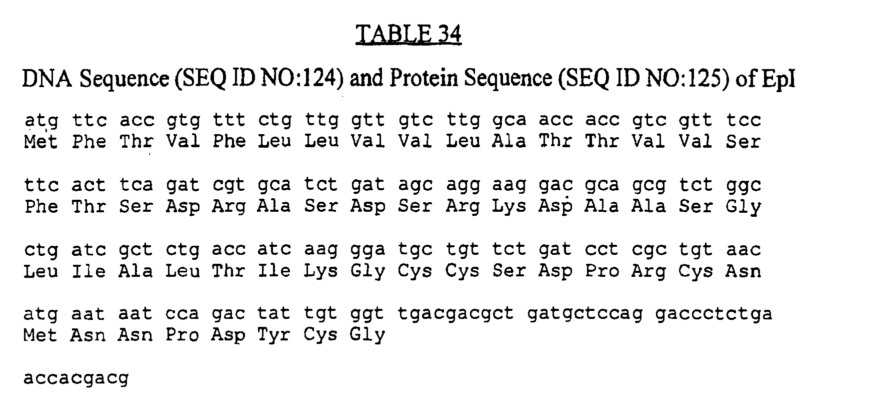

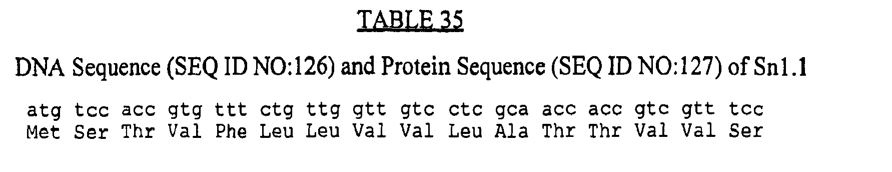

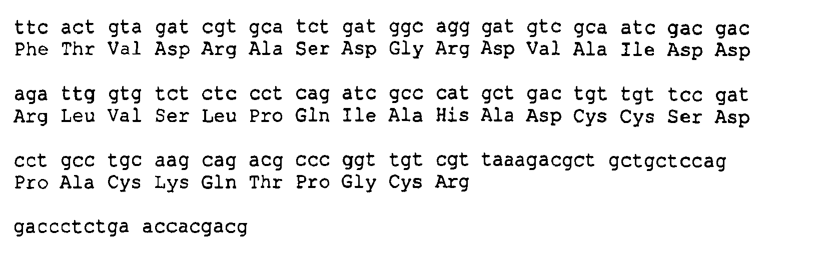

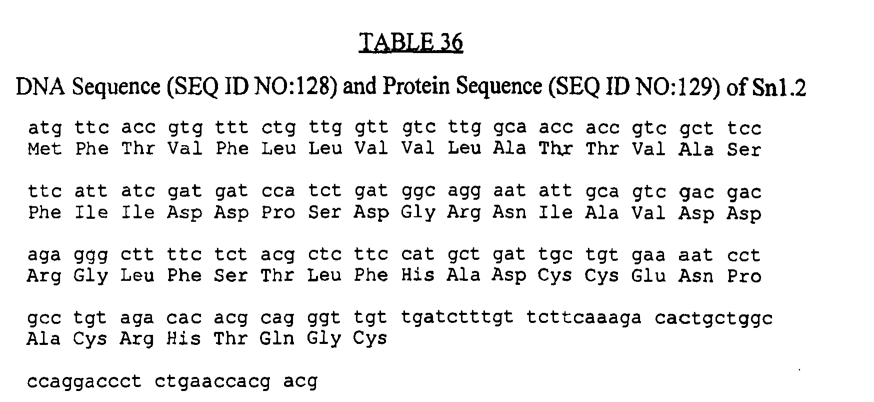

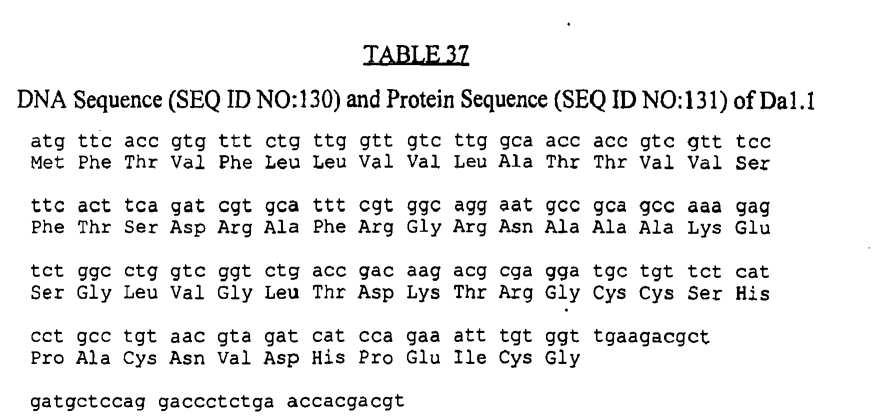

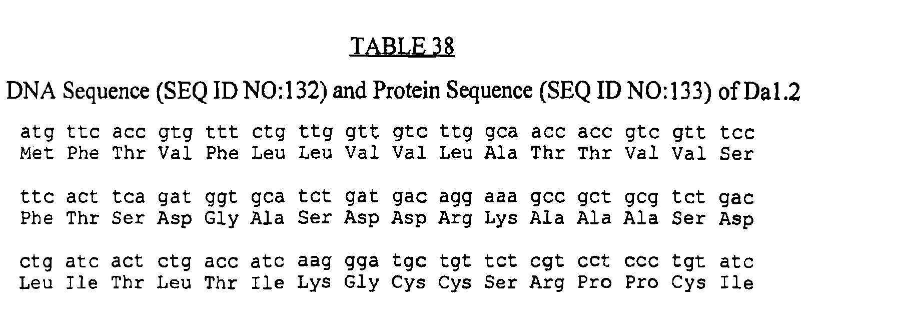

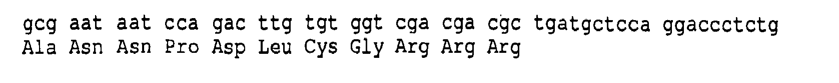

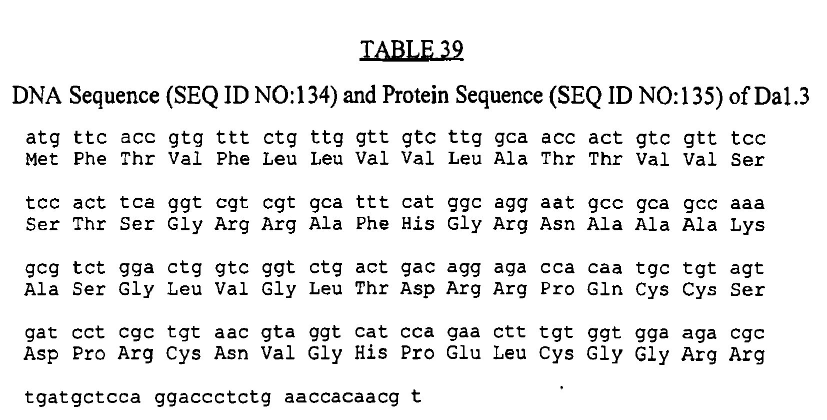

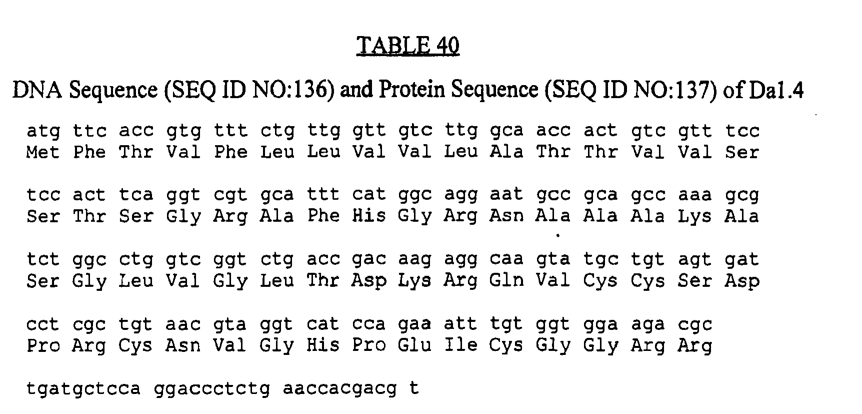

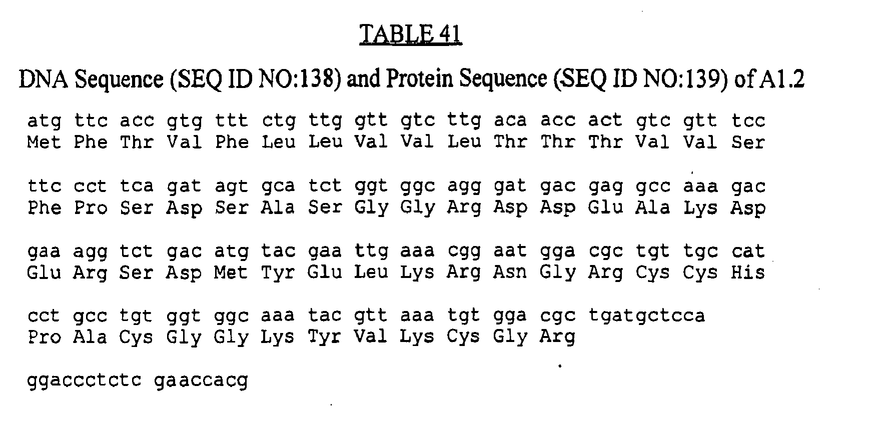

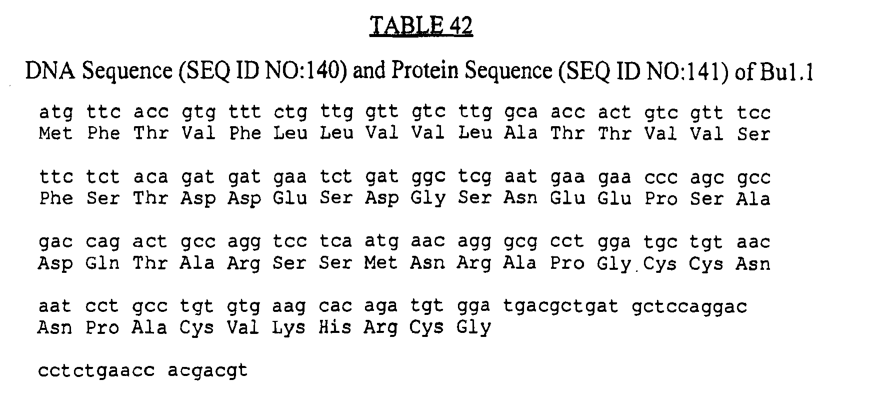

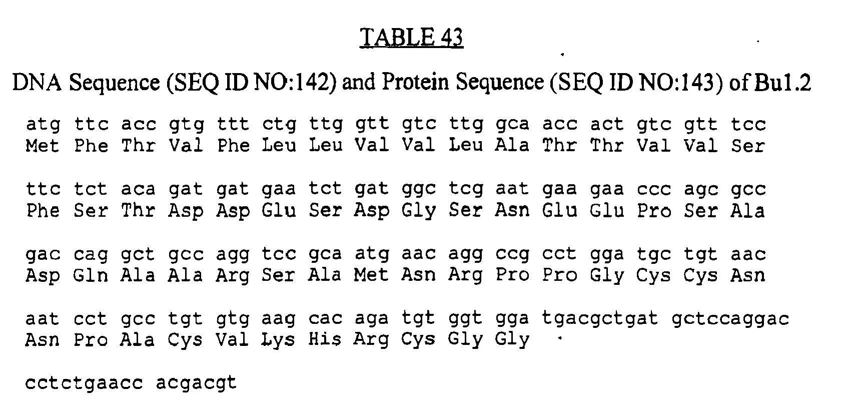

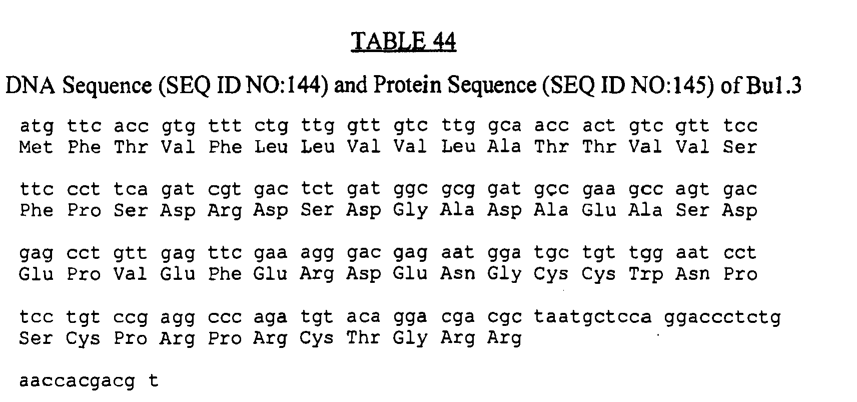

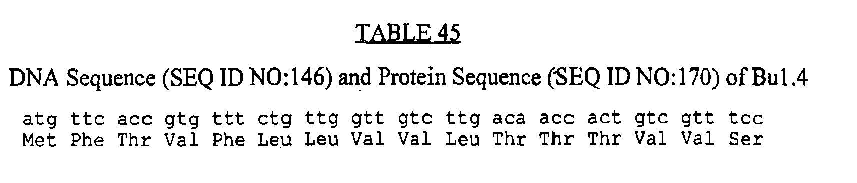

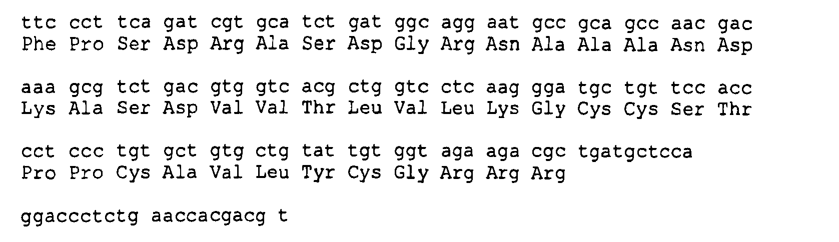

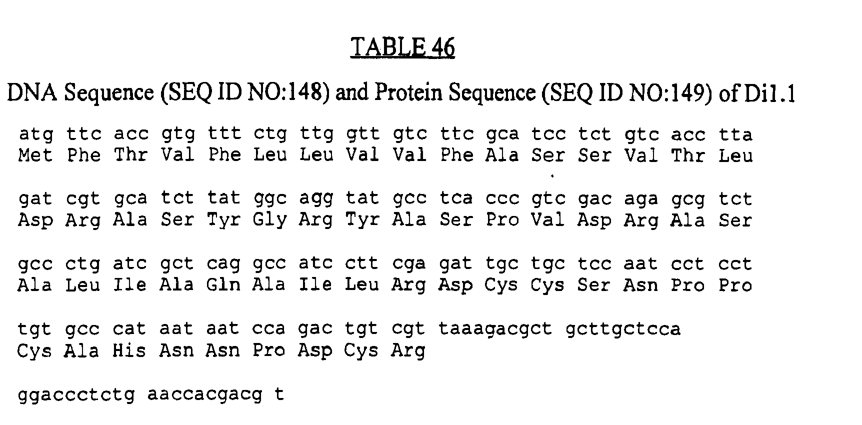

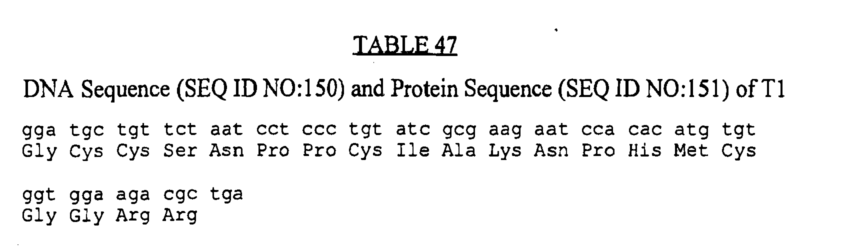

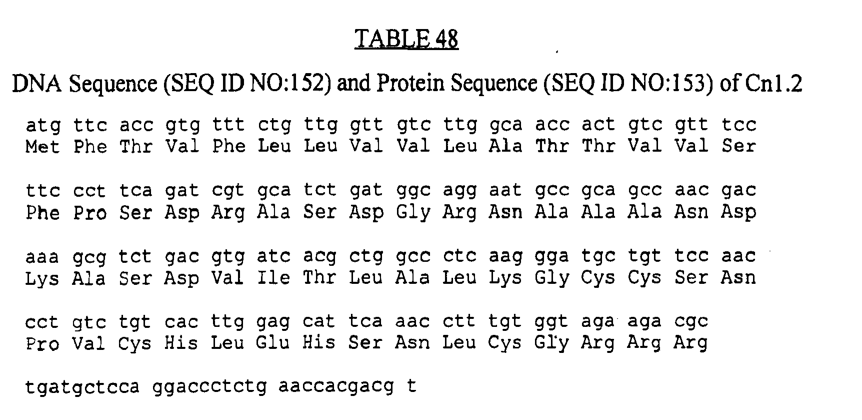

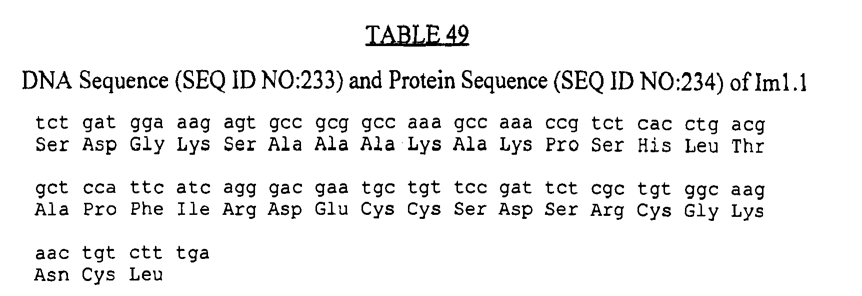

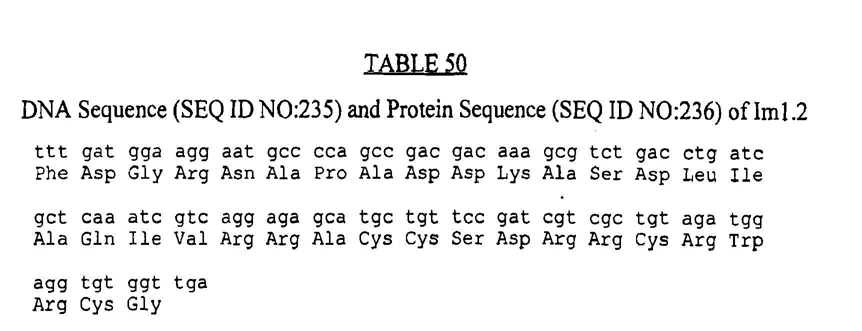

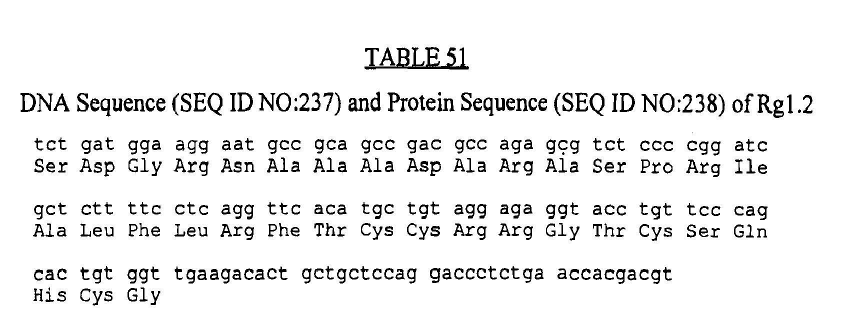

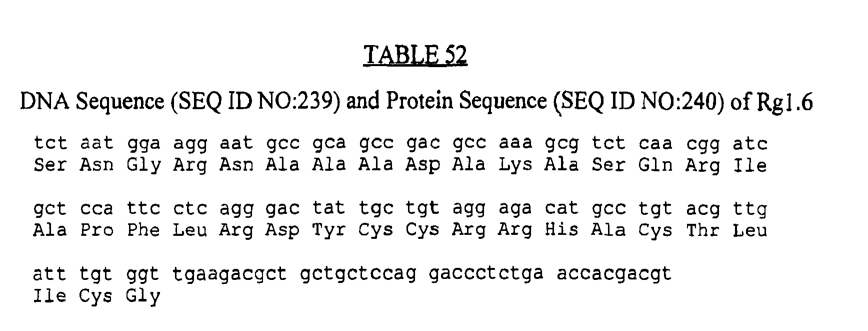

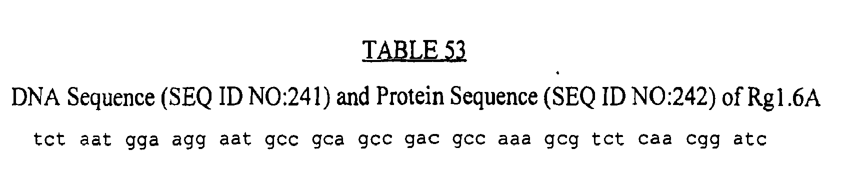

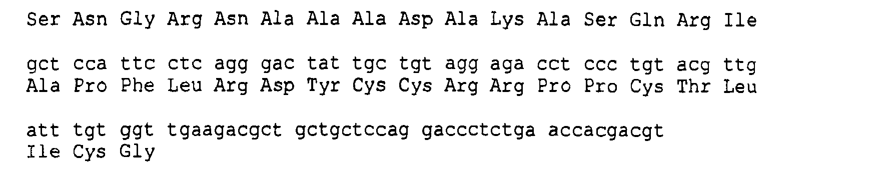

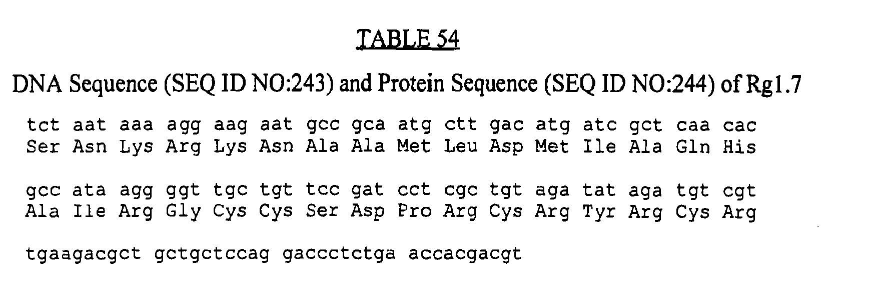

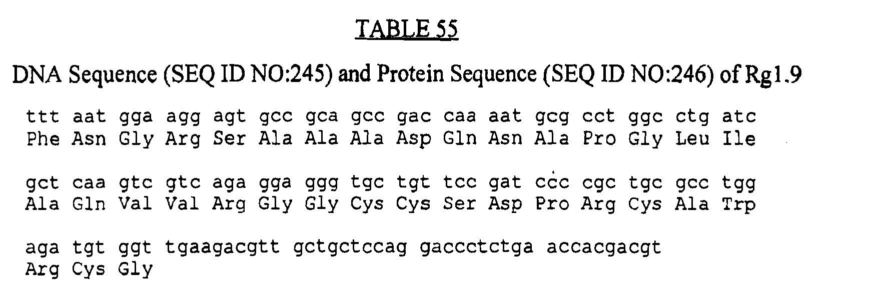

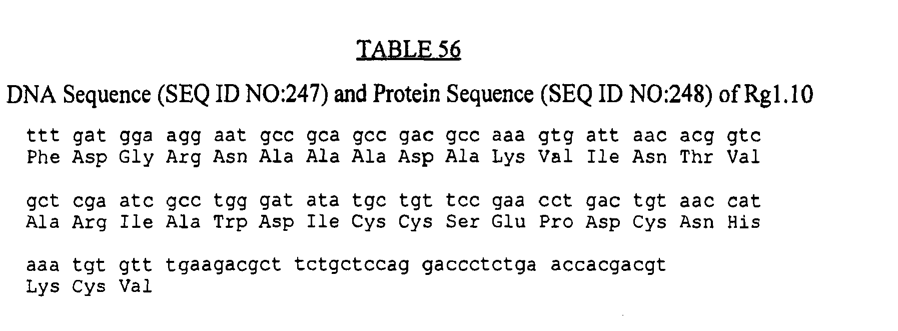

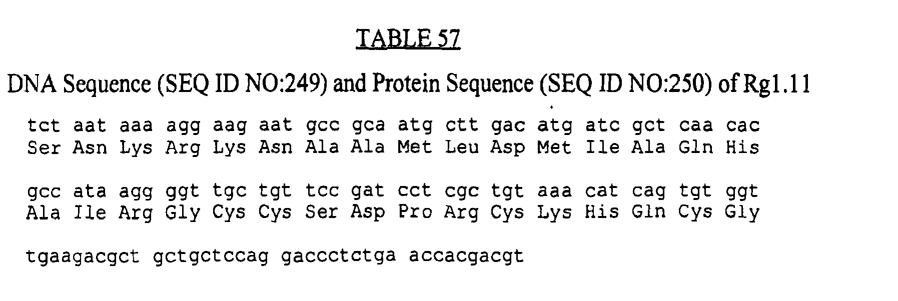

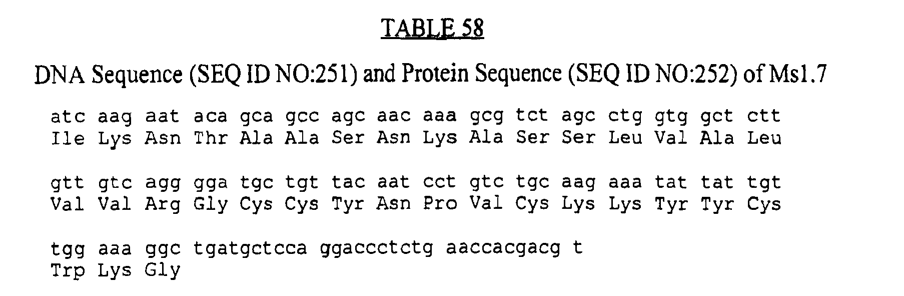

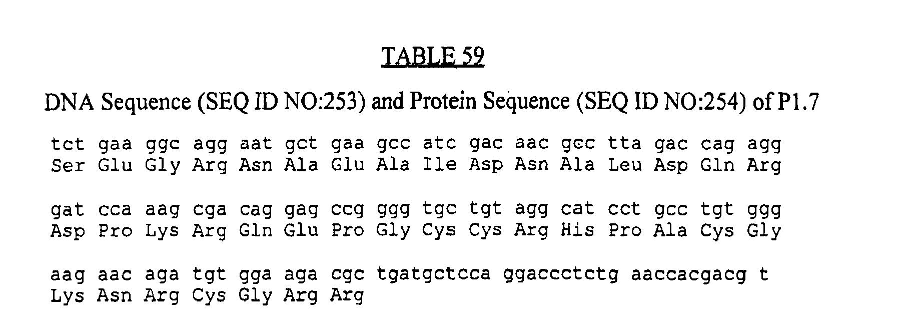

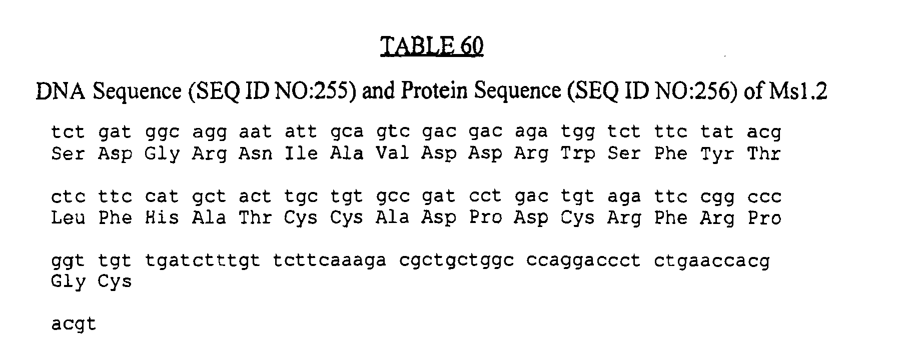

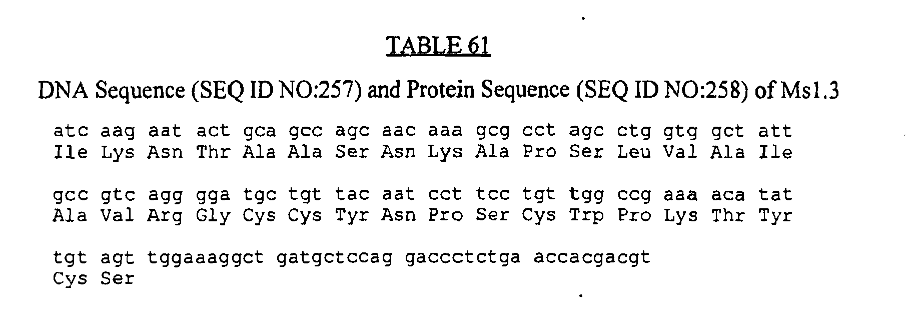

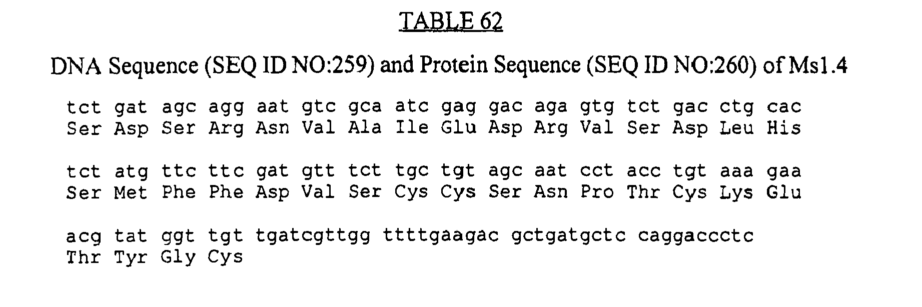

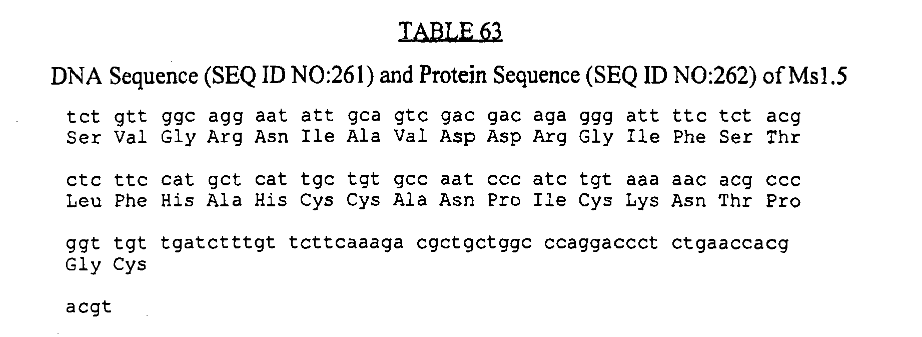

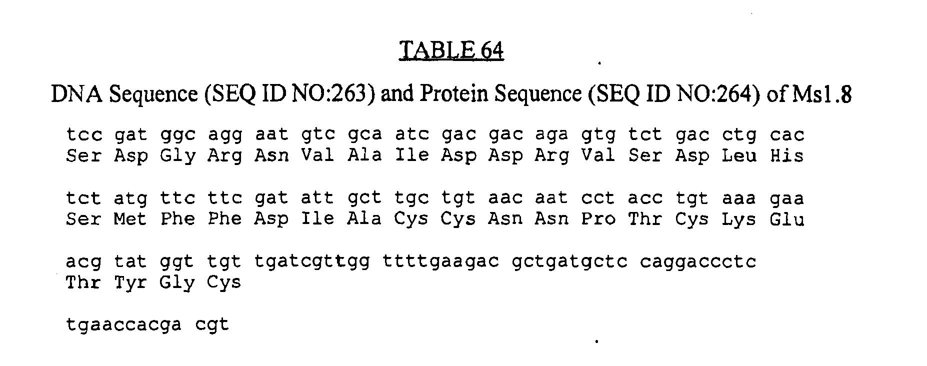

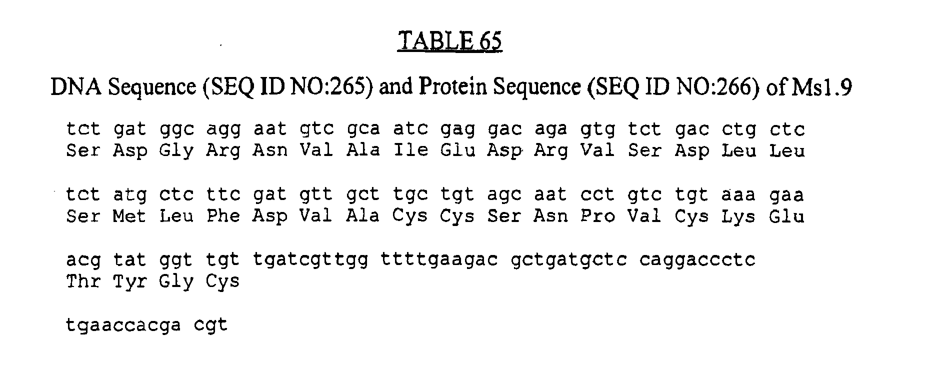

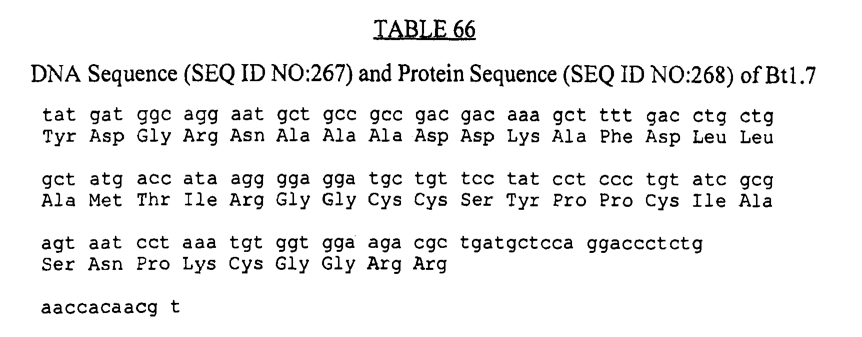

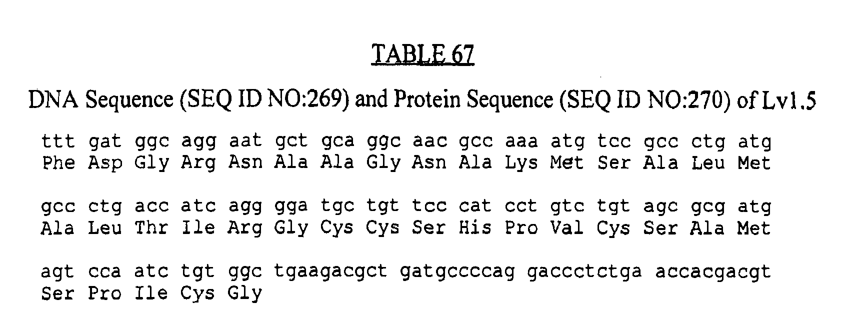

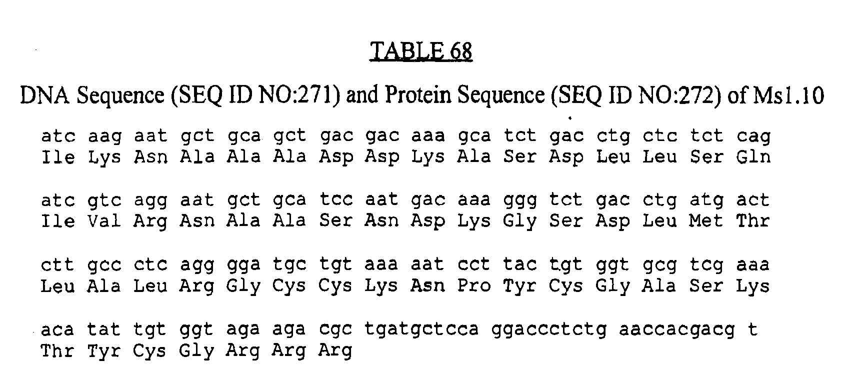

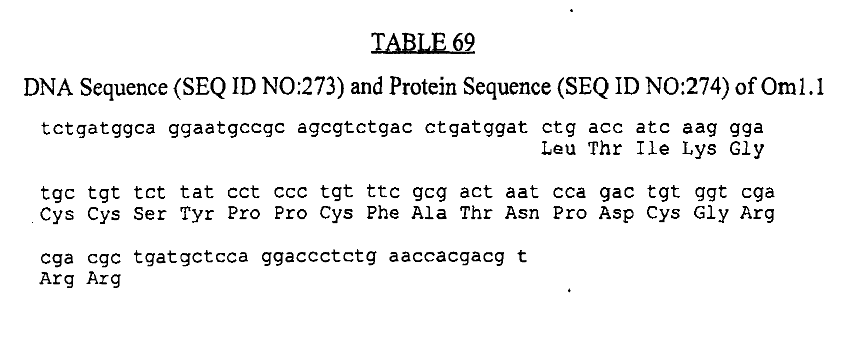

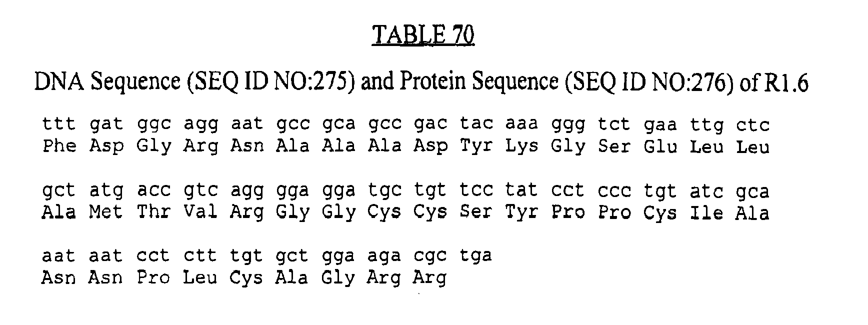

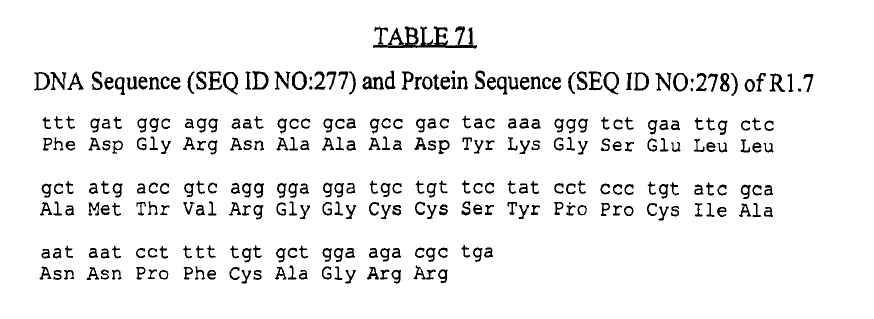

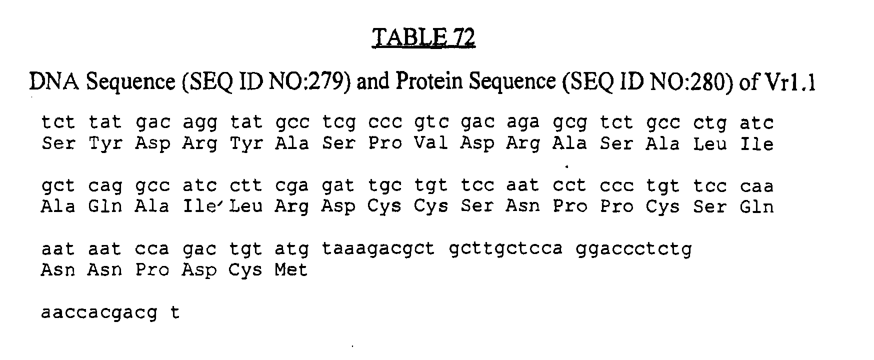

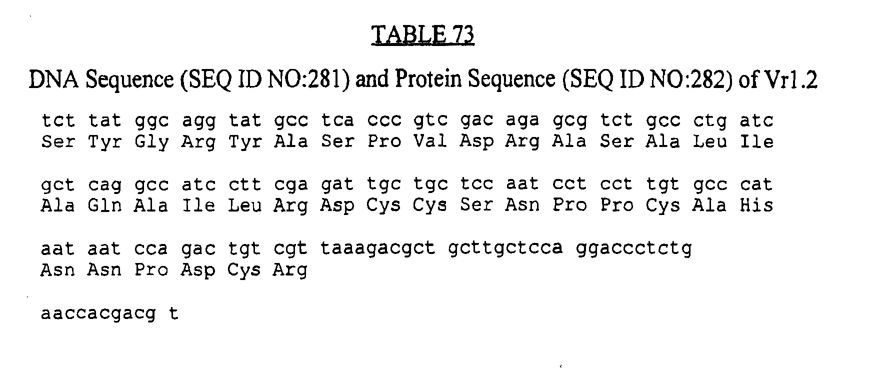

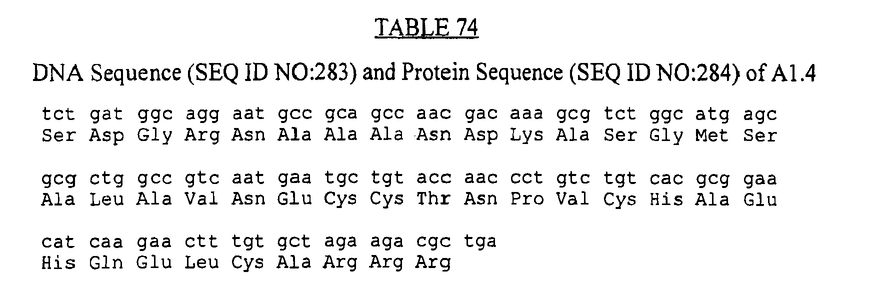

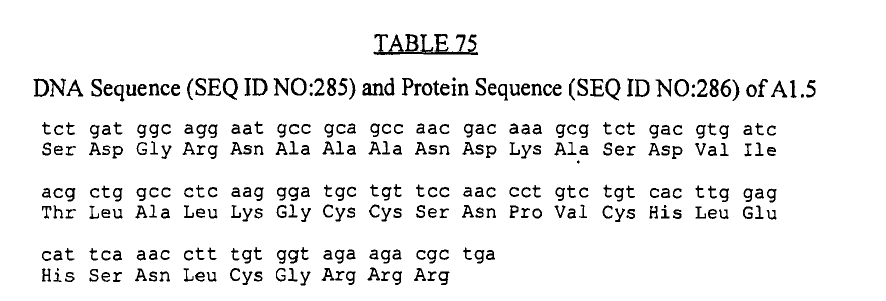

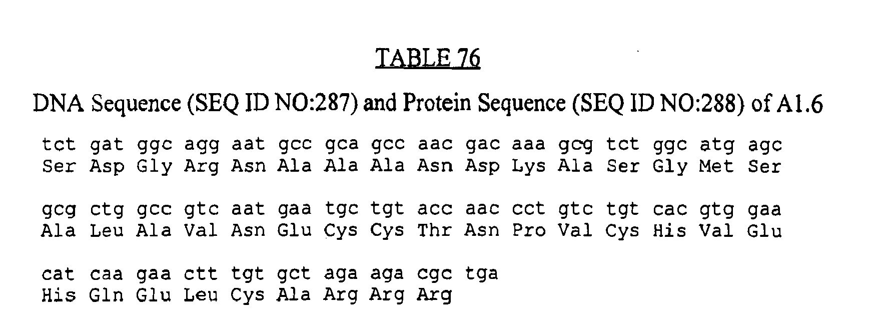

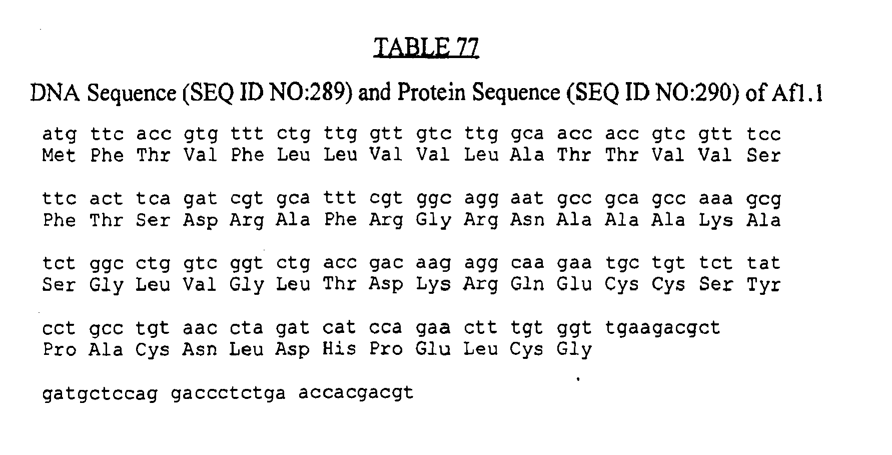

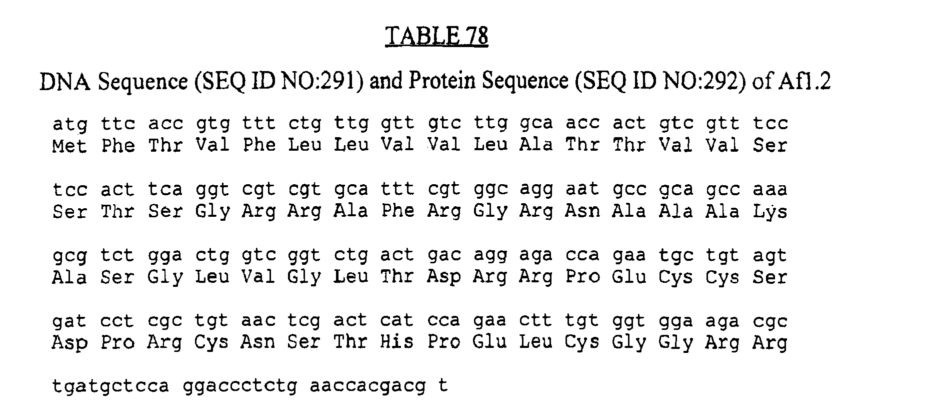

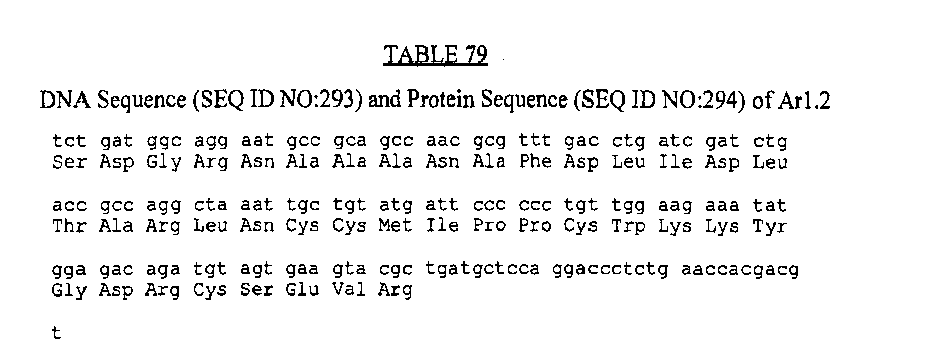

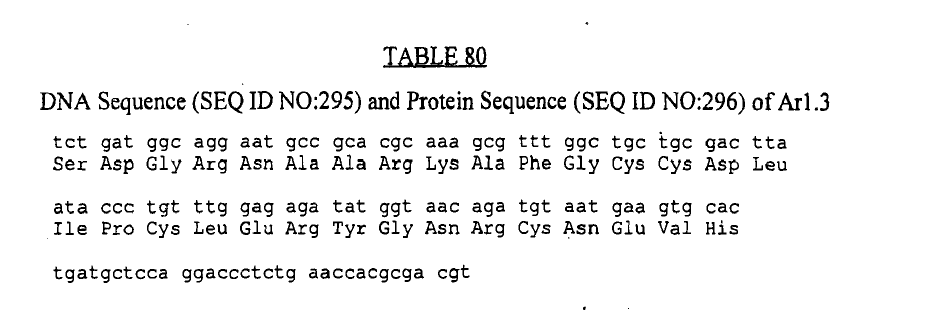

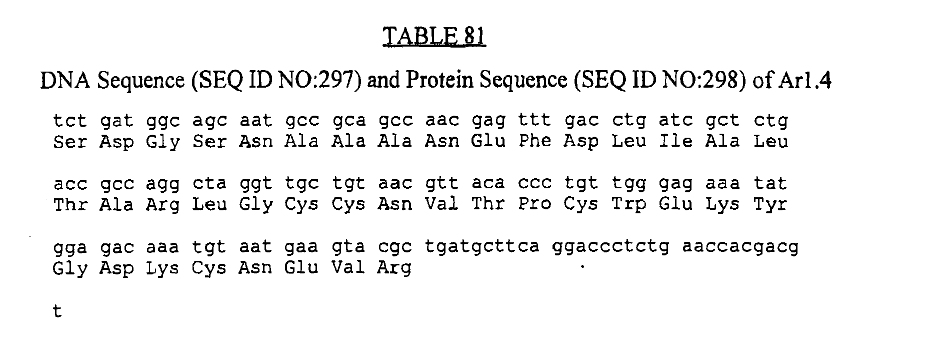

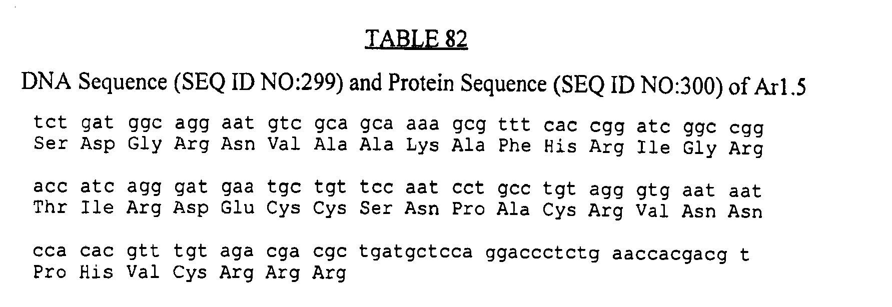

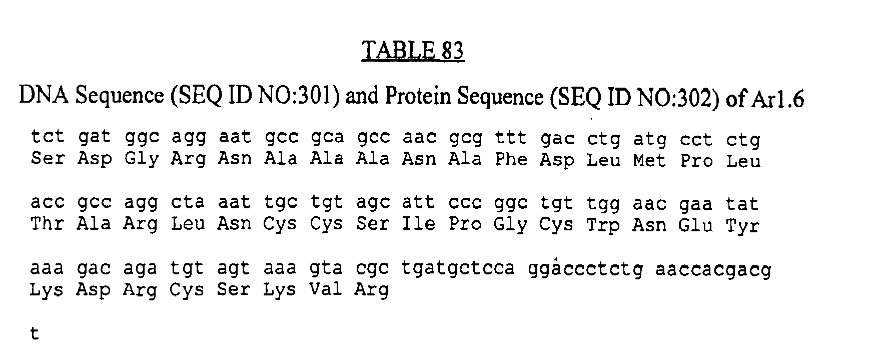

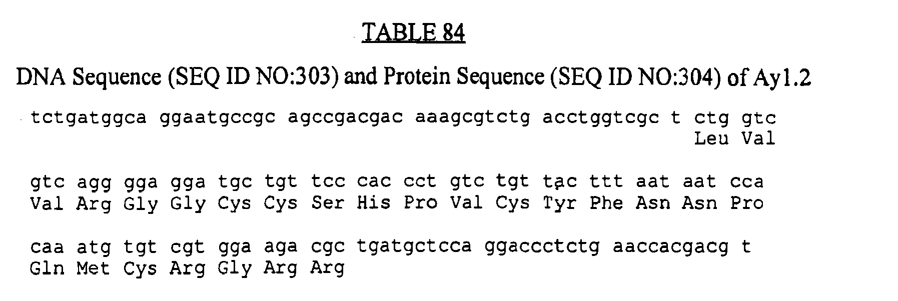

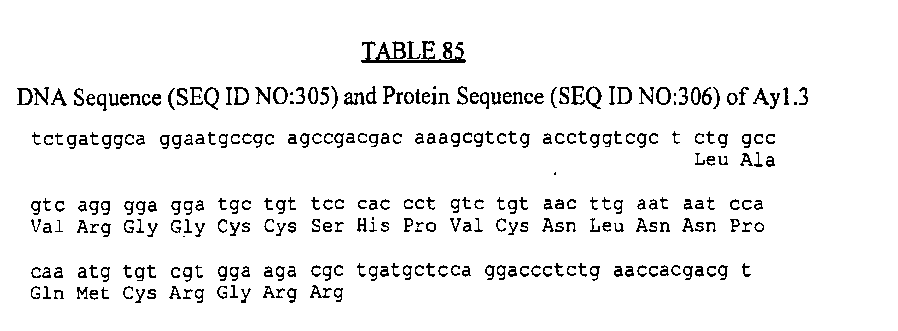

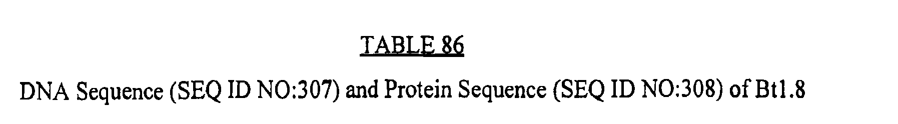

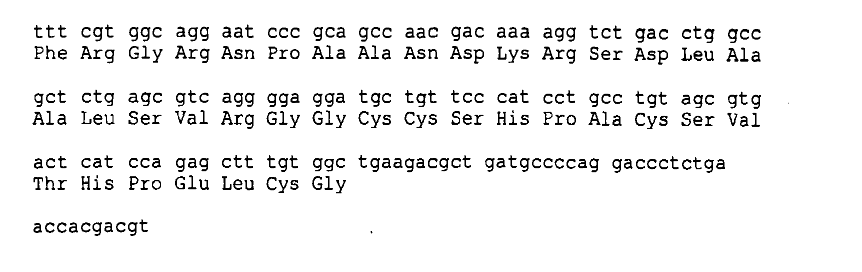

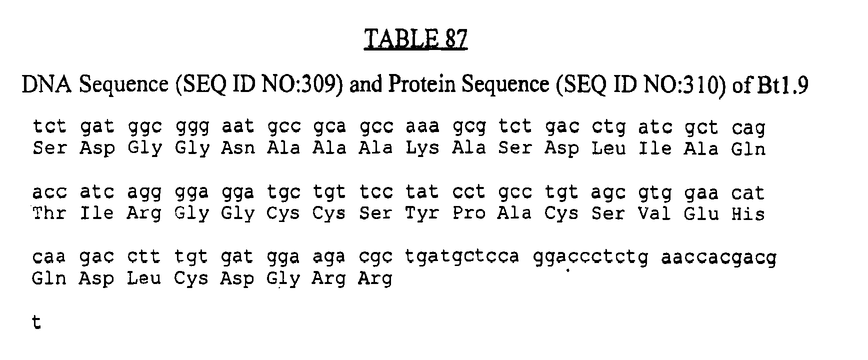

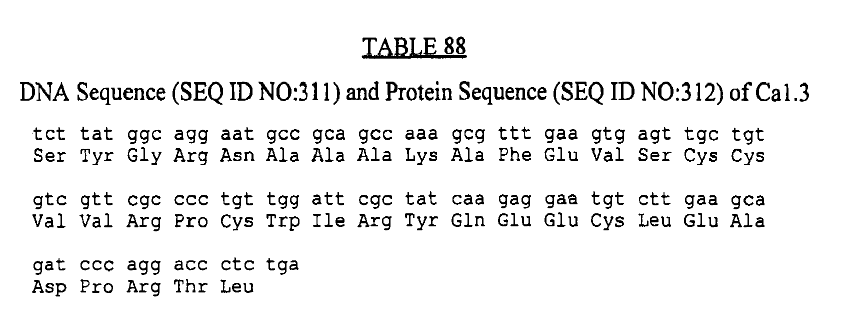

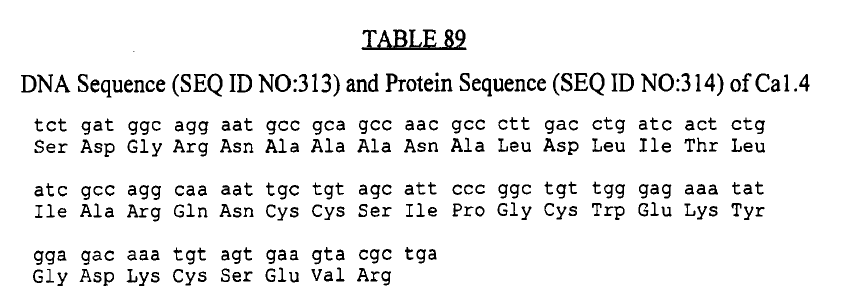

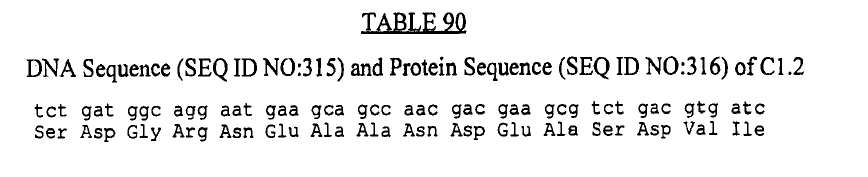

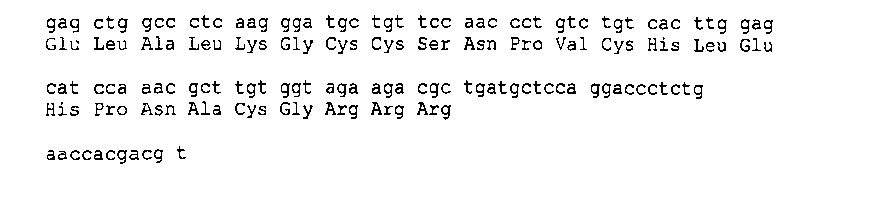

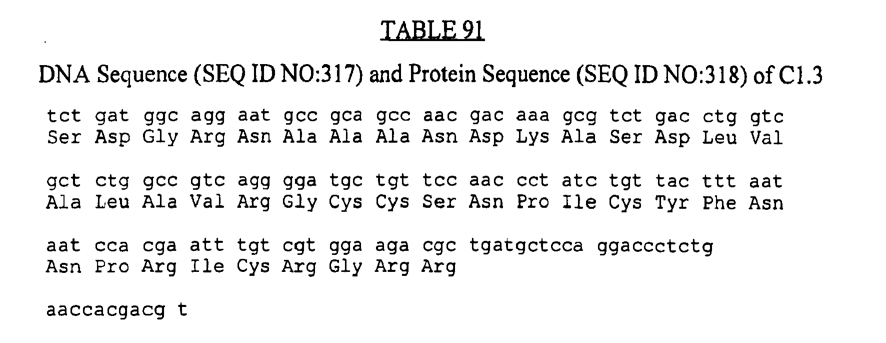

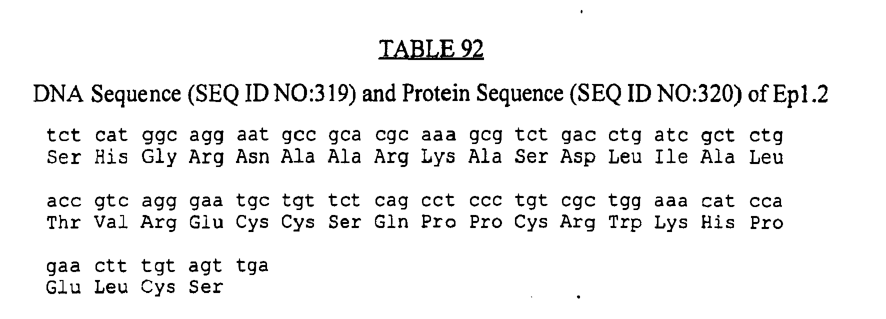

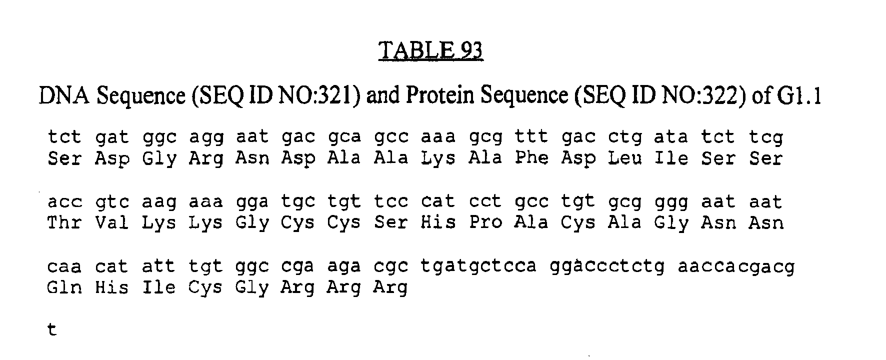

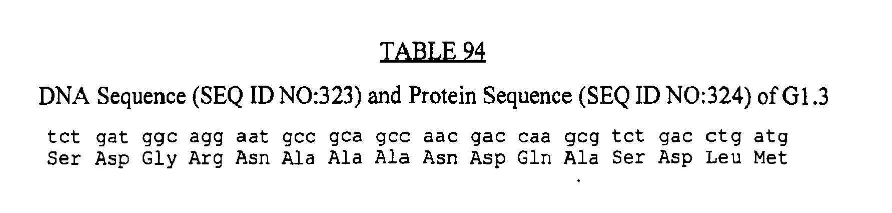

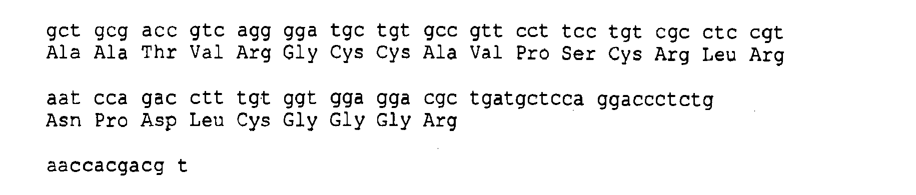

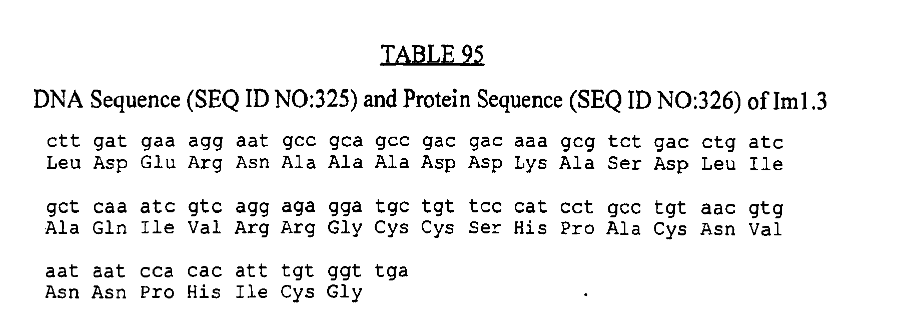

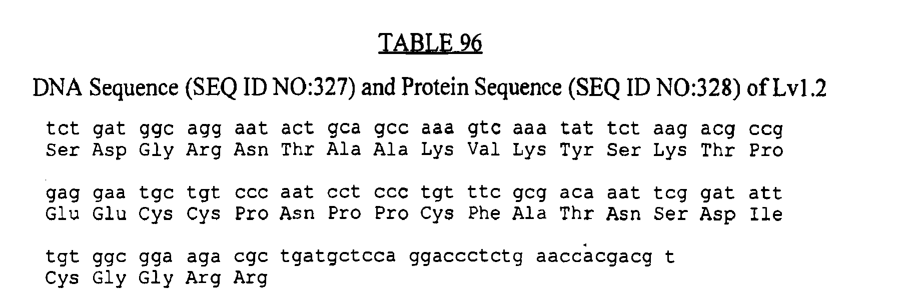

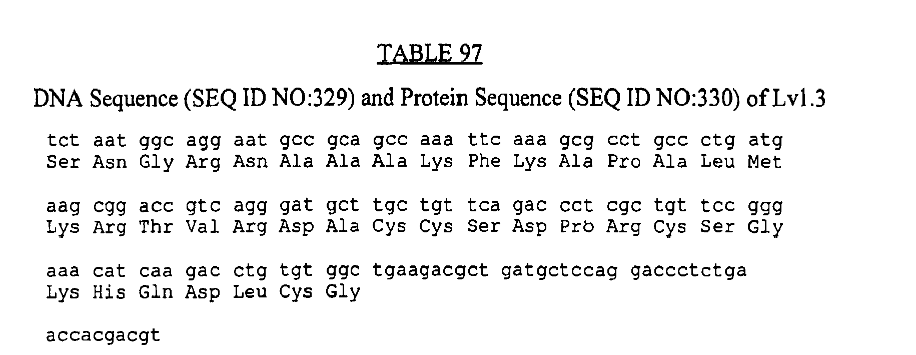

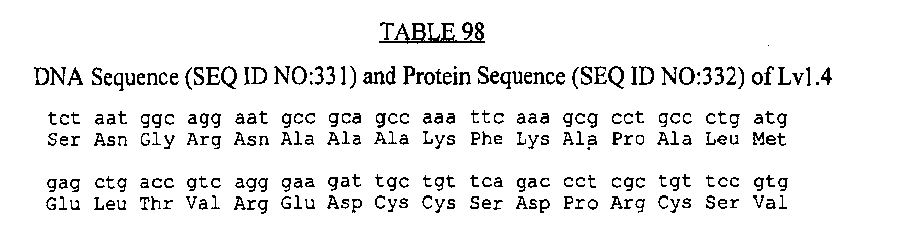

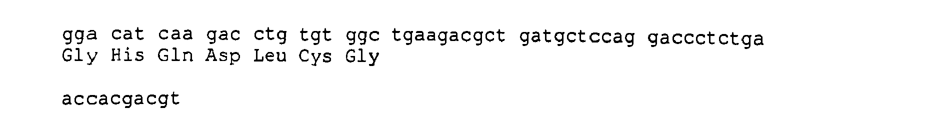

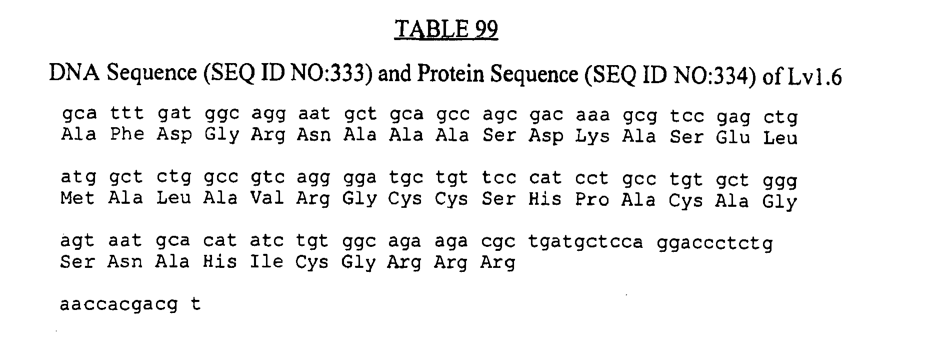

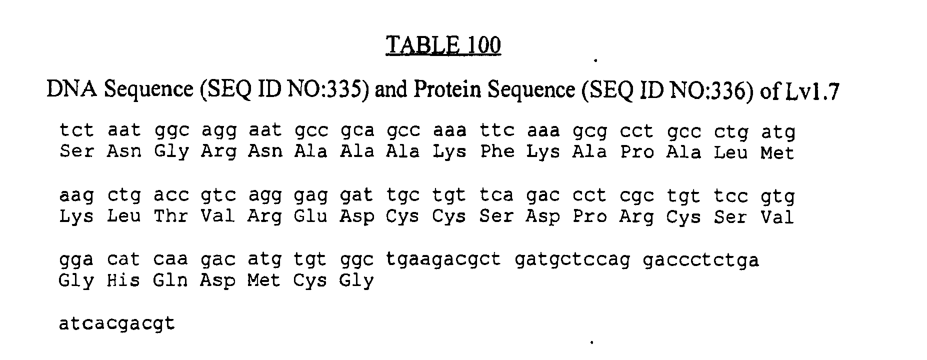

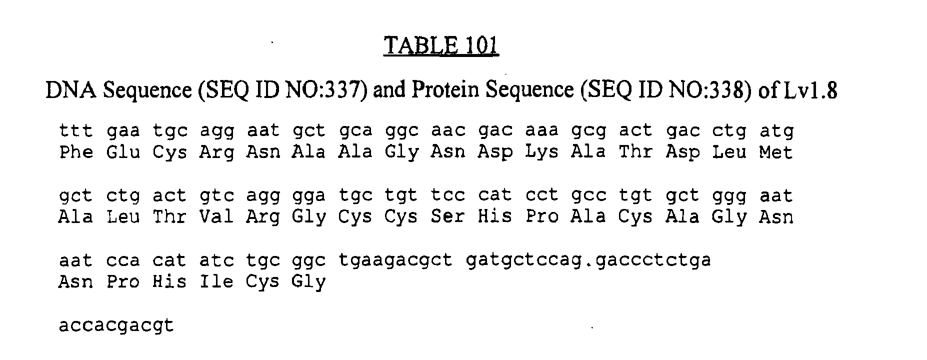

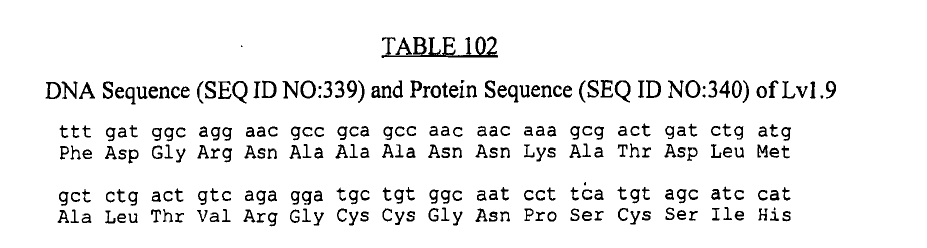

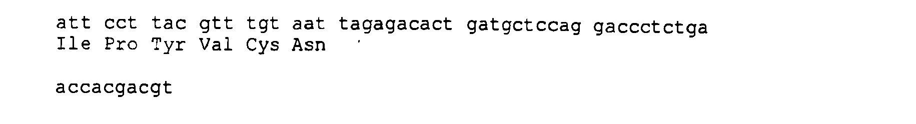

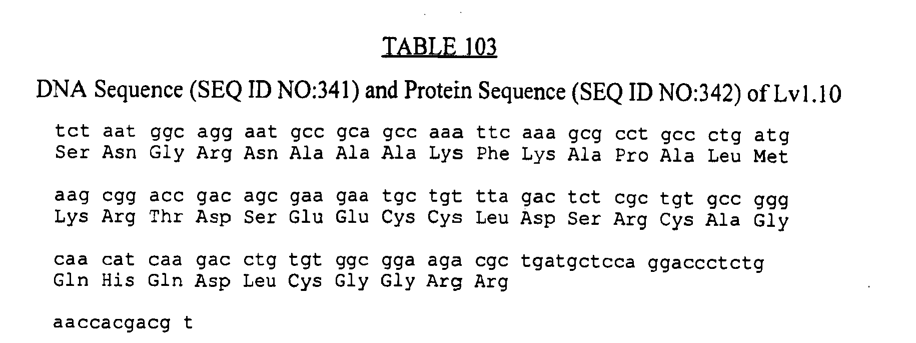

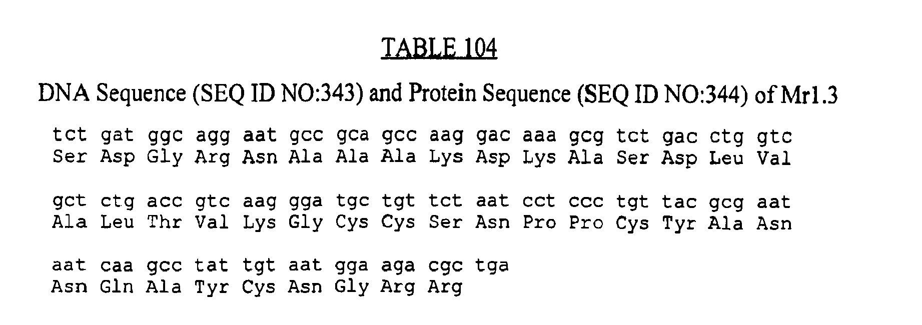

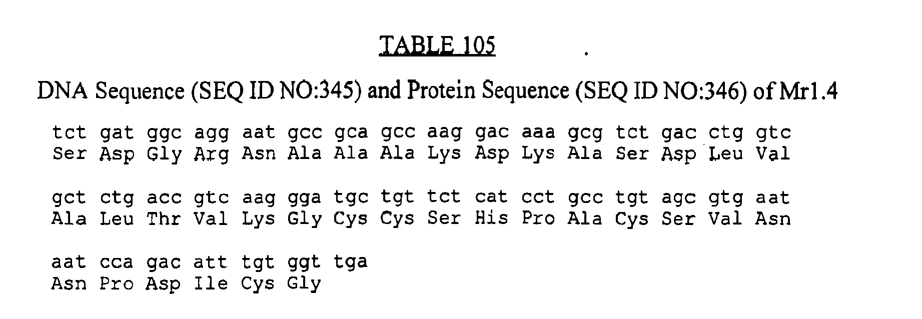

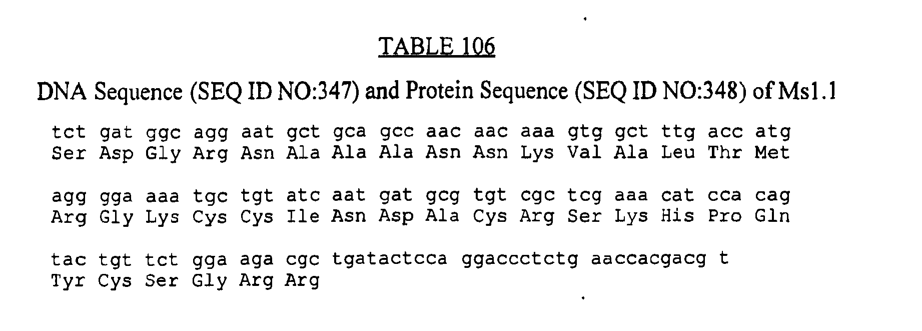

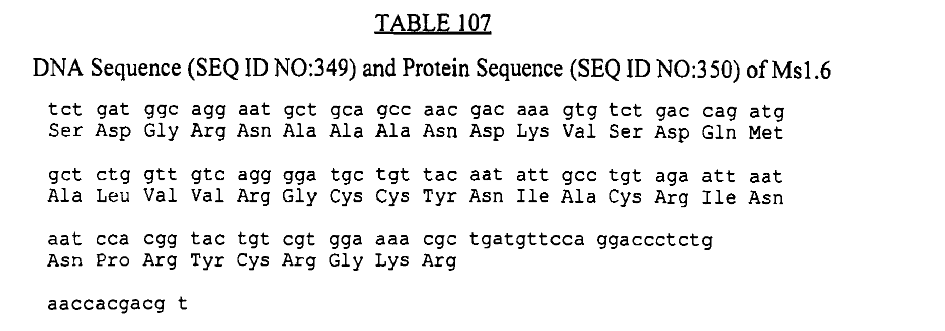

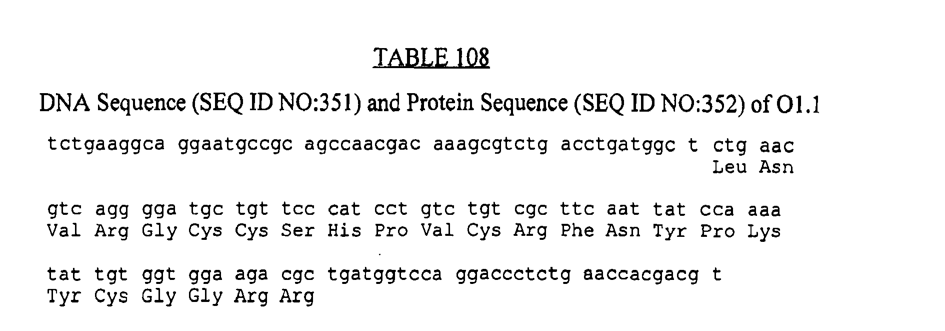

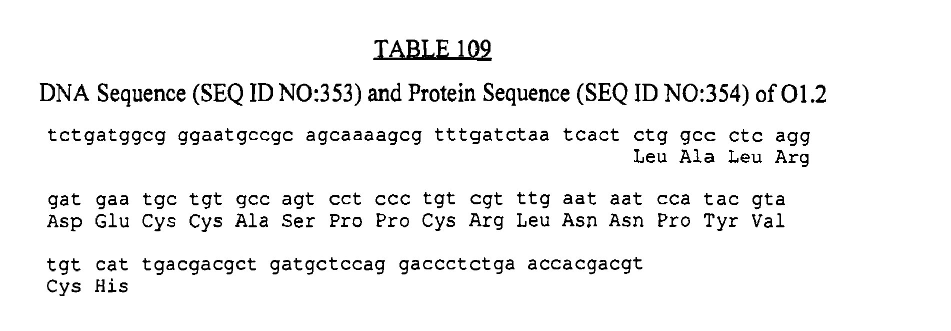

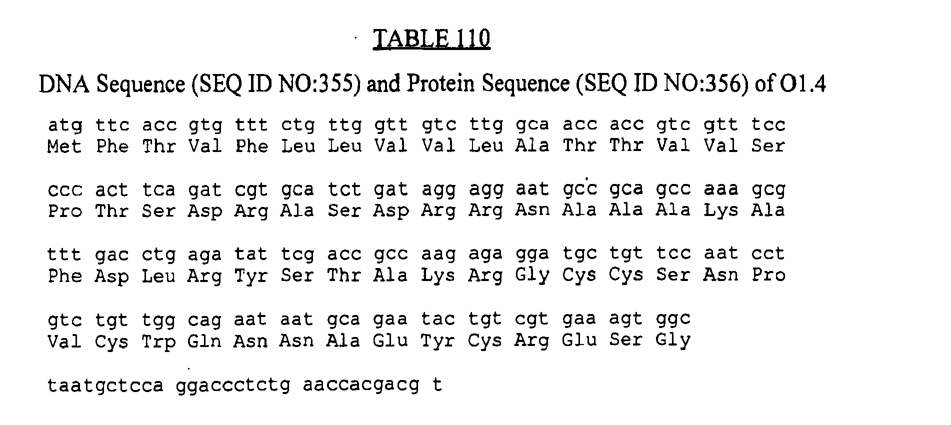

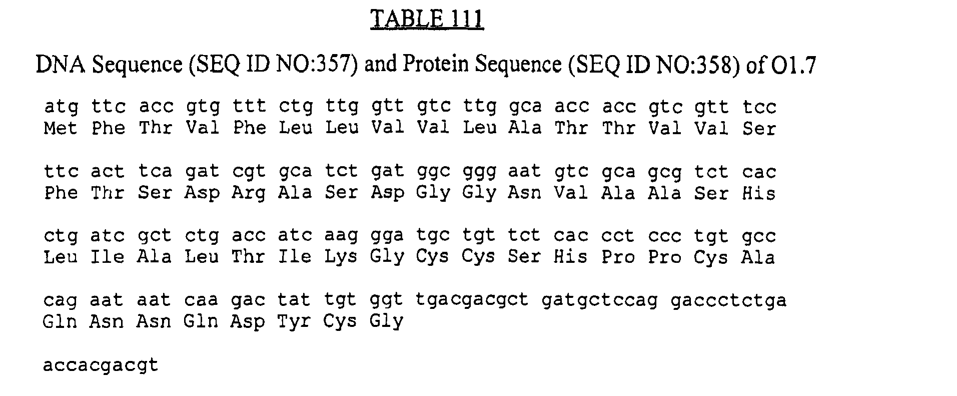

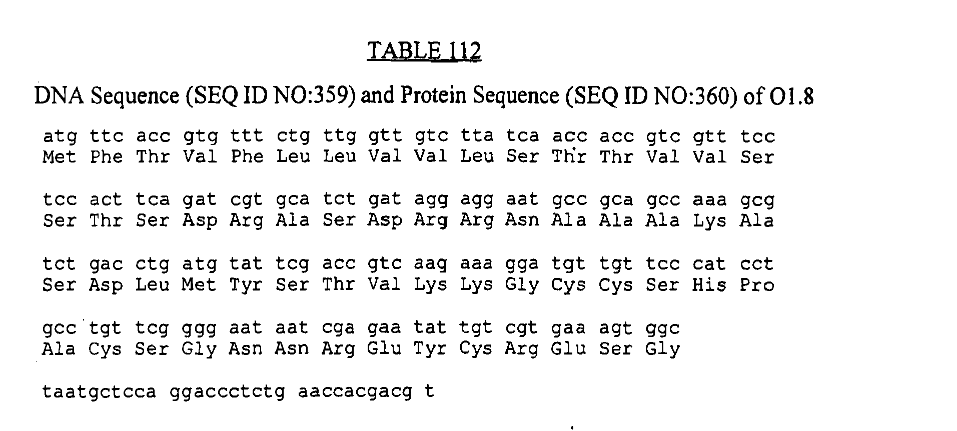

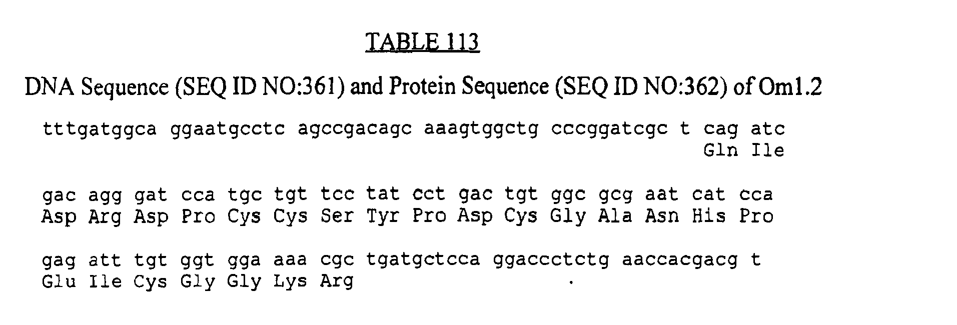

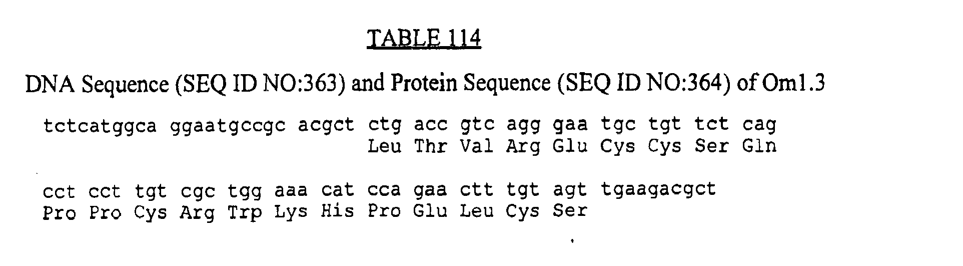

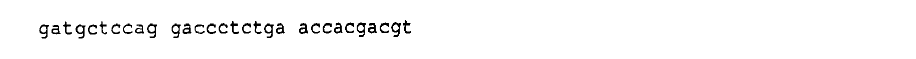

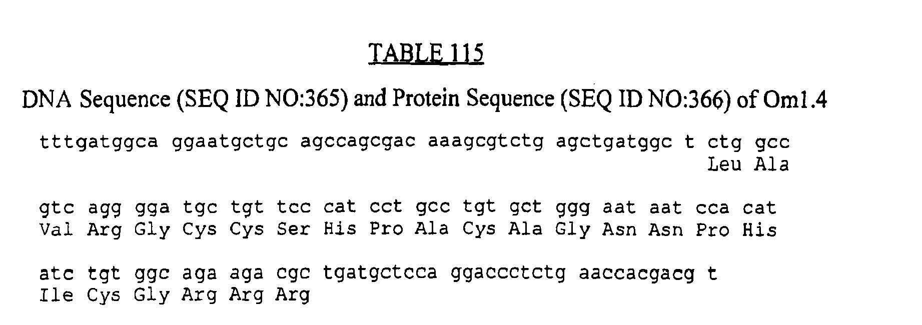

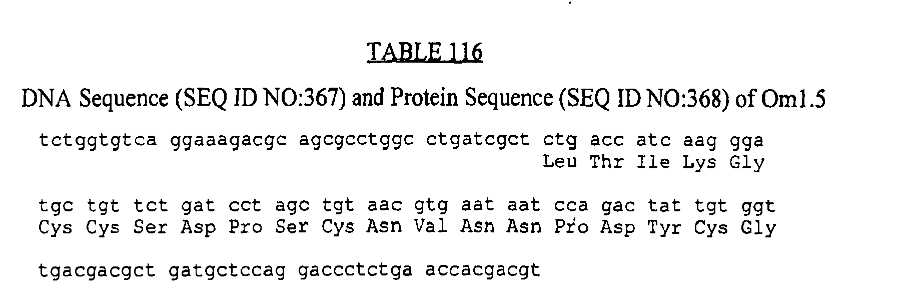

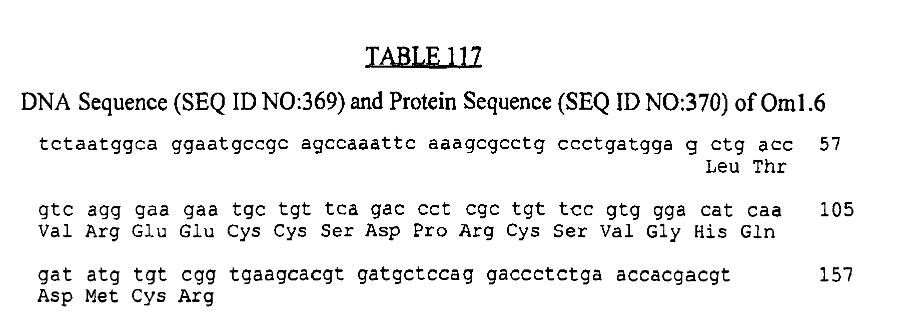

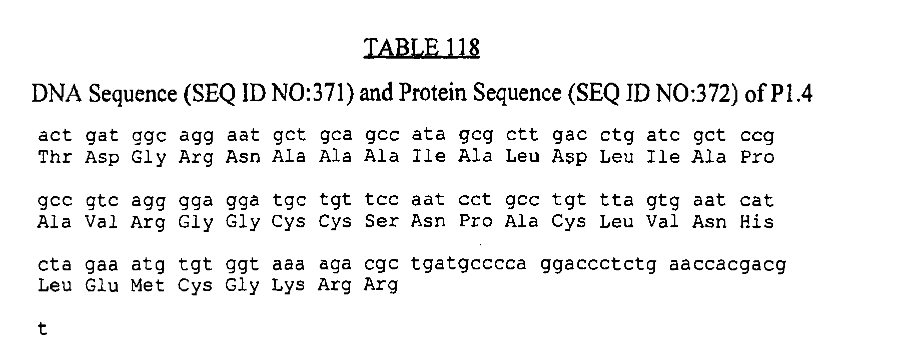

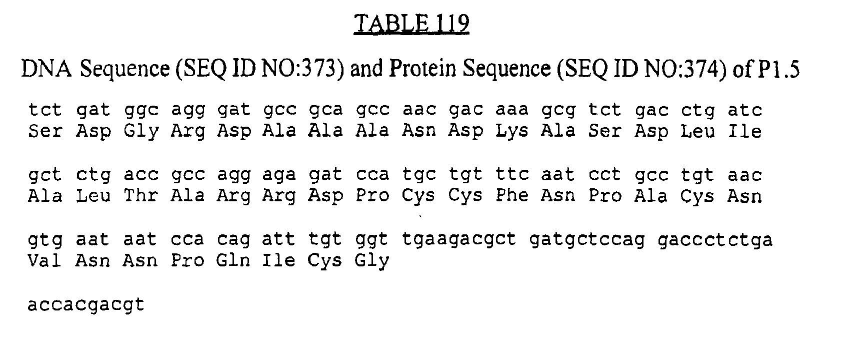

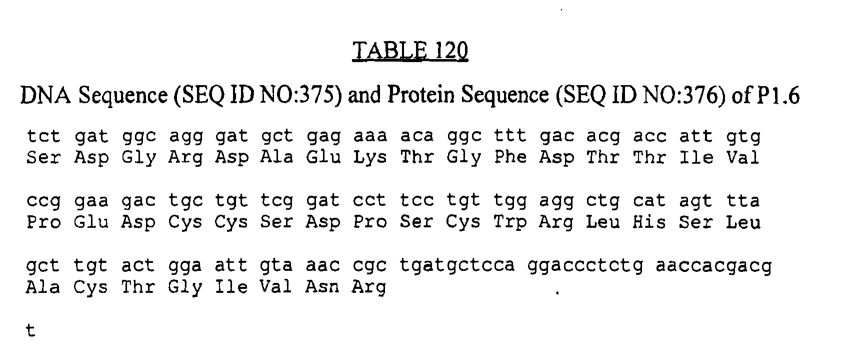

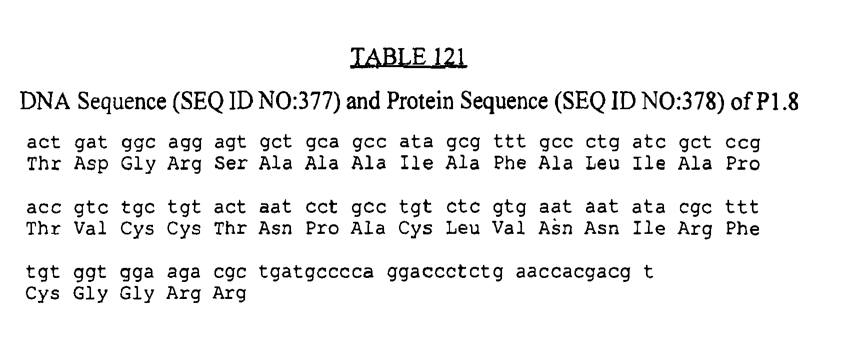

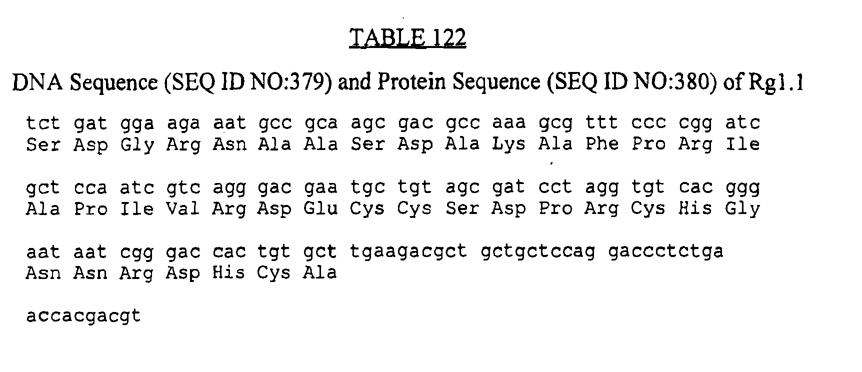

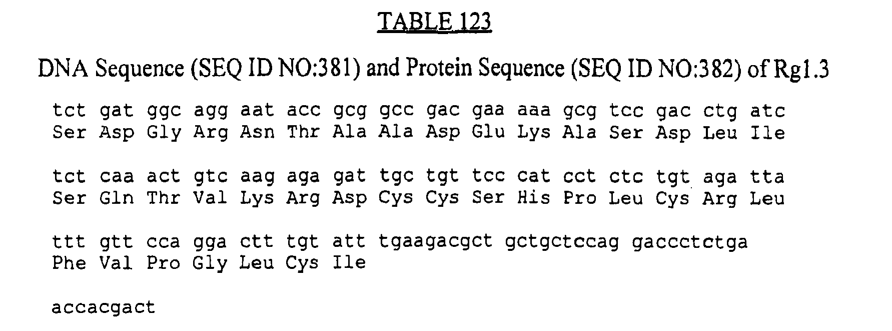

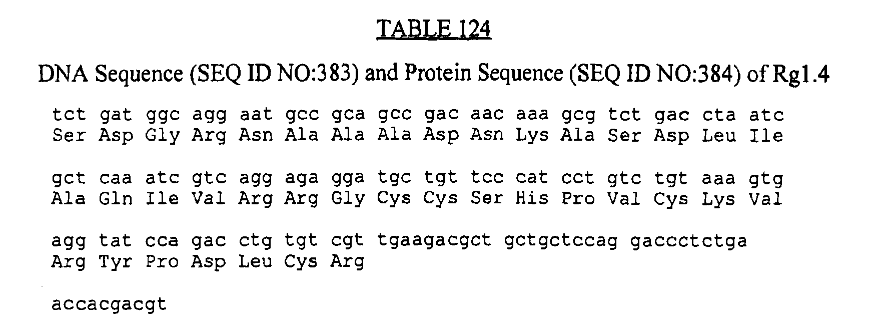

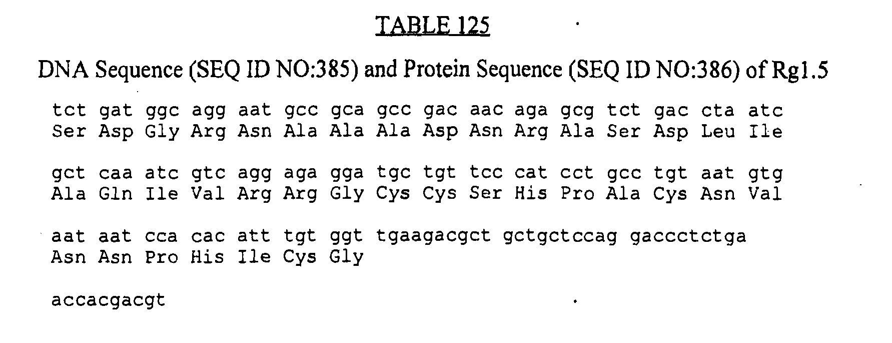

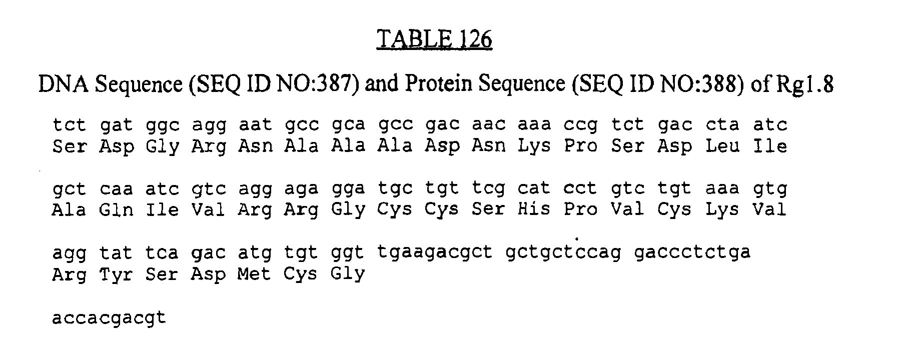

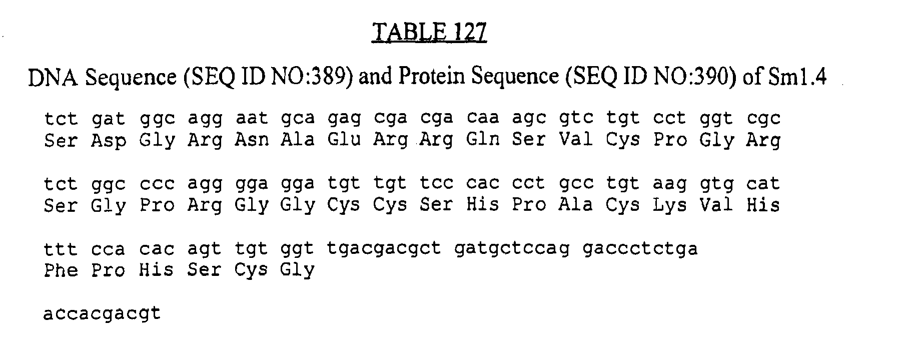

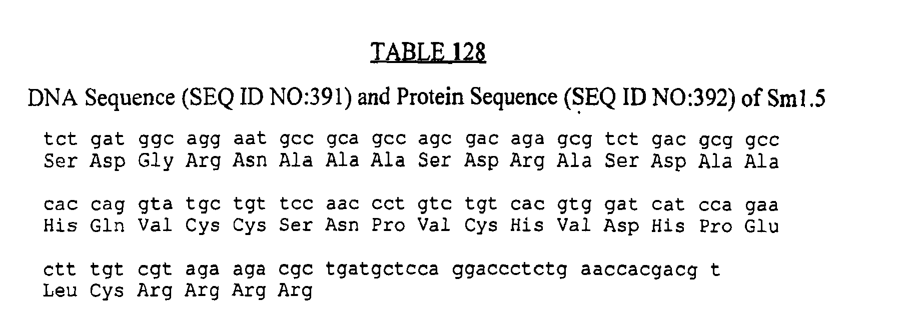

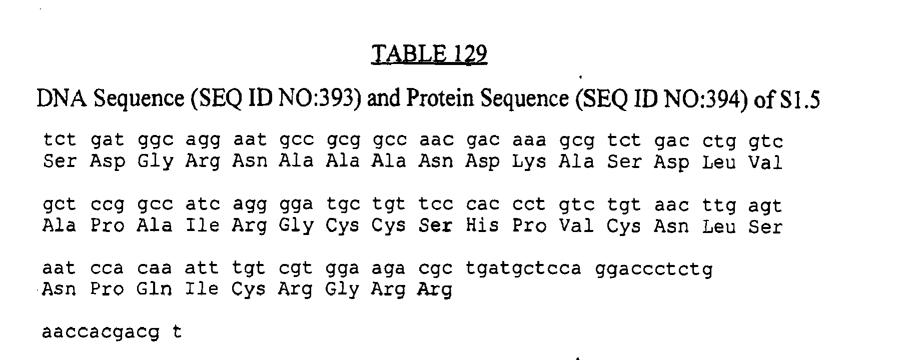

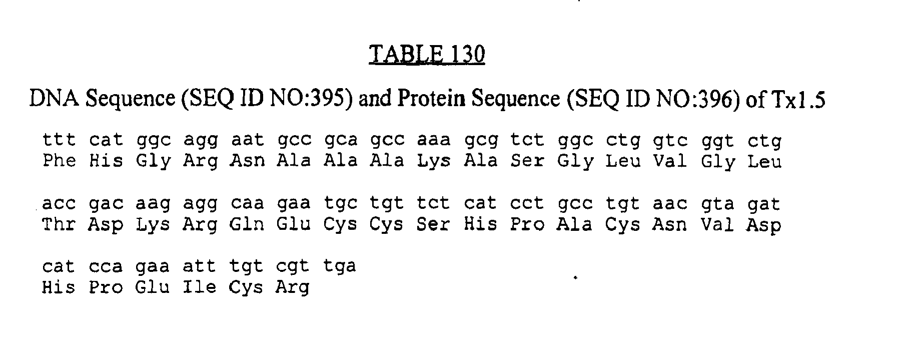

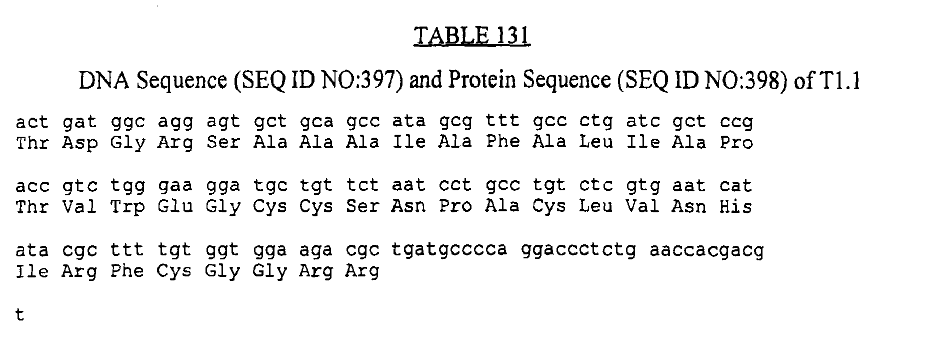

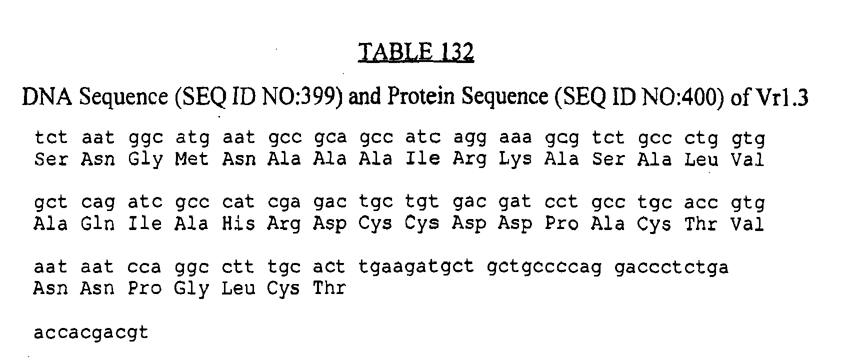

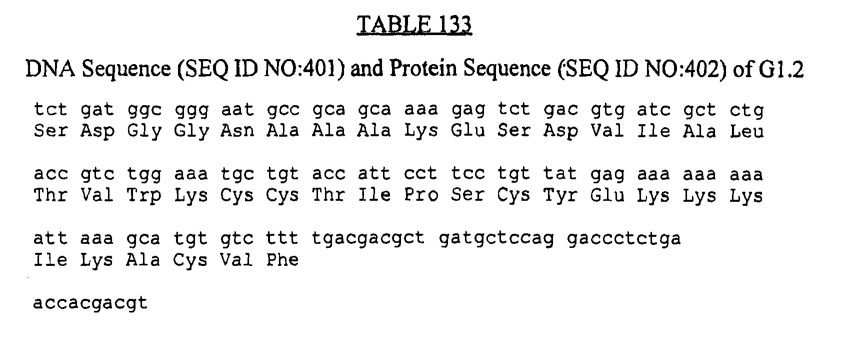

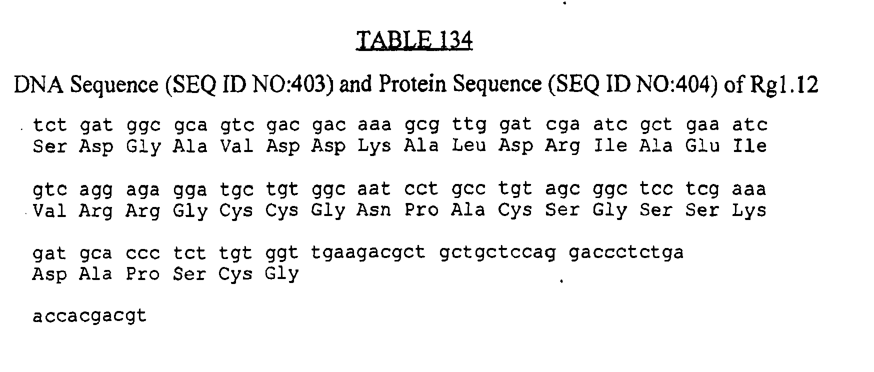

### LIST OF REFERENCES

Barnay, G. et al. (2000). J. Med. Chem.
Bitan, G. et al. (1997). J. Peptide Res. 49:421-426.
Blount, K. et al. (1992). Toxicon 30:835-842.
Bodansky et al. (1966). Chem. Ind. 38:1597-98.
Cartier, G.E. et al. (1996). J Biol. Chem. 271:7522-7528.
Cruz, L.J. at al. (1976). Verliger 18:302-308.
Cruz, L.J. et al. (1987). J. Biol. Chem. 260:9280-9288.
Fainzilber, M. et al. (1994). Biochemistry 33:9523-9529.
Gray, W.R. et al. (1981). J.Biol. Chem. 256:4734-4740.
Haack, J.A. et al. (1990). J. Biol. Chem. 265:6025-6029.
Horiki, K. et al. (1978). Chemistry Letters 165-68.
Hubry, V. et al. (1994). Reactive Polymers 22:231-241.
Jacobsen, R. et al. (1997). J Biol. Chem. 272:22531-22537.
Johnson, D.S. et al. (1995). Mol. Pharmacol. 48:194-199.
Kapoor (1970). J. Pharm. Sci. 59:1-27.
Kornreich, W.D. et al. (1986). U.S. Patent No. 4,569,967.
Luo, S. et al. (1998). J. Neurosci. 18:8571-8679.
Marshall, I.G. and Harvey, A.L. (1990). Toxicon 28:231-234.
Martinez, J.S. et al. (1995). Biochem. 34:14519-14526.
McIntosh, J.M. et al. (1982). Arch. Biochem. Biophys. 218:329-334.
Mena, E.E. et al, (1990). Neurosci. Lett. 118:241-244.
Methoden der Organischen Chemie (Houben-Weyl): Synthese von Peptiden, E. Wunsch (Ed.), Georg Thieme Verlag, Stuttgart, Ger. (1974).
Myers, R.A. et al. (1991). Biochemistry 30:9370-9377.
Nishiuchi, Y. et al. (1993). Int. J. Pept. Protein Res. 42:533-538.
Nowak, L. et al. (1984). Nature 307:462-465.
Olivera, B.M. et al. (1984). U.S. Patent 4,447,356.
Olivera, B.M. et al. (1985). Science 230:1338-1343.
Olivera, B.M. et al. (1996). U.S. Patent 5,514,774.
Rivier, J.R. et al. (1978). Biopolymers 17:1927-38.
Rivier, J.R. et al. (1987). Biochem. 26:8508-8512.
Sambrook, J. et al. (1989). Molecular Cloning: A Laboratory Manual, 2nd Ed., Cold Spring Harbor Laboratory, Cold Spring Harbor, NY.
Schroder & Lubke (1965). The Peptides 1:72-75, Academic Press, NY.
Shon, K.-J. et al. (1994). Biochemistry 33:11420-11425.
Stewart and Young, Solid-Phase Peptide Synthesis, Freeman & Co., San Francisco, CA (1969).
Vale et al. (1978). U.S. Patent 4,105,603.
Van de Steen, P. et al. (1998). Critical Rev. in Biochem. and Mol. Biol. 33:151-208*.*
Zafaralla, G.C. et al. (1988). Biochemistry 27:7102-7105.
Zhou L.M., et al. (1996). J. Neurochem. 66:620-628.
U.S. Patent No. 3,972,859.
U.S. Patent No. 3,842,067.
U.S. Patent No. 3,862,925.
U.S. Patent No. 5,550,050.
PCT Published Application WO 92/19195.
PCT Published Application WO 94/25503.
PCT Published Application WO 95/01203.
PCT Published Application WO 95/05452.
PCT Published Application WO 96/02286.
PCT Published Application WO 96/02646.
PCT Published Application WO 96/11698.
PCT Published Application WO 96/40871.
PCT Published Application WO 96/40959.
PCT Published Application WO 97/12635.

SEQUENCE LISTING

<110> University of Utah Research Foundation
      Cognetix, Inc.

<120> Alpha-Conotoxin Peptides

<130> Alphas 2

<140>
<141>

<150> US 60/118,381
<151> 1999-01-29

<160> 404

<170> PatentIn Ver. 2.0

<210> 1
<211> 17
<212> PRT
<213> Artificial Sequence

<220>
<223> Description of Artificial Sequence:Alpha-Conotoxin
      Peptide Generic Formula I

<220>
<221> SITE
<222> (1)..(3)
<223> Xaa at residue 1 is des-Xaa, Ile, Leu or Val; Xaa
      at residue 2 is des-Xaa, Ala or Gly; Xaa at
      residue 3 is des-Xaa, Gly, Trp (D or L), neo-Trp,
      halo-Trp or any unnatural aromatic amino acid.

<220>
<221> SITE
<222> (4)..(5)
<223> N-methyl-Lys, Xaa at residue 4 is des-Xaa, Gly,
      Trp (D or L), neo-Trp, halo-Trp or any unnatural
      aromatic amino acid; Xaa at residue 5 is Glu,
      gamma-carboxy-Glu (Gla), Asp, Ala, Thr, Ser, Gly,

<220>
<221> SITE
<222> (5)..(8)
<223> Ile, Tyr, nor-Tyr, mono-halo-Tyr, di-halo-Tyr,
      O-sulpho-Tyr, O-phospho-Tyr, nitro-Tyr or any
      unnatural hydroxy containing amino acid; Xaa at
      residue 8 is Ser, Thr, Arg, ornithine,

<220>
<221> SITE
<222> (8)..(9)
<223> homoarginine, Lys, N,N-dimethyl-Lys, N,N,N-
      trimethyl-Lys or any unnatural basic amino acid;
      Xaa at residue 9 is Asp, Glu, Gla, Arg, ornithine,
      homoarginine, Lys, N-methyl-Lys,N,N-dimethyl-

<220>
<221> SITE
<222> (9)..(11)
<223> Lys, N,N,N-trimethyl-Lys or any unnatural basic
      amino acid; Xaa at residue 10 is Ser, Thr, Asn,
      Ala, Gly, His, halo-His, Pro or hydroxy-Pro;
      Xaa at residue 11 is Thr, Ser, Ala, Asp, Asn,

<220>

<221> SITE
<222> (11)..(13)
<223> Pro, hydroxy-Pro, Arg, ornithine, homoarginine,
      Lys, N-methyl-Lys, N,N-dimethyl-Lys, N,N,N-
      trimethyl-Lys or any unnatural basic amino acid;
      Xaa at residue 13 is Gly, Ser, Thr, Ala, Asn,

<220>
<221> SITE
<222> (13)..(14)
<223> Arg, ornithine, homoarginine, Lys, N-methyl-Lys,
      N,N-dimethyl-Lys, N,N,N-trimethyl-Lys or any
      unnatural basic amino acid; Xaa at residue 14 is
      Gln, Leu, His, halo-His, Trp (D or L), halo-Trp,

<220>
<221> SITE
<222> (14)
<223> neo-Trp, Tyr, nor-Tyr, mono-halo-Tyr, di-halo-
      Tyr, O-sulpho-Tyr, O-phospho-Tyr, nitro-Tyr, Arg,
      ornithine, homoarginine, Lys, N-methyl-Lys, N,N-
      dimethyl-Lys, N,N,N-trimethyl-Lys, any unnatural

<220>
<221> SITE
<222> (14)..(15)
<223> basic amino acid or any unnatural aromatic amino
      acid; Xaa at residue 15 is Asn, His, halo-His,
      Ile, Leu, Val, Gln, Arg, ornithine, homoarginine,
      Lys, N-methyl-Lys, N,N-dimethyl-Lys, N,N,N-

<220>
<221> SITE
<222> (15)..(17)
<223> trimethyl-Lys or any unnatural basic amino acid;
      Xaa at residue 17 is des-Xaa, Val, Ile, Leu, Arg,
      ornithine, homoarginine, Lys, N-methyl-Lys, N,N-
      dimethyl-Lys, N,N,N-trimethyl-Lys or any

<220>
<221> SITE
<222> (17)
<223> unnatural basic amino acid.

<400> 1
Xaa Xaa Xaa Xaa Xaa Cys Cys Xaa Xaa Xaa Xaa Cys Xaa Xaa Xaa Cys
  1               5                   10                  15

Xaa



<210> 2
<211> 21
<212> PRT
<213> Artificial Sequence

<220>
<223> Description of Artificial Sequence:Alpha-Conotoxin
      Peptide Generic Formula II.

<220>
<221> SITE
<222> (1)..(3)
<223> Xaa at residue 1 is des-Xaa, Asp, Glu or gamma-
      carboxy-Glu (Gla); Xaa at residue 2 is des-Xaa,
      Gln, Ala, Asp, Glu, Gla; Xaa at residue 3 is des-
      Xaa, Gly, Ala, Asp, Glu, Gla, Pro or hydroxy-Pro.

<220>
<221> SITE
<222> (4)..(7)
<223> Xaa at residue 4 is des-Xaa, Gly, Glu, Gla, Gln,
      Asp, Asn, Pro or hydroxy-Pro; Xaa at residue 7 is
      Ser, Thr, Gly, Glu, Gla, Asn, Trp (D or L),
      neo-Trp, halo-Trp, Arg, ornithine, homoarginine,

<220>
<221> SITE
<222> (7)
<223> Lys, N-methyl-Lys, N,N-dimethyl-Lys, N,N,N-
      trimethyl-Lys, any unnatural basic amino acid,
      Tyr, nor-Tyr, mono-halo-Tyr, di-halo-Tyr,
      O-sulpho-Tyr, O-phospho-Tyr, nitro-Tyr or any

<220>
<221> SITE
<222> (7)..(8)
<223> unnatural hydroxy containing amino acid; Xaa at
      residue 8 is Asp, Asn, His, halo-His, Thr, Ser,
      Tyr, nor-Tyr, mono-halo-Tyr, di-halo-Tyr,
      O-sulpho-Tyr, O-phospho-Tyr, nitro-Tyr or any

<220>
<221> SITE
<222> (8)..(10)
<223> unnatural hydroxy containing amino acid; Xaa at
      residue 9 is Pro or hydroxy-Pro; Xaa at residue
      10 is Ala, Ser, Thr, Asp, Val, Ile, Pro, hydroxy-
      Pro, Tyr, nor-Tyr, mono-halo-Tyr, di-halo-Tyr,

<220>
<221> SITE
<222> (10)..(12)
<223> O-sulpho-Tyr, O-phospho-Tyr, nitro-Tyr or any
      unnatural hydroxy containing amino acid; Xaa at
      residue 12 is Gly, Ile, Leu, Val, Ala, Thr, Ser,
      Pro, hydroxy-Pro, Phe, Trp (D or L), neo-Trp,

<220>
<221> SITE
<222> (12)..(13)
<223> halo-Trp, Arg, ornithine, homoarginine, Lys, N-
      methyl-Lys, N,N-dimethyl-Lys, N,N,N-trimethyl-
      Lys, any unnatural basic amino acid or any
      unnatural aromatic amino acid; Xaa at residue 13

<220>
<221> SITE
<222> (13)
<223> is Ala, Asn, Phe, Pro, hydroxy-Pro, Glu, Gla,
      Gln, His, halo-His, Val, Ser, Thr, Arg,
      ornithine, homoarginine, Lys, N-methyl-Lys, N,N-
      dimethyl-Lys, N,N,N-trimethyl-Lys or any

<220>
<221> SITE
<222> (13)..(14)
<223> unnatural basic amino acid; Xaa at residue 14 is
      Thr, Ser, His, halo-His, Leu, Ile, Val, Asn, Met,
      Pro, hydroxy-Pro, Arg, ornithine, homoarginine,
      Lys, N-methyl-Lys, N,N-dimethyl-Lys, N,N,N-

<220>
<221> SITE
<222> (14)..(15)
<223> trimethyl-Lys, any unnatural basic amino acid,
      Tyr, nor-Tyr, mono-halo-Tyr, di-halo-Tyr,

O-sulpho-Tyr, O-phospho-Tyr, nitro-Tyr or any
      unnatural hydroxy containing amino acid; Xaa at

<220>
<221> SITE
<222> (15)
<223> residue 15 is Asn, Pro, hydroxy-Pro, Gln, Ser,
      Thr, Arg, ornithine, homoarginine, Lys, N-methyl-
      Lys, N,N-dimethyl-Lys N,N,N-trimethyl-Lys, any
      unnatural basic amino acid, Tyr, nor-Tyr, mono-

<220>
<221> SITE
<222> (15)..(16)
<223> halo-Tyr, di-halo-Tyr, O-sulpho-Tyr, O-phospho-
      Tyr, nitro-Tyr or any unnatural hydroxy contain-
      ing amino acid; Xaa at residue 16 is des-Xaa,
      Gly, Thr, Ser, Pro, hydroxy-Pro, Tyr, nor-Tyr,

<220>
<221> SITE
<222> (16)..(17)
<223> mono-halo-Tyr, di-halo-Tyr, O-sulpho-Tyr,
      O-phospho-Tyr, nitro-Tyr or any unnatural hydroxy
      containing amino acid; Xaa at residue 17 is des-
      Xaa, Ile, Val, Asp, Leu, Phe, Arg, ornithine,

<220>
<221> SITE
<222> (17)
<223> homoarginine, Lys, N-methyl-Lys, N,N-dimethyl-
      Lys, N,N,N-trimethyl-Lys, any unnatural basic
      amino acid, Tyr, nor-Tyr, mono-halo-Tyr, di-halo-
      Tyr, O-sulpho-Tyr, O-phospho-Tyr, nitro-Tyr or

<220>
<221> SITE
<222> (17)..(19)
<223> any unnatural hydroxy containing amino acid; Xaa
      at residue 19 is des-Xaa, Gly, Ala, Met, Ser,
      Thr, Trp (D or L), neo-Trp, halo-Trp, any
      unnatural aromatic amino acid, Arg, ornithine,

<220>
<221> SITE
<222> (19)..(20)
<223> homoarginine, Lys, N-methyl-Lys, N,N-dimethyl-
      Lys, N,N,N-trimethyl-Lys or any unnatural basic
      amino acid; Xaa at residue 20 is des-Xaa, Trp
      (D or L), neo-Trp, halo-Trp, any unnatural

<220>
<221> SITE
<222> (20)..(21)
<223> aromatic amino acid, Arg, ornithine, homo-
      arginine, Lys, N-methyl-Lys, N,N-dimethyl-Lys,
      N,N,N-trimethyl-Lys or any unnatural basic amino
      acid; Xaa at residue 21 is des-Xaa, Arg,

<220>
<221> SITE
<222> (21)
<223> ornithine, homoarginine, Lys, N-methyl-Lys,
      N,N-dimethyl-Lys, N,N,N-trimethyl-Lys or any
      unnatural basic amino acid.

<400> 2
Xaa Xaa Xaa Xaa Cys Cys Xaa Xaa Xaa Xaa Cys Xaa Xaa Xaa Xaa Xaa
1               5                   10                  15

Xaa Cys Xaa Xaa Xaa
            20

<210> 3
<211> 28
<212> PRT
<213> Artificial Sequence

<220>
<223> Description of Artificial Sequence:Alpha-Conotoxin
      Peptide Generic Formula III.

<220>
<221> SITE
<222> (1)..(3)
<223> Xaa at residue 1 is des-Xaa, Ser or Thr; Xaa at
      residue 2 is des-Xaa, Asp, Glu, gamma-carboxy-Glu
      (Gla), Asn, Ser or Thr; Xaa at residue 3 is des-
      Xaa, Ala, Gly, Asn, Ser, Thr, Pro, hydroxy-Pro,

<220>
<221> SITE
<222> (3)..(4)
<223> Arg, ornithine, homoarginine, Lys, N-methyl-Lys,
      N,N-dimethyl-Lys, N,N,N-trimethyl-Lys or any
      unnatural basic amino acid; Xaa at residue 4 is
      des-Xaa, Ala, Val, Leu, Ile, Gly, Glu, Gla, Gln,

<220>
<221> SITE
<222> (4)..(5)
<223> Asp, Asn, Phe, Pro, hydroxy-Pro or any unnatural
      aromatic amino acid; Xaa at residue 5 is des-Xaa,
      Thr, Ser, Asp, Glu, Gla, Gln, Gly, Val, Asp, Asn,
      Ala, Pro, hydroxy-Pro, Arg, ornithine, homo-

<220>
<221> SITE
<222> (5)..(8)
<223> arginine, Lys, N-methyl-Lys, N,N-dimethyl-Lys,
      N,N,N-trimethyl-Lys or any unnatural basic amino
      acid; Xaa at residue 8 is Thr, Ser, Asp, Asn, Met,
      Val, Ala, Gly, Leu, Ile, Phe, any unnatural

<220>
<221> SITE
<222> (8)..(9)
<223> aromatic amino acid, Pro, hydroxy-Pro, Tyr, nor-
      Tyr, mono-halo-Tyr, di-halo-Tyr, O-sulpho-Tyr,
      O-phospho-Tyr, nitro-Tyr or any unnatural hydroxy
      containing amino acid; Xaa at residue 9 is  Ile,

<220>
<221> SITE
<222> (9)
<223> Leu, Val, Ser, Thr, Gln, Asn, Asp, Arg, His,
      halo-His, Phe,  any unnatural aromatic amino
      acid, homoarginine, ornithine, Lys, N-methyl-Lys,
      N,N-dimethyl-Lys, N,N,N-trimethyl-Lys, any

<220>
<221> SITE
<222> (9)..(10)
<223> unnatural basic amino acid, Tyr, nor-Tyr, mono-
      halo-Tyr, di-halo-Tyr, O-sulpho-Tyr, O-phospho-
      Tyr, nitro-Tyr or any unnatural hydroxy contain-
      ing amino acid; Xaa at residue 10 is Pro, hyroxy-

<220>
<221> SITE
<222> (10)
<223> Pro, Ser, Thr, Ile, Asp, Leu, Val, Gly, Ala, Phe,
      any unnatural aromatic amino acid, Arg, ornithine,
      homoarginine, Lys, N-methyl-Lys, N,N-dimethyl-Lys,
      N,N,N-trimethyl-Lys or any unnatural basic amino

<220>
<221> SITE
<222> (11)
<223> acid; Xaa at residue 11 is Val, Ala, Gly, Ile,
      Leu, Asp, Ser, Thr, Pro, hydroxy-Pro, Arg,
      ornithine, homoarginine, Lys, N-methyl-Lys,
      N,N-dimethyl-Lys, N,N,N-trimethyl-Lys or any

<220>
<221> SITE
<222> (11)..(13)
<223> unnatural basic amino acid; Xaa at residue 13 is
      His, halo-His, Arg, homoarginine, ornithine, Lys,
      N-methyl-Lys, N,N-dimethyl-Lys, N,N,N-trimethyl-
      Lys, any unnatural basic amino acid, Asn, Ala,

<220>
<221> SITE
<222> (13)
<223> Ser, Thr, Phe, Ile, Leu, Gly, Trp (D or L), neo-
      Trp, halo-Trp, any unnatural aromatic amino acid,
      Tyr, nor-Tyr, mono-halo-Tyr, di-halo-Tyr,
      O-sulpho-Tyr, O-phospho-Tyr, nitro-Tyr or any

<220>
<221> SITE
<222> (13)..(14)
<223> unnatural hydroxy containing amino acid; Xaa at
      residue 14 is Leu, Gln, Val, Ile, Gly, Met, Ala,
      Lys, N-methyl-Lys, N,N-dimethyl-Lys, N,N,N-tri-
      methyl-Lys, Ser, Thr, Arg, homoarginine, orni-

<220>
<221> SITE
<222> (14)..(15)
<223> thine, any unnatural basic amino acid, Asn, Glu,
      Gla, Gln, Phe, Trp (D or L), neo-Trp, halo-Trp or
      any unnatural aromatic amino acid; Xaa at residue
      15 is Glu, Gla, Gln, Asn, Asp, Pro, hydroxy-Pro,

<220>
<221> SITE
<222> (15)
<223> Ser, Gly, Thr, Lys, N-methyl-Lys, N,N-dimethyl-
      Lys, N,N,N-trimethyl-Lys, Arg, homoarginine,
      ornithine, any unnatural basic amino acid, Phe,
      His, halo-His, any unnatural aromatic acid, Leu,

<220>
<221> SITE
<222> (15)..(16)
<223> Met, Gly, Ala, Tyr, nor-Tyr, mono-halo-Tyr, di-
      halo-Tyr, O-sulpho-Tyr, O-phospho-Tyr, nitro-Tyr
      or any unnatural hydroxy containing amino acid;
      Xaa at residue 16 is His, halo-His, Asn, Thr,

<220>
<221> SITE
<222> (16)
<223> Ser, Ile, Val, Leu, Phe, any unnatural aromatic

amino acid, Arg, homoarginine, ornithine, Lys, N-
      methyl-Lys, N,N-dimethyl-Lys, N,N,N-trimethyl-Lys,
      any unnatural basic amino acid, Tyr, nor-Try,

<220>
<221> SITE
<222> (16)..(17)
<223> mono-halo-Tyr, di-halo-Tyr, O-sulpho-Tyr,
      O-phospho-Tyr, nitro-Tyr or any unnatural hydroxy
      containing amino acid; Xaa at residue 17 is Ser,
      Thr, Ala, Gln, Pro, hydroxy-Pro, Gly, Ile, Leu,

<220>
<221> SITE
<222> (17)..(18)
<223> Arg, ornithine, homoarginine, Lys, N-methyl-Lys,
      N,N-dimethyl-Lys, N,N,N-trimethyl-Lys or any
      unnatural basic amino acid; Xaa at residue 18 is
      Asn, Glu, Gla, Asp, Gly, His, halo-His, Ala, Leu,

<220>
<221> SITE
<222> (18)
<223> Gln, Arg,  ornithine, homoarginine, Lys, N-methyl-
      Lys, N,N-dimethyl-Lys, N,N,N-trimethyl-Lys, any
      unnatural basic amino acid, Tyr, nor-Tyr, mono-
      halo-Tyr, di-halo-Tyr, O-sulpho-Tyr, O-phospho-

<220>
<221> SITE
<222> (18)..(19)
<223> Tyr, nitro-Tyr or any unnatural hydroxy containing
      amino acid; Xaa at residue 19 is Met, Ile, Thr,
      Ser, Val, Leu, Pro, hydroxy-Pro, Phe, any
      unnatural aromatic amino acid, Tyr, nor-Tyr, mono-

<220>
<221> SITE
<222> (19)
<223> halo-Tyr, di-halo-Tyr, O-sulpho-Tyr, O-phospho-
      Tyr, nitro-Tyr, any unnatural hydroxy containing
      amino acid, Glu, Gla, Ala, His, halo-His, Arg,
      ornithine, homoarginine, Lys, N-methyl-Lys, N,N-

<220>
<221> SITE
<222> (21)
<223> dimethyl-Lys, N,N,N-trimethyl-Lys or any unnatural
      basic amino acid; Xaa at residue 21 is des-Xaa,
      Gly, Asp, Asn, Ala, Ile, Leu, Ser, Thr, His, halo-
      His, Arg, ornithine, homoarginine, Lys, N-methyl-

<220>
<221> SITE
<222> (21)..(22)
<223> Lys, N,N-dimethyl-Lys, N,N,N-trimethyl-Lys or any
      unnatural basic amino acid; Xaa at residue 22 is
      des-Xaa, Gly, Glu, Gla, Gln, Trp (D or L), neo-Trp,
      halo-Trp, any unnatural aromatic amino acid, Arg,

<220>
<221> SITE
<222> (22)..(23)
<223> ornithine, homoarginine, Lys, N-methyl-Lys, N,N-
      dimethyl-Lys, N,N,N-trimethyl-Lys or any unnatural
      basic amino acid; Xaa at residue 23 is des-Xaa,
      Ser, Thr, Val, Ile, Ala, Arg, ornithine, homo-

<220>

<221> SITE
<222> (23)..(24)
<223> arginine, Lys, N-methyl-Lys, N,N-dimethyl-Lys,
      N,N,N-trimethyl-Lys or any unnatural basic amino
      acid; Xaa at residue 24 is des-Xaa, Val, Asp, His,
      halo-His, Arg, ornithine, homoarginine, Lys, N-

<220>
<221> SITE
<222> (24)..(26)
<223> methyl-Lys, N,N-dimethyl-Lys, N,N,N-trimethyl-Lys
      or any unnatural basic amino acid; Xaa at residue
      25 is des-Xaa, Asn, Pro or hydroxy-Pro; Xaa at
      residue 26 is des-Xaa, Arg, ornithine, homo-

<220>
<221> SITE
<222> (26)..(28)
<223> arginine, Lys, N-methyl-Lys, N,N-dimethyl-Lys,
      N,N,N-trimethyl-Lys or any unnatural basic amino
      acid; Xaa at residue 27 is des-Xaa, Ser or Thr;
      Xaa at residue 28 is des-Xaa, Leu, Ile or Val.

<400> 3
Xaa Xaa Xaa Xaa Xaa Cys Cys Xaa Xaa Xaa Xaa Cys Xaa Xaa Xaa Xaa
1               5                   10                  15

Xaa Xaa Xaa Cys Xaa Xaa Xaa Xaa Xaa Xaa Xaa Xaa
            20                  25


<210> 4
<211> 14
<212> PRT
<213> Conus imperialis

<220>
<221> SITE
<222> (2)..(11)
<223> Xaa at residue 2 is Glu or gamma-carboxy-Glu; Xaa
      at residue 11 is Lys, N-methyl-Lys,
      N,N-dimethyl-Lys or N,N,N-trimethyl-Lys.

<400> 4
Asp Xaa Cys Cys Ser Asp Ser Arg Cys Gly Xaa Asn Cys Leu
1               5                   10


<210> 5
<211> 12
<212> PRT
<213> Conus imperialis

<220>
<221> SITE
<222> (10)
<223> Xaa at residue 10 is Trp (D or L) or halo-Trp.

<400> 5
Ala Cys Cys Ser Asp Arg Arg Cys Arg Xaa Arg Cys
1               5                   10


<210> 6
<211> 13
<212> PRT
<213> Conus regius

<400> 6

Phe Thr Cys Cys Arg Arg Gly Thr Cys Ser Gln His Cys
1               5                   10

<210> 7
<211> 13
<212> PRT
<213> Conus regius

<220>
<221> SITE
<222> (2)
<223> Xaa at residue 2 is Tyr, nor-Tyr, mono-halo-Tyr,
      di-halo-Tyr, O-sulpho-Tyr, O-phospho-Tyr or
      nitro-Tyr.

<400> 7
Asp Xaa Cys Cys Arg Arg His Ala Cys Thr Leu Ile Cys
1               5                   10

<210> 8
<211> 13
<212> PRT
<213> Conus regius

<220>
<221> SITE
<222> (2)..(8)
<223> Xaa at residue 2 is Tyr, nor-Tyr, mono-halo-Tyr,
      di-halo-Tyr, O-sulpho-Tyr, O-phospho-Tyr or
      nitro-Tyr; Xaa at residues 7 and 8 is Pro or
      hydroxy-Pro.

<400> 8
Asp Xaa Cys Cys Arg Arg Xaa Xaa Cys Thr Leu Ile Cys
1               5                   10

<210> 9
<211> 13
<212> PRT
<213> Conus regius

<220>
<221> SITE
<222> (6)..(10)
<223> Xaa at residue 6 is Pro or hdroxy-Pro; Xaa at
      residue 10 is Tyr, nor-Tyr, mono-halo-Tyr,
      di-halo-Tyr, O-sulpho-Tyr, O-phospho-Tyr or
      nitro-Tyr.

<400> 9
Gly Cys Cys Ser Asp Xaa Arg Cys Arg Xaa Arg Cys Arg
1               5                   10

<210> 10
<211> 13
<212> PRT
<213> Conus regius

<220>
<221> SITE
<222> (7)..(11)
<223> Xaa at residue 7 is Pro or hydroxy-Pro; Xaa at
      residue 11 is Trp (D or L) or halo-Trp.

<400> 10

Gly Gly Cys Cys Ser Asp Xaa Arg Cys Ala Xaa Arg Cys
1               5                   10

<210> 11
<211> 17
<212> PRT
<213> Conus regius

<220>
<221> SITE
<222> (3)..(10)
<223> Xaa at residue 3 is Trp (D or L) or halo-Trp; Xaa
      at residue 9 is Glu or gamma-carboxy-Glu; Xaa at
      residue 10 is Pro or hydroxy-Pro.

<220>
<221> SITE
<222> (15)
<223> Xaa at residue 15 is Lys, N-methyl-Lys,
      N,N-dimethyl-Lys or N,N,N-trimethyl-Lys.

<400> 11
Ile Ala Xaa Asp Ile Cys Cys Ser Xaa Xaa Asp Cys Asn His Xaa Cys
1               5                   10                  15

Val

<210> 12
<211> 12
<212> PRT
<213> Conus regius

<220>
<221> SITE
<222> (6)..(9)
<223> Xaa at residue 6 is Pro or hydroxy-Pro; Xaa at
      residue 9 is Lys, N-methyl-Lys, N,N-dimethyl-Lys
      or N,N,N-trimethyl-Lys.

<400> 12
Gly Cys Cys Ser Asp Xaa Arg Cys Xaa His Gln Cys
1               5                   10

<210> 13
<211> 14
<212> PRT
<213> Conus sponsalis

<220>
<221> SITE
<222> (5)..(11)
<223> Xaa at residues 5 and 11 is Pro or hydroxy-Pro;
      Xaa at residue 8 is Lys, N-methyl-Lys,
      N.N-dimethyl-Lys or N.N.n-trimethyl-Lys.

<400> 13
Cys Cys Ser Asp Xaa Ala Cys Xaa Gln Thr Xaa Gly Cys Arg
1               5                   10

<210> 14
<211> 13
<212> PRT
<213> Conus sponsalis

<220>
<221> SITE
<222> (3)..(5)
<223> Xaa at residue 3 is Glu or gamma-carboxy-Glu; Xaa
      at residue 5 is Pro or hydroxy-Pro.

<400> 14
Cys Cys Xaa Asn Xaa Ala Cys Arg His Thr Gln Gly Cys
  1               5                   10


<210> 15
<211> 13
<212> PRT
<213> Conus sulcatus

<220>
<221> SITE
<222> (4)..(12)
<223> Xaa at residue 4 is Trp or halo-Trp; Xaa at
      residue 6 is Pro or hydroxy-Pro; Xaa at residue 12
      is Tyr, nor-Tyr, mono-halo-Tyr, di-halo-Tyr,
      O-sulpho-Tyr, O-phospho-Tyr or nitro-Tyr.

<400> 15
Gly Cys Cys Xaa His Xaa Ala Cys Gly Arg His Xaa Cys
  1               5                   10


<210> 16
<211> 14
<212> PRT
<213> Conus achatinus

<220>
<221> SITE
<222> (2)..(11)
<223> Xaa at residues 2 and 7 is Pro or hydroxy-Pro; Xaa
      at residue 11 is Lys, N-methyl-Lys,
      N,N-dimethyl-Lys or N,N,N-trimethyl-Lys.

<400> 16
Ala Xaa Cys Cys Asn Asn Xaa Ala Cys Val Xaa His Arg Cys
  1               5                   10


<210> 17
<211> 15
<212> PRT
<213> Conus bullatus

<220>
<221> SITE
<222> (2)..(12)
<223> Xaa at residues 2 and 8 is Pro or hydroxy-Pro; Xaa
      at residue 12 is Lys, N-methyl-Lys,
      N,N-dimethyl-Lys or N,N,N-trimethyl-Lys.

<400> 17
Ala Xaa Gly Cys Cys Asn Asn Xaa Ala Cys Val Xaa His Arg Cys
  1               5                   10                  15


<210> 18
<211> 14
<212> PRT
<213> Conus bullatus

<220>

<221> SITE
<222> (1)..(11)
<223> Xaa at residues 1, 2 and 7 is Pro or hydroxy-Pro;
      Xaa at residue 11 is Lys, N-methyl-Lys,
      N,N-dimethyl-Lys or N,N,N-trimethyl-Lys.

<400> 18
Xaa Xaa Cys Cys Asn Asn Xaa Ala Cys Val Xaa His Arg Cys
1               5                   10


<210> 19
<211> 16
<212> PRT
<213> Conus bullatus

<220>
<221> SITE
<222> (2)..(13)
<223> Xaa at residue 2 is Glu or gamma-carboxy-Glu; Xaa
      at residue 6 is Trp or halo-Trp; Xaa at residues 8
      11 and 13 is Pro or hydroxy-Pro.

<400> 19
Asp Xaa Asn Cys Cys Xaa Asn Xaa Ser Cys Xaa Arg Xaa Arg Cys Thr
1               5                   10                  15


<210> 20
<211> 13
<212> PRT
<213> Conus bullatus

<220>
<221> SITE
<222> (6)..(12)
<223> Xaa at residues 6 and 7 is Pro or hydroxy-Pro; Xaa
      at residue 12 is Tyr, nor-Tyr, mono-halo-Tyr,
      di-halo-Tyr, O-sulpho-Tyr, O-phospho-Tyr or
      nitro-Tyr.

<400> 20
Gly Cys Cys Ser Arg Xaa Xaa Cys Ala Val Leu Xaa Cys
1               5                   10


<210> 21
<211> 13
<212> PRT
<213> Conus circumcisus

<220>
<221> SITE
<222> (6)
<223> Xaa at residue 6 is Pro or hydroxy-Pro.

<400> 21
Gly Cys Cys Gly Asn Xaa Asp Cys Thr Ser His Ser Cys
1               5                   10


<210> 22
<211> 16
<212> PRT
<213> Conus stercusmuscarum

<220>
<221> SITE
<222> (6)..(11)

<223> Xaa at residue 6 is Pro or hydroxy-Pro; Xaa at
      residue 11 is Glu or gamma-carboxy-Glu.

<400> 22
Gly Cys Cys Ser Asn Xaa Val Cys His Leu Xaa His Ser Asn Met Cys
1               5                   10                  15


<210> 23
<211> 17
<212> PRT
<213> Conus obscurus

<220>
<221> SITE
<222> (6)..(15)
<223> Xaa at residue 6 is Pro or hydroxy-Pro; Xaa at
      residue 14 is Glu or gamma-carboxy-Glu; Xaa at
      residue 15 is Tyr, nor-Tyr, mono-halo-Tyr,
      di-halo-Tyr, O-sulpho-Tyr, O-phospho-Tyr or

<220>
<221> SITE
<222> (15)
<223> nitro-Tyr.

<400> 23
Gly Cys Cys Ser Asn Xaa Val Cys Arg Gln Asn Asn Ala Xaa Xaa Cys
1               5                   10                  15

Arg


<210> 24
<211> 18
<212> PRT
<213> Conus textile

<220>
<221> SITE
<222> (1)..(15)
<223> Xaa at residues 1, 7 and 14 is Pro or hydroxy-Pro;
      Xaa at residue 15 is Glu or gamma-carboxy-Glu.

<400> 24
Xaa Gln Cys Cys Ser His Xaa Ala Cys Asn Val Asp His Xaa Xaa Ile
1               5                   10                  15

Cys Arg


<210> 25
<211> 18
<212> PRT
<213> Conus radiatus

<220>
<221> SITE
<222> (1)..(15)
<223> Xaa at residues 1, 7 and 14 is Pro or hydroxy-Pro;
      Xaa at residues 2 and 15 is Glu or
      gamma-carboxy-Glu.

<400> 25
Xaa Xaa Cys Cys Ser His Xaa Ala Cys Asn Val Asp His Xaa Xaa Ile
1               5                   10                  15

Cys Arg

<210> 26
<211> 18
<212> PRT
<213> Conus radiatus

<220>
<221> SITE
<222> (1)..(15)
<223> Xaa at residues 1, 7 and 14 is Pro or hydroxy-Pro;
      Xaa at residue 15 is Glu or gamma-carboxy-Glu.

<400> 26
Xaa Gln Cys Cys Ser His Xaa Ala Cys Asn Val Asp His Xaa Xaa Ile
1               5                   10                  15

Cys Asp

<210> 27
<211> 18
<212> PRT
<213> Conus omaria

<220>
<221> SITE
<222> (1)..(15)
<223> Xaa at residues 1, 7 and 14 is Pro or hydroxy-Pro;
      Xaa at residue 15 is Glu or gamma-carboxy-Glu.

<400> 27
Xaa Arg Cys Cys Ser His Xaa Ala Cys Asn Val Asp His Xaa Xaa Ile
1               5                   10                  15

Cys Arg

<210> 28
<211> 18
<212> PRT
<213> Conus omaria

<220>
<221> SITE
<222> (1)..(14)
<223> Xaa at residues 1, 7 and 14 is Pro or hydroxy-Pro.

<400> 28
Xaa Gln Cys Cys Ser His Xaa Ala Cys Asn Val Asp His Xaa Gly Ile
1               5                   10                  15

Cys Arg

<210> 29
<211> 18
<212> PRT
<213> Conus omaria

<220>
<221> SITE
<222> (1)..(15)
<223> Xaa at residues 1, 7 and 14 is Pro or hydroxy-Pro;

Xaa at residue 15 is Glu or gamma-carboxy-Glu.

<400> 29
Xaa Gln Cys Cys Ser His Xaa Ala Cys Asn Val Asp His Xaa Xaa Thr
1               5                   10                  15

Cys Arg

<210> 30
<211> 18
<212> PRT
<213> Conus omaria

<220>
<221> SITE
<222> (1)..(15)
<223> Xaa at residues 1, 7 and 14 is Pro or hydroxy-Pro;
      Xaa at residue 15 is Glu or gamma-carboxy-Glu.

<400> 30
Xaa Gln Cys Cys Ser His Xaa Ala Cys Asn Val Asp His Xaa Xaa Val
1               5                   10                  15

Cys Arg

<210> 31
<211> 18
<212> PRT
<213> Conus omaria

<220>
<221> SITE
<222> (1)..(15)
<223> Xaa at residues 1, 7 and 14 is Pro or hydroxy-Pro;
      Xaa at residue 15 is Glu or gamma-carboxy-Glu.

<400> 31
Xaa Gln Cys Cys Ser His Xaa Ala Cys Asn Ile Asp His Xaa Xaa Ile
1               5                   10                  15

Cys Arg

<210> 32
<211> 21
<212> PRT
<213> Conus omaria

<220>
<221> SITE
<222> (1)..(15)
<223> Xaa at residues 1, 7 and 14 is Pro or hydroxy-Pro;
      Xaa at residue 15 is Glu or gamma-carboxy-Glu.

<400> 32
Xaa Gln Cys Cys Ser His Xaa Ala Cys Asn Val Asp His Xaa Xaa Ile
1               5                   10                  15

Cys Arg Arg Arg Arg
            20

<210> 33
<211> 17

<212> PRT
<213> Conus betulinus

<220>
<221> SITE
<222> (7)..(15)
<223> Xaa at residues 7 and 14 is Pro or hydroxy-Pro;
      Xaa at residue 15 is Glu or gamma-carboxy-Glu.

<400> 33
Gly Gly Cys Cys Ser His Xaa Ala Cys Ala Val Asn His Xaa Xaa Leu
1               5                   10                  15

Cys




<210> 34
<211> 16
<212> PRT
<213> Conus betulinus

<220>
<221> SITE
<222> (6)..(14)
<223> Xaa at residues 6 and 13 is Pro or hydroxy-Pro;
      Xaa at residue 14 is Glu or gamma-carboxy-Glu.

<400> 34
Gly Cys Cys Ser His Xaa Ala Cys Ser Val Asn His Xaa Xaa Leu Cys
1               5                   10                  15


<210> 35
<211> 16
<212> PRT
<213> Conus dalli

<220>
<221> SITE
<222> (6)..(14)
<223> Xaa at residues 6 and 13 is Pro or hydroxy-Pro;
      Xaa at residue 14 is Glu or gamma-carboxy-Glu.

<400> 35
Gly Cys Cys Ser His Xaa Ala Cys Asn Val Asp His Xaa Xaa Ile Cys
1               5                   10                  15


<210> 36
<211> 19
<212> PRT
<213> Conus obscurus

<220>
<221> SITE
<222> (6)..(18)
<223> Xaa at residues 6 and 15 is Pro or hydroxy-Pro;
      Xaa at reside 11 is Lys, N,-methyl-Lys,
      N,N-dimethyl-Lys or N,N,N-trimethyl-Lys; Xaa at
      residues 14 and 18 is Glu or gamma-carboxy-Glu.

<400> 36
Gly Cys Cys Ser His Xaa Ala Cys Ser Gly Xaa Thr Gln Xaa Xaa Cys
1               5                   10                  15

Arg Xaa Ser

<210> 37
<211> 18
<212> PRT
<213> Conus tulipa

<220>
<221> SITE
<222> (1)..(14)
<223> Xaa at residues 1, 6 and 13 is Pro or hydroxy-Pro;
      Xaa at residue 14 is Glu or gamma-carboxy-Glu.

<400> 37
Xaa Cys Cys Ser His Xaa Ala Cys Ser Gly Asn Asn Xaa Xaa Phe Cys
  1               5                   10                  15

Arg Gln



<210> 38
<211> 18
<212> PRT
<213> Conus tulipa

<220>
<221> SITE
<222> (6)..(14)
<223> Xaa at residues 6 and 13 is Pro or hydroxy-Pro;
      Xaa at residue 14 is Glu or gamma-carboxy-Glu.

<400> 38
Gly Cys Cys Ser His Xaa Ala Cys Ser Gly Asn Asn Xaa Xaa Phe Cys
  1               5                   10                  15

Arg Gln



<210> 39
<211> 16
<212> PRT
<213> Conus pennaceus

<220>
<221> SITE
<222> (6)..(15)
<223> Xaa at residues 6, 7 and 13 is Pro or hydroxy-Pro;
      Xaa at residue 15 is Tyr, nor-Tyr, mono-halo-Tyr,
      di-halo-Tyr, O-sulpho-Tyr, O-phospho-Tyr or
      nitro-Tyr.

<400> 39
Gly Cys Cys Ser His Xaa Xaa Cys Ala Met Asn Asn Xaa Asp Xaa Cys
  1               5                   10                  15


<210> 40
<211> 16
<212> PRT
<213> Conus pennaceus

<220>
<221> SITE
<222> (6)..(15)
<223> Xaa at residuew 6, 7 and 13 is Pro or hydroxy-Pro;
      Xaa at residue 15 is Tyr, nor-Tyr, mono-halo-Tyr,
      di-halo-Tyr, O-sulpho-Tyr, O-phospho-Tyr or
      nitro-Tyr.

<400> 40
Gly Cys Cys Ser His Xaa Xaa Cys Phe Leu Asn Asn Xaa Asp Xaa Cys
1               5                   10                  15

<210> 41
<211> 17
<212> PRT
<213> Conus textile

<220>
<221> SITE
<222> (6)..(13)
<223> Xaa at residues 6, 7 and 13 is Pro or hydroxy-Pro;
      Xaa at residue 11 is Lys, N-methyl-Lys,
      N,N-dimethyl-Lys or N.N.N-trimethyl-Lys.

<400> 41
Gly Cys Cys Ser Asn Xaa Xaa Cys Ile Ala Xaa Asn Xaa His Met Cys
1               5                   10                  15

Gly

<210> 42
<211> 16
<212> PRT
<213> Conus distans

<220>
<221> SITE
<222> (6)..(13)
<223> Xaa at residues 6, 7 and 13 is Pro or hydroxy-Pro.

<400> 42
Gly Cys Cys Ser Asn Xaa Xaa Cys Ala His Asn Asn Xaa Asp Cys Arg
1               5                   10                  15

<210> 43
<211> 17
<212> PRT
<213> Conus tulipa

<220>
<221> SITE
<222> (6)..(13)
<223> Xaa at residues 6 and 13 is Pro or hydroxy-Pro.

<400> 43
Gly Cys Cys Ser Asn Xaa Ala Cys Ala Gly Asn Asn Xaa His Val Cys
1               5                   10                  15

Arg

<210> 44
<211> 16
<212> PRT
<213> Conus dalli

<220>
<221> SITE
<222> (6)..(13)
<223> Xaa at residues 6 and 13 is Pro or hydroxy-Pro.

<400> 44
Gly Cys Cys Ser Arg Xaa Ala Cys Ile Ala Asn Asn Xaa Asp Leu Cys
1               5                   10                  15

<210> 45
<211> 20
<212> PRT
<213> Conus circumcisus

<220>
<221> SITE
<222> (6)..(14)
<223> Xaa at residues 6 and 13 is Pro or hydroxy-Pro;
      Xaa at residues 11 and 14 is Glu or
      gamma-carboxy-Glu.

<400> 45
Gly Cys Cys Ser Asn Xaa Val Cys His Val Xaa His Xaa Xaa Leu Cys
1               5                   10                  15

Arg Arg Arg Arg
            20

<210> 46
<211> 18
<212> PRT
<213> Conus sulcatus

<220>
<221> SITE
<222> (7)..(15)
<223> Xaa at residues 7, 12 and 14 is Pro or
      hydroxy-Pro; Xaa at residue 11 is Lys,
      N-methyl-Lys, N,N-dimethyl-Lys or
      N,N,N-trimethyl-Lys; Xaa at residue 15 is Glu or

<220>
<221> SITE
<222> (7)..(15)
<223> gamma-carboxy-Glu.

<400> 46
Gly Gly Cys Cys Ser Phe Xaa Ala Cys Arg Xaa Xaa Arg Xaa Xaa Met
1               5                   10                  15

Cys Gly

<210> 47
<211> 18
<212> PRT
<213> Conus textile

<220>
<221> SITE
<222> (1)..(15)
<223> Xaa at residues 1, 7 and 14 is Pro or hydroxy-Pro;
      Xaa at residues 2 and 15 is Glu or
      gamma-carboxy-Glu.

<400> 47
Xaa Xaa Cys Cys Ser Asp Xaa Arg Cys Asn Ser Ser His Xaa Xaa Leu
1               5                   10                  15

Cys Arg

<210> 48
<211> 18
<212> PRT
<213> Conus dalli

<220>
<221> SITE
<222> (1)..(15)
<223> Xaa at residues 1, 7 and 14 is Pro or hydroxy-pro;
      Xaa at residue 15 is Glu or gamma-carboxy-Glu.

<400> 48
Xaa Gln Cys Cys Ser Asp Xaa Arg Cys Asn Val Gly His Xaa Xaa Leu
1               5                   10                  15

Cys Gly



<210> 49
<211> 18
<212> PRT
<213> Conus dalli

<220>
<221> SITE
<222> (1)..(15)
<223> Xaa at residue 1 is Gln or pyro-Glu; Xaa at
      residues 7 and 14 is Pro or hydroxy-Pro; Xaa at
      residue 15 is Glu or gamma-carboxy-Glu.

<400> 49
Xaa Val Cys Cys Ser Asp Xaa Arg Cys Asn Val Gly His Xaa Xaa Ile
1               5                   10                  15

Cys Gly



<210> 50
<211> 16
<212> PRT
<213> Conus textile

<220>
<221> SITE
<222> (6)..(13)
<223> Xaa at residues 6, 7 and 13 is Pro or hydroxy-Pro.

<400> 50
Gly Cys Cys Ser Arg Xaa Xaa Cys Ile Ala Asn Asn Xaa Asp Leu Cys
1               5                   10                  15


<210> 51
<211> 18
<212> PRT
<213> Conus omaria

<220>
<221> SITE
<222> (1)..(15)
<223> Xaa at residues 1 and 14 is Pro or hydroxy-Pro;
      Xaa at residue 15 is Glu or gamma-carboxy-Glu.

<400> 51
Xaa Gln Cys Cys Ser His Leu Ala Cys Asn Val Asp His Xaa Xaa Ile

1                   5                   10                  15

Cys Arg




<210> 52
<211> 19
<212> PRT
<213> Conus sulcatus

<220>
<221> SITE
<222> (5)..(14)
<223> Xaa at residue 5 is Tyr, nor-Tyr, mono-halo-Tyr,
      di-halo-Tyr, O-sulpho-Tyr, O-phospho-Tyr or
      nitro-Tyr; Xaa at residue 13 is Pro or
      hydroxy-Pro; Xaa at residue 14 is Glu or



<220>
<221> SITE
<222> (14)..(18)
<223> gamma-carboxy-Glu; Xaa at residue 18 is Trp or
      halo-Trp.

<400> 52
Gly Cys Cys Ser Xaa Phe Asp Cys Arg Met Met Phe Xaa Xaa Met Cys
1                   5                   10                  15

Gly Xaa Arg




<210> 53
<211> 18
<212> PRT
<213> Conus sulcatus

<220>
<221> SITE
<222> (11)..(12)
<223> Xaa at residue 11 is Lys, N-methyl-Lys,
      N,N-dimethyl-Lys or N,N,N-trimethyl-Lys; Xaa at
      residue 12 is Tyr, nor-Tyr, mono-halo-Tyr,
      di-halo-Tyr, O-sulpho-Tyr, O-phospho-Tyr or

<220>
<221> SITE
<222> (12)..(15)
<223> nitro-Tyr; Xaa at residue 14 is Pro or hydroxy-
      Pro; Xaa at residue 15 is Glu or gamma-carboxy-
      Glu.

<400> 53
Gly Gly Cys Cys Ser Phe Ala Ala Cys Arg Xaa Xaa Arg Xaa Xaa Met
1                   5                   10                  15

Cys Gly




<210> 54
<211> 20
<212> PRT
<213> Conus sulcatus

<220>
<221> SITE

<222> (7)..(15)
<223> Xaa at residue 7 is Pro or hydroxy-Pro; Xaa at
      residue 10 is Tyr, nor-Tyr, mono-halo-Tyr,
      di-halo-Tyr, O-sulpho-Tyr, O-phospho-Tyr or
      nitro-Tyr; Xaa at residue 15 is Glu or

<220>
<221> SITE
<222> (7)..(15)
<223> gamma-carboxy-Glu.

<400> 54
Gly Gly Cys Cys Phe His Xaa Val Cys Xaa Ile Asn Leu Leu Xaa Met
  1               5                  10                  15

Cys Arg Gln Arg
            20


<210> 55
<211> 19
<212> PRT
<213> Conus betulinus

<220>
<221> SITE
<222> (7)..(15)
<223> Xaa at residues 7, 11 and 14 is Tyr, nor-Tyr,
      mono-halo-Tyr, di-halo-Tyr, O-sulpho-Tyr,
      O-phospho-Tyr; Xaa at residues 8, 9 and 15 is Pro
      or hydroxy-Pro.

<220>
<221> SITE
<222> (12)..(16)
<223> Xaa at residues 12 and 16 is Glu or
      gamma-carboxy-Glu.

<400> 55
Ser Ala Thr Cys Cys Asn Xaa Xaa Xaa Cys Xaa Xaa Thr Xaa Xaa Xaa
  1               5                  10                  15

Ser Cys Leu



<210> 56
<211> 17
<212> PRT
<213> Conus betulinus

<220>
<221> SITE
<222> (5)..(13)
<223> Xaa at residues 5 and 12 is Tyr, no-Tyr,
      mono-halo-Tyr, di-halo-Tyr, O-sulpho-Tyr,
      O-phospho-Tyr or nitro-Tyr; Xaa at residues 6, 7
      and 13 is Pro or hydroxy-Pro.

<220>
<221> SITE
<222> (10)..(14)
<223> Xaa at residues 10 and 14 is Glu or
      gamma-carboxy-Glu.

<400> 56
Ala Cys Cys Ala Xaa Xaa Xaa Cys Phe Xaa Ala Xaa Xaa Xaa Arg Cys
  1               5                  10                  15

Leu

<210> 57
<211> 19
<212> PRT
<213> Conus betulinus

<220>
<221> SITE
<222> (3)..(16)
<223> Xaa at residues 3, 12 and 16 is Glu or
      gamma-carboxy-Glu; Xaa at residues 6, 7, 11 and 14
      is Tyr, nor-Tyr, mono-halo-Tyr, di-halo-Tyr,
      O-sulpho-Tyr, O-phospho-Tyr or nitro-Tyr.

<220>
<221> SITE
<222> (8)..(15)
<223> Xaa at residues 8, 9 and 15 is Pro or hydroxy-Pro.

<400> 57
Asn Ala Xaa Cys Cys Xaa Xaa Xaa Xaa Cys Xaa Xaa Ala Xaa Xaa Xaa
1               5                   10                  15

Ile Cys Leu

<210> 58
<211> 227
<212> DNA
<213> Conus magus

<220>
<221> CDS
<222> (1)..(189)

<400> 58
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca gat cgt gca tct gat ggc agg aat gcc gca gcc aac gac     96
Phe Pro Ser Asp Arg Ala Ser Asp Gly Arg Asn Ala Ala Ala Asn Asp
            20                  25                  30

aaa gcg tct gac gtg atc acg ctg gcc ctc aag gga tgc tgt tcc aac     144
Lys Ala Ser Asp Val Ile Thr Leu Ala Leu Lys Gly Cys Cys Ser Asn
        35                  40                  45

cct gtc tgt cac ttg gag cat tca aac ctt tgt ggt aga aga cgc         189
Pro Val Cys His Leu Glu His Ser Asn Leu Cys Gly Arg Arg Arg
    50                  55                  60

tgatgctcca ggaccctctg aaccacgacg ttcgagca                           227

<210> 59
<211> 63
<212> PRT
<213> Conus magus

<400> 59
Met Phe Thr Val Phe Leu Leu Val Val Leu Ala Thr Thr Val Val Ser
1               5                   10                  15

Phe Pro Ser Asp Arg Ala Ser Asp Gly Arg Asn Ala Ala Ala Asn Asp

                20                  25                  30

Lys Ala Ser Asp Val Ile Thr Leu Ala Leu Lys Gly Cys Cys Ser Asn
            35                  40                  45

Pro Val Cys His Leu Glu His Ser Asn Leu Cys Gly Arg Arg Arg
    50                  55                  60


<210> 60
<211> 208
<212> DNA
<213> Conus aulicus

<220>
<221> CDS
<222> (1)..(168)

<400> 60
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc act tca gat cgt gca tct gat ggc agg aag gac gca gcg tct ggc      96
Phe Thr Ser Asp Arg Ala Ser Asp Gly Arg Lys Asp Ala Ala Ser Gly
            20                  25                  30

ctg atc gct ctg acc atc aag gga tgc tgt tct tat cct ccc tgt ttc      144
Leu Ile Ala Leu Thr Ile Lys Gly Cys Cys Ser Tyr Pro Pro Cys Phe
        35                  40                  45

gcg act aat tca gac tat tgt ggt tgacgacgct gatgctccag gaccctctga     198
Ala Thr Asn Ser Asp Tyr Cys Gly
    50                  55

accacgacgt                                                           208


<210> 61
<211> 56
<212> PRT
<213> Conus aulicus

<400> 61
Met Phe Thr Val Phe Leu Leu Val Val Leu Ala Thr Thr Val Val Ser
1               5                   10                  15

Phe Thr Ser Asp Arg Ala Ser Asp Gly Arg Lys Asp Ala Ala Ser Gly
            20                  25                  30

Leu Ile Ala Leu Thr Ile Lys Gly Cys Cys Ser Tyr Pro Pro Cys Phe
        35                  40                  45

Ala Thr Asn Ser Asp Tyr Cys Gly
    50                  55


<210> 62
<211> 205
<212> DNA
<213> Conus aulicus

<220>
<221> CDS
<222> (1)..(174)

<400> 62
atg ttc acc gtg ttt ctg ttg gtc gtc ttg gca acc acc gtc gtt tcc      48
Met Phe Thr Val Phe Leu Leu Val Val Leu Ala Thr Thr Val Val Ser

1                 5                   10                  15

ttc act tca gat cgt gca tct gat ggc agg aag gac gca gcg tct ggc     96
Phe Thr Ser Asp Arg Ala Ser Asp Gly Arg Lys Asp Ala Ala Ser Gly
            20                  25                  30

ctg att gct ctg acc atg aag gga tgc tgt tct tat cct ccc tgt ttc     144
Leu Ile Ala Leu Thr Met Lys Gly Cys Cys Ser Tyr Pro Pro Cys Phe
        35                  40                  45

gcg act aat cca gac tgt ggt cga cga cgc tgatgctcca ggaccctctg      194
Ala Thr Asn Pro Asp Cys Gly Arg Arg Arg
    50                  55

aaccacgacg t                                                        205


<210> 63
<211> 58
<212> PRT
<213> Conus aulicus

<400> 63
Met Phe Thr Val Phe Leu Leu Val Val Leu Ala Thr Thr Val Val Ser
1               5                   10                  15

Phe Thr Ser Asp Arg Ala Ser Asp Gly Arg Lys Asp Ala Ala Ser Gly
            20                  25                  30

Leu Ile Ala Leu Thr Met Lys Gly Cys Cys Ser Tyr Pro Pro Cys Phe
        35                  40                  45

Ala Thr Asn Pro Asp Cys Gly Arg Arg Arg
    50                  55


<210> 64
<211> 223
<212> DNA
<213> Conus textile

<220>
<221> CDS
<222> (1)..(192)

<400> 64
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc tct tca ggt cgt agt aca ttt cgt ggc agg aat gcc gca gcc aaa     96
Phe Ser Ser Gly Arg Ser Thr Phe Arg Gly Arg Asn Ala Ala Ala Lys
            20                  25                  30

gcg tct ggc ctg gtc agt ctg act gac agg aga cca gaa tgc tgt agt     144
Ala Ser Gly Leu Val Ser Leu Thr Asp Arg Arg Pro Glu Cys Cys Ser
        35                  40                  45

gat cct cgc tgt aac tcg agt cat cca gaa ctt tgt ggt gga aga cgc     192
Asp Pro Arg Cys Asn Ser Ser His Pro Glu Leu Cys Gly Gly Arg Arg
    50                  55                  60

tgatgctcca ggaccctctg aaccacgacg t                                  223


<210> 65
<211> 64
<212> PRT
<213> Conus textile

<400> 65
Met Phe Thr Val Phe Leu Leu Val Val Leu Ala Thr Thr Val Val Ser
1               5                   10                  15

Phe Ser Ser Gly Arg Ser Thr Phe Arg Gly Arg Asn Ala Ala Ala Lys
            20                  25                  30

Ala Ser Gly Leu Val Ser Leu Thr Asp Arg Arg Pro Glu Cys Cys Ser
        35                  40                  45

Asp Pro Arg Cys Asn Ser Ser His Pro Glu Leu Cys Gly Gly Arg Arg
    50                  55                  60


<210> 66
<211> 244
<212> DNA
<213> Conus textile

<220>
<221> CDS
<222> (1)..(168)

<400> 66
atg ttc acc gtg ttt ctg ttg gtt gtc ttg gca acc gcc gtc gtt tcc      48
Met Phe Thr Val Phe Leu Leu Val Val Leu Ala Thr Ala Val Val Ser
1               5                   10                  15

ttc act tca gat cgt gca tct gat gac ggg aaa gcc gct gcg tct gac      96
Phe Thr Ser Asp Arg Ala Ser Asp Asp Gly Lys Ala Ala Ala Ser Asp
            20                  25                  30

ctg atc act ctg acc atc aag gga tgc tgt tct cgt cct ccc tgt atc      144
Leu Ile Thr Leu Thr Ile Lys Gly Cys Cys Ser Arg Pro Pro Cys Ile
        35                  40                  45

gcg aat aat cca gac ttg tgt ggt tgacgacgct gatgctccag aacggtctga     198
Ala Asn Asn Pro Asp Leu Cys Gly
    50                  55

accacgacgt tcgagcaatg ttcaccgtgt ttctgttggt tgtctt                   244


<210> 67
<211> 56
<212> PRT
<213> Conus textile

<400> 67
Met Phe Thr Val Phe Leu Leu Val Val Leu Ala Thr Ala Val Val Ser
1               5                   10                  15

Phe Thr Ser Asp Arg Ala Ser Asp Asp Gly Lys Ala Ala Ala Ser Asp
            20                  25                  30

Leu Ile Thr Leu Thr Ile Lys Gly Cys Cys Ser Arg Pro Pro Cys Ile
        35                  40                  45

Ala Asn Asn Pro Asp Leu Cys Gly
    50                  55


<210> 68
<211> 223
<212> DNA
<213> Conus textile

<220>

<221> CDS
<222> (1)..(183)

<400> 68
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca ggt cgt agt aca ttt cgt ggc agg aat gcc gca gcc aaa     96
Phe Thr Ser Gly Arg Ser Thr Phe Arg Gly Arg Asn Ala Ala Ala Lys
            20                  25                  30

gcg tct ggc ctg gtc agt ctg act gac agg aga cca caa tgc tgt tct     144
Ala Ser Gly Leu Val Ser Leu Thr Asp Arg Arg Pro Gln Cys Cys Ser
        35                  40                  45

cat cct gcc tgt aac gta gat cat cca gaa att tgt cgt tgaagacgct     193
His Pro Ala Cys Asn Val Asp His Pro Glu Ile Cys Arg
    50                  55                  60

gatgctccag gaccctctga accacgacgt                                   223


<210> 69
<211> 61
<212> PRT
<213> Conus textile

<400> 69
Met Phe Thr Val Phe Leu Leu Val Val Leu Ala Thr Thr Val Val Ser
1               5                   10                  15

Phe Thr Ser Gly Arg Ser Thr Phe Arg Gly Arg Asn Ala Ala Ala Lys
            20                  25                  30

Ala Ser Gly Leu Val Ser Leu Thr Asp Arg Arg Pro Gln Cys Cys Ser
        35                  40                  45

His Pro Ala Cys Asn Val Asp His Pro Glu Ile Cys Arg
    50                  55                  60


<210> 70
<211> 223
<212> DNA
<213> Conus radiatus

<220>
<221> CDS
<222> (1)..(183)

<400> 70
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca ggt cgt cgt aca ttt cat ggc agg aat gcc gca gcc aaa     96
Phe Thr Ser Gly Arg Arg Thr Phe His Gly Arg Asn Ala Ala Ala Lys
            20                  25                  30

gcg tct ggc ctg gtc agt ctg act gac agg aga cca gaa tgc tgt tct     144
Ala Ser Gly Leu Val Ser Leu Thr Asp Arg Arg Pro Glu Cys Cys Ser
        35                  40                  45

cat cct gcc tgt aac gta gat cat cca gaa att tgt cgt tgaagacgct     193
His Pro Ala Cys Asn Val Asp His Pro Glu Ile Cys Arg
    50                  55                  60

gatgctccag gaccctctga accacgacgt                                   223

<210> 71
<211> 61
<212> PRT
<213> Conus radiatus

<400> 71
Met Phe Thr Val Phe Leu Leu Val Val Leu Ala Thr Thr Val Val Ser
1               5                   10                  15

Phe Thr Ser Gly Arg Arg Thr Phe His Gly Arg Asn Ala Ala Ala Lys
            20                  25                  30

Ala Ser Gly Leu Val Ser Leu Thr Asp Arg Arg Pro Glu Cys Cys Ser
        35                  40                  45

His Pro Ala Cys Asn Val Asp His Pro Glu Ile Cys Arg
    50                  55                  60


<210> 72
<211> 223
<212> DNA
<213> Conus radiatus

<220>
<221> CDS
<222> (1)..(183)

<400> 72
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca ggt cgt agt aca ttt cgt ggc agg aat gcc gca gcc aaa     96
Phe Thr Ser Gly Arg Ser Thr Phe Arg Gly Arg Asn Ala Ala Ala Lys
            20                  25                  30

gcg tct ggc ctg gtc agt ctg act gac agg aga cca caa tgc tgt tct     144
Ala Ser Gly Leu Val Ser Leu Thr Asp Arg Arg Pro Gln Cys Cys Ser
        35                  40                  45

cat cct gcc tgt aac gta gat cat cca gaa att tgc gat tgaagacgct      193
His Pro Ala Cys Asn Val Asp His Pro Glu Ile Cys Asp
    50                  55                  60

gatgctccag gaccctctga accacgacgt                                    223


<210> 73
<211> 61
<212> PRT
<213> Conus radiatus

<400> 73
Met Phe Thr Val Phe Leu Leu Val Val Leu Ala Thr Thr Val Val Ser
1               5                   10                  15

Phe Thr Ser Gly Arg Ser Thr Phe Arg Gly Arg Asn Ala Ala Ala Lys
            20                  25                  30

Ala Ser Gly Leu Val Ser Leu Thr Asp Arg Arg Pro Gln Cys Cys Ser
        35                  40                  45

His Pro Ala Cys Asn Val Asp His Pro Glu Ile Cys Asp
    50                  55                  60

<210> 74
<211> 218
<212> DNA
<213> Conus striatus

<220>
<221> CDS
<222> (1)..(171)

<400> 74
atg ttc act gtg ttt ctg ttg gtt gtc ttg gca atc act gtc gtt tcc     48
Met Phe Thr Val Phe Leu Leu Val Val Leu Ala Ile Thr Val Val Ser
1               5                   10                  15

ttc cct tta gat cgt gaa tct gat ggc gcg aat gcc gaa gcc cgc acc     96
Phe Pro Leu Asp Arg Glu Ser Asp Gly Ala Asn Ala Glu Ala Arg Thr
            20                  25                  30

cac gat cat gag aag cac gca ctg gac cgg aat gga tgc tgt agg aat     144
His Asp His Glu Lys His Ala Leu Asp Arg Asn Gly Cys Cys Arg Asn
        35                  40                  45

cct gcc tgt gag agc cac aga tgt ggt tgacgacgct gatgctccag           191
Pro Ala Cys Glu Ser His Arg Cys Gly
    50                  55

gaccctctga accacgacgt tcgagca                                       218


<210> 75
<211> 57
<212> PRT
<213> Conus striatus

<400> 75
Met Phe Thr Val Phe Leu Leu Val Val Leu Ala Ile Thr Val Val Ser
1               5                   10                  15

Phe Pro Leu Asp Arg Glu Ser Asp Gly Ala Asn Ala Glu Ala Arg Thr
            20                  25                  30

His Asp His Glu Lys His Ala Leu Asp Arg Asn Gly Cys Cys Arg Asn
        35                  40                  45

Pro Ala Cys Glu Ser His Arg Cys Gly
    50                  55


<210> 76
<211> 227
<212> DNA
<213> Conus bandanus

<220>
<221> CDS
<222> (1)..(180)

<400> 76
atg ttc acc atg ttt ctg ttg gtt gtc ttg gca acc act gtc gtt tcc     48
Met Phe Thr Met Phe Leu Leu Val Val Leu Ala Thr Thr Val Val Ser
1               5                   10                  15

ttc gct tca gat cgt gca tct gat ggc agg aat gcc gca gcc aag gac     96
Phe Ala Ser Asp Arg Ala Ser Asp Gly Arg Asn Ala Ala Ala Lys Asp
            20                  25                  30

aaa gcg tct gac ctg gtc gct ctg acc gtc aag gga tgc tgt tct cat     144
Lys Ala Ser Asp Leu Val Ala Leu Thr Val Lys Gly Cys Cys Ser His
        35                  40                  45

cct gcc tgt agc gtg aat aat cca gac att tgt ggt tgaagacgct        190
Pro Ala Cys Ser Val Asn Asn Pro Asp Ile Cys Gly
     50                  55                  60

gatgctccag gaccctctga accacgacgt tcgagca                             227


<210> 77
<211> 60
<212> PRT
<213> Conus bandanus

<400> 77
Met Phe Thr Met Phe Leu Leu Val Val Leu Ala Thr Thr Val Val Ser
  1               5                  10                  15

Phe Ala Ser Asp Arg Ala Ser Asp Gly Arg Asn Ala Ala Ala Lys Asp
             20                  25                  30

Lys Ala Ser Asp Leu Val Ala Leu Thr Val Lys Gly Cys Cys Ser His
         35                  40                  45

Pro Ala Cys Ser Val Asn Asn Pro Asp Ile Cys Gly
     50                  55                  60


<210> 78
<211> 104
<212> DNA
<213> Conus bandanus

<220>
<221> CDS
<222> (1)..(54)

<400> 78
aaa gaa tgc tgt act cat cct gcc tgt cac gtg agt cat cca gaa ctc    48
Lys Glu Cys Cys Thr His Pro Ala Cys His Val Ser His Pro Glu Leu
  1               5                  10                  15

tgt ggt tgaaaagcga cgtgacgctc caggaccctc tgaaccacga cgttcgagca    104
Cys Gly


<210> 79
<211> 18
<212> PRT
<213> Conus bandanus

<400> 79
Lys Glu Cys Cys Thr His Pro Ala Cys His Val Ser His Pro Glu Leu
  1               5                  10                  15

Cys Gly



<210> 80
<211> 206
<212> DNA
<213> Conus bandanus

<220>
<221> CDS
<222> (1)..(171)

<400> 80
atg ttc acc gtg ttt ctg ttg gtt gtc ttg gca act gct gtt ctt cca    48

Met Phe Thr Val Phe Leu Leu Val Val Leu Ala Thr Ala Val Leu Pro
1               5                   10                  15

gtc act tta gat cgt gca tct gat gga agg aat gca gca gcc aac gcc     96
Val Thr Leu Asp Arg Ala Ser Asp Gly Arg Asn Ala Ala Ala Asn Ala
            20                  25                  30

aaa acg cct cgc ctg atc gcg cca ttc atc agg gat tat tgc tgt cat     144
Lys Thr Pro Arg Leu Ile Ala Pro Phe Ile Arg Asp Tyr Cys Cys His
        35                  40                  45

aga ggt ccc tgt atg gta tgg tgt ggt tgaagccgct gctgctccag           191
Arg Gly Pro Cys Met Val Trp Cys Gly
    50                  55

gaccctctga accac                                                    206


<210> 81
<211> 57
<212> PRT
<213> Conus bandanus

<400> 81
Met Phe Thr Val Phe Leu Leu Val Val Leu Ala Thr Ala Val Leu Pro
1               5                   10                  15

Val Thr Leu Asp Arg Ala Ser Asp Gly Arg Asn Ala Ala Ala Asn Ala
            20                  25                  30

Lys Thr Pro Arg Leu Ile Ala Pro Phe Ile Arg Asp Tyr Cys Cys His
        35                  40                  45

Arg Gly Pro Cys Met Val Trp Cys Gly
    50                  55


<210> 82
<211> 174
<212> DNA
<213> Conus caracteristicus

<220>
<221> CDS
<222> (1)..(171)

<400> 82
atg ttc acc gtg ttt ctg ttg gtt gtc ttg gca acc act gtg gtt tcc     48
Met Phe Thr Val Phe Leu Leu Val Val Leu Ala Thr Thr Val Val Ser
1               5                   10                  15

ttc act tca gat cgt gct tct gat ggc agg aat gcc gca gcc aac gcg     96
Phe Thr Ser Asp Arg Ala Ser Asp Gly Arg Asn Ala Ala Ala Asn Ala
            20                  25                  30

ttt gac ctg atc gct ctg atc gcc agg caa aat tgc tgt agc att ccc     144
Phe Asp Leu Ile Ala Leu Ile Ala Arg Gln Asn Cys Cys Ser Ile Pro
        35                  40                  45

agc tgt tgg gag aaa tat aaa tgt agt taa                             174
Ser Cys Trp Glu Lys Tyr Lys Cys Ser
    50                  55


<210> 83
<211> 57
<212> PRT
<213> Conus caracteristicus

<400> 83
Met Phe Thr Val Phe Leu Leu Val Val Leu Ala Thr Thr Val Val Ser
1               5                   10                  15

Phe Thr Ser Asp Arg Ala Ser Asp Gly Arg Asn Ala Ala Ala Asn Ala
            20                  25                  30

Phe Asp Leu Ile Ala Leu Ile Ala Arg Gln Asn Cys Cys Ser Ile Pro
        35                  40                  45

Ser Cys Trp Glu Lys Tyr Lys Cys Ser
    50                  55


<210> 84
<211> 219
<212> DNA
<213> Conus caracteristicus

<220>
<221> CDS
<222> (1)..(189)

<400> 84
atg ttc acc gtg ttt ctg ttg gtt gtc ttg gca acc act gtg gtt tcc     48
Met Phe Thr Val Phe Leu Leu Val Val Leu Ala Thr Thr Val Val Ser
1               5                   10                  15

ttc act tca gat cgt gcg tct gaa ggc agg aat gct gca gcc aag gac     96
Phe Thr Ser Asp Arg Ala Ser Glu Gly Arg Asn Ala Ala Ala Lys Asp
            20                  25                  30

aaa gcg tct gac ctg gtg gct ctg aca gtc agg gga tgc tgt gcc att     144
Lys Ala Ser Asp Leu Val Ala Leu Thr Val Arg Gly Cys Cys Ala Ile
        35                  40                  45

cgt gaa tgt cgc ttg cag aat gca gcg tat tgt ggt gga ata tac         189
Arg Glu Cys Arg Leu Gln Asn Ala Ala Tyr Cys Gly Gly Ile Tyr
    50                  55                  60

tgatgctcca ggaccctctg aaccacgacg                                    219


<210> 85
<211> 63
<212> PRT
<213> Conus caracteristicus

<400> 85
Met Phe Thr Val Phe Leu Leu Val Val Leu Ala Thr Thr Val Val Ser
1               5                   10                  15

Phe Thr Ser Asp Arg Ala Ser Glu Gly Arg Asn Ala Ala Ala Lys Asp
            20                  25                  30

Lys Ala Ser Asp Leu Val Ala Leu Thr Val Arg Gly Cys Cys Ala Ile
        35                  40                  45

Arg Glu Cys Arg Leu Gln Asn Ala Ala Tyr Cys Gly Gly Ile Tyr
    50                  55                  60


<210> 86
<211> 217
<212> DNA
<213> Conus tulipa

<220>
<221> CDS

<222> (1)..(186)

<400> 86
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca gat att gca act gag ggc agg aat gcc gca gcc aaa gcg     96
Phe Pro Ser Asp Ile Ala Thr Glu Gly Arg Asn Ala Ala Ala Lys Ala
            20                  25                  30

ttt gac ctg ata tct tcg atc gtc aag aaa gga tgc tgt tcc cat cct     144
Phe Asp Leu Ile Ser Ser Ile Val Lys Lys Gly Cys Cys Ser His Pro
        35                  40                  45

gcc tgt tcg ggg aat aat cca gaa ttt tgt cgt caa ggt cgc             186
Ala Cys Ser Gly Asn Asn Pro Glu Phe Cys Arg Gln Gly Arg
    50                  55                  60

tgatgctcca ggaccctctg aaccacgacg t                                  217


<210> 87
<211> 62
<212> PRT
<213> Conus tulipa

<400> 87
Met Phe Thr Val Phe Leu Leu Val Val Leu Ala Thr Thr Val Val Ser
1               5                   10                  15

Phe Pro Ser Asp Ile Ala Thr Glu Gly Arg Asn Ala Ala Ala Lys Ala
            20                  25                  30

Phe Asp Leu Ile Ser Ser Ile Val Lys Lys Gly Cys Cys Ser His Pro
        35                  40                  45

Ala Cys Ser Gly Asn Asn Pro Glu Phe Cys Arg Gln Gly Arg
    50                  55                  60


<210> 88
<211> 217
<212> DNA
<213> Conus tulipa

<220>
<221> CDS
<222> (1)..(186)

<400> 88
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca gat ata gca act gag ggc agg aat gcc gca gcc aaa gcg     96
Phe Pro Ser Asp Ile Ala Thr Glu Gly Arg Asn Ala Ala Ala Lys Ala
            20                  25                  30

ttt gac ctg ata tct tcg atc gtc agg aaa gga tgc tgt tcc aat ccc     144
Phe Asp Leu Ile Ser Ser Ile Val Arg Lys Gly Cys Cys Ser Asn Pro
        35                  40                  45

gcc tgt gcg ggg aat aat cca cat gtt tgt cgt caa ggt cgc             186
Ala Cys Ala Gly Asn Asn Pro His Val Cys Arg Gln Gly Arg
    50                  55                  60

tgatgctcca ggaccctctg aaccacgacg t                                  217

<210> 89
<211> 62
<212> PRT
<213> Conus tulipa

<400> 89
Met Phe Thr Val Phe Leu Leu Val Val Leu Ala Thr Thr Val Val Ser
1               5                   10                  15

Phe Pro Ser Asp Ile Ala Thr Glu Gly Arg Asn Ala Ala Ala Lys Ala
            20                  25                  30

Phe Asp Leu Ile Ser Ser Ile Val Arg Lys Gly Cys Cys Ser Asn Pro
        35                  40                  45

Ala Cys Ala Gly Asn Asn Pro His Val Cys Arg Gln Gly Arg
    50                  55                  60


<210> 90
<211> 226
<212> DNA
<213> Conus sulcatus

<220>
<221> CDS
<222> (1)..(195)

<400> 90
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc aat tca gat cgt gat cca gca tta ggt ggc agg aat gct gca gcc      96
Phe Asn Ser Asp Arg Asp Pro Ala Leu Gly Gly Arg Asn Ala Ala Ala
            20                  25                  30

aaa gcg tct gac aag atc gct tcg acc ctc aag aga aga gga tgc tgt      144
Lys Ala Ser Asp Lys Ile Ala Ser Thr Leu Lys Arg Arg Gly Cys Cys
        35                  40                  45

tcg tat ttt gac tgt aga atg atg ttt cca gaa atg tgt ggt tgg cga      192
Ser Tyr Phe Asp Cys Arg Met Met Phe Pro Glu Met Cys Gly Trp Arg
    50                  55                  60

ggc tgatgctcca ggaccctctg aaccacgacg t                               226
Gly
65


<210> 91
<211> 65
<212> PRT
<213> Conus sulcatus

<400> 91
Met Phe Thr Val Phe Leu Leu Val Val Leu Ala Thr Thr Val Val Ser
1               5                   10                  15

Phe Asn Ser Asp Arg Asp Pro Ala Leu Gly Gly Arg Asn Ala Ala Ala
            20                  25                  30

Lys Ala Ser Asp Lys Ile Ala Ser Thr Leu Lys Arg Arg Gly Cys Cys
        35                  40                  45

Ser Tyr Phe Asp Cys Arg Met Met Phe Pro Glu Met Cys Gly Trp Arg
    50                  55                  60

Gly
65


<210> 92
<211> 226
<212> DNA
<213> Conus sulcatus

<220>
<221> CDS
<222> (1)..(195)

<400> 92
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
  1               5                  10                  15

ttc aat tca gat cgt gat cca gca tta ggt ggc agg aat gct gca gcc     96
Phe Asn Ser Asp Arg Asp Pro Ala Leu Gly Gly Arg Asn Ala Ala Ala
             20                  25                  30

ata gcg tct gac aag atc gct tcg acc ctc agg aga gga gga tgc tgt    144
Ile Ala Ser Asp Lys Ile Ala Ser Thr Leu Arg Arg Gly Gly Cys Cys
         35                  40                  45

tct ttt cct gcc tgt aga aag tat cgt cca gaa atg tgt ggt gga cga    192
Ser Phe Pro Ala Cys Arg Lys Tyr Arg Pro Glu Met Cys Gly Gly Arg
     50                  55                  60

cgc tgatgctcca ggaccctctg aaccacgacg t                             226
Arg
65


<210> 93
<211> 65
<212> PRT
<213> Conus sulcatus

<400> 93
Met Phe Thr Val Phe Leu Leu Val Val Leu Ala Thr Thr Val Val Ser
  1               5                  10                  15

Phe Asn Ser Asp Arg Asp Pro Ala Leu Gly Gly Arg Asn Ala Ala Ala
             20                  25                  30

Ile Ala Ser Asp Lys Ile Ala Ser Thr Leu Arg Arg Gly Gly Cys Cys
         35                  40                  45

Ser Phe Pro Ala Cys Arg Lys Tyr Arg Pro Glu Met Cys Gly Gly Arg
     50                  55                  60

Arg
65


<210> 94
<211> 211
<212> DNA
<213> Conus sulcatus

<220>
<221> CDS
<222> (1)..(180)

<400> 94
atg ttc acc gtg ttt ctg ttg gtt gtc ttg gca acc acc gtc gtt tcc     48
Met Phe Thr Val Phe Leu Leu Val Val Leu Ala Thr Thr Val Val Ser

1                   5                   10                  15

ttc act tca gat cat gaa tct gat cgc ggt gat gcc caa acc atc caa      96
Phe Thr Ser Asp His Glu Ser Asp Arg Gly Asp Ala Gln Thr Ile Gln
                20                  25                  30

gaa gtg ttt gag atg ttc gct ctg gac agc gat gga tgc tgt tgg cat      144
Glu Val Phe Glu Met Phe Ala Leu Asp Ser Asp Gly Cys Cys Trp His
            35                  40                  45

cct gct tgt ggc aga cac tat tgt ggt cga aga cgc tgatgctcca           190
Pro Ala Cys Gly Arg His Tyr Cys Gly Arg Arg Arg
    50                  55                  60

ggaccctctg aaccacgacg t                                              211


<210> 95
<211> 60
<212> PRT
<213> Conus sulcatus

<400> 95
Met Phe Thr Val Phe Leu Leu Val Val Leu Ala Thr Thr Val Val Ser
1               5                   10                  15

Phe Thr Ser Asp His Glu Ser Asp Arg Gly Asp Ala Gln Thr Ile Gln
                20                  25                  30

Glu Val Phe Glu Met Phe Ala Leu Asp Ser Asp Gly Cys Cys Trp His
            35                  40                  45

Pro Ala Cys Gly Arg His Tyr Cys Gly Arg Arg Arg
    50                  55                  60


<210> 96
<211> 202
<212> DNA
<213> Conus sulcatus

<220>
<221> CDS
<222> (1)..(195)

<400> 96
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc aat tca gat cgt gat cca gca tta ggt ggc agg aat gct gca gcc      96
Phe Asn Ser Asp Arg Asp Pro Ala Leu Gly Gly Arg Asn Ala Ala Ala
                20                  25                  30

ata gcg tct gac aag atc gct tcg acc ctc agg aga gga gga tgc tgt      144
Ile Ala Ser Asp Lys Ile Ala Ser Thr Leu Arg Arg Gly Gly Cys Cys
            35                  40                  45

tct ttt gct gcc tgt aga aag tat cgt cca gaa atg tgt ggt gga cga      192
Ser Phe Ala Ala Cys Arg Lys Tyr Arg Pro Glu Met Cys Gly Gly Arg
    50                  55                  60

cgc tgatgct                                                          202
Arg
65


<210> 97
<211> 65

<212> PRT
<213> Conus sulcatus

<400> 97
Met Phe Thr Val Phe Leu Leu Val Val Leu Ala Thr Thr Val Val Ser
1               5                   10                  15

Phe Asn Ser Asp Arg Asp Pro Ala Leu Gly Gly Arg Asn Ala Ala Ala
            20                  25                  30

Ile Ala Ser Asp Lys Ile Ala Ser Thr Leu Arg Arg Gly Gly Cys Cys
        35                  40                  45

Ser Phe Ala Ala Cys Arg Lys Tyr Arg Pro Glu Met Cys Gly Gly Arg
    50                  55                  60

Arg
65


<210> 98
<211> 220
<212> DNA
<213> Conus sulcatus

<220>
<221> CDS
<222> (1)..(189)

<400> 98
atg ttc acc gtg ttt ctg ttg gtt ctc ttg gca acc acc gtc gtt tcc      48
Met Phe Thr Val Phe Leu Leu Val Leu Leu Ala Thr Thr Val Val Ser
1               5                   10                  15

ttc aat tca gat cgt gca tta ggt ggc agg aat gct gca gcc aaa gcg      96
Phe Asn Ser Asp Arg Ala Leu Gly Gly Arg Asn Ala Ala Ala Lys Ala
            20                  25                  30

tct gac aag atc ctt tcg aac ctc agg aga gga gga tgc tgt ttt cat      144
Ser Asp Lys Ile Leu Ser Asn Leu Arg Arg Gly Gly Cys Cys Phe His
        35                  40                  45

cct gtc tgt tac atc aat ctt cta gaa atg tgt cgt caa cga ggc          189
Pro Val Cys Tyr Ile Asn Leu Leu Glu Met Cys Arg Gln Arg Gly
    50                  55                  60

tgatcgtcca ggaccctctg aaccacgacg t                                   220


<210> 99
<211> 63
<212> PRT
<213> Conus sulcatus

<400> 99
Met Phe Thr Val Phe Leu Leu Val Leu Leu Ala Thr Thr Val Val Ser
1               5                   10                  15

Phe Asn Ser Asp Arg Ala Leu Gly Gly Arg Asn Ala Ala Ala Lys Ala
            20                  25                  30

Ser Asp Lys Ile Leu Ser Asn Leu Arg Arg Gly Gly Cys Cys Phe His
        35                  40                  45

Pro Val Cys Tyr Ile Asn Leu Leu Glu Met Cys Arg Gln Arg Gly
    50                  55                  60


<210> 100

<211> 208
<212> DNA
<213> Conus consors

<220>
<221> CDS
<222> (1)..(177)

<400> 100
atg ttc acc gtg ttt ctg ttg gtt gtc ttg aca acc act gtc gtt tcc     48
Met Phe Thr Val Phe Leu Leu Val Val Leu Thr Thr Thr Val Val Ser
1               5                   10                  15

ttc cct tca gat agt gca tct gat gtc agg gat gac gaa gcc aaa gac     96
Phe Pro Ser Asp Ser Ala Ser Asp Val Arg Asp Asp Glu Ala Lys Asp
            20                  25                  30

gaa agg tct gac atg tac aaa tcg aaa cgg aat gga cgc tgt tgc cat     144
Glu Arg Ser Asp Met Tyr Lys Ser Lys Arg Asn Gly Arg Cys Cys His
        35                  40                  45

cct gcc tgt ggc aaa cac ttt agt tgt gga cgc tgatgctcca ggaccctctg 197
Pro Ala Cys Gly Lys His Phe Ser Cys Gly Arg
    50                  55

aaccacgacg t                                                        208


<210> 101
<211> 59
<212> PRT
<213> Conus consors

<400> 101
Met Phe Thr Val Phe Leu Leu Val Val Leu Thr Thr Thr Val Val Ser
1               5                   10                  15

Phe Pro Ser Asp Ser Ala Ser Asp Val Arg Asp Asp Glu Ala Lys Asp
            20                  25                  30

Glu Arg Ser Asp Met Tyr Lys Ser Lys Arg Asn Gly Arg Cys Cys His
        35                  40                  45

Pro Ala Cys Gly Lys His Phe Ser Cys Gly Arg
    50                  55


<210> 102
<211> 219
<212> DNA
<213> Conus stercusmuscarum

<220>
<221> CDS
<222> (1)..(189)

<400> 102
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

tcc cct tca gat cgt gca tct gat ggc agg aat gcc gca gcc aac gag     96
Ser Pro Ser Asp Arg Ala Ser Asp Gly Arg Asn Ala Ala Ala Asn Glu
            20                  25                  30

aaa gcg tct gac gtg atc gcg ctg gcc ctc aag gga tgc tgt tcc aac     144
Lys Ala Ser Asp Val Ile Ala Leu Ala Leu Lys Gly Cys Cys Ser Asn
        35                  40                  45

cct gtc tgt cac ctg gag cat tca aac atg tgt ggt aga aga cgc        189
Pro Val Cys His Leu Glu His Ser Asn Met Cys Gly Arg Arg Arg
     50                  55                  60

tgatgctcca ggaccctctg aaccacgacg                                   219


<210> 103
<211> 63
<212> PRT
<213> Conus stercusmuscarum

<400> 103
Met Phe Thr Val Phe Leu Leu Val Val Leu Ala Thr Thr Val Val Ser
1               5                   10                  15

Ser Pro Ser Asp Arg Ala Ser Asp Gly Arg Asn Ala Ala Ala Asn Glu
            20                  25                  30

Lys Ala Ser Asp Val Ile Ala Leu Ala Leu Lys Gly Cys Cys Ser Asn
        35                  40                  45

Pro Val Cys His Leu Glu His Ser Asn Met Cys Gly Arg Arg Arg
     50                  55                  60


<210> 104
<211> 248
<212> DNA
<213> Conus betulinus

<220>
<221> CDS
<222> (1)..(180)

<400> 104
atg ttc tcc gtg ttt ctg ttg gtt gtc ttg gca acc act gtc gtt tcc    48
Met Phe Ser Val Phe Leu Leu Val Val Leu Ala Thr Thr Val Val Ser
1               5                   10                  15

tcc act tca ggt ggt gca tct ggt ggc agg aag gct gca gcc aaa gcg    96
Ser Thr Ser Gly Gly Ala Ser Gly Gly Arg Lys Ala Ala Ala Lys Ala
            20                  25                  30

tct aac cgg atc gct ctg acc gtc agg agt gca aca tgc tgt aat tat    144
Ser Asn Arg Ile Ala Leu Thr Val Arg Ser Ala Thr Cys Cys Asn Tyr
        35                  40                  45

cct ccc tgt tac gag act tat cca gaa agt tgt ctg taacgtgaat         190
Pro Pro Cys Tyr Glu Thr Tyr Pro Glu Ser Cys Leu
     50                  55                  60

catccagagc tttgtggctg aagacactga tgctccagga ccctctgaac cacgacgt    248


<210> 105
<211> 60
<212> PRT
<213> Conus betulinus

<400> 105
Met Phe Ser Val Phe Leu Leu Val Val Leu Ala Thr Thr Val Val Ser
1               5                   10                  15

Ser Thr Ser Gly Gly Ala Ser Gly Gly Arg Lys Ala Ala Ala Lys Ala
            20                  25                  30

Ser Asn Arg Ile Ala Leu Thr Val Arg Ser Ala Thr Cys Cys Asn Tyr
        35                  40                  45

Pro Pro Cys Tyr Glu Thr Tyr Pro Glu Ser Cys Leu
     50                  55                  60


<210> 106
<211> 223
<212> DNA
<213> Conus betulinus

<220>
<221> CDS
<222> (1)..(183)

<400> 106
atg ttc acc gtg ttt ctg ttg gtt gtc ttg gca acc act gtg gtt tcc      48
Met Phe Thr Val Phe Leu Leu Val Val Leu Ala Thr Thr Val Val Ser
  1               5                  10                  15

ttc act tca ggt cgt gca ttt cgt ggc agg aat cgc gca gcc gac gac      96
Phe Thr Ser Gly Arg Ala Phe Arg Gly Arg Asn Arg Ala Ala Asp Asp
             20                  25                  30

aaa agg tct gac ctg gcc gct ctg agc gtc agg gga gga tgc tgt tcc     144
Lys Arg Ser Asp Leu Ala Ala Leu Ser Val Arg Gly Gly Cys Cys Ser
         35                  40                  45

cat cct gcc tgt gcg gtg aat cat cca gag ctt tgt ggc tgaagacgct      193
His Pro Ala Cys Ala Val Asn His Pro Glu Leu Cys Gly
     50                  55                  60

gatgccccag gaccctctga accacgacgt                                    223


<210> 107
<211> 61
<212> PRT
<213> Conus betulinus

<400> 107
Met Phe Thr Val Phe Leu Leu Val Val Leu Ala Thr Thr Val Val Ser
  1               5                  10                  15

Phe Thr Ser Gly Arg Ala Phe Arg Gly Arg Asn Arg Ala Ala Asp Asp
             20                  25                  30

Lys Arg Ser Asp Leu Ala Ala Leu Ser Val Arg Gly Gly Cys Cys Ser
         35                  40                  45

His Pro Ala Cys Ala Val Asn His Pro Glu Leu Cys Gly
     50                  55                  60


<210> 108
<211> 248
<212> DNA
<213> Conus betulinus

<220>
<221> CDS
<222> (1)..(180)

<400> 108
atg ttc acc gtg ttt ctg ttg gtt gtc ttg gca acc act gtc gtt tcc      48
Met Phe Thr Val Phe Leu Leu Val Val Leu Ala Thr Thr Val Val Ser
  1               5                  10                  15

ttc act tca ggt cgt gca tct ggt ggc agg aat gct gca gcc aaa gcg      96
Phe Thr Ser Gly Arg Ala Ser Gly Gly Arg Asn Ala Ala Ala Lys Ala

20                  25                  30

tct aac cgg atc gct atg gcc atc agc agt gga gca tgc tgt gca tat    144
Ser Asn Arg Ile Ala Met Ala Ile Ser Ser Gly Ala Cys Cys Ala Tyr
        35                  40                  45

cct ccc tgt ttc gag gct tat cca gaa aga tgt ctg taacgtgaat         190
Pro Pro Cys Phe Glu Ala Tyr Pro Glu Arg Cys Leu
    50                  55                  60

catccagacc tttgtggctg aagacgctga tgccccagga ccctctgaac cacgacgt    248


<210> 109
<211> 60
<212> PRT
<213> Conus betulinus

<400> 109
Met Phe Thr Val Phe Leu Leu Val Val Leu Ala Thr Thr Val Val Ser
1               5                   10                  15

Phe Thr Ser Gly Arg Ala Ser Gly Gly Arg Asn Ala Ala Ala Lys Ala
            20                  25                  30

Ser Asn Arg Ile Ala Met Ala Ile Ser Ser Gly Ala Cys Cys Ala Tyr
        35                  40                  45

Pro Pro Cys Phe Glu Ala Tyr Pro Glu Arg Cys Leu
    50                  55                  60


<210> 110
<211> 223
<212> DNA
<213> Conus betulinus

<220>
<221> CDS
<222> (1)..(192)

<400> 110
atg ttc acc gtg ttt ctg ttg gtt gtc ttg gca acc act gtc gtt tcc    48
Met Phe Thr Val Phe Leu Leu Val Val Leu Ala Thr Thr Val Val Ser
1               5                   10                  15

ttc act tca gat cgt gca ttt cgt ggc agg aat tcc gca gcc aac gac    96
Phe Thr Ser Asp Arg Ala Phe Arg Gly Arg Asn Ser Ala Ala Asn Asp
            20                  25                  30

aaa agg tct gac ctg gcc gct ctg agc gtc agg aga gga tgc tgc tcc    144
Lys Arg Ser Asp Leu Ala Ala Leu Ser Val Arg Arg Gly Cys Cys Ser
        35                  40                  45

cat ccc gcc tgt agc gtg aat cat cca gag ctt tgt ggt aga aga cgc    192
His Pro Ala Cys Ser Val Asn His Pro Glu Leu Cys Gly Arg Arg Arg
    50                  55                  60

tgatgcccca ggaccctctg aaccacgacg t                                 223


<210> 111
<211> 64
<212> PRT
<213> Conus betulinus

<400> 111
Met Phe Thr Val Phe Leu Leu Val Val Leu Ala Thr Thr Val Val Ser
1               5                   10                  15

Phe Thr Ser Asp Arg Ala Phe Arg Gly Arg Asn Ser Ala Ala Asn Asp
            20                  25                  30

Lys Arg Ser Asp Leu Ala Ala Leu Ser Val Arg Arg Gly Cys Cys Ser
        35                  40                  45

His Pro Ala Cys Ser Val Asn His Pro Glu Leu Cys Gly Arg Arg Arg
    50                  55                  60


<210> 112
<211> 248
<212> DNA
<213> Conus betulinus

<220>
<221> CDS
<222> (1)..(180)

<400> 112
atg ttc acc gtg ttt ctg ttg gtt gtc ttg gca acc act gtc gtt tcc      48
Met Phe Thr Val Phe Leu Leu Val Val Leu Ala Thr Thr Val Val Ser
1               5                   10                  15

ttc act tca ggt cgt gca tct ggt ggc agg aat gct gca gcc aaa gcg      96
Phe Thr Ser Gly Arg Ala Ser Gly Gly Arg Asn Ala Ala Ala Lys Ala
            20                  25                  30

tct aac cgg atc gct ctg atc gtc agg aat gca gaa tgc tgt tat tat      144
Ser Asn Arg Ile Ala Leu Ile Val Arg Asn Ala Glu Cys Cys Tyr Tyr
        35                  40                  45

cct ccc tgt tac gag gct tat cca gaa att tgt ctg taacgtgaat           190
Pro Pro Cys Tyr Glu Ala Tyr Pro Glu Ile Cys Leu
    50                  55                  60

catccagacc tttgtggctg aagaccctga tgctccagga ccctctgaac cacgacgt      248


<210> 113
<211> 60
<212> PRT
<213> Conus betulinus

<400> 113
Met Phe Thr Val Phe Leu Leu Val Val Leu Ala Thr Thr Val Val Ser
1               5                   10                  15

Phe Thr Ser Gly Arg Ala Ser Gly Gly Arg Asn Ala Ala Ala Lys Ala
            20                  25                  30

Ser Asn Arg Ile Ala Leu Ile Val Arg Asn Ala Glu Cys Cys Tyr Tyr
        35                  40                  45

Pro Pro Cys Tyr Glu Ala Tyr Pro Glu Ile Cys Leu
    50                  55                  60


<210> 114
<211> 207
<212> DNA
<213> Conus pennaceus

<220>
<221> CDS
<222> (1)..(168)

<400> 114

atg ttc acc gtg ttt ctg ttg gtt gtc ttg gca acc acc gtc att tcc     48
Met Phe Thr Val Phe Leu Leu Val Val Leu Ala Thr Thr Val Ile Ser
1               5                   10                  15

ttc act tca gat cgt gca tct gat ggc ggg aat gcc gca gcg tct gac     96
Phe Thr Ser Asp Arg Ala Ser Asp Gly Gly Asn Ala Ala Ala Ser Asp
            20                  25                  30

ctg atc gct ctg acc atc aag gga tgc tgt tct cat cct ccc tgt gcc     144
Leu Ile Ala Leu Thr Ile Lys Gly Cys Cys Ser His Pro Pro Cys Ala
        35                  40                  45

atg aat aat cca gac tat tgt ggt tgacgacgct gatgctccag gaccctctga    198
Met Asn Asn Pro Asp Tyr Cys Gly
    50                  55

accacgacg                                                           207


<210> 115
<211> 56
<212> PRT
<213> Conus pennaceus

<400> 115
Met Phe Thr Val Phe Leu Leu Val Val Leu Ala Thr Thr Val Ile Ser
1               5                   10                  15

Phe Thr Ser Asp Arg Ala Ser Asp Gly Gly Asn Ala Ala Ala Ser Asp
            20                  25                  30

Leu Ile Ala Leu Thr Ile Lys Gly Cys Cys Ser His Pro Pro Cys Ala
        35                  40                  45

Met Asn Asn Pro Asp Tyr Cys Gly
    50                  55


<210> 116
<211> 207
<212> DNA
<213> Conus pennaceus

<220>
<221> CDS
<222> (1)..(168)

<400> 116
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca gat cgt gca tct gat ggc ggg aat gcc gca atg tct gac     96
Phe Thr Ser Asp Arg Ala Ser Asp Gly Gly Asn Ala Ala Met Ser Asp
            20                  25                  30

ctg atc gct ctg acc atc aag gga tgc tgt tct cat cct ccc tgt ttc     144
Leu Ile Ala Leu Thr Ile Lys Gly Cys Cys Ser His Pro Pro Cys Phe
        35                  40                  45

ctg aat aat cca gac tat tgt ggt tgacgacgct gatgctccag gaccctctga    198
Leu Asn Asn Pro Asp Tyr Cys Gly
    50                  55

accacgacg                                                           207


<210> 117
<211> 56

<212> PRT
<213> Conus pennaceus

<400> 117
Met Phe Thr Val Phe Leu Leu Val Val Leu Ala Thr Thr Val Val Ser
1               5                   10                  15

Phe Thr Ser Asp Arg Ala Ser Asp Gly Gly Asn Ala Ala Met Ser Asp
            20                  25                  30

Leu Ile Ala Leu Thr Ile Lys Gly Cys Cys Ser His Pro Pro Cys Phe
        35                  40                  45

Leu Asn Asn Pro Asp Tyr Cys Gly
    50                  55


<210> 118
<211> 210
<212> DNA
<213> Conus stercusmuscarum

<220>
<221> CDS
<222> (1)..(171)

<400> 118
atg ttc acc gtg ttt ctg ttg gtt gtc ttg gca acc act gtc gtt tcc      48
Met Phe Thr Val Phe Leu Leu Val Val Leu Ala Thr Thr Val Val Ser
1               5                   10                  15

ttc cct tca gat cgt gaa tct gat ggc gcg aat gac gaa gcc cgc acc      96
Phe Pro Ser Asp Arg Glu Ser Asp Gly Ala Asn Asp Glu Ala Arg Thr
            20                  25                  30

gac gag cct gag gag cac gga ccg gac agg aat gga tgc tgt agg aat      144
Asp Glu Pro Glu Glu His Gly Pro Asp Arg Asn Gly Cys Cys Arg Asn
        35                  40                  45

cct gcc tgt gag agc cac aga tgt ggt tgacgacgct gatgctccag            191
Pro Ala Cys Glu Ser His Arg Cys Gly
    50                  55

gaccctctga accacgacg                                                 210


<210> 119
<211> 57
<212> PRT
<213> Conus stercusmuscarum

<400> 119
Met Phe Thr Val Phe Leu Leu Val Val Leu Ala Thr Thr Val Val Ser
1               5                   10                  15

Phe Pro Ser Asp Arg Glu Ser Asp Gly Ala Asn Asp Glu Ala Arg Thr
            20                  25                  30

Asp Glu Pro Glu Glu His Gly Pro Asp Arg Asn Gly Cys Cys Arg Asn
        35                  40                  45

Pro Ala Cys Glu Ser His Arg Cys Gly
    50                  55


<210> 120
<211> 210
<212> DNA
<213> Conus circumcisus

<220>
<221> CDS
<222> (1)..(180)

<400> 120
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca gat cgt gca tct gat ggc agg aat gcc gca gcc agc gac     96
Phe Pro Ser Asp Arg Ala Ser Asp Gly Arg Asn Ala Ala Ala Ser Asp
            20                  25                  30

aga gcg tct gac gcg gcc cac cag gga tgc tgt tcc aac cct gtc tgt     144
Arg Ala Ser Asp Ala Ala His Gln Gly Cys Cys Ser Asn Pro Val Cys
        35                  40                  45

cac gtg gaa cat cca gaa ctt tgt cgt aga aga cgc tgatgctcca          190
His Val Glu His Pro Glu Leu Cys Arg Arg Arg Arg
    50                  55                  60

ggaccctctg aaccacgacg                                               210


<210> 121
<211> 60
<212> PRT
<213> Conus circumcisus

<400> 121
Met Phe Thr Val Phe Leu Leu Val Val Leu Ala Thr Thr Val Val Ser
1               5                   10                  15

Phe Pro Ser Asp Arg Ala Ser Asp Gly Arg Asn Ala Ala Ala Ser Asp
            20                  25                  30

Arg Ala Ser Asp Ala Ala His Gln Gly Cys Cys Ser Asn Pro Val Cys
        35                  40                  45

His Val Glu His Pro Glu Leu Cys Arg Arg Arg Arg
    50                  55                  60


<210> 122
<211> 213
<212> DNA
<213> Conus circumcisus

<220>
<221> CDS
<222> (1)..(174)

<400> 122
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca aat cgt gaa tct gat ggc gcg aat gcc gaa gtc cgc acc     96
Phe Pro Ser Asn Arg Glu Ser Asp Gly Ala Asn Ala Glu Val Arg Thr
            20                  25                  30

gac gag cct gag gag cac gac gaa ctg ggc ggg aat gga tgc tgt ggg     144
Asp Glu Pro Glu Glu His Asp Glu Leu Gly Gly Asn Gly Cys Cys Gly
        35                  40                  45

aat cct gac tgt acg agc cac agt tgt gat tgacgacgct gatgctccag       194
Asn Pro Asp Cys Thr Ser His Ser Cys Asp
    50                  55

gaccctctga accacgacg                                                213

<210> 123
<211> 58
<212> PRT
<213> Conus circumcisus

<400> 123
Met Phe Thr Val Phe Leu Leu Val Val Leu Ala Thr Thr Val Val Ser
1               5                   10                  15

Phe Pro Ser Asn Arg Glu Ser Asp Gly Ala Asn Ala Glu Val Arg Thr
            20                  25                  30

Asp Glu Pro Glu Glu His Asp Glu Leu Gly Gly Asn Gly Cys Cys Gly
        35                  40                  45

Asn Pro Asp Cys Thr Ser His Ser Cys Asp
    50                  55


<210> 124
<211> 207
<212> DNA
<213> Conus episcopatus

<220>
<221> CDS
<222> (1)..(168)

<400> 124
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc act tca gat cgt gca tct gat agc agg aag gac gca gcg tct ggc      96
Phe Thr Ser Asp Arg Ala Ser Asp Ser Arg Lys Asp Ala Ala Ser Gly
            20                  25                  30

ctg atc gct ctg acc atc aag gga tgc tgt tct gat cct cgc tgt aac     144
Leu Ile Ala Leu Thr Ile Lys Gly Cys Cys Ser Asp Pro Arg Cys Asn
        35                  40                  45

atg aat aat cca gac tat tgt ggt tgacgacgct gatgctccag gaccctctga    198
Met Asn Asn Pro Asp Tyr Cys Gly
    50                  55

accacgacg                                                           207


<210> 125
<211> 56
<212> PRT
<213> Conus episcopatus

<400> 125
Met Phe Thr Val Phe Leu Leu Val Val Leu Ala Thr Thr Val Val Ser
1               5                   10                  15

Phe Thr Ser Asp Arg Ala Ser Asp Ser Arg Lys Asp Ala Ala Ser Gly
            20                  25                  30

Leu Ile Ala Leu Thr Ile Lys Gly Cys Cys Ser Asp Pro Arg Cys Asn
        35                  40                  45

Met Asn Asn Pro Asp Tyr Cys Gly
    50                  55

<210> 126
<211> 213
<212> DNA
<213> Conus sponsalis

<220>
<221> CDS
<222> (1)..(174)

<400> 126
atg tcc acc gtg ttt ctg ttg gtt gtc ctc gca acc acc gtc gtt tcc     48
Met Ser Thr Val Phe Leu Leu Val Val Leu Ala Thr Thr Val Val Ser
1               5                   10                  15

ttc act gta gat cgt gca tct gat ggc agg gat gtc gca atc gac gac     96
Phe Thr Val Asp Arg Ala Ser Asp Gly Arg Asp Val Ala Ile Asp Asp
            20                  25                  30

aga ttg gtg tct ctc cct cag atc gcc cat gct gac tgt tgt tcc gat     144
Arg Leu Val Ser Leu Pro Gln Ile Ala His Ala Asp Cys Cys Ser Asp
        35                  40                  45

cct gcc tgc aag cag acg ccc ggt tgt cgt taaagacgct gctgctccag      194
Pro Ala Cys Lys Gln Thr Pro Gly Cys Arg
    50                  55

gaccctctga accacgacg                                                213


<210> 127
<211> 58
<212> PRT
<213> Conus sponsalis

<400> 127
Met Ser Thr Val Phe Leu Leu Val Val Leu Ala Thr Thr Val Val Ser
1               5                   10                  15

Phe Thr Val Asp Arg Ala Ser Asp Gly Arg Asp Val Ala Ile Asp Asp
            20                  25                  30

Arg Leu Val Ser Leu Pro Gln Ile Ala His Ala Asp Cys Cys Ser Asp
        35                  40                  45

Pro Ala Cys Lys Gln Thr Pro Gly Cys Arg
    50                  55


<210> 128
<211> 221
<212> DNA
<213> Conus sponsalis

<220>
<221> CDS
<222> (1)..(168)

<400> 128
atg ttc acc gtg ttt ctg ttg gtt gtc ttg gca acc acc gtc gct tcc     48
Met Phe Thr Val Phe Leu Leu Val Val Leu Ala Thr Thr Val Ala Ser
1               5                   10                  15

ttc att atc gat gat cca tct gat ggc agg aat att gca gtc gac gac     96
Phe Ile Ile Asp Asp Pro Ser Asp Gly Arg Asn Ile Ala Val Asp Asp
            20                  25                  30

aga ggg ctt ttc tct acg ctc ttc cat gct gat tgc tgt gaa aat cct     144

Arg Gly Leu Phe Ser Thr Leu Phe His Ala Asp Cys Cys Glu Asn Pro
            35                  40                  45

gcc tgt aga cac acg cag ggt tgt tgatctttgt tcttcaaaga cactgctggc    198
Ala Cys Arg His Thr Gln Gly Cys
        50                  55

ccaggaccct ctgaaccacg acg                                           221


<210> 129
<211> 56
<212> PRT
<213> Conus sponsalis

<400> 129
Met Phe Thr Val Phe Leu Leu Val Val Leu Ala Thr Thr Val Ala Ser
1               5                   10                  15

Phe Ile Ile Asp Asp Pro Ser Asp Gly Arg Asn Ile Ala Val Asp Asp
                20                  25                  30

Arg Gly Leu Phe Ser Thr Leu Phe His Ala Asp Cys Cys Glu Asn Pro
            35                  40                  45

Ala Cys Arg His Thr Gln Gly Cys
        50                  55


<210> 130
<211> 220
<212> DNA
<213> Conus dalli

<220>
<221> CDS
<222> (1)..(180)

<400> 130
atg ttc acc gtg ttt ctg ttg gtt gtc ttg gca acc acc gtc gtt tcc    48
Met Phe Thr Val Phe Leu Leu Val Val Leu Ala Thr Thr Val Val Ser
1               5                   10                  15

ttc act tca gat cgt gca ttt cgt ggc agg aat gcc gca gcc aaa gag    96
Phe Thr Ser Asp Arg Ala Phe Arg Gly Arg Asn Ala Ala Ala Lys Glu
                20                  25                  30

tct ggc ctg gtc ggt ctg acc gac aag acg cga gga tgc tgt tct cat    144
Ser Gly Leu Val Gly Leu Thr Asp Lys Thr Arg Gly Cys Cys Ser His
            35                  40                  45

cct gcc tgt aac gta gat cat cca gaa att tgt ggt tgaagacgct         190
Pro Ala Cys Asn Val Asp His Pro Glu Ile Cys Gly
        50                  55                  60

gatgctccag gaccctctga accacgacgt                                    220


<210> 131
<211> 60
<212> PRT
<213> Conus dalli

<400> 131
Met Phe Thr Val Phe Leu Leu Val Val Leu Ala Thr Thr Val Val Ser
1               5                   10                  15

Phe Thr Ser Asp Arg Ala Phe Arg Gly Arg Asn Ala Ala Ala Lys Glu
                20                  25                  30

Ser Gly Leu Val Gly Leu Thr Asp Lys Thr Arg Gly Cys Cys Ser His
        35                  40                  45

Pro Ala Cys Asn Val Asp His Pro Glu Ile Cys Gly
    50                  55                  60


<210> 132
<211> 208
<212> DNA
<213> Conus dalli

<220>
<221> CDS
<222> (1)..(177)

<400> 132
atg ttc acc gtg ttt ctg ttg gtt gtc ttg gca acc acc gtc gtt tcc    48
Met Phe Thr Val Phe Leu Leu Val Val Leu Ala Thr Thr Val Val Ser
  1               5                  10                  15

ttc act tca gat ggt gca tct gat gac agg aaa gcc gct gcg tct gac    96
Phe Thr Ser Asp Gly Ala Ser Asp Asp Arg Lys Ala Ala Ala Ser Asp
             20                  25                  30

ctg atc act ctg acc atc aag gga tgc tgt tct cgt cct ccc tgt atc    144
Leu Ile Thr Leu Thr Ile Lys Gly Cys Cys Ser Arg Pro Pro Cys Ile
        35                  40                  45

gcg aat aat cca gac ttg tgt ggt cga cga cgc tgatgctcca ggaccctctg 197
Ala Asn Asn Pro Asp Leu Cys Gly Arg Arg Arg
    50                  55

aaccacgacg t                                                       208


<210> 133
<211> 59
<212> PRT
<213> Conus dalli

<400> 133
Met Phe Thr Val Phe Leu Leu Val Val Leu Ala Thr Thr Val Val Ser
  1               5                  10                  15

Phe Thr Ser Asp Gly Ala Ser Asp Asp Arg Lys Ala Ala Ala Ser Asp
             20                  25                  30

Leu Ile Thr Leu Thr Ile Lys Gly Cys Cys Ser Arg Pro Pro Cys Ile
        35                  40                  45

Ala Asn Asn Pro Asp Leu Cys Gly Arg Arg Arg
    50                  55


<210> 134
<211> 223
<212> DNA
<213> Conus dalli

<220>
<221> CDS
<222> (1)..(192)

<400> 134
atg ttc acc gtg ttt ctg ttg gtt gtc ttg gca acc act gtc gtt tcc    48
Met Phe Thr Val Phe Leu Leu Val Val Leu Ala Thr Thr Val Val Ser
  1               5                  10                  15

tcc act tca ggt cgt cgt gca ttt cat ggc agg aat gcc gca gcc aaa     96
Ser Thr Ser Gly Arg Arg Ala Phe His Gly Arg Asn Ala Ala Ala Lys
            20                  25                  30

gcg tct gga ctg gtc ggt ctg act gac agg aga cca caa tgc tgt agt     144
Ala Ser Gly Leu Val Gly Leu Thr Asp Arg Arg Pro Gln Cys Cys Ser
        35                  40                  45

gat cct cgc tgt aac gta ggt cat cca gaa ctt tgt ggt gga aga cgc     192
Asp Pro Arg Cys Asn Val Gly His Pro Glu Leu Cys Gly Gly Arg Arg
    50                  55                  60

tgatgctcca ggaccctctg aaccacaacg t                                  223


<210> 135
<211> 64
<212> PRT
<213> Conus dalli

<400> 135
Met Phe Thr Val Phe Leu Leu Val Val Leu Ala Thr Thr Val Val Ser
1               5                   10                  15

Ser Thr Ser Gly Arg Arg Ala Phe His Gly Arg Asn Ala Ala Ala Lys
            20                  25                  30

Ala Ser Gly Leu Val Gly Leu Thr Asp Arg Arg Pro Gln Cys Cys Ser
        35                  40                  45

Asp Pro Arg Cys Asn Val Gly His Pro Glu Leu Cys Gly Gly Arg Arg
    50                  55                  60


<210> 136
<211> 220
<212> DNA
<213> Conus dalli

<220>
<221> CDS
<222> (1)..(189)

<400> 136
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

tcc act tca ggt cgt gca ttt cat ggc agg aat gcc gca gcc aaa gcg     96
Ser Thr Ser Gly Arg Ala Phe His Gly Arg Asn Ala Ala Ala Lys Ala
            20                  25                  30

tct ggc ctg gtc ggt ctg acc gac aag agg caa gta tgc tgt agt gat     144
Ser Gly Leu Val Gly Leu Thr Asp Lys Arg Gln Val Cys Cys Ser Asp
        35                  40                  45

cct cgc tgt aac gta ggt cat cca gaa att tgt ggt gga aga cgc         189
Pro Arg Cys Asn Val Gly His Pro Glu Ile Cys Gly Gly Arg Arg
    50                  55                  60

tgatgctcca ggaccctctg aaccacgacg t                                  220


<210> 137
<211> 63
<212> PRT
<213> Conus dalli

<400> 137
Met Phe Thr Val Phe Leu Leu Val Val Leu Ala Thr Thr Val Val Ser
1               5                   10                  15

Ser Thr Ser Gly Arg Ala Phe His Gly Arg Asn Ala Ala Ala Lys Ala
            20                  25                  30

Ser Gly Leu Val Gly Leu Thr Asp Lys Arg Gln Val Cys Cys Ser Asp
        35                  40                  45

Pro Arg Cys Asn Val Gly His Pro Glu Ile Cys Gly Gly Arg Arg
    50                  55                  60


<210> 138
<211> 208
<212> DNA
<213> Conus achatinus

<220>
<221> CDS
<222> (1)..(180)

<400> 138
atg ttc acc gtg ttt ctg ttg gtt gtc ttg aca acc act gtc gtt tcc      48
Met Phe Thr Val Phe Leu Leu Val Val Leu Thr Thr Thr Val Val Ser
1               5                   10                  15

ttc cct tca gat agt gca tct ggt ggc agg gat gac gag gcc aaa gac      96
Phe Pro Ser Asp Ser Ala Ser Gly Gly Arg Asp Asp Glu Ala Lys Asp
            20                  25                  30

gaa agg tct gac atg tac gaa ttg aaa cgg aat gga cgc tgt tgc cat      144
Glu Arg Ser Asp Met Tyr Glu Leu Lys Arg Asn Gly Arg Cys Cys His
        35                  40                  45

cct gcc tgt ggt ggc aaa tac gtt aaa tgt gga cgc tgatgctcca           190
Pro Ala Cys Gly Gly Lys Tyr Val Lys Cys Gly Arg
    50                  55                  60

ggaccctctc gaaccacg                                                  208


<210> 139
<211> 60
<212> PRT
<213> Conus achatinus

<400> 139
Met Phe Thr Val Phe Leu Leu Val Val Leu Thr Thr Thr Val Val Ser
1               5                   10                  15

Phe Pro Ser Asp Ser Ala Ser Gly Gly Arg Asp Asp Glu Ala Lys Asp
            20                  25                  30

Glu Arg Ser Asp Met Tyr Glu Leu Lys Arg Asn Gly Arg Cys Cys His
        35                  40                  45

Pro Ala Cys Gly Gly Lys Tyr Val Lys Cys Gly Arg
    50                  55                  60


<210> 140
<211> 211
<212> DNA
<213> Conus bullatus

<220>
<221> CDS

<222> (1)..(174)

<400> 140
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc tct aca gat gat gaa tct gat ggc tcg aat gaa gaa ccc agc gcc     96
Phe Ser Thr Asp Asp Glu Ser Asp Gly Ser Asn Glu Glu Pro Ser Ala
            20                  25                  30

gac cag act gcc agg tcc tca atg aac agg gcg cct gga tgc tgt aac     144
Asp Gln Thr Ala Arg Ser Ser Met Asn Arg Ala Pro Gly Cys Cys Asn
        35                  40                  45

aat cct gcc tgt gtg aag cac aga tgt gga tgacgctgat gctccaggac      194
Asn Pro Ala Cys Val Lys His Arg Cys Gly
    50                  55

cctctgaacc acgacgt                                                 211


<210> 141
<211> 58
<212> PRT
<213> Conus bullatus

<400> 141
Met Phe Thr Val Phe Leu Leu Val Val Leu Ala Thr Thr Val Val Ser
1               5                   10                  15

Phe Ser Thr Asp Asp Glu Ser Asp Gly Ser Asn Glu Glu Pro Ser Ala
            20                  25                  30

Asp Gln Thr Ala Arg Ser Ser Met Asn Arg Ala Pro Gly Cys Cys Asn
        35                  40                  45

Asn Pro Ala Cys Val Lys His Arg Cys Gly
    50                  55


<210> 142
<211> 214
<212> DNA
<213> Conus bullatus

<220>
<221> CDS
<222> (1)..(177)

<400> 142
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc tct aca gat gat gaa tct gat ggc tcg aat gaa gaa ccc agc gcc     96
Phe Ser Thr Asp Asp Glu Ser Asp Gly Ser Asn Glu Glu Pro Ser Ala
            20                  25                  30

gac cag gct gcc agg tcc gca atg aac agg ccg cct gga tgc tgt aac     144
Asp Gln Ala Ala Arg Ser Ala Met Asn Arg Pro Pro Gly Cys Cys Asn
        35                  40                  45

aat cct gcc tgt gtg aag cac aga tgt ggt gga tgacgctgat gctccaggac 197
Asn Pro Ala Cys Val Lys His Arg Cys Gly Gly
    50                  55

cctctgaacc acgacgt                                                 214

<210> 143
<211> 59
<212> PRT
<213> Conus bullatus

<400> 143
Met Phe Thr Val Phe Leu Leu Val Val Leu Ala Thr Thr Val Val Ser
1               5                   10                  15

Phe Ser Thr Asp Asp Glu Ser Asp Gly Ser Asn Glu Glu Pro Ser Ala
            20                  25                  30

Asp Gln Ala Ala Arg Ser Ala Met Asn Arg Pro Pro Gly Cys Cys Asn
        35                  40                  45

Asn Pro Ala Cys Val Lys His Arg Cys Gly Gly
    50                  55


<210> 144
<211> 208
<212> DNA
<213> Conus bullatus

<220>
<221> CDS
<222> (1)..(177)

<400> 144
atg ttc acc gtg ttt ctg ttg gtt gtc ttg gca acc act gtc gtt tcc    48
Met Phe Thr Val Phe Leu Leu Val Val Leu Ala Thr Thr Val Val Ser
1               5                   10                  15

ttc cct tca gat cgt gac tct gat ggc gcg gat gcc gaa gcc agt gac    96
Phe Pro Ser Asp Arg Asp Ser Asp Gly Ala Asp Ala Glu Ala Ser Asp
            20                  25                  30

gag cct gtt gag ttc gaa agg gac gag aat gga tgc tgt tgg aat cct    144
Glu Pro Val Glu Phe Glu Arg Asp Glu Asn Gly Cys Cys Trp Asn Pro
        35                  40                  45

tcc tgt ccg agg ccc aga tgt aca gga cga cgc taatgctcca ggaccctctg 197
Ser Cys Pro Arg Pro Arg Cys Thr Gly Arg Arg
    50                  55

aaccacgacg t                                                       208


<210> 145
<211> 59
<212> PRT
<213> Conus bullatus

<400> 145
Met Phe Thr Val Phe Leu Leu Val Val Leu Ala Thr Thr Val Val Ser
1               5                   10                  15

Phe Pro Ser Asp Arg Asp Ser Asp Gly Ala Asp Ala Glu Ala Ser Asp
            20                  25                  30

Glu Pro Val Glu Phe Glu Arg Asp Glu Asn Gly Cys Cys Trp Asn Pro
        35                  40                  45

Ser Cys Pro Arg Pro Arg Cys Thr Gly Arg Arg
    50                  55


<210> 146

<211> 211
<212> DNA
<213> Conus bullatus

<220>
<221> CDS
<222> (1)..(180)

<400> 146
atg ttc acc gtg ttt ctg ttg gtt gtc ttg aca acc act gtc gtt tcc     48
Met Phe Thr Val Phe Leu Leu Val Val Leu Thr Thr Thr Val Val Ser
1               5                   10                  15

ttc cct tca gat cgt gca tct gat ggc agg aat gcc gca gcc aac gac     96
Phe Pro Ser Asp Arg Ala Ser Asp Gly Arg Asn Ala Ala Ala Asn Asp
            20                  25                  30

aaa gcg tct gac gtg gtc acg ctg gtc ctc aag gga tgc tgt tcc acc     144
Lys Ala Ser Asp Val Val Thr Leu Val Leu Lys Gly Cys Cys Ser Thr
        35                  40                  45

cct ccc tgt gct gtg ctg tat tgt ggt aga aga cgc tgatgctcca          190
Pro Pro Cys Ala Val Leu Tyr Cys Gly Arg Arg Arg
    50                  55                  60

ggaccctctg aaccacgacg t                                             211


<210> 147
<211> 60
<212> PRT
<213> Conus bullatus

<400> 147
Met Phe Thr Val Phe Leu Leu Val Val Leu Thr Thr Thr Val Val Ser
1               5                   10                  15

Phe Pro Ser Asp Arg Ala Ser Asp Gly Arg Asn Ala Ala Ala Asn Asp
            20                  25                  30

Lys Ala Ser Asp Val Val Thr Leu Val Leu Lys Gly Cys Cys Ser Thr
        35                  40                  45

Pro Pro Cys Ala Val Leu Tyr Cys Gly Arg Arg Arg
    50                  55                  60


<210> 148
<211> 212
<212> DNA
<213> Conus distans

<220>
<221> CDS
<222> (1)..(171)

<400> 148
atg ttc acc gtg ttt ctg ttg gtt gtc ttc gca tcc tct gtc acc tta     48
Met Phe Thr Val Phe Leu Leu Val Val Phe Ala Ser Ser Val Thr Leu
1               5                   10                  15

gat cgt gca tct tat ggc agg tat gcc tca ccc gtc gac aga gcg tct     96
Asp Arg Ala Ser Tyr Gly Arg Tyr Ala Ser Pro Val Asp Arg Ala Ser
            20                  25                  30

gcc ctg atc gct cag gcc atc ctt cga gat tgc tgc tcc aat cct cct     144
Ala Leu Ile Ala Gln Ala Ile Leu Arg Asp Cys Cys Ser Asn Pro Pro
        35                  40                  45

tgt gcc cat aat aat cca gac tgt cgt taaagacgct gcttgctcca        191
Cys Ala His Asn Asn Pro Asp Cys Arg
     50                  55

ggaccctctg aaccacgacg t                                          212


<210> 149
<211> 57
<212> PRT
<213> Conus distans

<400> 149
Met Phe Thr Val Phe Leu Leu Val Val Phe Ala Ser Ser Val Thr Leu
1               5                   10                  15

Asp Arg Ala Ser Tyr Gly Arg Tyr Ala Ser Pro Val Asp Arg Ala Ser
            20                  25                  30

Ala Leu Ile Ala Gln Ala Ile Leu Arg Asp Cys Cys Ser Asn Pro Pro
        35                  40                  45

Cys Ala His Asn Asn Pro Asp Cys Arg
    50                  55


<210> 150
<211> 63
<212> DNA
<213> Conus textile

<220>
<221> CDS
<222> (1)..(60)

<400> 150
gga tgc tgt tct aat cct ccc tgt atc gcg aag aat cca cac atg tgt    48
Gly Cys Cys Ser Asn Pro Pro Cys Ile Ala Lys Asn Pro His Met Cys
1               5                   10                  15

ggt gga aga cgc tga                                                63
Gly Gly Arg Arg
            20


<210> 151
<211> 20
<212> PRT
<213> Conus textile

<400> 151
Gly Cys Cys Ser Asn Pro Pro Cys Ile Ala Lys Asn Pro His Met Cys
1               5                   10                  15

Gly Gly Arg Arg
            20


<210> 152
<211> 220
<212> DNA
<213> Conus consors

<220>
<221> CDS
<222> (1)..(189)

<400> 152
atg ttc acc gtg ttt ctg ttg gtt gtc ttg gca acc act gtc gtt tcc    48

Met Phe Thr Val Phe Leu Leu Val Val Leu Ala Thr Thr Val Val Ser
1               5                   10                  15

ttc cct tca gat cgt gca tct gat ggc agg aat gcc gca gcc aac gac     96
Phe Pro Ser Asp Arg Ala Ser Asp Gly Arg Asn Ala Ala Ala Asn Asp
            20                  25                  30

aaa gcg tct gac gtg atc acg ctg gcc ctc aag gga tgc tgt tcc aac     144
Lys Ala Ser Asp Val Ile Thr Leu Ala Leu Lys Gly Cys Cys Ser Asn
        35                  40                  45

cct gtc tgt cac ttg gag cat tca aac ctt tgt ggt aga aga cgc         189
Pro Val Cys His Leu Glu His Ser Asn Leu Cys Gly Arg Arg Arg
    50                  55                  60

tgatgctcca ggaccctctg aaccacgacg t                                  220

<210> 153
<211> 63
<212> PRT
<213> Conus consors

<400> 153
Met Phe Thr Val Phe Leu Leu Val Val Leu Ala Thr Thr Val Val Ser
1               5                   10                  15

Phe Pro Ser Asp Arg Ala Ser Asp Gly Arg Asn Ala Ala Ala Asn Asp
            20                  25                  30

Lys Ala Ser Asp Val Ile Thr Leu Ala Leu Lys Gly Cys Cys Ser Asn
        35                  40                  45

Pro Val Cys His Leu Glu His Ser Asn Leu Cys Gly Arg Arg Arg
    50                  55                  60

<210> 154
<211> 15
<212> PRT
<213> Conus musicus

<220>
<221> SITE
<222> (4)..(12)
<223> Xaa at residues 4, 11 and 12 is Tyr, nor-Tyr,
      mono-halo-Tyr, di-halo-Tyr, O-sulpho-Tyr,
      O-phospho-Tyr or nitro-Tyr. Xaa at residue 6 is
      Pro or hydroxy-Pro.

<220>
<221> SITE
<222> (9)..(15)
<223> Xaa at residues 9, 10 and 15 is Lys, N-methyl-Lys,
      N,N-dimethyl-Lys or N,N,N-trimethyl-Lys; Xaa at
      residue 14 is Trp (D or L) or halo-Trp.

<400> 154
Gly Cys Cys Xaa Asn Xaa Val Cys Xaa Xaa Xaa Xaa Cys Xaa Xaa
1               5                   10                  15

<210> 155
<211> 16
<212> PRT
<213> Conus purpurascens

<220>
<221> SITE

<222> (1)..(3)
<223> Xaa at residue 1 is Gln or pyro-Glu; Xaa at
      residue 2 is Glu or gamma-carboxy-Glu; Xaa at
      residues 3 and 9 is Pro or hydroxy-Pro.

<220>
<221> SITE
<222> (13)
<223> Xaa at residue 13 is Lys, N-methyl-Lys,
      N,N-dimethyl-Lys or N,N,N-trimethyl-Lys.

<400> 155
Xaa Xaa Xaa Gly Cys Cys Arg His Xaa Ala Cys Gly Xaa Asn Arg Cys
  1               5                   10                  15


<210> 156
<211> 13
<212> PRT
<213> Conus musicus

<220>
<221> SITE
<222> (5)..(11)
<223> Xaa at residues 5 and 11 is Pro or hydroxy-Pro.

<400> 156
Cys Cys Ala Asp Xaa Asp Cys Arg Phe Arg Xaa Gly Cys
  1               5                   10


<210> 157
<211> 17
<212> PRT
<213> Conus musicus

<220>
<221> SITE
<222> (4)..(13)
<223> Xaa at residues 4 and 13 is Tyr, nor-Tyr,
      mono-halo-Tyr, di-halo-Tyr, O-sulpho-Tyr,
      O-phospho-Tyr or nitro-Tyr; Xaa at residues 6 and
      10 is Pro or hydroxy-Pro.

<220>
<221> SITE
<222> (9)..(17)
<223> Xaa at residues 9 and 16 is Trp (D or L) or
      halo-Trp; Xaa at residues 11 and 17 is Lys,
      N-methyl-Lys, N,N-dimethyl-Lys or
      N,N,N-trimethyl-Lys.

<400> 157
Gly Cys Cys Xaa Asn Xaa Ser Cys Xaa Xaa Xaa Thr Xaa Cys Ser Xaa
  1               5                   10                  15

Xaa


<210> 158
<211> 13
<212> PRT
<213> Conus musicus

<220>
<221> SITE
<222> (5)..(8)
<223> Xaa at residue 5 is Pro or hydroxy-Pro; Xaa at

residue 8 is Lys, N-methyl-Lys, N,N-dimethyl-Lys
      or N,N,N-trimethyl-Lys.

<220>
<221> SITE
<222> (9)..(11)
<223> Xaa at residue 9 is Glu or gamma-carboxy-Glu; Xaa
      at residue 11 is Tyr, nor-Tyr, mono-halo-Tyr,
      di-halo-Tyr, O-sulpho-Tyr, O-phospho-Tyr or
      nitro-Tyr.

<400> 158
Cys Cys Ser Asn Xaa Thr Cys Xaa Xaa Thr Xaa Gly Cys
1               5                   10


<210> 159
<211> 13
<212> PRT
<213> Conus musicus

<220>
<221> SITE
<222> (5)..(11)
<223> Xaa at residues 5 and 11 is Pro or hydroxy-Pro;
      Xaa at residue 8 is Lys, N-methyl-Lys,
      N,N-dimethyl-Lys or N,N,N-trimethyl-Lys.

<400> 159
Cys Cys Ala Asn Xaa Ile Cys Xaa Asn Thr Xaa Gly Cys
1               5                   10


<210> 160
<211> 13
<212> PRT
<213> Conus musicus

<220>
<221> SITE
<222> (5)..(8)
<223> Xaa at residue 5 is Pro or hydroxy-Pro; Xaa at
      residue 8 is Lys, N-methyl-Lys, N,N-dimethyl-Lys
      or N,N,N-trimethyl-Lys.

<220>
<221> SITE
<222> (9)..(11)
<223> Xaa at residue 9 is Glu or gamma-carboxy-Glu; Xaa
      at residue 11 is Tyr, mono-halo-Tyr, di-halo-Tyr,
      O-sulpho-Tyr, O-phospho-Tyr or nitro-Tyr.

<400> 160
Cys Cys Asn Asn Xaa Thr Cys Xaa Xaa Thr Xaa Gly Cys
1               5                   10


<210> 161
<211> 13
<212> PRT
<213> Conus musicus

<220>
<221> SITE
<222> (5)..(8)
<223> Xaa at residue 5 is Pro or hydroxy-Pro; Xaa at
      residue 8 is Lys, N-methyl-Lys, N,N-dimethyl-Lys
      or N,N,N-trimethyl-Lys.

<220>
<221> SITE
<222> (9)..(11)
<223> Xaa at residue 9 is Glu or gamma-carboxy-Glu; Xaa
      at residue 11 is Tyr, nor-Tyr, mono-halo-Tyr,
      di-halo-Tyr, O-sulpho-Tyr, O-phospho-Tyr or
      nitro-Tyr.

<400> 161
Cys Cys Ser Asn Xaa Val Cys Xaa Xaa Thr Xaa Gly Cys
1               5                   10


<210> 162
<211> 17
<212> PRT
<213> Conus betulinus

<220>
<221> SITE
<222> (6)..(14)
<223> Xaa at residue 6 is Tyr, nor-Tyr, mono-halo-Tyr,
      di-halo-Tyr, O-sulpho-Tyr, O-phospho-Tyr or
      nitro-Tyr; Xaa at residues 7, 8 and 14 is Pro or
      hydroxy-Pro.

<220>
<221> SITE
<222> (15)
<223> Xaa at residue 15 is Lys, N-methyl-Lys,
      N,N-dimethyl-Lys or N,N,N-trimethyl-Lys.

<400> 162
Gly Gly Cys Cys Ser Xaa Xaa Xaa Cys Ile Ala Ser Asn Xaa Xaa Cys
1               5                   10                  15

Gly



<210> 163
<211> 15
<212> PRT
<213> Conus lividus

<220>
<221> SITE
<222> (6)..(13)
<223> Xaa at residues 6 and 13 is Pro or hydroxy-Pro.

<400> 163
Gly Cys Cys Ser His Xaa Val Cys Ser Ala Met Ser Xaa Ile Cys
1               5                   10                  15


<210> 164
<211> 15
<212> PRT
<213> Conus musicus

<220>
<221> SITE
<222> (4)..(12)
<223> Xaa at residues 4 and 12 is Lys, N-methyl-Lys,
      N,N-dimethyl-Lys or N,N,N-trimethyl-Lys; Xaa at
      residue 6 is Pro or hydroxy-Pro.

<220>
<221> SITE

<222> (7)..(14)
<223> Xaa at residues 7 and 14 is Tyr, mono-halo-Tyr,
      di-halo-Tyr, O-sulpho-Tyr, O-phospho-Tyr or
      nitro-Tyr.

<400> 164
Gly Cys Cys Xaa Asn Xaa Xaa Cys Gly Ala Ser Xaa Thr Xaa Cys
1               5                   10                  15


<210> 165
<211> 15
<212> PRT
<213> Conus omaria

<220>
<221> SITE
<222> (5)..(13)
<223> Xaa at residue 5 is Tyr, nor-Tyr, mono-halo-Tyr,
      di-halo-Tyr, O-sulpho-Tyr, O-phospho-Tyr or
      nitro-Tyr; Xaa at residues 6, 7 and 13 is Pro or
      hydroxy-Pro.

<400> 165
Gly Cys Cys Ser Xaa Xaa Xaa Cys Phe Ala Thr Asn Xaa Asp Cys
1               5                   10                  15


<210> 166
<211> 17
<212> PRT
<213> Conus radiatus

<220>
<221> SITE
<222> (6)..(14)
<223> Xaa at residue 6 is Tyr, nor-Tyr, mono-halo-Tyr,
      di-halo-Tyr, O-sulpho-Tyr, O-phospho-Tyr or
      nitro-Tyr; Xaa at residues 7, 8 and 14 is Pro or
      hydroxy-Pro.

<400> 166
Gly Gly Cys Cys Ser Xaa Xaa Xaa Cys Ile Ala Asn Asn Xaa Leu Cys
1               5                   10                  15

Ala



<210> 167
<211> 17
<212> PRT
<213> Conus radiatus

<220>
<221> SITE
<222> (6)..(14)
<223> Xaa at residue 6 is Tyr, nor-Tyr, mono-halo-Tyr,
      di-halo-Tyr, O-sulpho-Tyr, O-phospho-Tyr or
      nitro-Tyr; Xaa at residues 7, 8 and 14 is Pro or
      hydroxy-Pro.

<400> 167
Gly Gly Cys Cys Ser Xaa Xaa Xaa Cys Ile Ala Asn Asn Xaa Phe Cys
1               5                   10                  15

Ala

<210> 168
<211> 16
<212> PRT
<213> Conus virgo

<220>
<221> SITE
<222> (6)..(13)
<223> Xaa at residues 6, 7 and 13 is Pro or hydroxy-Pro.

<400> 168
Asp Cys Cys Ser Asn Xaa Xaa Cys Ser Gln Asn Asn Xaa Asp Cys Met
1               5                   10                  15


<210> 169
<211> 16
<212> PRT
<213> Conus virgo

<220>
<221> SITE
<222> (6)..(13)
<223> Xaa at residues 6, 7 and 13 is Pro or hydroxy-Pro.

<400> 169
Asp Cys Cys Ser Asn Xaa Xaa Cys Ala His Asn Asn Xaa Asp Cys Arg
1               5                   10                  15


<210> 170
<211> 20
<212> PRT
<213> Conus achatinus

<220>
<221> SITE
<222> (1)..(14)
<223> Xaa at residues 1, 11 and 14 is Glu or
      gamma-carboxy-Glu; Xaa at residue 6 is Pro or
      hydroxy-Pro.

<400> 170
Xaa Cys Cys Thr Asn Xaa Val Cys His Ala Xaa His Gln Xaa Leu Cys
1               5                   10                  15

Ala Arg Arg Arg
            20


<210> 171
<211> 16
<212> PRT
<213> Conus achatinus

<220>
<221> SITE
<222> (6)..(10)
<223> Xaa at residue 6 is Pro or hydroxy-Pro; Xaa at
      residue 10 is Glu or gamma-carboxy-Glu.

<400> 171
Gly Cys Cys Ser Asn Xaa Val Cys His Leu Xaa His Ser Asn Leu Cys
1               5                   10                  15


<210> 172
<211> 20

<212> PRT
<213> Conus achatinus

<220>
<221> SITE
<222> (1)..(14)
<223> Xaa at residues 1, 11 and 14 is Glu or
      gamma-carboxy-Glu; Xaa at residue 6 is Pro or
      hydroxy-Pro.

<400> 172
Xaa Cys Cys Thr Asn Xaa Val Cys His Val Xaa His Gln Xaa Leu Cys
1               5                   10                  15

Ala Arg Arg Arg
            20


<210> 173
<211> 17
<212> PRT
<213> Conus ammiralis

<220>
<221> SITE
<222> (1)..(15)
<223> Xaa at residue 1 is Gln or pyro-Glu; Xaa at
      residues 2 and 15 is Glu or gamma-carboxy-Glu; Xaa
      at residue 6 is Tyr, nor-Tyr, mono-halo-Tyr,
      di-halo-Tyr, O-sulpho-Tyr, O-phospho-Tyr or


<220>
<221> SITE
<222> (6)..(14)
<223> nitro-Tyr; Xaa at residues 7 and 14 is Pro or
      hydroxy-Pro.

<400> 173
Xaa Xaa Cys Cys Ser Xaa Xaa Ala Cys Asn Leu Asp His Xaa Xaa Leu
1               5                   10                  15

Cys



<210> 174
<211> 18
<212> PRT
<213> Conus ammiralis

<220>
<221> SITE
<222> (1)..(15)
<223> Xaa at residues 1, 7 and 14 is Pro or hydroxy-Pro;
      Xaa at residues 2 and 15 is Glu or
      gamma-carboxy-Glu.

<400> 174
Xaa Xaa Cys Cys Ser Asp Xaa Arg Cys Asn Ser Thr His Xaa Xaa Leu
1               5                   10                  15

Cys Gly



<210> 175
<211> 21
<212> PRT

<213> Conus arenatus

<220>
<221> SITE
<222> (7)..(12)
<223> Xaa at residues 7 and 8 is Pro or hydroxy-Pro; Xaa
      at residue 10 is Trp (D or L) or halo-Trp; Xaa at
      residues 11 and 12 is Lys, N-methyl-Lys,
      N,N-dimethyl-Lys or N,N,N-trimethyl-Lys.

<220>
<221> SITE
<222> (13)..(19)
<223> Xaa at residue 13 is Tyr, nor-Tyr, mono-halo-Tyr,
      di-halo-Tyr, O-sulpho-Tyr, O-phospho-Tyr or
      nitro-Tyr ; Xaa at residue 19 is Glu or
      gamma-carboxy-Glu.

<400> 175
Leu Asn Cys Cys Met Ile Xaa Xaa Cys Xaa Xaa Xaa Xaa Gly Asp Arg
1               5                   10                  15

Cys Ser Xaa Val Arg
            20


<210> 176
<211> 22
<212> PRT
<213> Conus arenatus

<220>
<221> SITE
<222> (9)..(20)
<223> Xaa at residue 9 is Pro or hydroxy-Pro; Xaa at
      residues 12 and 20 is Glu or gamma-carboxy-Glu;
      Xaa at residue 14 is Tyr, nor-Tyr, mono-halo-Tyr,
      di-halo-Tyr, O-sulpho-Tyr, O-phospho-Tyr or

<220>
<221> SITE
<222> (9)..(20)
<223> nitro-Tyr.

<400> 176
Ala Phe Gly Cys Cys Asp Leu Ile Xaa Cys Leu Xaa Arg Xaa Gly Asn
1               5                   10                  15

Arg Cys Asn Xaa Val His
            20


<210> 177
<211> 21
<212> PRT
<213> Conus arenatus

<220>
<221> SITE
<222> (8)..(16)
<223> Xaa at residue 8 is Pro or hydroxy-Pro; Xaa at
      residue 10 is Trp (D or L) or halo-Trp; Xaa at
      residues 12 and 16 is Lys, N-methyl-Lys,
      N,N-dimethyl-Lys or N,N,N-trimethyl-Lys.

<220>
<221> SITE
<222> (11)..(19)
<223> Xaa at residues 11 and 19 is Glu or

gamma-carboxy-Glu; Xaa at residue 13 is Tyr,
mono-halo-Tyr, di-halo-Tyr, O-sulpho-Tyr,
O-phospho-Tyr or nitro-Tyr.

<400> 177
Leu Gly Cys Cys Asn Val Thr Xaa Cys Xaa Xaa Xaa Xaa Gly Asp Xaa
1               5                   10                  15

Cys Asn Xaa Val Arg
            20


<210> 178
<211> 20
<212> PRT
<213> Conus arenatus

<220>
<221> SITE
<222> (2)..(14)
<223> Xaa at residue 2 is Glu or gamma-carboxy-Glu; Xaa
      at residues 7 and 14 is Pro or hydroxy-Pro.

<400> 178
Asp Xaa Cys Cys Ser Asn Xaa Ala Cys Arg Val Asn Asn Xaa His Val
1               5                   10                  15

Cys Arg Arg Arg
            20


<210> 179
<211> 21
<212> PRT
<213> Conus arenatus

<220>
<221> SITE
<222> (7)..(13)
<223> Xaa at residue 7 is Pro or hydroxy-Pro; Xaa at
      residue 10 is Trp (D or L) or halo-Trp; Xaa at
      residue 12 is Glu or gamma-carboxy-Glu; Xaa at
      residue 13 is Tyr, nor-Tyr, mono-halo-Tyr,

<220>
<221> SITE
<222> (13)..(19)
<223> di-halo-Tyr, O-sulpho-Tyr, O-phospho-Tyr or
      nitro-Tyr; Xaa at residues 14 and 19 is Lys,
      N-methyl-Lys, N,N-dimethyl-Lys or
      N,N,N-trimethyl-Lys.

<400> 179
Leu Asn Cys Cys Ser Ile Xaa Gly Cys Xaa Asn Xaa Xaa Xaa Asp Arg
1               5                   10                  15

Cys Ser Xaa Val Arg
            20


<210> 180
<211> 18
<212> PRT
<213> Conus aurisiacus

<220>
<221> SITE
<222> (7)..(14)
<223> Xaa at residues 7 and 14 is Pro or hydroxy-Pro;

Xaa at residue 10 is Tyr, mono-halo-Tyr,
      di-halo-Tyr, O-sulpho-Tyr, O-phospho-Tyr or
      nitro-Tyr.

<400> 180
Gly Gly Cys Cys Ser His Xaa Val Cys Xaa Phe Asn Asn Xaa Gln Met
1               5                   10                  15

Cys Arg

<210> 181
<211> 18
<212> PRT
<213> Conus aurisiacus

<220>
<221> SITE
<222> (7)..(14)
<223> Xaa at residues 7 and 14 is Pro or hydroxy-Pro.

<400> 181
Gly Gly Cys Cys Ser His Xaa Val Cys Asn Leu Asn Asn Xaa Gln Met
1               5                   10                  15

Cys Arg

<210> 182
<211> 17
<212> PRT
<213> Conus bandanus

<220>
<221> SITE
<222> (6)..(15)
<223> Xaa at residues 6 and 7 is Pro or hydroxy-Pro; Xaa
      at residues 9 and 15 is Tyr, mono-halo-Tyr,
      di-halo-Tyr, O-sulpho-Tyr, O-phospho-Tyr or
      nitro-Tyr.

<400> 182
Gly Cys Cys Ser His Xaa Xaa Cys Xaa Ala Asn Asn Gln Ala Xaa Cys
1               5                   10                  15

Asn

<210> 183
<211> 17
<212> PRT
<213> Conus betulinus

<220>
<221> SITE
<222> (7)..(15)
<223> Xaa at residues 7 and 14 is Pro and hydroxy-Pro;
      Xaa at residue 15 is Glu or gamma-carboxy-Glu.

<400> 183
Gly Gly Cys Cys Ser His Xaa Ala Cys Ser Val Thr His Xaa Xaa Leu
1               5                   10                  15

Cys

<210> 184
<211> 18
<212> PRT
<213> Conus betulinus

<220>
<221> SITE
<222> (6)..(12)
<223> Xaa at residue 6 is Tyr, nor-Tyr, mono-halo-Tyr,
      di-halo-Tyr, O-sulpho-Tyr, O-phospho-Tyr or
      nitro-Tyr; Xaa at residue 7 is Pro and
      hydroxy-Pro; Xaa at residue 12 is Glu or

<220>
<221> SITE
<222> (6)..(12)
<223> gamma-carboxy-Glu.

<400> 184
Gly Gly Cys Cys Ser Xaa Xaa Ala Cys Ser Val Xaa His Gln Asp Leu
  1               5                   10                  15

Cys Asp



<210> 185
<211> 25
<212> PRT
<213> Conus caracteristicus

<220>
<221> SITE
<222> (8)..(22)
<223> Xaa at residues 8 and 22 is Pro or hydroxy-Pro;
      Xaa at residue 10 is Trp (D or L) or halo-Trp; Xaa
      at residue 13 is Tyr, nor-Tyr, mono-halo-Tyr,
      di-halo-Tyr, O-sulpho-Tyr, O-phospho-Tyr or


<220>
<221> SITE
<222> (8)..(22)
<223> nitro-Tyr; Xaa at residues 15, 16 and 19 is Glu
      or gamma-carboxy-Glu.

<400> 185
Val Ser Cys Cys Val Val Arg Xaa Cys Xaa Ile Arg Xaa Gln Xaa Xaa
  1               5                   10                  15

Cys Leu Xaa Ala Asp Xaa Arg Thr Leu
            20                  25


<210> 186
<211> 21
<212> PRT
<213> Conus caracteristicus

<220>
<221> SITE
<222> (1)..(19)
<223> Xaa at residue 1 is Gln or pyro-Glu; Xaa at
      residue 7 is Pro or hydroxy-Pro; Xaa at residue 10
      is Trp (D or L) or halo-Trp; Xaa at residues 11
      and 19 is Glu or gamma-carboxy-Glu.

<220>

<221> SITE
<222> (12)..(16)
<223> Xaa at residues 12 and 16 is Lys, N-methyl-Lys,
      N,N-dimethyl-Lys or N,N,N-trimethyl-Lys; Xaa at
      residue 13 is Tyr, mono-halo-Tyr, di-halo-Tyr,
      O-sulpho-Tyr, O-phospho-Tyr or nitro-Tyr.

<400> 186
Xaa Asn Cys Cys Ser Ile Xaa Gly Cys Xaa Xaa Xaa Xaa Gly Asp Xaa
1               5                   10                  15

Cys Ser Xaa Val Arg
            20


<210> 187
<211> 16
<212> PRT
<213> Conus catus

<220>
<221> SITE
<222> (6)..(13)
<223> Xaa at residues 6 and 13 is Pro or hydroxy-Pro;
      Xaa at residue 11 is Glu or gamma-carboxy-Glu.

<400> 187
Gly Cys Cys Ser Asn Xaa Val Cys His Leu Xaa His Xaa Asn Ala Cys
1               5                   10                  15


<210> 188
<211> 17
<212> PRT
<213> Conus catus

<220>
<221> SITE
<222> (6)..(13)
<223> Xaa at residues 6 and 13 is Pro or hydroxy-Pro;
      Xaa at residue 9 is Tyr, nor-Tyr, mono-halo-Tyr,
      di-halo-Tyr, O-sulpho-Tyr, O-phospho-Tyr or
      nitro-Tyr.

<400> 188
Gly Cys Cys Ser Asn Xaa Ile Cys Xaa Phe Asn Asn Xaa Arg Ile Cys
1               5                   10                  15

Arg


<210> 189
<211> 17
<212> PRT
<213> Conus episcopatus

<220>
<221> SITE
<222> (1)..(14)
<223> Xaa at residues 1 and 14 is Glu or
      gamma-carboxy-Glu; Xaa at residues 6, 7 and 13 is
      Pro or hydroxy-Pro; Xaa at residue 10 is Trp (D or
      L) or halo-Trp.

<220>
<221> SITE
<222> (11)
<223> Xaa at residue 11 is Lys, N-methyl-Lys,

N,N-dimethyl-Lys or N,N,N-trimethyl-Lys.

<400> 189
Xaa Cys Cys Ser Gln Xaa Xaa Cys Arg Xaa Xaa His Xaa Xaa Leu Cys
1               5                   10                  15

Ser

<210> 190
<211> 16
<212> PRT
<213> Conus geographus

<220>
<221> SITE
<222> (6)
<223> Xaa at residue 6 is Pro or hydroxy-Pro.

<400> 190
Gly Cys Cys Ser His Xaa Ala Cys Ala Gly Asn Asn Gln His Ile Cys
1               5                   10                  15

<210> 191
<211> 18
<212> PRT
<213> Conus geographus

<220>
<221> SITE
<222> (6)..(13)
<223> Xaa at residues 6 and 13 is Pro or hydroxy-Pro.

<400> 191
Gly Cys Cys Ala Val Xaa Ser Cys Arg Leu Arg Asn Xaa Asp Leu Cys
1               5                   10                  15

Gly Gly

<210> 192
<211> 16
<212> PRT
<213> Conus imperialis

<220>
<221> NP_BIND
<222> (6)..(13)
<223> Xaa at residues 6 and 13 is Pro or hydroxy-Pro.

<400> 192
Gly Cys Cys Ser His Xaa Ala Cys Asn Val Asn Asn Xaa His Ile Cys
1               5                   10                  15

<210> 193
<211> 20
<212> PRT
<213> Conus lividus

<220>
<221> SITE
<222> (2)..(10)
<223> Xaa at residues 2, 7, 9 and 10 is Pro or
      hydroxy-Pro; Xaa at residues 3 and 4 is Glu or
      gamma-carboxy-Glu.

<400> 193
Thr Xaa Xaa Xaa Cys Cys Xaa Asn Xaa Xaa Cys Phe Ala Thr Asn Ser
1               5                   10                  15

Asp Ile Cys Gly
            20


<210> 194
<211> 17
<212> PRT
<213> Conus lividus

<220>
<221> SITE
<222> (7)..(12)
<223> Xaa at residue 7 is Pro or hydroxy-Pro; Xaa at
      residue 12 is Lys, N-methyl-Lys, N,N-dimethyl-Lys
      or N,N,N-trimethyl-Lys.

<400> 194
Asp Ala Cys Cys Ser Asp Xaa Arg Cys Ser Gly Xaa His Gln Asp Leu
1               5                   10                  15

Cys



<210> 195
<211> 17
<212> PRT
<213> Conus lividus

<220>
<221> SITE
<222> (1)..(7)
<223> Xaa at residue 1 is Glu or gamma-carboxy-Glu; Xaa
      at residue 7 is Pro or hydroxy-Pro.

<400> 195
Xaa Asp Cys Cys Ser Asp Xaa Arg Cys Ser Val Gly His Gln Asp Leu
1               5                   10                  15

Cys



<210> 196
<211> 16
<212> PRT
<213> Conus lividus

<220>
<221> SITE
<222> (6)
<223> Xaa at residue 6 is Pro or hydroxy-Pro.

<400> 196
Gly Cys Cys Ser His Xaa Ala Cys Ala Gly Ser Asn Ala His Ile Cys
1               5                   10                  15


<210> 197
<211> 17
<212> PRT
<213> Conus lividus

<220>

<221> SITE
<222> (1)..(7)
<223> Xaa at residue 1 is Glu or gamma-carboxy-Glu; Xaa
      at residue 7 is Pro or hydroxy-Pro.

<400> 197
Xaa Asp Cys Cys Ser Asp Xaa Arg Cys Ser Val Gly His Gln Asp Met
1               5                   10                  15

Cys

<210> 198
<211> 16
<212> PRT
<213> Conus lividus

<220>
<221> SITE
<222> (6)..(13)
<223> Xaa at residues 6 and 13 is Pro or hydroxy-Pro.

<400> 198
Gly Cys Cys Ser His Xaa Ala Cys Ala Gly Asn Asn Xaa His Ile Cys
1               5                   10                  15

<210> 199
<211> 17
<212> PRT
<213> Conus lividus

<220>
<221> SITE
<222> (6)..(14)
<223> Xaa at residues 6 and 13 is Pro or hydroxy-Pro;
      Xaa at residue 14 is Tyr, mono-halo-Tyr,
      di-halo-Tyr, O-sulpho-Tyr, O-phospho-Tyr or
      nitro-Tyr.

<400> 199
Gly Cys Cys Gly Asn Xaa Ser Cys Ser Ile His Ile Xaa Xaa Val Cys
1               5                   10                  15

Asn

<210> 200
<211> 21
<212> PRT
<213> Conus lividus

<220>
<221> SITE
<222> (4)..(5)
<223> Xaa at residues 4 and 5 is Glu or
      gamma-carboxy-Glu.

<400> 200
Thr Asp Ser Xaa Xaa Cys Cys Leu Asp Ser Arg Cys Ala Gly Gln His
1               5                   10                  15

Gln Asp Leu Cys Gly
            20

<210> 201

<211> 17
<212> PRT
<213> Conus marmoreus

<220>
<221> SITE
<222> (6)..(15)
<223> Xaa at residues 6 and 7 is Pro or hydroxy-Pro; Xaa
      at residues 9 and 15 is Tyr, mono-halo-Tyr,
      di-halo-Tyr, O-sulpho-Tyr, O-phospho-Tyr or
      nitro-Tyr.

<400> 201
Gly Cys Cys Ser Asn Xaa Xaa Cys Xaa Ala Asn Asn Gln Ala Xaa Cys
1               5                   10                  15

Asn



<210> 202
<211> 16
<212> PRT
<213> Conus marmoreus

<220>
<221> SITE
<222> (6)..(13)
<223> Xaa at residues 6 and 13 is Pro or hydroxy-Pro.

<400> 202
Gly Cys Cys Ser His Xaa Ala Cys Ser Val Asn Asn Xaa Asp Ile Cys
1               5                   10                  15


<210> 203
<211> 18
<212> PRT
<213> Conus musicus

<220>
<221> SITE
<222> (2)..(15)
<223> Xaa at residues 2 and 12 is Lys, N-methyl-Lys,
      N,N-dimethyl-Lys or N,N,N-trimethyl-Lys; Xaa at
      residue 14 is Pro or hydroxy-Pro.

<220>
<221> SITE
<222> (16)
<223> Xaa at residue 16 is Tyr, mono-halo-Tyr,
      di-halo-Tyr, O-sulpho-Tyr, O-phospho-Tyr or
      nitro-Tyr.

<400> 203
Gly Xaa Cys Cys Ile Asn Asp Ala Cys Arg Ser Xaa His Xaa Gln Xaa
1               5                   10                  15

Cys Ser



<210> 204
<211> 17
<212> PRT
<213> Conus musicus

<220>
<221> SITE

<222> (4)..(15)
<223> Xaa at residues 4 and 15 is Tyr, nor-Tyr,
      mono-halo-Tyr, di-halo-Tyr, O-sulpho-Tyr,
      O-phospho-Tyr or nitro-Tyr; Xaa at residue 13 is
      Pro or hydroxy-Pro.

<400> 204
Gly Cys Cys Xaa Asn Ile Ala Cys Arg Ile Asn Asn Xaa Arg Xaa Cys
1               5                   10                  15

Arg

<210> 205
<211> 17
<212> PRT
<213> Conus obscurus

<220>
<221> SITE
<222> (6)..(15)
<223> Xaa at residues 6 and 13 is Pro or hydroxy-Pro;
      Xaa at residues 12 and 15 is Tyr, nor-Tyr,
      mono-halo-Tyr, di-halo-Tyr, O-sulpho-Tyr,
      O-phospho-Tyr or nitro-Tyr.

<220>
<221> SITE
<222> (14)
<223> Xaa at residue 14 is Lys, N-methyl-Lys,
      N,N-dimethyl-Lys or N,N,N-trimethyl-Lys.

<400> 205
Gly Cys Cys Ser His Xaa Val Cys Arg Phe Asn Xaa Xaa Xaa Xaa Cys
1               5                   10                  15

Gly

<210> 206
<211> 18
<212> PRT
<213> Conus obscurus

<220>
<221> SITE
<222> (2)..(15)
<223> Xaa at residue 2 is Glu or gamma-carboxy-Glu; Xaa
      at residues 7, 8 and 14 is Pro or hydroxy-Pro; Xaa
      at residue 15 is Tyr, nor-Tyr, mono-halo-Tyr,
      di-halo-Tyr, O-sulpho-Tyr, O-phospho-Tyr or

<220>
<221> SITE
<222> (2)..(15)
<223> nitro-Tyr

<400> 206
Asp Xaa Cys Cys Ala Ser Xaa Xaa Cys Arg Leu Asn Asn Xaa Xaa Val
1               5                   10                  15

Cys His

<210> 207
<211> 19

<212> PRT
<213> Conus obscurus

<220>
<221> SITE
<222> (6)..(18)
<223> Xaa at residue 6 is Pro or hydroxy-Pro; Xaa at
      residue 9 is Trp (D or L) or halo-Trp; Xaa at
      residues 14 and 18 is Glu or gamma-carboxy-Glu.

<220>
<221> SITE
<222> (15)
<223> Xaa at residue 15 is Tyr, nor-Tyr, mono-halo-Tyr,
      di-halo-Tyr, O-sulpho-Tyr, O-phospho-Tyr or
      nitro-Tyr.

<400> 207
Gly Cys Cys Ser Asn Xaa Val Cys Xaa Gln Asn Asn Ala Xaa Xaa Cys
  1               5                  10                  15

Arg Xaa Ser



<210> 208
<211> 16
<212> PRT
<213> Conus obscurus

<220>
<221> SITE
<222> (6)..(15)
<223> Xaa at residues 6 and 7 is Pro or hydroxy-Pro; Xaa
      at residue 15 is Tyr, nor-Tyr, mono-halo-Tyr,
      di-halo-Tyr, O-sulpho-Tyr, O-phospho-Tyr or
      nitro-Tyr.

<400> 208
Gly Cys Cys Ser His Xaa Xaa Cys Ala Gln Asn Asn Gln Asp Xaa Cys
  1               5                  10                  15


<210> 209
<211> 19
<212> PRT
<213> Conus obscurus

<220>
<221> SITE
<222> (6)..(15)
<223> Xaa at residue 6 is Pro or hydroxy-Pro; Xaa at
      residues 14 and 18 is Glu or gamma-carboxy-Glu;
      Xaa at residue 15 is Tyr, nor-Tyr, mono-halo-Tyr,
      di-halo-Tyr, O-sulpho-Tyr, O-phospho-Tyr or

<220>
<221> SITE
<222> (6)..(15)
<223> nitro-Tyr.

<400> 209
Gly Cys Cys Ser His Xaa Ala Cys Ser Gly Asn Asn Arg Xaa Xaa Cys
  1               5                  10                  15

Arg Xaa Ser

<210> 210
<211> 18
<212> PRT
<213> Conus omaria

<220>
<221> SITE
<222> (2)..(15)
<223> Xaa at residues 2, 7 and 14 is Pro or hydroxy-Pro;
      Xaa at residue 6 is Tyr, nor-Tyr, mono-halo-Tyr,
      di-halo-Tyr, O-sulpho-Tyr, O-phospho-Tyr or
      nitro-Tyr; Xaa at residue 15 is Glu or

<220>
<221> SITE
<222> (2)..(15)
<223> gamma-carboxy-Glu

<400> 210
Asp Xaa Cys Cys Ser Xaa Xaa Asp Cys Gly Ala Asn His Xaa Xaa Ile
1               5                   10                  15

Cys Gly




<210> 211
<211> 17
<212> PRT
<213> Conus omaria

<220>
<221> SITE
<222> (1)..(14)
<223> Xaa at residues 1 and 14 is Glu or
      gamma-carboxy-Glu; Xaa at residues 6, 7 and 13 is
      Pro or hydroxy-Pro; Xaa at residue 10 is Trp (D or
      L) or halo-Trp.

<220>
<221> SITE
<222> (11)
<223> Xaa at residue 11 is Lys, N-methyl-Lys,
      N,N-dimethyl-Lys or N,N,N-trimethyl-Lys.

<400> 211
Xaa Cys Cys Ser Gln Xaa Xaa Cys Arg Xaa Xaa His Xaa Xaa Leu Cys
1               5                   10                  15

Ser




<210> 212
<211> 16
<212> PRT
<213> Conus omaria

<220>
<221> SITE
<222> (6)..(13)
<223> Xaa at residues 6 and 13 is Pro or hydroxy-Pro.

<400> 212
Gly Cys Cys Ser His Xaa Ala Cys Ala Gly Asn Asn Xaa His Ile Cys
1               5                   10                  15


<210> 213

<211> 16
<212> PRT
<213> Conus omaria

<220>
<221> SITE
<222> (6)..(15)
<223> Xaa at residues 6 and 13 is Pro or hydroxy-Pro;
      Xaa at residue 15 is Tyr, nor-Tyr, mono-halo-Tyr,
      di-halo-Tyr, O-sulpho-Tyr, O-phospho-Tyr or
      nitro-Tyr.

<400> 213
Gly Cys Cys Ser Asp Xaa Ser Cys Asn Val Asn Asn Xaa Asp Xaa Cys
1               5                   10                  15


<210> 214
<211> 18
<212> PRT
<213> Conus omaria

<220>
<221> SITE
<222> (1)..(7)
<223> Xaa at residues 1 and 2 is Glu or
      gamma-carboxy-Glu; Xaa at residue 7 is Pro or
      hydroxy-Pro.

<400> 214
Xaa Xaa Cys Cys Ser Asp Xaa Arg Cys Ser Val Gly His Gln Asp Met
1               5                   10                  15

Cys Arg


<210> 215
<211> 17
<212> PRT
<213> Conus purpurascens

<220>
<221> SITE
<222> (7)..(15)
<223> Xaa at residue 7 is Pro or hydroxy-Pro; Xaa at
      residue 15 is Glu or gamma-carboxy-Glu.

<400> 215
Gly Gly Cys Cys Ser Asn Xaa Ala Cys Leu Val Asn His Leu Xaa Met
1               5                   10                  15

Cys


<210> 216
<211> 18
<212> PRT
<213> Conus purpurascens

<220>
<221> SITE
<222> (3)..(15)
<223> Xaa at residues 3, 8 and 15 is Pro or hydroxy-Pro.

<400> 216
Arg Asp Xaa Cys Cys Phe Asn Xaa Ala Cys Asn Val Asn Asn Xaa Gln
1               5                   10                  15

Ile Cys



<210> 217
<211> 21
<212> PRT
<213> Conus purpurascens

<220>
<221> SITE
<222> (5)..(8)
<223> Xaa at residue 5 is Pro or hydroxy-Pro; Xaa at
      residue 8 is Trp (D or L) or halo-Trp.

<400> 217
Cys Cys Ser Asp Xaa Ser Cys Xaa Arg Leu His Ser Leu Ala Cys Thr
  1               5                  10                  15

Gly Ile Val Asn Arg
             20


<210> 218
<211> 16
<212> PRT
<213> Conus purpurascens

<220>
<221> SITE
<222> (5)
<223> Xaa at residue 5 is Pro or hydroxy-Pro.

<400> 218
Cys Cys Thr Asn Xaa Ala Cys Leu Val Asn Asn Ile Arg Phe Cys Gly
  1               5                  10                  15


<210> 219
<211> 18
<212> PRT
<213> Conus regius

<220>
<221> SITE
<222> (2)..(7)
<223> Xaa at residue 2 is Glu or gamma-carboxy-Glu; Xaa
      at residue 7 is Pro or hydroxy-Pro.

<400> 219
Asp Xaa Cys Cys Ser Asp Xaa Arg Cys His Gly Asn Asn Arg Asp His
  1               5                  10                  15

Cys Ala



<210> 220
<211> 17
<212> PRT
<213> Conus regius

<220>
<221> SITE
<222> (6)..(13)
<223> Xaa at residues 6 and 13 is Pro or hydroxy-Pro.

<400> 220

Asp Cys Cys Ser His Xaa Leu Cys Arg Leu Phe Val Xaa Gly Leu Cys
1               5                   10                  15

Ile

<210> 221
<211> 17
<212> PRT
<213> Conus regius

<220>
<221> SITE
<222> (6)..(13)
<223> Xaa at residues 6 and 13 is Pro or hydroxy-Pro;
      Xaa at residue 9 is Lys, N-methyl-Lys,
      N,N-dimethyl-Lys or N,N,N-trimethyl-Lys.

<220>
<221> SITE
<222> (12)
<223> Xaa at residue 12 is Tyr, nor-Tyr, mono-halo-Tyr,
      di-halo-Tyr, O-sulpho-Tyr, O-phospho-Tyr or
      nitro-Tyr.

<400> 221
Gly Cys Cys Ser His Xaa Val Cys Xaa Val Arg Xaa Xaa Asp Leu Cys
1               5                   10                  15

Arg

<210> 222
<211> 16
<212> PRT
<213> Conus regius

<220>
<221> SITE
<222> (6)..(13)
<223> Xaa at residues 6 and 13 is Pro or hydroxy-Pro.

<400> 222
Gly Cys Cys Ser His Xaa Ala Cys Asn Val Asn Asn Xaa His Ile Cys
1               5                   10                  15

<210> 223
<211> 16
<212> PRT
<213> Conus regius

<220>
<221> SITE
<222> (6)..(12)
<223> Xaa at residue 6 is Pro or hydroxy-Pro; Xaa at
      residue 12 is Tyr, nor-Tyr, nor-Tyr,
      mono-halo-Tyr, di-halo-Tyr, O-sulpho-Tyr,
      O-phospho-Tyr or nitro-Tyr.

<220>
<221> SITE
<222> (9)
<223> Xaa at residue 9 is Lys, N-methyl-Lys,
      N,N-dimethyl-Lys or N,N,N-trimethyl-Lys.

<400> 223

Gly Cys Cys Ser His Xaa Val Cys Xaa Val Arg Xaa Ser Asp Met Cys
1               5                   10                  15

<210> 224
<211> 17
<212> PRT
<213> Conus stercusmuscarum

<220>
<221> SITE
<222> (7)..(14)
<223> Xaa at residues 7 and 14 is Pro or hydroxy-Pro;
      Xaa at residue 10 is Lys, N-methyl-Lys,
      N,N-dimethyl-Lys or N,N,N-trimethyl-Lys.

<400> 224
Gly Gly Cys Cys Ser His Xaa Ala Cys Xaa Val His Phe Xaa His Ser
1               5                   10                  15

Cys

<210> 225
<211> 20
<212> PRT
<213> Conus stercusmuscarum

<220>
<221> SITE
<222> (6)..(14)
<223> Xaa at residues 6 and 13 is Pro or hydroxy-Pro;
      Xaa at residue 14 is Glu or gamma-carboxy-Glu.

<400> 225
Val Cys Cys Ser Asn Xaa Val Cys His Val Asp His Xaa Xaa Leu Cys
1               5                   10                  15

Arg Arg Arg Arg
            20

<210> 226
<211> 17
<212> PRT
<213> Conus striatus

<220>
<221> SITE
<222> (6)..(13)
<223> Xaa at residues 6 and 13 is Pro or hydroxy-Pro.

<400> 226
Gly Cys Cys Ser His Xaa Val Cys Asn Leu Ser Asn Xaa Gln Ile Cys
1               5                   10                  15

Arg

<210> 227
<211> 18
<212> PRT
<213> Conus textile

<220>
<221> SITE
<222> (1)..(15)

<223> Xaa at residue 1 is Gln or pyro-Glu; Xaa at
      residues 2 and 15 is Glu or gamma-carboxy-Glu; Xaa
      at residues 7 and 14 is Pro or hydroxy-Pro.

<400> 227
Xaa Xaa Cys Cys Ser His Xaa Ala Cys Asn Val Asp His Xaa Xaa Ile
1               5                   10                  15

Cys Arg




<210> 228
<211> 17
<212> PRT
<213> Conus tulipa

<220>
<221> SITE
<222> (6)
<223> Xaa at residue 6 is Pro or hydroxy-Pro.

<400> 228
Gly Cys Cys Ser Asn Xaa Ala Cys Leu Val Asn His Ile Arg Phe Cys
1               5                   10                  15

Gly




<210> 229
<211> 17
<212> PRT
<213> Conus virgo

<220>
<221> SITE
<222> (6)..(13)
<223> Xaa at residues 6 and 13 is Pro or hydroxy-Pro.

<400> 229
Asp Cys Cys Asp Asp Xaa Ala Cys Thr Val Asn Asn Xaa Gly Leu Cys
1               5                   10                  15

Thr




<210> 230
<211> 20
<212> PRT
<213> Conus textile

<220>
<221> SITE
<222> (6)..(13)
<223> Xaa at residues 6, 7 and 13 is Pro or hydroxy-Pro;
      Xaa at residue 11 is Lys, N-methyl-Lys,
      N,N-dimethyl-Lys or N,N,N-trimethyl-Lys.

<400> 230
Gly Cys Cys Ser Asn Xaa Xaa Cys Ile Ala Xaa Asn Xaa His Met Cys
1               5                   10                  15

Gly Gly Arg Arg
            20


<210> 231

<211> 18
<212> PRT
<213> Conus geographus

<220>
<221> SITE
<222> (5)..(9)
<223> Xaa at residue 5 is Pro or hydroxy-Pro; Xaa at
      residue 8 is Tyr, nor-Tyr, mono-halo-Tyr,
      di-halo-Tyr, O-sulpho-Tyr, O-phospho-Tyr or
      nitro-Tyr; Xaa at residue 9 is Glu or

<220>
<221> SITE
<222> (9)..(14)
<223> gamma-carboxy-Glu; Xaa at residues 10, 11, 12 and
      14 is Lys, N-methyl-Lys, N,N-dimethyl-Lys or
      N,N,N-trimethyl-Lys.

<400> 231
Cys Cys Thr Ile Xaa Ser Cys Xaa Xaa Xaa Xaa Xaa Ile Xaa Ala Cys
1               5                   10                  15

Val Phe




<210> 232
<211> 18
<212> PRT
<213> Conus regius

<220>
<221> SITE
<222> (6)..(16)
<223> Xaa at residues 6 and 16 is Pro or hydroxy-Pro;
      Xaa at residue 13 is Lys, N-methyl-Lys,
      N,N-dimethyl-Lys or N,N,N-trimethyl-Lys.

<400> 232
Gly Cys Cys Gly Asn Xaa Ala Cys Ser Gly Ser Ser Xaa Asp Ala Xaa
1               5                   10                  15

Ser Cys




<210> 233
<211> 108
<212> DNA
<213> Conus imperialis

<220>
<221> CDS
<222> (1)..(105)

<400> 233
tct gat gga aag agt gcc gcg gcc aaa gcc aaa ccg tct cac ctg acg     48
Ser Asp Gly Lys Ser Ala Ala Ala Lys Ala Lys Pro Ser His Leu Thr
1               5                   10                  15

gct cca ttc atc agg gac gaa tgc tgt tcc gat tct cgc tgt ggc aag     96
Ala Pro Phe Ile Arg Asp Glu Cys Cys Ser Asp Ser Arg Cys Gly Lys
            20                  25                  30

aac tgt ctt tga                                                     108
Asn Cys Leu
        35

<210> 234
<211> 35
<212> PRT
<213> Conus imperialis

<400> 234
Ser Asp Gly Lys Ser Ala Ala Ala Lys Ala Lys Pro Ser His Leu Thr
1               5                   10                  15

Ala Pro Phe Ile Arg Asp Glu Cys Cys Ser Asp Ser Arg Cys Gly Lys
            20                  25                  30

Asn Cys Leu
        35


<210> 235
<211> 108
<212> DNA
<213> Conus imperialis

<220>
<221> CDS
<222> (1)..(105)

<400> 235
ttt gat gga agg aat gcc cca gcc gac gac aaa gcg tct gac ctg atc     48
Phe Asp Gly Arg Asn Ala Pro Ala Asp Asp Lys Ala Ser Asp Leu Ile
1               5                   10                  15

gct caa atc gtc agg aga gca tgc tgt tcc gat cgt cgc tgt aga tgg     96
Ala Gln Ile Val Arg Arg Ala Cys Cys Ser Asp Arg Arg Cys Arg Trp
            20                  25                  30

agg tgt ggt tga                                                     108
Arg Cys Gly
        35


<210> 236
<211> 35
<212> PRT
<213> Conus imperialis

<400> 236
Phe Asp Gly Arg Asn Ala Pro Ala Asp Asp Lys Ala Ser Asp Leu Ile
1               5                   10                  15

Ala Gln Ile Val Arg Arg Ala Cys Cys Ser Asp Arg Arg Cys Arg Trp
            20                  25                  30

Arg Cys Gly
        35


<210> 237
<211> 145
<212> DNA
<213> Conus regius

<220>
<221> CDS
<222> (1)..(105)

<400> 237
tct gat gga agg aat gcc gca gcc gac gcc aga gcg tct ccc cgg atc     48
Ser Asp Gly Arg Asn Ala Ala Ala Asp Ala Arg Ala Ser Pro Arg Ile
1               5                   10                  15

gct ctt ttc ctc agg ttc aca tgc tgt agg aga ggt acc tgt tcc cag    96
Ala Leu Phe Leu Arg Phe Thr Cys Cys Arg Arg Gly Thr Cys Ser Gln
            20                  25                  30

cac tgt ggt tgaagacact gctgctccag gaccctctga accacgacgt          145
His Cys Gly
        35


<210> 238
<211> 35
<212> PRT
<213> Conus regius

<400> 238
Ser Asp Gly Arg Asn Ala Ala Ala Asp Ala Arg Ala Ser Pro Arg Ile
1               5                   10                  15

Ala Leu Phe Leu Arg Phe Thr Cys Cys Arg Arg Gly Thr Cys Ser Gln
            20                  25                  30

His Cys Gly
        35


<210> 239
<211> 145
<212> DNA
<213> Conus regius

<220>
<221> CDS
<222> (1)..(105)

<400> 239
tct aat gga agg aat gcc gca gcc gac gcc aaa gcg tct caa cgg atc    48
Ser Asn Gly Arg Asn Ala Ala Ala Asp Ala Lys Ala Ser Gln Arg Ile
1               5                   10                  15

gct cca ttc ctc agg gac tat tgc tgt agg aga cat gcc tgt acg ttg    96
Ala Pro Phe Leu Arg Asp Tyr Cys Cys Arg Arg His Ala Cys Thr Leu
            20                  25                  30

att tgt ggt tgaagacgct gctgctccag gaccctctga accacgacgt          145
Ile Cys Gly
        35


<210> 240
<211> 35
<212> PRT
<213> Conus regius

<400> 240
Ser Asn Gly Arg Asn Ala Ala Ala Asp Ala Lys Ala Ser Gln Arg Ile
1               5                   10                  15

Ala Pro Phe Leu Arg Asp Tyr Cys Cys Arg Arg His Ala Cys Thr Leu
            20                  25                  30

Ile Cys Gly
        35


<210> 241
<211> 145
<212> DNA
<213> Conus regius

<220>
<221> CDS
<222> (1)..(105)

<400> 241
tct aat gga agg aat gcc gca gcc gac gcc aaa gcg tct caa cgg atc      48
Ser Asn Gly Arg Asn Ala Ala Ala Asp Ala Lys Ala Ser Gln Arg Ile
1               5                   10                  15

gct cca ttc ctc agg gac tat tgc tgt agg aga cct ccc tgt acg ttg      96
Ala Pro Phe Leu Arg Asp Tyr Cys Cys Arg Arg Pro Pro Cys Thr Leu
            20                  25                  30

att tgt ggt tgaagacgct gctgctccag gaccctctga accacgacgt              145
Ile Cys Gly
        35


<210> 242
<211> 35
<212> PRT
<213> Conus regius

<400> 242
Ser Asn Gly Arg Asn Ala Ala Ala Asp Ala Lys Ala Ser Gln Arg Ile
1               5                   10                  15

Ala Pro Phe Leu Arg Asp Tyr Cys Cys Arg Arg Pro Pro Cys Thr Leu
            20                  25                  30

Ile Cys Gly
        35


<210> 243
<211> 136
<212> DNA
<213> Conus regius

<220>
<221> CDS
<222> (1)..(96)

<400> 243
tct aat aaa agg aag aat gcc gca atg ctt gac atg atc gct caa cac      48
Ser Asn Lys Arg Lys Asn Ala Ala Met Leu Asp Met Ile Ala Gln His
1               5                   10                  15

gcc ata agg ggt tgc tgt tcc gat cct cgc tgt aga tat aga tgt cgt      96
Ala Ile Arg Gly Cys Cys Ser Asp Pro Arg Cys Arg Tyr Arg Cys Arg
            20                  25                  30

tgaagacgct gctgctccag gaccctctga accacgacgt                          136


<210> 244
<211> 32
<212> PRT
<213> Conus regius

<400> 244
Ser Asn Lys Arg Lys Asn Ala Ala Met Leu Asp Met Ile Ala Gln His
1               5                   10                  15

Ala Ile Arg Gly Cys Cys Ser Asp Pro Arg Cys Arg Tyr Arg Cys Arg
            20                  25                  30

<210> 245
<211> 145
<212> DNA
<213> Conus regius

<220>
<221> CDS
<222> (1)..(105)

<400> 245
ttt aat gga agg agt gcc gca gcc gac caa aat gcg cct ggc ctg atc     48
Phe Asn Gly Arg Ser Ala Ala Ala Asp Gln Asn Ala Pro Gly Leu Ile
1               5                   10                  15

gct caa gtc gtc aga gga ggg tgc tgt tcc gat ccc cgc tgc gcc tgg     96
Ala Gln Val Val Arg Gly Gly Cys Cys Ser Asp Pro Arg Cys Ala Trp
            20                  25                  30

aga tgt ggt tgaagacgtt gctgctccag gaccctctga accacgacgt             145
Arg Cys Gly
        35


<210> 246
<211> 35
<212> PRT
<213> Conus regius

<400> 246
Phe Asn Gly Arg Ser Ala Ala Ala Asp Gln Asn Ala Pro Gly Leu Ile
1               5                   10                  15

Ala Gln Val Val Arg Gly Gly Cys Cys Ser Asp Pro Arg Cys Ala Trp
            20                  25                  30

Arg Cys Gly
        35


<210> 247
<211> 145
<212> DNA
<213> Conus regius

<220>
<221> CDS
<222> (1)..(105)

<400> 247
ttt gat gga agg aat gcc gca gcc gac gcc aaa gtg att aac acg gtc     48
Phe Asp Gly Arg Asn Ala Ala Ala Asp Ala Lys Val Ile Asn Thr Val
1               5                   10                  15

gct cga atc gcc tgg gat ata tgc tgt tcc gaa cct gac tgt aac cat     96
Ala Arg Ile Ala Trp Asp Ile Cys Cys Ser Glu Pro Asp Cys Asn His
            20                  25                  30

aaa tgt gtt tgaagacgct tctgctccag gaccctctga accacgacgt             145
Lys Cys Val
        35


<210> 248
<211> 35
<212> PRT
<213> Conus regius

<400> 248
Phe Asp Gly Arg Asn Ala Ala Ala Asp Ala Lys Val Ile Asn Thr Val

1                 5                   10                  15

Ala Arg Ile Ala Trp Asp Ile Cys Cys Ser Glu Pro Asp Cys Asn His
            20                  25                  30

Lys Cys Val
        35


<210> 249
<211> 136
<212> DNA
<213> Conus regius

<220>
<221> CDS
<222> (1)..(96)

<400> 249
tct aat aaa agg aag aat gcc gca atg ctt gac atg atc gct caa cac    48
Ser Asn Lys Arg Lys Asn Ala Ala Met Leu Asp Met Ile Ala Gln His
1               5                   10                  15

gcc ata agg ggt tgc tgt tcc gat cct cgc tgt aaa cat cag tgt ggt    96
Ala Ile Arg Gly Cys Cys Ser Asp Pro Arg Cys Lys His Gln Cys Gly
            20                  25                  30

tgaagacgct gctgctccag gaccctctga accacgacgt                        136


<210> 250
<211> 32
<212> PRT
<213> Conus regius

<400> 250
Ser Asn Lys Arg Lys Asn Ala Ala Met Leu Asp Met Ile Ala Gln His
1               5                   10                  15

Ala Ile Arg Gly Cys Cys Ser Asp Pro Arg Cys Lys His Gln Cys Gly
            20                  25                  30


<210> 251
<211> 136
<212> DNA
<213> Conus musicus

<220>
<221> CDS
<222> (1)..(105)

<400> 251
atc aag aat aca gca gcc agc aac aaa gcg tct agc ctg gtg gct ctt    48
Ile Lys Asn Thr Ala Ala Ser Asn Lys Ala Ser Ser Leu Val Ala Leu
1               5                   10                  15

gtt gtc agg gga tgc tgt tac aat cct gtc tgc aag aaa tat tat tgt    96
Val Val Arg Gly Cys Cys Tyr Asn Pro Val Cys Lys Lys Tyr Tyr Cys
            20                  25                  30

tgg aaa ggc tgatgctcca ggaccctctg aaccacgacg t                     136
Trp Lys Gly
        35


<210> 252
<211> 35
<212> PRT

<213> Conus musicus

<400> 252
Ile Lys Asn Thr Ala Ala Ser Asn Lys Ala Ser Ser Leu Val Ala Leu
1               5                   10                  15

Val Val Arg Gly Cys Cys Tyr Asn Pro Val Cys Lys Lys Tyr Tyr Cys
            20                  25                  30

Trp Lys Gly
        35


<210> 253
<211> 148
<212> DNA
<213> Conus purpurascens

<220>
<221> CDS
<222> (1)..(117)

<400> 253
tct gaa ggc agg aat gct gaa gcc atc gac aac gcc tta gac cag agg     48
Ser Glu Gly Arg Asn Ala Glu Ala Ile Asp Asn Ala Leu Asp Gln Arg
1               5                   10                  15

gat cca aag cga cag gag ccg ggg tgc tgt agg cat cct gcc tgt ggg     96
Asp Pro Lys Arg Gln Glu Pro Gly Cys Cys Arg His Pro Ala Cys Gly
            20                  25                  30

aag aac aga tgt gga aga cgc tgatgctcca ggaccctctg aaccacgacg t     148
Lys Asn Arg Cys Gly Arg Arg
        35


<210> 254
<211> 39
<212> PRT
<213> Conus purpurascens

<400> 254
Ser Glu Gly Arg Asn Ala Glu Ala Ile Asp Asn Ala Leu Asp Gln Arg
1               5                   10                  15

Asp Pro Lys Arg Gln Glu Pro Gly Cys Cys Arg His Pro Ala Cys Gly
            20                  25                  30

Lys Asn Arg Cys Gly Arg Arg
        35


<210> 255
<211> 156
<212> DNA
<213> Conus musicus

<220>
<221> CDS
<222> (1)..(102)

<400> 255
tct gat ggc agg aat att gca gtc gac gac aga tgg tct ttc tat acg     48
Ser Asp Gly Arg Asn Ile Ala Val Asp Asp Arg Trp Ser Phe Tyr Thr
1               5                   10                  15

ctc ttc cat gct act tgc tgt gcc gat cct gac tgt aga ttc cgg ccc     96
Leu Phe His Ala Thr Cys Cys Ala Asp Pro Asp Cys Arg Phe Arg Pro
            20                  25                  30

ggt tgt tgatctttgt tcttcaaaga cgctgctggc ccaggaccct ctgaaccacg     152
Gly Cys

acgt                                                                  156


<210> 256
<211> 34
<212> PRT
<213> Conus musicus

<400> 256
Ser Asp Gly Arg Asn Ile Ala Val Asp Asp Arg Trp Ser Phe Tyr Thr
1               5                   10                  15

Leu Phe His Ala Thr Cys Cys Ala Asp Pro Asp Cys Arg Phe Arg Pro
            20                  25                  30

Gly Cys



<210> 257
<211> 142
<212> DNA
<213> Conus musicus

<220>
<221> CDS
<222> (1)..(102)

<400> 257
atc aag aat act gca gcc agc aac aaa gcg cct agc ctg gtg gct att     48
Ile Lys Asn Thr Ala Ala Ser Asn Lys Ala Pro Ser Leu Val Ala Ile
1               5                   10                  15

gcc gtc agg gga tgc tgt tac aat cct tcc tgt tgg ccg aaa aca tat     96
Ala Val Arg Gly Cys Cys Tyr Asn Pro Ser Cys Trp Pro Lys Thr Tyr
            20                  25                  30

tgt agt tggaaaggct gatgctccag gaccctctga accacgacgt                 142
Cys Ser


<210> 258
<211> 34
<212> PRT
<213> Conus musicus

<400> 258
Ile Lys Asn Thr Ala Ala Ser Asn Lys Ala Pro Ser Leu Val Ala Ile
1               5                   10                  15

Ala Val Arg Gly Cys Cys Tyr Asn Pro Ser Cys Trp Pro Lys Thr Tyr
            20                  25                  30

Cys Ser



<210> 259
<211> 161
<212> DNA
<213> Conus musicus

<220>
<221> CDS
<222> (1)..(108)

<400> 259
tct gat agc agg aat gtc gca atc gag gac aga gtg tct gac ctg cac    48
Ser Asp Ser Arg Asn Val Ala Ile Glu Asp Arg Val Ser Asp Leu His
1               5                   10                  15

tct atg ttc ttc gat gtt tct tgc tgt agc aat cct acc tgt aaa gaa    96
Ser Met Phe Phe Asp Val Ser Cys Cys Ser Asn Pro Thr Cys Lys Glu
            20                  25                  30

acg tat ggt tgt tgatcgttgg ttttgaagac gctgatgctc caggaccctc        148
Thr Tyr Gly Cys
        35

tgaaccacga cgt                                                     161


<210> 260
<211> 36
<212> PRT
<213> Conus musicus

<400> 260
Ser Asp Ser Arg Asn Val Ala Ile Glu Asp Arg Val Ser Asp Leu His
1               5                   10                  15

Ser Met Phe Phe Asp Val Ser Cys Cys Ser Asn Pro Thr Cys Lys Glu
            20                  25                  30

Thr Tyr Gly Cys
        35


<210> 261
<211> 156
<212> DNA
<213> Conus musicus

<220>
<221> CDS
<222> (1)..(102)

<400> 261
tct gtt ggc agg aat att gca gtc gac gac aga ggg att ttc tct acg    48
Ser Val Gly Arg Asn Ile Ala Val Asp Asp Arg Gly Ile Phe Ser Thr
1               5                   10                  15

ctc ttc cat gct cat tgc tgt gcc aat ccc atc tgt aaa aac acg ccc    96
Leu Phe His Ala His Cys Cys Ala Asn Pro Ile Cys Lys Asn Thr Pro
            20                  25                  30

ggt tgt tgatctttgt tcttcaaaga cgctgctggc ccaggaccct ctgaaccacg     152
Gly Cys

acgt                                                               156


<210> 262
<211> 34
<212> PRT
<213> Conus musicus

<400> 262
Ser Val Gly Arg Asn Ile Ala Val Asp Asp Arg Gly Ile Phe Ser Thr
1               5                   10                  15

Leu Phe His Ala His Cys Cys Ala Asn Pro Ile Cys Lys Asn Thr Pro
            20                  25                  30

Gly Cys

<210> 263
<211> 161
<212> DNA
<213> Conus musicus

<220>
<221> CDS
<222> (1)..(108)

<400> 263
tcc gat ggc agg aat gtc gca atc gac gac aga gtg tct gac ctg cac     48
Ser Asp Gly Arg Asn Val Ala Ile Asp Asp Arg Val Ser Asp Leu His
1               5                   10                  15

tct atg ttc ttc gat att gct tgc tgt aac aat cct acc tgt aaa gaa     96
Ser Met Phe Phe Asp Ile Ala Cys Cys Asn Asn Pro Thr Cys Lys Glu
            20                  25                  30

acg tat ggt tgt tgatcgttgg ttttgaagac gctgatgctc caggaccctc        148
Thr Tyr Gly Cys
        35

tgaaccacga cgt                                                     161

<210> 264
<211> 36
<212> PRT
<213> Conus musicus

<400> 264
Ser Asp Gly Arg Asn Val Ala Ile Asp Asp Arg Val Ser Asp Leu His
1               5                   10                  15

Ser Met Phe Phe Asp Ile Ala Cys Cys Asn Asn Pro Thr Cys Lys Glu
            20                  25                  30

Thr Tyr Gly Cys
        35

<210> 265
<211> 161
<212> DNA
<213> Conus musicus

<220>
<221> CDS
<222> (1)..(108)

<400> 265
tct gat ggc agg aat gtc gca atc gag gac aga gtg tct gac ctg ctc     48
Ser Asp Gly Arg Asn Val Ala Ile Glu Asp Arg Val Ser Asp Leu Leu
1               5                   10                  15

tct atg ctc ttc gat gtt gct tgc tgt agc aat cct gtc tgt aaa gaa     96
Ser Met Leu Phe Asp Val Ala Cys Cys Ser Asn Pro Val Cys Lys Glu
            20                  25                  30

acg tat ggt tgt tgatcgttgg ttttgaagac gctgatgctc caggaccctc        148
Thr Tyr Gly Cys
        35

tgaaccacga cgt                                                     161

<210> 266
<211> 36
<212> PRT
<213> Conus musicus

<400> 266
Ser Asp Gly Arg Asn Val Ala Ile Glu Asp Arg Val Ser Asp Leu Leu
1               5                   10                  15

Ser Met Leu Phe Asp Val Ala Cys Cys Ser Asn Pro Val Cys Lys Glu
            20                  25                  30

Thr Tyr Gly Cys
        35


<210> 267
<211> 154
<212> DNA
<213> Conus betulinus

<220>
<221> CDS
<222> (1)..(123)

<400> 267
tat gat ggc agg aat gct gcc gcc gac gac aaa gct ttt gac ctg ctg      48
Tyr Asp Gly Arg Asn Ala Ala Ala Asp Asp Lys Ala Phe Asp Leu Leu
1               5                   10                  15

gct atg acc ata agg gga gga tgc tgt tcc tat cct ccc tgt atc gcg      96
Ala Met Thr Ile Arg Gly Gly Cys Cys Ser Tyr Pro Pro Cys Ile Ala
            20                  25                  30

agt aat cct aaa tgt ggt gga aga cgc tgatgctcca ggaccctctg           143
Ser Asn Pro Lys Cys Gly Gly Arg Arg
        35                  40

aaccacaacg t                                                         154


<210> 268
<211> 41
<212> PRT
<213> Conus betulinus

<400> 268
Tyr Asp Gly Arg Asn Ala Ala Ala Asp Asp Lys Ala Phe Asp Leu Leu
1               5                   10                  15

Ala Met Thr Ile Arg Gly Gly Cys Cys Ser Tyr Pro Pro Cys Ile Ala
            20                  25                  30

Ser Asn Pro Lys Cys Gly Gly Arg Arg
        35                  40


<210> 269
<211> 151
<212> DNA
<213> Conus lividus

<220>
<221> CDS
<222> (1)..(111)

<400> 269
ttt gat ggc agg aat gct gca ggc aac gcc aaa atg tcc gcc ctg atg      48

Phe Asp Gly Arg Asn Ala Ala Gly Asn Ala Lys Met Ser Ala Leu Met
1               5                   10                  15

gcc ctg acc atc agg gga tgc tgt tcc cat cct gtc tgt agc gcg atg     96
Ala Leu Thr Ile Arg Gly Cys Cys Ser His Pro Val Cys Ser Ala Met
            20                  25                  30

agt cca atc tgt ggc tgaagacgct gatgccccag gaccctctga accacgacgt     151
Ser Pro Ile Cys Gly
        35

<210> 270
<211> 37
<212> PRT
<213> Conus lividus

<400> 270
Phe Asp Gly Arg Asn Ala Ala Gly Asn Ala Lys Met Ser Ala Leu Met
1               5                   10                  15

Ala Leu Thr Ile Arg Gly Cys Cys Ser His Pro Val Cys Ser Ala Met
            20                  25                  30

Ser Pro Ile Cys Gly
        35

<210> 271
<211> 196
<212> DNA
<213> Conus musicus

<220>
<221> CDS
<222> (1)..(165)

<400> 271
atc aag aat gct gca gct gac gac aaa gca tct gac ctg ctc tct cag     48
Ile Lys Asn Ala Ala Ala Asp Asp Lys Ala Ser Asp Leu Leu Ser Gln
1               5                   10                  15

atc gtc agg aat gct gca tcc aat gac aaa ggg tct gac ctg atg act     96
Ile Val Arg Asn Ala Ala Ser Asn Asp Lys Gly Ser Asp Leu Met Thr
            20                  25                  30

ctt gcc ctc agg gga tgc tgt aaa aat cct tac tgt ggt gcg tcg aaa     144
Leu Ala Leu Arg Gly Cys Cys Lys Asn Pro Tyr Cys Gly Ala Ser Lys
        35                  40                  45

aca tat tgt ggt aga aga cgc tgatgctcca ggaccctctg aaccacgacg t     196
Thr Tyr Cys Gly Arg Arg Arg
    50                  55

<210> 272
<211> 55
<212> PRT
<213> Conus musicus

<400> 272
Ile Lys Asn Ala Ala Ala Asp Asp Lys Ala Ser Asp Leu Leu Ser Gln
1               5                   10                  15

Ile Val Arg Asn Ala Ala Ser Asn Asp Lys Gly Ser Asp Leu Met Thr
            20                  25                  30

Leu Ala Leu Arg Gly Cys Cys Lys Asn Pro Tyr Cys Gly Ala Ser Lys
        35                  40                  45

Thr Tyr Cys Gly Arg Arg Arg
    50                  55


<210> 273
<211> 139
<212> DNA
<213> Conus omaria

<220>
<221> CDS
<222> (40)..(108)

<400> 273
tctgatggca ggaatgccgc agcgtctgac ctgatggat ctg acc atc aag gga    54
                                           Leu Thr Ile Lys Gly
                                            1               5

tgc tgt tct tat cct ccc tgt ttc gcg act aat cca gac tgt ggt cga    102
Cys Cys Ser Tyr Pro Pro Cys Phe Ala Thr Asn Pro Asp Cys Gly Arg
                10                  15                  20

cga cgc tgatgctcca ggaccctctg aaccacgacg t                         139
Arg Arg


<210> 274
<211> 23
<212> PRT
<213> Conus omaria

<400> 274
Leu Thr Ile Lys Gly Cys Cys Ser Tyr Pro Pro Cys Phe Ala Thr Asn
 1               5                  10                  15

Pro Asp Cys Gly Arg Arg Arg
            20


<210> 275
<211> 126
<212> DNA
<213> Conus radiatus

<220>
<221> CDS
<222> (1)..(123)

<400> 275
ttt gat ggc agg aat gcc gca gcc gac tac aaa ggg tct gaa ttg ctc    48
Phe Asp Gly Arg Asn Ala Ala Ala Asp Tyr Lys Gly Ser Glu Leu Leu
 1               5                  10                  15

gct atg acc gtc agg gga gga tgc tgt tcc tat cct ccc tgt atc gca    96
Ala Met Thr Val Arg Gly Gly Cys Cys Ser Tyr Pro Pro Cys Ile Ala
            20                  25                  30

aat aat cct ctt tgt gct gga aga cgc tga                            126
Asn Asn Pro Leu Cys Ala Gly Arg Arg
        35                  40


<210> 276
<211> 41
<212> PRT
<213> Conus radiatus

<400> 276

Phe Asp Gly Arg Asn Ala Ala Ala Asp Tyr Lys Gly Ser Glu Leu Leu
1               5                   10                  15

Ala Met Thr Val Arg Gly Gly Cys Cys Ser Tyr Pro Pro Cys Ile Ala
            20                  25                  30

Asn Asn Pro Leu Cys Ala Gly Arg Arg
        35                  40


<210> 277
<211> 126
<212> DNA
<213> Conus radiatus

<220>
<221> CDS
<222> (1)..(123)

<400> 277
ttt gat ggc agg aat gcc gca gcc gac tac aaa ggg tct gaa ttg ctc     48
Phe Asp Gly Arg Asn Ala Ala Ala Asp Tyr Lys Gly Ser Glu Leu Leu
1               5                   10                  15

gct atg acc gtc agg gga gga tgc tgt tcc tat cct ccc tgt atc gca     96
Ala Met Thr Val Arg Gly Gly Cys Cys Ser Tyr Pro Pro Cys Ile Ala
            20                  25                  30

aat aat cct ttt tgt gct gga aga cgc tga                             126
Asn Asn Pro Phe Cys Ala Gly Arg Arg
        35                  40


<210> 278
<211> 41
<212> PRT
<213> Conus radiatus

<400> 278
Phe Asp Gly Arg Asn Ala Ala Ala Asp Tyr Lys Gly Ser Glu Leu Leu
1               5                   10                  15

Ala Met Thr Val Arg Gly Gly Cys Cys Ser Tyr Pro Pro Cys Ile Ala
            20                  25                  30

Asn Asn Pro Phe Cys Ala Gly Arg Arg
        35                  40


<210> 279
<211> 155
<212> DNA
<213> Conus virgo

<220>
<221> CDS
<222> (1)..(114)

<400> 279
tct tat gac agg tat gcc tcg ccc gtc gac aga gcg tct gcc ctg atc     48
Ser Tyr Asp Arg Tyr Ala Ser Pro Val Asp Arg Ala Ser Ala Leu Ile
1               5                   10                  15

gct cag gcc atc ctt cga gat tgc tgt tcc aat cct ccc tgt tcc caa     96
Ala Gln Ala Ile Leu Arg Asp Cys Cys Ser Asn Pro Pro Cys Ser Gln
            20                  25                  30

aat aat cca gac tgt atg taaagacgct gcttgctcca ggaccctctg            144
Asn Asn Pro Asp Cys Met

        35

aaccacgacg t                                                    155


<210> 280
<211> 38
<212> PRT
<213> Conus virgo

<400> 280
Ser Tyr Asp Arg Tyr Ala Ser Pro Val Asp Arg Ala Ser Ala Leu Ile
1               5                   10                  15

Ala Gln Ala Ile Leu Arg Asp Cys Cys Ser Asn Pro Pro Cys Ser Gln
            20                  25                  30

Asn Asn Pro Asp Cys Met
        35


<210> 281
<211> 155
<212> DNA
<213> Conus virgo

<220>
<221> CDS
<222> (1)..(114)

<400> 281
tct tat ggc agg tat gcc tca ccc gtc gac aga gcg tct gcc ctg atc    48
Ser Tyr Gly Arg Tyr Ala Ser Pro Val Asp Arg Ala Ser Ala Leu Ile
1               5                   10                  15

gct cag gcc atc ctt cga gat tgc tgc tcc aat cct cct tgt gcc cat    96
Ala Gln Ala Ile Leu Arg Asp Cys Cys Ser Asn Pro Pro Cys Ala His
            20                  25                  30

aat aat cca gac tgt cgt taaagacgct gcttgctcca ggaccctctg          144
Asn Asn Pro Asp Cys Arg
        35

aaccacgacg t                                                    155


<210> 282
<211> 38
<212> PRT
<213> Conus virgo

<400> 282
Ser Tyr Gly Arg Tyr Ala Ser Pro Val Asp Arg Ala Ser Ala Leu Ile
1               5                   10                  15

Ala Gln Ala Ile Leu Arg Asp Cys Cys Ser Asn Pro Pro Cys Ala His
            20                  25                  30

Asn Asn Pro Asp Cys Arg
        35


<210> 283
<211> 126
<212> DNA
<213> Conus achatinus

<220>
<221> CDS

<222> (1)..(123)

<400> 283
tct gat ggc agg aat gcc gca gcc aac gac aaa gcg tct ggc atg agc     48
Ser Asp Gly Arg Asn Ala Ala Ala Asn Asp Lys Ala Ser Gly Met Ser
1               5                   10                  15

gcg ctg gcc gtc aat gaa tgc tgt acc aac cct gtc tgt cac gcg gaa     96
Ala Leu Ala Val Asn Glu Cys Cys Thr Asn Pro Val Cys His Ala Glu
            20                  25                  30

cat caa gaa ctt tgt gct aga aga cgc tga                             126
His Gln Glu Leu Cys Ala Arg Arg Arg
        35                  40


<210> 284
<211> 41
<212> PRT
<213> Conus achatinus

<400> 284
Ser Asp Gly Arg Asn Ala Ala Ala Asn Asp Lys Ala Ser Gly Met Ser
1               5                   10                  15

Ala Leu Ala Val Asn Glu Cys Cys Thr Asn Pro Val Cys His Ala Glu
            20                  25                  30

His Gln Glu Leu Cys Ala Arg Arg Arg
        35                  40


<210> 285
<211> 126
<212> DNA
<213> Conus achatinus

<220>
<221> CDS
<222> (1)..(123)

<400> 285
tct gat ggc agg aat gcc gca gcc aac gac aaa gcg tct gac gtg atc     48
Ser Asp Gly Arg Asn Ala Ala Ala Asn Asp Lys Ala Ser Asp Val Ile
1               5                   10                  15

acg ctg gcc ctc aag gga tgc tgt tcc aac cct gtc tgt cac ttg gag     96
Thr Leu Ala Leu Lys Gly Cys Cys Ser Asn Pro Val Cys His Leu Glu
            20                  25                  30

cat tca aac ctt tgt ggt aga aga cgc tga                             126
His Ser Asn Leu Cys Gly Arg Arg Arg
        35                  40


<210> 286
<211> 41
<212> PRT
<213> Conus achatinus

<400> 286
Ser Asp Gly Arg Asn Ala Ala Ala Asn Asp Lys Ala Ser Asp Val Ile
1               5                   10                  15

Thr Leu Ala Leu Lys Gly Cys Cys Ser Asn Pro Val Cys His Leu Glu
            20                  25                  30

His Ser Asn Leu Cys Gly Arg Arg Arg
        35                  40

<210> 287
<211> 126
<212> DNA
<213> Conus achatinus

<220>
<221> CDS
<222> (1)..(123)

<400> 287
tct gat ggc agg aat gcc gca gcc aac gac aaa gcg tct ggc atg agc     48
Ser Asp Gly Arg Asn Ala Ala Ala Asn Asp Lys Ala Ser Gly Met Ser
1               5                   10                  15

gcg ctg gcc gtc aat gaa tgc tgt acc aac cct gtc tgt cac gtg gaa     96
Ala Leu Ala Val Asn Glu Cys Cys Thr Asn Pro Val Cys His Val Glu
            20                  25                  30

cat caa gaa ctt tgt gct aga aga cgc tga                             126
His Gln Glu Leu Cys Ala Arg Arg Arg
        35                  40


<210> 288
<211> 41
<212> PRT
<213> Conus achatinus

<400> 288
Ser Asp Gly Arg Asn Ala Ala Ala Asn Asp Lys Ala Ser Gly Met Ser
1               5                   10                  15

Ala Leu Ala Val Asn Glu Cys Cys Thr Asn Pro Val Cys His Val Glu
            20                  25                  30

His Gln Glu Leu Cys Ala Arg Arg Arg
        35                  40


<210> 289
<211> 220
<212> DNA
<213> Conus ammiralis

<220>
<221> CDS
<222> (1)..(180)

<400> 289
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca gat cgt gca ttt cgt ggc agg aat gcc gca gcc aaa gcg     96
Phe Thr Ser Asp Arg Ala Phe Arg Gly Arg Asn Ala Ala Ala Lys Ala
            20                  25                  30

tct ggc ctg gtc ggt ctg acc gac aag agg caa gaa tgc tgt tct tat     144
Ser Gly Leu Val Gly Leu Thr Asp Lys Arg Gln Glu Cys Cys Ser Tyr
        35                  40                  45

cct gcc tgt aac cta gat cat cca gaa ctt tgt ggt tgaagacgct          190
Pro Ala Cys Asn Leu Asp His Pro Glu Leu Cys Gly
    50                  55                  60

gatgctccag gaccctctga accacgacgt                                    220

<210> 290
<211> 60
<212> PRT
<213> Conus ammiralis

<400> 290
Met Phe Thr Val Phe Leu Leu Val Val Leu Ala Thr Thr Val Val Ser
1               5                   10                  15

Phe Thr Ser Asp Arg Ala Phe Arg Gly Arg Asn Ala Ala Ala Lys Ala
            20                  25                  30

Ser Gly Leu Val Gly Leu Thr Asp Lys Arg Gln Glu Cys Cys Ser Tyr
        35                  40                  45

Pro Ala Cys Asn Leu Asp His Pro Glu Leu Cys Gly
    50                  55                  60


<210> 291
<211> 223
<212> DNA
<213> Conus ammiralis

<220>
<221> CDS
<222> (1)..(192)

<400> 291
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

tcc act tca ggt cgt cgt gca ttt cgt ggc agg aat gcc gca gcc aaa     96
Ser Thr Ser Gly Arg Arg Ala Phe Arg Gly Arg Asn Ala Ala Ala Lys
            20                  25                  30

gcg tct gga ctg gtc ggt ctg act gac agg aga cca gaa tgc tgt agt     144
Ala Ser Gly Leu Val Gly Leu Thr Asp Arg Arg Pro Glu Cys Cys Ser
        35                  40                  45

gat cct cgc tgt aac tcg act cat cca gaa ctt tgt ggt gga aga cgc     192
Asp Pro Arg Cys Asn Ser Thr His Pro Glu Leu Cys Gly Gly Arg Arg
    50                  55                  60

tgatgctcca ggaccctctg aaccacgacg t                                  223


<210> 292
<211> 64
<212> PRT
<213> Conus ammiralis

<400> 292
Met Phe Thr Val Phe Leu Leu Val Val Leu Ala Thr Thr Val Val Ser
1               5                   10                  15

Ser Thr Ser Gly Arg Arg Ala Phe Arg Gly Arg Asn Ala Ala Ala Lys
            20                  25                  30

Ala Ser Gly Leu Val Gly Leu Thr Asp Arg Arg Pro Glu Cys Cys Ser
        35                  40                  45

Asp Pro Arg Cys Asn Ser Thr His Pro Glu Leu Cys Gly Gly Arg Arg
    50                  55                  60


<210> 293

<211> 151
<212> DNA
<213> Conus arenatus

<220>
<221> CDS
<222> (1)..(120)

<400> 293
tct gat ggc agg aat gcc gca gcc aac gcg ttt gac ctg atc gat ctg     48
Ser Asp Gly Arg Asn Ala Ala Ala Asn Ala Phe Asp Leu Ile Asp Leu
1               5                   10                  15

acc gcc agg cta aat tgc tgt atg att ccc ccc tgt tgg aag aaa tat     96
Thr Ala Arg Leu Asn Cys Cys Met Ile Pro Pro Cys Trp Lys Lys Tyr
            20                  25                  30

gga gac aga tgt agt gaa gta cgc tgatgctcca ggaccctctg aaccacgacg   150
Gly Asp Arg Cys Ser Glu Val Arg
        35                  40

t                                                                   151


<210> 294
<211> 40
<212> PRT
<213> Conus arenatus

<400> 294
Ser Asp Gly Arg Asn Ala Ala Ala Asn Ala Phe Asp Leu Ile Asp Leu
1               5                   10                  15

Thr Ala Arg Leu Asn Cys Cys Met Ile Pro Pro Cys Trp Lys Lys Tyr
            20                  25                  30

Gly Asp Arg Cys Ser Glu Val Arg
        35                  40


<210> 295
<211> 126
<212> DNA
<213> Conus arenatus

<220>
<221> CDS
<222> (1)..(93)

<400> 295
tct gat ggc agg aat gcc gca cgc aaa gcg ttt ggc tgc tgc gac tta     48
Ser Asp Gly Arg Asn Ala Ala Arg Lys Ala Phe Gly Cys Cys Asp Leu
1               5                   10                  15

ata ccc tgt ttg gag aga tat ggt aac aga tgt aat gaa gtg cac         93
Ile Pro Cys Leu Glu Arg Tyr Gly Asn Arg Cys Asn Glu Val His
            20                  25                  30

tgatgctcca ggaccctctg aaccacgcga cgt                               126


<210> 296
<211> 31
<212> PRT
<213> Conus arenatus

<400> 296
Ser Asp Gly Arg Asn Ala Ala Arg Lys Ala Phe Gly Cys Cys Asp Leu
1               5                   10                  15

Ile Pro Cys Leu Glu Arg Tyr Gly Asn Arg Cys Asn Glu Val His
            20                  25                  30

<210> 297
<211> 151
<212> DNA
<213> Conus arenatus

<220>
<221> CDS
<222> (1)..(120)

<400> 297
tct gat ggc agc aat gcc gca gcc aac gag ttt gac ctg atc gct ctg    48
Ser Asp Gly Ser Asn Ala Ala Ala Asn Glu Phe Asp Leu Ile Ala Leu
1               5                   10                  15

acc gcc agg cta ggt tgc tgt aac gtt aca ccc tgt tgg gag aaa tat    96
Thr Ala Arg Leu Gly Cys Cys Asn Val Thr Pro Cys Trp Glu Lys Tyr
            20                  25                  30

gga gac aaa tgt aat gaa gta cgc tgatgcttca ggaccctctg aaccacgacg   150
Gly Asp Lys Cys Asn Glu Val Arg
        35                  40

t                                                                  151

<210> 298
<211> 40
<212> PRT
<213> Conus arenatus

<400> 298
Ser Asp Gly Ser Asn Ala Ala Ala Asn Glu Phe Asp Leu Ile Ala Leu
1               5                   10                  15

Thr Ala Arg Leu Gly Cys Cys Asn Val Thr Pro Cys Trp Glu Lys Tyr
            20                  25                  30

Gly Asp Lys Cys Asn Glu Val Arg
        35                  40

<210> 299
<211> 148
<212> DNA
<213> Conus arenatus

<220>
<221> CDS
<222> (1)..(117)

<400> 299
tct gat ggc agg aat gtc gca gca aaa gcg ttt cac cgg atc ggc cgg    48
Ser Asp Gly Arg Asn Val Ala Ala Lys Ala Phe His Arg Ile Gly Arg
1               5                   10                  15

acc atc agg gat gaa tgc tgt tcc aat cct gcc tgt agg gtg aat aat    96
Thr Ile Arg Asp Glu Cys Cys Ser Asn Pro Ala Cys Arg Val Asn Asn
            20                  25                  30

cca cac gtt tgt aga cga cgc tgatgctcca ggaccctctg aaccacgacg t     148
Pro His Val Cys Arg Arg Arg
        35

<210> 300
<211> 39
<212> PRT
<213> Conus arenatus

<400> 300
Ser Asp Gly Arg Asn Val Ala Ala Lys Ala Phe His Arg Ile Gly Arg
1               5                   10                  15

Thr Ile Arg Asp Glu Cys Cys Ser Asn Pro Ala Cys Arg Val Asn Asn
            20                  25                  30

Pro His Val Cys Arg Arg Arg
        35


<210> 301
<211> 151
<212> DNA
<213> Conus arenatus

<220>
<221> CDS
<222> (1)..(120)

<400> 301
tct gat ggc agg aat gcc gca gcc aac gcg ttt gac ctg atg cct ctg     48
Ser Asp Gly Arg Asn Ala Ala Ala Asn Ala Phe Asp Leu Met Pro Leu
1               5                   10                  15

acc gcc agg cta aat tgc tgt agc att ccc ggc tgt tgg aac gaa tat     96
Thr Ala Arg Leu Asn Cys Cys Ser Ile Pro Gly Cys Trp Asn Glu Tyr
            20                  25                  30

aaa gac aga tgt agt aaa gta cgc tgatgctcca ggaccctctg aaccacgacg   150
Lys Asp Arg Cys Ser Lys Val Arg
        35                  40

t                                                                   151


<210> 302
<211> 40
<212> PRT
<213> Conus arenatus

<400> 302
Ser Asp Gly Arg Asn Ala Ala Ala Asn Ala Phe Asp Leu Met Pro Leu
1               5                   10                  15

Thr Ala Arg Leu Asn Cys Cys Ser Ile Pro Gly Cys Trp Asn Glu Tyr
            20                  25                  30

Lys Asp Arg Cys Ser Lys Val Arg
        35                  40


<210> 303
<211> 157
<212> DNA
<213> Conus aurisiacus

<220>
<221> CDS
<222> (52)..(126)

<400> 303
tctgatggca ggaatgccgc agccgacgac aaagcgtctg acctggtcgc t ctg gtc    57
                                                        Leu Val

                                                        1

gtc agg gga gga tgc tgt tcc cac cct gtc tgt tac ttt aat aat cca    105
Val Arg Gly Gly Cys Cys Ser His Pro Val Cys Tyr Phe Asn Asn Pro
        5                   10                  15

caa atg tgt cgt gga aga cgc tgatgctcca ggaccctctg aaccacgacg t    157
Gln Met Cys Arg Gly Arg Arg
    20                  25


<210> 304
<211> 25
<212> PRT
<213> Conus aurisiacus

<400> 304
Leu Val Val Arg Gly Gly Cys Cys Ser His Pro Val Cys Tyr Phe Asn
1               5                   10                  15

Asn Pro Gln Met Cys Arg Gly Arg Arg
            20                  25


<210> 305
<211> 157
<212> DNA
<213> Conus aurisiacus

<220>
<221> CDS
<222> (52)..(126)

<400> 305
tctgatggca ggaatgccgc agccgacgac aaagcgtctg acctggtcgc t ctg gcc   57
                                                         Leu Ala
                                                         1

gtc agg gga gga tgc tgt tcc cac cct gtc tgt aac ttg aat aat cca    105
Val Arg Gly Gly Cys Cys Ser His Pro Val Cys Asn Leu Asn Asn Pro
        5                   10                  15

caa atg tgt cgt gga aga cgc tgatgctcca ggaccctctg aaccacgacg t    157
Gln Met Cys Arg Gly Arg Arg
    20                  25


<210> 306
<211> 25
<212> PRT
<213> Conus aurisiacus

<400> 306
Leu Ala Val Arg Gly Gly Cys Cys Ser His Pro Val Cys Asn Leu Asn
1               5                   10                  15

Asn Pro Gln Met Cys Arg Gly Arg Arg
            20                  25


<210> 307
<211> 157
<212> DNA
<213> Conus betulinus

<220>
<221> CDS
<222> (1)..(117)

<400> 307
ttt cgt ggc agg aat ccc gca gcc aac gac aaa agg tct gac ctg gcc    48
Phe Arg Gly Arg Asn Pro Ala Ala Asn Asp Lys Arg Ser Asp Leu Ala
1               5                   10                  15

gct ctg agc gtc agg gga gga tgc tgt tcc cat cct gcc tgt agc gtg    96
Ala Leu Ser Val Arg Gly Gly Cys Cys Ser His Pro Ala Cys Ser Val
            20                  25                  30

act cat cca gag ctt tgt ggc tgaagacgct gatgccccag gaccctctga      147
Thr His Pro Glu Leu Cys Gly
        35

accacgacgt                                                        157


<210> 308
<211> 39
<212> PRT
<213> Conus betulinus

<400> 308
Phe Arg Gly Arg Asn Pro Ala Ala Asn Asp Lys Arg Ser Asp Leu Ala
1               5                   10                  15

Ala Leu Ser Val Arg Gly Gly Cys Cys Ser His Pro Ala Cys Ser Val
            20                  25                  30

Thr His Pro Glu Leu Cys Gly
        35


<210> 309
<211> 151
<212> DNA
<213> Conus betulinus

<220>
<221> CDS
<222> (1)..(120)

<400> 309
tct gat ggc ggg aat gcc gca gcc aaa gcg tct gac ctg atc gct cag    48
Ser Asp Gly Gly Asn Ala Ala Ala Lys Ala Ser Asp Leu Ile Ala Gln
1               5                   10                  15

acc atc agg gga gga tgc tgt tcc tat cct gcc tgt agc gtg gaa cat    96
Thr Ile Arg Gly Gly Cys Cys Ser Tyr Pro Ala Cys Ser Val Glu His
            20                  25                  30

caa gac ctt tgt gat gga aga cgc tgatgctcca ggaccctctg aaccacgacg  150
Gln Asp Leu Cys Asp Gly Arg Arg
        35                  40

t                                                                 151


<210> 310
<211> 40
<212> PRT
<213> Conus betulinus

<400> 310
Ser Asp Gly Gly Asn Ala Ala Ala Lys Ala Ser Asp Leu Ile Ala Gln
1               5                   10                  15

Thr Ile Arg Gly Gly Cys Cys Ser Tyr Pro Ala Cys Ser Val Glu His
            20                  25                  30

Gln Asp Leu Cys Asp Gly Arg Arg
        35                  40


<210> 311
<211> 114
<212> DNA
<213> Conus caracteristicus

<220>
<221> CDS
<222> (1)..(111)

<400> 311
tct tat ggc agg aat gcc gca gcc aaa gcg ttt gaa gtg agt tgc tgt     48
Ser Tyr Gly Arg Asn Ala Ala Ala Lys Ala Phe Glu Val Ser Cys Cys
1               5                   10                  15

gtc gtt cgc ccc tgt tgg att cgc tat caa gag gaa tgt ctt gaa gca     96
Val Val Arg Pro Cys Trp Ile Arg Tyr Gln Glu Glu Cys Leu Glu Ala
            20                  25                  30

gat ccc agg acc ctc tga                                             114
Asp Pro Arg Thr Leu
        35


<210> 312
<211> 37
<212> PRT
<213> Conus caracteristicus

<400> 312
Ser Tyr Gly Arg Asn Ala Ala Ala Lys Ala Phe Glu Val Ser Cys Cys
1               5                   10                  15

Val Val Arg Pro Cys Trp Ile Arg Tyr Gln Glu Glu Cys Leu Glu Ala
            20                  25                  30

Asp Pro Arg Thr Leu
        35


<210> 313
<211> 123
<212> DNA
<213> Conus caracteristicus

<220>
<221> CDS
<222> (1)..(120)

<400> 313
tct gat ggc agg aat gcc gca gcc aac gcc ctt gac ctg atc act ctg     48
Ser Asp Gly Arg Asn Ala Ala Ala Asn Ala Leu Asp Leu Ile Thr Leu
1               5                   10                  15

atc gcc agg caa aat tgc tgt agc att ccc ggc tgt tgg gag aaa tat     96
Ile Ala Arg Gln Asn Cys Cys Ser Ile Pro Gly Cys Trp Glu Lys Tyr
            20                  25                  30

gga gac aaa tgt agt gaa gta cgc tga                                 123
Gly Asp Lys Cys Ser Glu Val Arg
        35                  40


<210> 314
<211> 40
<212> PRT

<213> Conus caracteristicus

<400> 314
Ser Asp Gly Arg Asn Ala Ala Ala Asn Ala Leu Asp Leu Ile Thr Leu
1               5                   10                  15

Ile Ala Arg Gln Asn Cys Cys Ser Ile Pro Gly Cys Trp Glu Lys Tyr
            20                  25                  30

Gly Asp Lys Cys Ser Glu Val Arg
        35                  40


<210> 315
<211> 154
<212> DNA
<213> Conus catus

<220>
<221> CDS
<222> (1)..(123)

<400> 315
tct gat ggc agg aat gaa gca gcc aac gac gaa gcg tct gac gtg atc      48
Ser Asp Gly Arg Asn Glu Ala Ala Asn Asp Glu Ala Ser Asp Val Ile
1               5                   10                  15

gag ctg gcc ctc aag gga tgc tgt tcc aac cct gtc tgt cac ttg gag      96
Glu Leu Ala Leu Lys Gly Cys Cys Ser Asn Pro Val Cys His Leu Glu
            20                  25                  30

cat cca aac gct tgt ggt aga aga cgc tgatgctcca ggaccctctg           143
His Pro Asn Ala Cys Gly Arg Arg Arg
        35                  40

aaccacgacg t                                                         154


<210> 316
<211> 41
<212> PRT
<213> Conus catus

<400> 316
Ser Asp Gly Arg Asn Glu Ala Ala Asn Asp Glu Ala Ser Asp Val Ile
1               5                   10                  15

Glu Leu Ala Leu Lys Gly Cys Cys Ser Asn Pro Val Cys His Leu Glu
            20                  25                  30

His Pro Asn Ala Cys Gly Arg Arg Arg
        35                  40


<210> 317
<211> 154
<212> DNA
<213> Conus catus

<220>
<221> CDS
<222> (1)..(123)

<400> 317
tct gat ggc agg aat gcc gca gcc aac gac aaa gcg tct gac ctg gtc      48
Ser Asp Gly Arg Asn Ala Ala Ala Asn Asp Lys Ala Ser Asp Leu Val
1               5                   10                  15

gct ctg gcc gtc agg gga tgc tgt tcc aac cct atc tgt tac ttt aat      96

Ala Leu Ala Val Arg Gly Cys Cys Ser Asn Pro Ile Cys Tyr Phe Asn
            20                  25                  30

aat cca cga att tgt cgt gga aga cgc tgatgctcca ggaccctctg        143
Asn Pro Arg Ile Cys Arg Gly Arg Arg
        35                  40

aaccacgacg t                                                      154


<210> 318
<211> 41
<212> PRT
<213> Conus catus

<400> 318
Ser Asp Gly Arg Asn Ala Ala Ala Asn Asp Lys Ala Ser Asp Leu Val
1               5                   10                  15

Ala Leu Ala Val Arg Gly Cys Cys Ser Asn Pro Ile Cys Tyr Phe Asn
            20                  25                  30

Asn Pro Arg Ile Cys Arg Gly Arg Arg
        35                  40


<210> 319
<211> 111
<212> DNA
<213> Conus episcopatus

<220>
<221> CDS
<222> (1)..(108)

<400> 319
tct cat ggc agg aat gcc gca cgc aaa gcg tct gac ctg atc gct ctg   48
Ser His Gly Arg Asn Ala Ala Arg Lys Ala Ser Asp Leu Ile Ala Leu
1               5                   10                  15

acc gtc agg gaa tgc tgt tct cag cct ccc tgt cgc tgg aaa cat cca   96
Thr Val Arg Glu Cys Cys Ser Gln Pro Pro Cys Arg Trp Lys His Pro
            20                  25                  30

gaa ctt tgt agt tga                                               111
Glu Leu Cys Ser
        35


<210> 320
<211> 36
<212> PRT
<213> Conus episcopatus

<400> 320
Ser His Gly Arg Asn Ala Ala Arg Lys Ala Ser Asp Leu Ile Ala Leu
1               5                   10                  15

Thr Val Arg Glu Cys Cys Ser Gln Pro Pro Cys Arg Trp Lys His Pro
            20                  25                  30

Glu Leu Cys Ser
        35


<210> 321
<211> 151
<212> DNA
<213> Conus geographus

<220>
<221> CDS
<222> (1)..(120)

<400> 321
tct gat ggc agg aat gac gca gcc aaa gcg ttt gac ctg ata tct tcg     48
Ser Asp Gly Arg Asn Asp Ala Ala Lys Ala Phe Asp Leu Ile Ser Ser
1               5                   10                  15

acc gtc aag aaa gga tgc tgt tcc cat cct gcc tgt gcg ggg aat aat     96
Thr Val Lys Lys Gly Cys Cys Ser His Pro Ala Cys Ala Gly Asn Asn
            20                  25                  30

caa cat att tgt ggc cga aga cgc tgatgctcca ggaccctctg aaccacgacg    150
Gln His Ile Cys Gly Arg Arg Arg
        35                  40

t                                                                   151


<210> 322
<211> 40
<212> PRT
<213> Conus geographus

<400> 322
Ser Asp Gly Arg Asn Asp Ala Ala Lys Ala Phe Asp Leu Ile Ser Ser
1               5                   10                  15

Thr Val Lys Lys Gly Cys Cys Ser His Pro Ala Cys Ala Gly Asn Asn
            20                  25                  30

Gln His Ile Cys Gly Arg Arg Arg
        35                  40


<210> 323
<211> 154
<212> DNA
<213> Conus geographus

<220>
<221> CDS
<222> (1)..(123)

<400> 323
tct gat ggc agg aat gcc gca gcc aac gac caa gcg tct gac ctg atg     48
Ser Asp Gly Arg Asn Ala Ala Ala Asn Asp Gln Ala Ser Asp Leu Met
1               5                   10                  15

gct gcg acc gtc agg gga tgc tgt gcc gtt cct tcc tgt cgc ctc cgt     96
Ala Ala Thr Val Arg Gly Cys Cys Ala Val Pro Ser Cys Arg Leu Arg
            20                  25                  30

aat cca gac ctt tgt ggt gga gga cgc tgatgctcca ggaccctctg           143
Asn Pro Asp Leu Cys Gly Gly Gly Arg
        35                  40

aaccacgacg t                                                        154


<210> 324
<211> 41
<212> PRT
<213> Conus geographus

<400> 324
Ser Asp Gly Arg Asn Ala Ala Ala Asn Asp Gln Ala Ser Asp Leu Met

1                   5                   10                  15

Ala Ala Thr Val Arg Gly Cys Cys Ala Val Pro Ser Cys Arg Leu Arg
                20                  25                  30

Asn Pro Asp Leu Cys Gly Gly Gly Arg
            35                  40


<210> 325
<211> 120
<212> DNA
<213> Conus imperialis

<220>
<221> CDS
<222> (1)..(117)

<400> 325
ctt gat gaa agg aat gcc gca gcc gac gac aaa gcg tct gac ctg atc     48
Leu Asp Glu Arg Asn Ala Ala Ala Asp Asp Lys Ala Ser Asp Leu Ile
  1               5                  10                  15

gct caa atc gtc agg aga gga tgc tgt tcc cat cct gcc tgt aac gtg     96
Ala Gln Ile Val Arg Arg Gly Cys Cys Ser His Pro Ala Cys Asn Val
                20                  25                  30

aat aat cca cac att tgt ggt tga                                     120
Asn Asn Pro His Ile Cys Gly
            35


<210> 326
<211> 39
<212> PRT
<213> Conus imperialis

<400> 326
Leu Asp Glu Arg Asn Ala Ala Ala Asp Asp Lys Ala Ser Asp Leu Ile
  1               5                  10                  15

Ala Gln Ile Val Arg Arg Gly Cys Cys Ser His Pro Ala Cys Asn Val
                20                  25                  30

Asn Asn Pro His Ile Cys Gly
            35


<210> 327
<211> 142
<212> DNA
<213> Conus lividus

<220>
<221> CDS
<222> (1)..(111)

<400> 327
tct gat ggc agg aat act gca gcc aaa gtc aaa tat tct aag acg ccg     48
Ser Asp Gly Arg Asn Thr Ala Ala Lys Val Lys Tyr Ser Lys Thr Pro
  1               5                  10                  15

gag gaa tgc tgt ccc aat cct ccc tgt ttc gcg aca aat tcg gat att     96
Glu Glu Cys Cys Pro Asn Pro Pro Cys Phe Ala Thr Asn Ser Asp Ile
                20                  25                  30

tgt ggc gga aga cgc tgatgctcca ggaccctctg aaccacgacg t              142
Cys Gly Gly Arg Arg
            35

<210> 328
<211> 37
<212> PRT
<213> Conus lividus

<400> 328
Ser Asp Gly Arg Asn Thr Ala Ala Lys Val Lys Tyr Ser Lys Thr Pro
1               5                   10                  15

Glu Glu Cys Cys Pro Asn Pro Pro Cys Phe Ala Thr Asn Ser Asp Ile
            20                  25                  30

Cys Gly Gly Arg Arg
        35


<210> 329
<211> 157
<212> DNA
<213> Conus lividus

<220>
<221> CDS
<222> (1)..(117)

<400> 329
tct aat ggc agg aat gcc gca gcc aaa ttc aaa gcg cct gcc ctg atg      48
Ser Asn Gly Arg Asn Ala Ala Ala Lys Phe Lys Ala Pro Ala Leu Met
1               5                   10                  15

aag cgg acc gtc agg gat gct tgc tgt tca gac cct cgc tgt tcc ggg      96
Lys Arg Thr Val Arg Asp Ala Cys Cys Ser Asp Pro Arg Cys Ser Gly
            20                  25                  30

aaa cat caa gac ctg tgt ggc tgaagacgct gatgctccag gaccctctga        147
Lys His Gln Asp Leu Cys Gly
        35

accacgacgt                                                          157


<210> 330
<211> 39
<212> PRT
<213> Conus lividus

<400> 330
Ser Asn Gly Arg Asn Ala Ala Ala Lys Phe Lys Ala Pro Ala Leu Met
1               5                   10                  15

Lys Arg Thr Val Arg Asp Ala Cys Cys Ser Asp Pro Arg Cys Ser Gly
            20                  25                  30

Lys His Gln Asp Leu Cys Gly
        35


<210> 331
<211> 157
<212> DNA
<213> Conus lividus

<220>
<221> CDS
<222> (1)..(117)

<400> 331

tct aat ggc agg aat gcc gca gcc aaa ttc aaa gcg cct gcc ctg atg     48
Ser Asn Gly Arg Asn Ala Ala Ala Lys Phe Lys Ala Pro Ala Leu Met
1               5                   10                  15

gag ctg acc gtc agg gaa gat tgc tgt tca gac cct cgc tgt tcc gtg     96
Glu Leu Thr Val Arg Glu Asp Cys Cys Ser Asp Pro Arg Cys Ser Val
            20                  25                  30

gga cat caa gac ctg tgt ggc tgaagacgct gatgctccag gaccctctga       147
Gly His Gln Asp Leu Cys Gly
        35

accacgacgt                                                          157


<210> 332
<211> 39
<212> PRT
<213> Conus lividus

<400> 332
Ser Asn Gly Arg Asn Ala Ala Ala Lys Phe Lys Ala Pro Ala Leu Met
1               5                   10                  15

Glu Leu Thr Val Arg Glu Asp Cys Cys Ser Asp Pro Arg Cys Ser Val
            20                  25                  30

Gly His Gln Asp Leu Cys Gly
        35


<210> 333
<211> 157
<212> DNA
<213> Conus lividus

<220>
<221> CDS
<222> (1)..(126)

<400> 333
gca ttt gat ggc agg aat gct gca gcc agc gac aaa gcg tcc gag ctg     48
Ala Phe Asp Gly Arg Asn Ala Ala Ala Ser Asp Lys Ala Ser Glu Leu
1               5                   10                  15

atg gct ctg gcc gtc agg gga tgc tgt tcc cat cct gcc tgt gct ggg     96
Met Ala Leu Ala Val Arg Gly Cys Cys Ser His Pro Ala Cys Ala Gly
            20                  25                  30

agt aat gca cat atc tgt ggc aga aga cgc tgatgctcca ggaccctctg      146
Ser Asn Ala His Ile Cys Gly Arg Arg Arg
        35                  40

aaccacgacg t                                                        157


<210> 334
<211> 42
<212> PRT
<213> Conus lividus

<400> 334
Ala Phe Asp Gly Arg Asn Ala Ala Ala Ser Asp Lys Ala Ser Glu Leu
1               5                   10                  15

Met Ala Leu Ala Val Arg Gly Cys Cys Ser His Pro Ala Cys Ala Gly
            20                  25                  30

Ser Asn Ala His Ile Cys Gly Arg Arg Arg

35                    40

<210> 335
<211> 157
<212> DNA
<213> Conus lividus

<220>
<221> CDS
<222> (1)..(117)

<400> 335
tct aat ggc agg aat gcc gca gcc aaa ttc aaa gcg cct gcc ctg atg      48
Ser Asn Gly Arg Asn Ala Ala Ala Lys Phe Lys Ala Pro Ala Leu Met
1               5                   10                  15

aag ctg acc gtc agg gag gat tgc tgt tca gac cct cgc tgt tcc gtg      96
Lys Leu Thr Val Arg Glu Asp Cys Cys Ser Asp Pro Arg Cys Ser Val
            20                  25                  30

gga cat caa gac atg tgt ggc tgaagacgct gatgctccag gaccctctga        147
Gly His Gln Asp Met Cys Gly
        35

atcacgacgt                                                          157


<210> 336
<211> 39
<212> PRT
<213> Conus lividus

<400> 336
Ser Asn Gly Arg Asn Ala Ala Ala Lys Phe Lys Ala Pro Ala Leu Met
1               5                   10                  15

Lys Leu Thr Val Arg Glu Asp Cys Cys Ser Asp Pro Arg Cys Ser Val
            20                  25                  30

Gly His Gln Asp Met Cys Gly
        35


<210> 337
<211> 154
<212> DNA
<213> Conus lividus

<220>
<221> CDS
<222> (1)..(114)

<400> 337
ttt gaa tgc agg aat gct gca ggc aac gac aaa gcg act gac ctg atg      48
Phe Glu Cys Arg Asn Ala Ala Gly Asn Asp Lys Ala Thr Asp Leu Met
1               5                   10                  15

gct ctg act gtc agg gga tgc tgt tcc cat cct gcc tgt gct ggg aat      96
Ala Leu Thr Val Arg Gly Cys Cys Ser His Pro Ala Cys Ala Gly Asn
            20                  25                  30

aat cca cat atc tgc ggc tgaagacgct gatgctccag gaccctctga            144
Asn Pro His Ile Cys Gly
        35

accacgacgt                                                          154

<210> 338
<211> 38
<212> PRT
<213> Conus lividus

<400> 338
Phe Glu Cys Arg Asn Ala Ala Gly Asn Asp Lys Ala Thr Asp Leu Met
1               5                   10                  15

Ala Leu Thr Val Arg Gly Cys Cys Ser His Pro Ala Cys Ala Gly Asn
            20                  25                  30

Asn Pro His Ile Cys Gly
        35


<210> 339
<211> 154
<212> DNA
<213> Conus lividus

<220>
<221> CDS
<222> (1)..(114)

<400> 339
ttt gat ggc agg aac gcc gca gcc aac aac aaa gcg act gat ctg atg      48
Phe Asp Gly Arg Asn Ala Ala Ala Asn Asn Lys Ala Thr Asp Leu Met
1               5                   10                  15

gct ctg act gtc aga gga tgc tgt ggc aat cct tca tgt agc atc cat      96
Ala Leu Thr Val Arg Gly Cys Cys Gly Asn Pro Ser Cys Ser Ile His
            20                  25                  30

att cct tac gtt tgt aat tagagacact gatgctccag gaccctctga             144
Ile Pro Tyr Val Cys Asn
        35

accacgacgt                                                           154


<210> 340
<211> 38
<212> PRT
<213> Conus lividus

<400> 340
Phe Asp Gly Arg Asn Ala Ala Ala Asn Asn Lys Ala Thr Asp Leu Met
1               5                   10                  15

Ala Leu Thr Val Arg Gly Cys Cys Gly Asn Pro Ser Cys Ser Ile His
            20                  25                  30

Ile Pro Tyr Val Cys Asn
        35


<210> 341
<211> 157
<212> DNA
<213> Conus lividus

<220>
<221> CDS
<222> (1)..(126)

<400> 341
tct aat ggc agg aat gcc gca gcc aaa ttc aaa gcg cct gcc ctg atg      48
Ser Asn Gly Arg Asn Ala Ala Ala Lys Phe Lys Ala Pro Ala Leu Met

1                 5                   10                  15

aag cgg acc gac agc gaa gaa tgc tgt tta gac tct cgc tgt gcc ggg     96
Lys Arg Thr Asp Ser Glu Glu Cys Cys Leu Asp Ser Arg Cys Ala Gly
            20                  25                  30

caa cat caa gac ctg tgt ggc gga aga cgc tgatgctcca ggaccctctg      146
Gln His Gln Asp Leu Cys Gly Gly Arg Arg
        35                  40

aaccacgacg t                                                        157


<210> 342
<211> 42
<212> PRT
<213> Conus lividus

<400> 342
Ser Asn Gly Arg Asn Ala Ala Ala Lys Phe Lys Ala Pro Ala Leu Met
1               5                   10                  15

Lys Arg Thr Asp Ser Glu Glu Cys Cys Leu Asp Ser Arg Cys Ala Gly
            20                  25                  30

Gln His Gln Asp Leu Cys Gly Gly Arg Arg
        35                  40


<210> 343
<211> 126
<212> DNA
<213> Conus marmoreus

<220>
<221> CDS
<222> (1)..(123)

<400> 343
tct gat ggc agg aat gcc gca gcc aag gac aaa gcg tct gac ctg gtc     48
Ser Asp Gly Arg Asn Ala Ala Ala Lys Asp Lys Ala Ser Asp Leu Val
1               5                   10                  15

gct ctg acc gtc aag gga tgc tgt tct aat cct ccc tgt tac gcg aat     96
Ala Leu Thr Val Lys Gly Cys Cys Ser Asn Pro Pro Cys Tyr Ala Asn
            20                  25                  30

aat caa gcc tat tgt aat gga aga cgc tga                             126
Asn Gln Ala Tyr Cys Asn Gly Arg Arg
        35                  40


<210> 344
<211> 41
<212> PRT
<213> Conus marmoreus

<400> 344
Ser Asp Gly Arg Asn Ala Ala Ala Lys Asp Lys Ala Ser Asp Leu Val
1               5                   10                  15

Ala Leu Thr Val Lys Gly Cys Cys Ser Asn Pro Pro Cys Tyr Ala Asn
            20                  25                  30

Asn Gln Ala Tyr Cys Asn Gly Arg Arg
        35                  40


<210> 345

<211> 117
<212> DNA
<213> Conus marmoreus

<220>
<221> CDS
<222> (1)..(114)

<400> 345
tct gat ggc agg aat gcc gca gcc aag gac aaa gcg tct gac ctg gtc     48
Ser Asp Gly Arg Asn Ala Ala Ala Lys Asp Lys Ala Ser Asp Leu Val
1               5                   10                  15

gct ctg acc gtc aag gga tgc tgt tct cat cct gcc tgt agc gtg aat     96
Ala Leu Thr Val Lys Gly Cys Cys Ser His Pro Ala Cys Ser Val Asn
            20                  25                  30

aat cca gac att tgt ggt tga                                         117
Asn Pro Asp Ile Cys Gly
        35


<210> 346
<211> 38
<212> PRT
<213> Conus marmoreus

<400> 346
Ser Asp Gly Arg Asn Ala Ala Ala Lys Asp Lys Ala Ser Asp Leu Val
1               5                   10                  15

Ala Leu Thr Val Lys Gly Cys Cys Ser His Pro Ala Cys Ser Val Asn
            20                  25                  30

Asn Pro Asp Ile Cys Gly
        35


<210> 347
<211> 145
<212> DNA
<213> Conus musicus

<220>
<221> CDS
<222> (1)..(114)

<400> 347
tct gat ggc agg aat gct gca gcc aac aac aaa gtg gct ttg acc atg     48
Ser Asp Gly Arg Asn Ala Ala Ala Asn Asn Lys Val Ala Leu Thr Met
1               5                   10                  15

agg gga aaa tgc tgt atc aat gat gcg tgt cgc tcg aaa cat cca cag     96
Arg Gly Lys Cys Cys Ile Asn Asp Ala Cys Arg Ser Lys His Pro Gln
            20                  25                  30

tac tgt tct gga aga cgc tgatactcca ggaccctctg aaccacgacg t          145
Tyr Cys Ser Gly Arg Arg
        35


<210> 348
<211> 38
<212> PRT
<213> Conus musicus

<400> 348
Ser Asp Gly Arg Asn Ala Ala Ala Asn Asn Lys Val Ala Leu Thr Met
1               5                   10                  15

Arg Gly Lys Cys Cys Ile Asn Asp Ala Cys Arg Ser Lys His Pro Gln
            20                  25                  30

Tyr Cys Ser Gly Arg Arg
        35

<210> 349
<211> 154
<212> DNA
<213> Conus musicus

<220>
<221> CDS
<222> (1)..(123)

<400> 349
tct gat ggc agg aat gct gca gcc aac gac aaa gtg tct gac cag atg    48
Ser Asp Gly Arg Asn Ala Ala Ala Asn Asp Lys Val Ser Asp Gln Met
1               5                   10                  15

gct ctg gtt gtc agg gga tgc tgt tac aat att gcc tgt aga att aat    96
Ala Leu Val Val Arg Gly Cys Cys Tyr Asn Ile Ala Cys Arg Ile Asn
            20                  25                  30

aat cca cgg tac tgt cgt gga aaa cgc tgatgttcca ggaccctctg          143
Asn Pro Arg Tyr Cys Arg Gly Lys Arg
        35                  40

aaccacgacg t                                                       154

<210> 350
<211> 41
<212> PRT
<213> Conus musicus

<400> 350
Ser Asp Gly Arg Asn Ala Ala Ala Asn Asp Lys Val Ser Asp Gln Met
1               5                   10                  15

Ala Leu Val Val Arg Gly Cys Cys Tyr Asn Ile Ala Cys Arg Ile Asn
            20                  25                  30

Asn Pro Arg Tyr Cys Arg Gly Lys Arg
        35                  40

<210> 351
<211> 154
<212> DNA
<213> Conus obscurus

<220>
<221> CDS
<222> (52)..(123)

<400> 351
tctgaaggca ggaatgccgc agccaacgac aaagcgtctg acctgatggc t ctg aac    57
                                                         Leu Asn
                                                         1

gtc agg gga tgc tgt tcc cat cct gtc tgt cgc ttc aat tat cca aaa    105
Val Arg Gly Cys Cys Ser His Pro Val Cys Arg Phe Asn Tyr Pro Lys
        5                   10                  15

tat tgt ggt gga aga cgc tgatggtcca ggaccctctg aaccacgacg t         154
Tyr Cys Gly Gly Arg Arg

    20

<210> 352
<211> 24
<212> PRT
<213> Conus obscurus

<400> 352
Leu Asn Val Arg Gly Cys Cys Ser His Pro Val Cys Arg Phe Asn Tyr
1               5                   10                  15

Pro Lys Tyr Cys Gly Gly Arg Arg
            20

<210> 353
<211> 151
<212> DNA
<213> Conus obscurus

<220>
<221> CDS
<222> (46)..(111)

<400> 353
tctgatggcg ggaatgccgc agcaaaagcg tttgatctaa tcact ctg gcc ctc agg 57
                                                  Leu Ala Leu Arg
                                                  1

gat gaa tgc tgt gcc agt cct ccc tgt cgt ttg aat aat cca tac gta   105
Asp Glu Cys Cys Ala Ser Pro Pro Cys Arg Leu Asn Asn Pro Tyr Val
5                   10                  15                  20

tgt cat tgacgacgct gatgctccag gaccctctga accacgacgt               151
Cys His

<210> 354
<211> 22
<212> PRT
<213> Conus obscurus

<400> 354
Leu Ala Leu Arg Asp Glu Cys Cys Ala Ser Pro Pro Cys Arg Leu Asn
1               5                   10                  15

Asn Pro Tyr Val Cys His
            20

<210> 355
<211> 217
<212> DNA
<213> Conus obscurus

<220>
<221> CDS
<222> (1)..(186)

<400> 355
atg ttc acc gtg ttt ctg ttg gtt gtc ttg gca acc acc gtc gtt tcc   48
Met Phe Thr Val Phe Leu Leu Val Val Leu Ala Thr Thr Val Val Ser
1               5                   10                  15

ccc act tca gat cgt gca tct gat agg agg aat gcc gca gcc aaa gcg   96
Pro Thr Ser Asp Arg Ala Ser Asp Arg Arg Asn Ala Ala Ala Lys Ala
            20                  25                  30

ttt gac ctg aga tat tcg acc gcc aag aga gga tgc tgt tcc aat cct    144
Phe Asp Leu Arg Tyr Ser Thr Ala Lys Arg Gly Cys Cys Ser Asn Pro
        35                  40                  45

gtc tgt tgg cag aat aat gca gaa tac tgt cgt gaa agt ggc             186
Val Cys Trp Gln Asn Asn Ala Glu Tyr Cys Arg Glu Ser Gly
    50                  55                  60

taatgctcca ggaccctctg aaccacgacg t                                  217


<210> 356
<211> 62
<212> PRT
<213> Conus obscurus

<400> 356
Met Phe Thr Val Phe Leu Leu Val Val Leu Ala Thr Thr Val Val Ser
1               5                   10                  15

Pro Thr Ser Asp Arg Ala Ser Asp Arg Arg Asn Ala Ala Ala Lys Ala
            20                  25                  30

Phe Asp Leu Arg Tyr Ser Thr Ala Lys Arg Gly Cys Cys Ser Asn Pro
        35                  40                  45

Val Cys Trp Gln Asn Asn Ala Glu Tyr Cys Arg Glu Ser Gly
    50                  55                  60


<210> 357
<211> 208
<212> DNA
<213> Conus obscurus

<220>
<221> CDS
<222> (1)..(168)

<400> 357
atg ttc acc gtg ttt ctg ttg gtt gtc ttg gca acc acc gtc gtt tcc    48
Met Phe Thr Val Phe Leu Leu Val Val Leu Ala Thr Thr Val Val Ser
1               5                   10                  15

ttc act tca gat cgt gca tct gat ggc ggg aat gtc gca gcg tct cac    96
Phe Thr Ser Asp Arg Ala Ser Asp Gly Gly Asn Val Ala Ala Ser His
            20                  25                  30

ctg atc gct ctg acc atc aag gga tgc tgt tct cac cct ccc tgt gcc    144
Leu Ile Ala Leu Thr Ile Lys Gly Cys Cys Ser His Pro Pro Cys Ala
        35                  40                  45

cag aat aat caa gac tat tgt ggt tgacgacgct gatgctccag gaccctctga   198
Gln Asn Asn Gln Asp Tyr Cys Gly
    50                  55

accacgacgt                                                          208


<210> 358
<211> 56
<212> PRT
<213> Conus obscurus

<400> 358
Met Phe Thr Val Phe Leu Leu Val Val Leu Ala Thr Thr Val Val Ser
1               5                   10                  15

Phe Thr Ser Asp Arg Ala Ser Asp Gly Gly Asn Val Ala Ala Ser His

20                  25                  30

Leu Ile Ala Leu Thr Ile Lys Gly Cys Cys Ser His Pro Pro Cys Ala
        35                  40                  45

Gln Asn Asn Gln Asp Tyr Cys Gly
    50                  55


<210> 359
<211> 217
<212> DNA
<213> Conus obscurus

<220>
<221> CDS
<222> (1)..(186)

<400> 359
atg ttc acc gtg ttt ctg ttg gtt gtc tta tca acc acc gtc gtt tcc    48
Met Phe Thr Val Phe Leu Leu Val Val Leu Ser Thr Thr Val Val Ser
1               5                   10                  15

tcc act tca gat cgt gca tct gat agg agg aat gcc gca gcc aaa gcg    96
Ser Thr Ser Asp Arg Ala Ser Asp Arg Arg Asn Ala Ala Ala Lys Ala
            20                  25                  30

tct gac ctg atg tat tcg acc gtc aag aaa gga tgt tgt tcc cat cct    144
Ser Asp Leu Met Tyr Ser Thr Val Lys Lys Gly Cys Cys Ser His Pro
        35                  40                  45

gcc tgt tcg ggg aat aat cga gaa tat tgt cgt gaa agt ggc            186
Ala Cys Ser Gly Asn Asn Arg Glu Tyr Cys Arg Glu Ser Gly
    50                  55                  60

taatgctcca ggaccctctg aaccacgacg t                                 217


<210> 360
<211> 62
<212> PRT
<213> Conus obscurus

<400> 360
Met Phe Thr Val Phe Leu Leu Val Val Leu Ser Thr Thr Val Val Ser
1               5                   10                  15

Ser Thr Ser Asp Arg Ala Ser Asp Arg Arg Asn Ala Ala Ala Lys Ala
            20                  25                  30

Ser Asp Leu Met Tyr Ser Thr Val Lys Lys Gly Cys Cys Ser His Pro
        35                  40                  45

Ala Cys Ser Gly Asn Asn Arg Glu Tyr Cys Arg Glu Ser Gly
    50                  55                  60


<210> 361
<211> 157
<212> DNA
<213> Conus omaria

<220>
<221> CDS
<222> (52)..(126)

<400> 361
tttgatggca ggaatgcctc agccgacagc aaagtggctg cccggatcgc t cag atc    57
                                                         Gln Ile

                                                         1

gac agg gat cca tgc tgt tcc tat cct gac tgt ggc gcg aat cat cca     105
Asp Arg Asp Pro Cys Cys Ser Tyr Pro Asp Cys Gly Ala Asn His Pro
        5                   10                  15

gag att tgt ggt gga aaa cgc tgatgctcca ggaccctctg aaccacgacg t     157
Glu Ile Cys Gly Gly Lys Arg
    20                  25


<210> 362
<211> 25
<212> PRT
<213> Conus omaria

<400> 362
Gln Ile Asp Arg Asp Pro Cys Cys Ser Tyr Pro Asp Cys Gly Ala Asn
1               5                   10                  15

His Pro Glu Ile Cys Gly Gly Lys Arg
            20                  25


<210> 363
<211> 128
<212> DNA
<213> Conus omaria

<220>
<221> CDS
<222> (26)..(88)

<400> 363
tctcatggca ggaatgccgc acgct ctg acc gtc agg gaa tgc tgt tct cag     52
                            Leu Thr Val Arg Glu Cys Cys Ser Gln
                            1               5

cct cct tgt cgc tgg aaa cat cca gaa ctt tgt agt tgaagacgct         98
Pro Pro Cys Arg Trp Lys His Pro Glu Leu Cys Ser
10                  15                  20

gatgctccag gaccctctga accacgacgt                                   128


<210> 364
<211> 21
<212> PRT
<213> Conus omaria

<400> 364
Leu Thr Val Arg Glu Cys Cys Ser Gln Pro Pro Cys Arg Trp Lys His
1               5                   10                  15

Pro Glu Leu Cys Ser
            20


<210> 365
<211> 154
<212> DNA
<213> Conus omaria

<220>
<221> CDS
<222> (52)..(123)

<400> 365
tttgatggca ggaatgctgc agccagcgac aaagcgtctg agctgatggc t ctg gcc   57

Leu Ala
1

gtc agg gga tgc tgt tcc cat cct gcc tgt gct ggg aat aat cca cat 105
Val Arg Gly Cys Cys Ser His Pro Ala Cys Ala Gly Asn Asn Pro His
        5                   10                  15

atc tgt ggc aga aga cgc tgatgctcca ggaccctctg aaccacgacg t 154
Ile Cys Gly Arg Arg Arg
    20

<210> 366
<211> 24
<212> PRT
<213> Conus omaria

<400> 366
Leu Ala Val Arg Gly Cys Cys Ser His Pro Ala Cys Ala Gly Asn Asn
1               5                   10                  15

Pro His Ile Cys Gly Arg Arg Arg
            20

<210> 367
<211> 142
<212> DNA
<213> Conus omaria

<220>
<221> CDS
<222> (40)..(102)

<400> 367
tctggtgtca ggaaagacgc agcgcctggc ctgatcgct ctg acc atc aag gga 54
Leu Thr Ile Lys Gly
1               5

tgc tgt tct gat cct agc tgt aac gtg aat aat cca gac tat tgt ggt 102
Cys Cys Ser Asp Pro Ser Cys Asn Val Asn Asn Pro Asp Tyr Cys Gly
                10                  15                  20

tgacgacgct gatgctccag gaccctctga accacgacgt 142

<210> 368
<211> 21
<212> PRT
<213> Conus omaria

<400> 368
Leu Thr Ile Lys Gly Cys Cys Ser Asp Pro Ser Cys Asn Val Asn Asn
1               5                   10                  15

Pro Asp Tyr Cys Gly
            20

<210> 369
<211> 157
<212> DNA
<213> Conus omaria

<220>
<221> CDS
<222> (52)..(117)

<400> 369

tctaatggca ggaatgccgc agccaaattc aaagcgcctg ccctgatgga g ctg acc   57
                                                          Leu Thr
                                                          1

gtc agg gaa gaa tgc tgt tca gac cct cgc tgt tcc gtg gga cat caa   105
Val Arg Glu Glu Cys Cys Ser Asp Pro Arg Cys Ser Val Gly His Gln
        5                   10                  15

gat atg tgt cgg tgaagcacgt gatgctccag gaccctctga accacgacgt       157
Asp Met Cys Arg
    20


<210> 370
<211> 22
<212> PRT
<213> Conus omaria

<400> 370
Leu Thr Val Arg Glu Glu Cys Cys Ser Asp Pro Arg Cys Ser Val Gly
1               5                   10                  15

His Gln Asp Met Cys Arg
            20


<210> 371
<211> 151
<212> DNA
<213> Conus purpurascens

<220>
<221> CDS
<222> (1)..(120)

<400> 371
act gat ggc agg aat gct gca gcc ata gcg ctt gac ctg atc gct ccg   48
Thr Asp Gly Arg Asn Ala Ala Ala Ile Ala Leu Asp Leu Ile Ala Pro
1               5                   10                  15

gcc gtc agg gga gga tgc tgt tcc aat cct gcc tgt tta gtg aat cat   96
Ala Val Arg Gly Gly Cys Cys Ser Asn Pro Ala Cys Leu Val Asn His
            20                  25                  30

cta gaa atg tgt ggt aaa aga cgc tgatgcccca ggaccctctg aaccacgacg  150
Leu Glu Met Cys Gly Lys Arg Arg
        35                  40

t                                                                 151


<210> 372
<211> 40
<212> PRT
<213> Conus purpurascens

<400> 372
Thr Asp Gly Arg Asn Ala Ala Ala Ile Ala Leu Asp Leu Ile Ala Pro
1               5                   10                  15

Ala Val Arg Gly Gly Cys Cys Ser Asn Pro Ala Cys Leu Val Asn His
            20                  25                  30

Leu Glu Met Cys Gly Lys Arg Arg
        35                  40


<210> 373
<211> 160

<212> DNA
<213> Conus purpurascens

<220>
<221> CDS
<222> (1)..(120)

<400> 373
tct gat ggc agg gat gcc gca gcc aac gac aaa gcg tct gac ctg atc     48
Ser Asp Gly Arg Asp Ala Ala Ala Asn Asp Lys Ala Ser Asp Leu Ile
1               5                   10                  15

gct ctg acc gcc agg aga gat cca tgc tgt ttc aat cct gcc tgt aac     96
Ala Leu Thr Ala Arg Arg Asp Pro Cys Cys Phe Asn Pro Ala Cys Asn
            20                  25                  30

gtg aat aat cca cag att tgt ggt tgaagacgct gatgctccag gaccctctga    150
Val Asn Asn Pro Gln Ile Cys Gly
        35                  40

accacgacgt                                                          160


<210> 374
<211> 40
<212> PRT
<213> Conus purpurascens

<400> 374
Ser Asp Gly Arg Asp Ala Ala Ala Asn Asp Lys Ala Ser Asp Leu Ile
1               5                   10                  15

Ala Leu Thr Ala Arg Arg Asp Pro Cys Cys Phe Asn Pro Ala Cys Asn
            20                  25                  30

Val Asn Asn Pro Gln Ile Cys Gly
        35                  40


<210> 375
<211> 151
<212> DNA
<213> Conus purpurascens

<220>
<221> CDS
<222> (1)..(120)

<400> 375
tct gat ggc agg gat gct gag aaa aca ggc ttt gac acg acc att gtg     48
Ser Asp Gly Arg Asp Ala Glu Lys Thr Gly Phe Asp Thr Thr Ile Val
1               5                   10                  15

ccg gaa gac tgc tgt tcg gat cct tcc tgt tgg agg ctg cat agt tta     96
Pro Glu Asp Cys Cys Ser Asp Pro Ser Cys Trp Arg Leu His Ser Leu
            20                  25                  30

gct tgt act gga att gta aac cgc tgatgctcca ggaccctctg aaccacgacg    150
Ala Cys Thr Gly Ile Val Asn Arg
        35                  40

t                                                                   151


<210> 376
<211> 40
<212> PRT
<213> Conus purpurascens

<400> 376
Ser Asp Gly Arg Asp Ala Glu Lys Thr Gly Phe Asp Thr Thr Ile Val
1               5                   10                  15

Pro Glu Asp Cys Cys Ser Asp Pro Ser Cys Trp Arg Leu His Ser Leu
            20                  25                  30

Ala Cys Thr Gly Ile Val Asn Arg
        35                  40


<210> 377
<211> 142
<212> DNA
<213> Conus purpurascens

<220>
<221> CDS
<222> (1)..(111)

<400> 377
act gat ggc agg agt gct gca gcc ata gcg ttt gcc ctg atc gct ccg     48
Thr Asp Gly Arg Ser Ala Ala Ala Ile Ala Phe Ala Leu Ile Ala Pro
1               5                   10                  15

acc gtc tgc tgt act aat cct gcc tgt ctc gtg aat aat ata cgc ttt     96
Thr Val Cys Cys Thr Asn Pro Ala Cys Leu Val Asn Asn Ile Arg Phe
            20                  25                  30

tgt ggt gga aga cgc tgatgcccca ggaccctctg aaccacgacg t             142
Cys Gly Gly Arg Arg
        35


<210> 378
<211> 37
<212> PRT
<213> Conus purpurascens

<400> 378
Thr Asp Gly Arg Ser Ala Ala Ala Ile Ala Phe Ala Leu Ile Ala Pro
1               5                   10                  15

Thr Val Cys Cys Thr Asn Pro Ala Cys Leu Val Asn Asn Ile Arg Phe
            20                  25                  30

Cys Gly Gly Arg Arg
        35


<210> 379
<211> 157
<212> DNA
<213> Conus regius

<220>
<221> CDS
<222> (1)..(117)

<400> 379
tct gat gga aga aat gcc gca agc gac gcc aaa gcg ttt ccc cgg atc     48
Ser Asp Gly Arg Asn Ala Ala Ser Asp Ala Lys Ala Phe Pro Arg Ile
1               5                   10                  15

gct cca atc gtc agg gac gaa tgc tgt agc gat cct agg tgt cac ggg     96
Ala Pro Ile Val Arg Asp Glu Cys Cys Ser Asp Pro Arg Cys His Gly
            20                  25                  30

aat aat cgg gac cac tgt gct tgaagacgct gctgctccag gaccctctga       147

Asn Asn Arg Asp His Cys Ala
        35

accacgacgt                                                          157


<210> 380
<211> 39
<212> PRT
<213> Conus regius

<400> 380
Ser Asp Gly Arg Asn Ala Ala Ser Asp Ala Lys Ala Phe Pro Arg Ile
1               5                   10                  15

Ala Pro Ile Val Arg Asp Glu Cys Cys Ser Asp Pro Arg Cys His Gly
            20                  25                  30

Asn Asn Arg Asp His Cys Ala
        35


<210> 381
<211> 156
<212> DNA
<213> Conus regius

<220>
<221> CDS
<222> (1)..(117)

<400> 381
tct gat ggc agg aat acc gcg gcc gac gaa aaa gcg tcc gac ctg atc    48
Ser Asp Gly Arg Asn Thr Ala Ala Asp Glu Lys Ala Ser Asp Leu Ile
1               5                   10                  15

tct caa act gtc aag aga gat tgc tgt tcc cat cct ctc tgt aga tta    96
Ser Gln Thr Val Lys Arg Asp Cys Cys Ser His Pro Leu Cys Arg Leu
            20                  25                  30

ttt gtt cca gga ctt tgt att tgaagacgct gctgctccag gaccctctga      147
Phe Val Pro Gly Leu Cys Ile
        35

accacgact                                                           156


<210> 382
<211> 39
<212> PRT
<213> Conus regius

<400> 382
Ser Asp Gly Arg Asn Thr Ala Ala Asp Glu Lys Ala Ser Asp Leu Ile
1               5                   10                  15

Ser Gln Thr Val Lys Arg Asp Cys Cys Ser His Pro Leu Cys Arg Leu
            20                  25                  30

Phe Val Pro Gly Leu Cys Ile
        35


<210> 383
<211> 157
<212> DNA
<213> Conus regius

<220>

<221> CDS
<222> (1)..(117)

<400> 383
tct gat ggc agg aat gcc gca gcc gac aac aaa gcg tct gac cta atc     48
Ser Asp Gly Arg Asn Ala Ala Ala Asp Asn Lys Ala Ser Asp Leu Ile
1               5                   10                  15

gct caa atc gtc agg aga gga tgc tgt tcc cat cct gtc tgt aaa gtg     96
Ala Gln Ile Val Arg Arg Gly Cys Cys Ser His Pro Val Cys Lys Val
            20                  25                  30

agg tat cca gac ctg tgt cgt tgaagacgct gctgctccag gaccctctga       147
Arg Tyr Pro Asp Leu Cys Arg
        35

accacgacgt                                                          157


<210> 384
<211> 39
<212> PRT
<213> Conus regius

<400> 384
Ser Asp Gly Arg Asn Ala Ala Ala Asp Asn Lys Ala Ser Asp Leu Ile
1               5                   10                  15

Ala Gln Ile Val Arg Arg Gly Cys Cys Ser His Pro Val Cys Lys Val
            20                  25                  30

Arg Tyr Pro Asp Leu Cys Arg
        35


<210> 385
<211> 157
<212> DNA
<213> Conus regius

<220>
<221> CDS
<222> (1)..(117)

<400> 385
tct gat ggc agg aat gcc gca gcc gac aac aga gcg tct gac cta atc     48
Ser Asp Gly Arg Asn Ala Ala Ala Asp Asn Arg Ala Ser Asp Leu Ile
1               5                   10                  15

gct caa atc gtc agg aga gga tgc tgt tcc cat cct gcc tgt aat gtg     96
Ala Gln Ile Val Arg Arg Gly Cys Cys Ser His Pro Ala Cys Asn Val
            20                  25                  30

aat aat cca cac att tgt ggt tgaagacgct gctgctccag gaccctctga       147
Asn Asn Pro His Ile Cys Gly
        35

accacgacgt                                                          157


<210> 386
<211> 39
<212> PRT
<213> Conus regius

<400> 386
Ser Asp Gly Arg Asn Ala Ala Ala Asp Asn Arg Ala Ser Asp Leu Ile
1               5                   10                  15

Ala Gln Ile Val Arg Arg Gly Cys Cys Ser His Pro Ala Cys Asn Val
            20                  25                  30

Asn Asn Pro His Ile Cys Gly
        35


<210> 387
<211> 157
<212> DNA
<213> Conus regius

<220>
<221> CDS
<222> (1)..(117)

<400> 387
tct gat ggc agg aat gcc gca gcc gac aac aaa ccg tct gac cta atc      48
Ser Asp Gly Arg Asn Ala Ala Ala Asp Asn Lys Pro Ser Asp Leu Ile
1               5                   10                  15

gct caa atc gtc agg aga gga tgc tgt tcg cat cct gtc tgt aaa gtg      96
Ala Gln Ile Val Arg Arg Gly Cys Cys Ser His Pro Val Cys Lys Val
            20                  25                  30

agg tat tca gac atg tgt ggt tgaagacgct gctgctccag gaccctctga      147
Arg Tyr Ser Asp Met Cys Gly
        35

accacgacgt                                                            157


<210> 388
<211> 39
<212> PRT
<213> Conus regius

<400> 388
Ser Asp Gly Arg Asn Ala Ala Ala Asp Asn Lys Pro Ser Asp Leu Ile
1               5                   10                  15

Ala Gln Ile Val Arg Arg Gly Cys Cys Ser His Pro Val Cys Lys Val
            20                  25                  30

Arg Tyr Ser Asp Met Cys Gly
        35


<210> 389
<211> 154
<212> DNA
<213> Conus stercusmuscarum

<220>
<221> CDS
<222> (1)..(114)

<400> 389
tct gat ggc agg aat gca gag cga cga caa agc gtc tgt cct ggt cgc      48
Ser Asp Gly Arg Asn Ala Glu Arg Arg Gln Ser Val Cys Pro Gly Arg
1               5                   10                  15

tct ggc ccc agg gga gga tgt tgt tcc cac cct gcc tgt aag gtg cat      96
Ser Gly Pro Arg Gly Gly Cys Cys Ser His Pro Ala Cys Lys Val His
            20                  25                  30

ttt cca cac agt tgt ggt tgacgacgct gatgctccag gaccctctga      144
Phe Pro His Ser Cys Gly
        35

accaccgacgt                                                          154

<210> 390
<211> 38
<212> PRT
<213> Conus stercusmuscarum

<400> 390
Ser Asp Gly Arg Asn Ala Glu Arg Arg Gln Ser Val Cys Pro Gly Arg
1               5                   10                  15

Ser Gly Pro Arg Gly Gly Cys Cys Ser His Pro Ala Cys Lys Val His
            20                  25                  30

Phe Pro His Ser Cys Gly
        35


<210> 391
<211> 145
<212> DNA
<213> Conus stercusmuscarum

<220>
<221> CDS
<222> (1)..(114)

<400> 391
tct gat ggc agg aat gcc gca gcc agc gac aga gcg tct gac gcg gcc    48
Ser Asp Gly Arg Asn Ala Ala Ala Ser Asp Arg Ala Ser Asp Ala Ala
1               5                   10                  15

cac cag gta tgc tgt tcc aac cct gtc tgt cac gtg gat cat cca gaa    96
His Gln Val Cys Cys Ser Asn Pro Val Cys His Val Asp His Pro Glu
            20                  25                  30

ctt tgt cgt aga aga cgc tgatgctcca ggaccctctg aaccacgacg t         145
Leu Cys Arg Arg Arg Arg
        35


<210> 392
<211> 38
<212> PRT
<213> Conus stercusmuscarum

<400> 392
Ser Asp Gly Arg Asn Ala Ala Ala Ser Asp Arg Ala Ser Asp Ala Ala
1               5                   10                  15

His Gln Val Cys Cys Ser Asn Pro Val Cys His Val Asp His Pro Glu
            20                  25                  30

Leu Cys Arg Arg Arg Arg
        35


<210> 393
<211> 154
<212> DNA
<213> Conus striatus

<220>
<221> CDS
<222> (1)..(123)

<400> 393

tct gat ggc agg aat gcc gcg gcc aac gac aaa gcg tct gac ctg gtc     48
Ser Asp Gly Arg Asn Ala Ala Ala Asn Asp Lys Ala Ser Asp Leu Val
1               5                   10                  15

gct ccg gcc atc agg gga tgc tgt tcc cac cct gtc tgt aac ttg agt     96
Ala Pro Ala Ile Arg Gly Cys Cys Ser His Pro Val Cys Asn Leu Ser
            20                  25                  30

aat cca caa att tgt cgt gga aga cgc tgatgctcca ggaccctctg          143
Asn Pro Gln Ile Cys Arg Gly Arg Arg
        35                  40

aaccacgacg t                                                        154


<210> 394
<211> 41
<212> PRT
<213> Conus striatus

<400> 394
Ser Asp Gly Arg Asn Ala Ala Ala Asn Asp Lys Ala Ser Asp Leu Val
1               5                   10                  15

Ala Pro Ala Ile Arg Gly Cys Cys Ser His Pro Val Cys Asn Leu Ser
            20                  25                  30

Asn Pro Gln Ile Cys Arg Gly Arg Arg
        35                  40


<210> 395
<211> 117
<212> DNA
<213> Conus textile

<220>
<221> CDS
<222> (1)..(114)

<400> 395
ttt cat ggc agg aat gcc gca gcc aaa gcg tct ggc ctg gtc ggt ctg     48
Phe His Gly Arg Asn Ala Ala Ala Lys Ala Ser Gly Leu Val Gly Leu
1               5                   10                  15

acc gac aag agg caa gaa tgc tgt tct cat cct gcc tgt aac gta gat     96
Thr Asp Lys Arg Gln Glu Cys Cys Ser His Pro Ala Cys Asn Val Asp
            20                  25                  30

cat cca gaa att tgt cgt tga                                         117
His Pro Glu Ile Cys Arg
        35


<210> 396
<211> 38
<212> PRT
<213> Conus textile

<400> 396
Phe His Gly Arg Asn Ala Ala Ala Lys Ala Ser Gly Leu Val Gly Leu
1               5                   10                  15

Thr Asp Lys Arg Gln Glu Cys Cys Ser His Pro Ala Cys Asn Val Asp
            20                  25                  30

His Pro Glu Ile Cys Arg
        35

<210> 397
<211> 151
<212> DNA
<213> Conus tulipa

<220>
<221> CDS
<222> (1)..(120)

<400> 397
act gat ggc agg agt gct gca gcc ata gcg ttt gcc ctg atc gct ccg     48
Thr Asp Gly Arg Ser Ala Ala Ala Ile Ala Phe Ala Leu Ile Ala Pro
1               5                   10                  15

acc gtc tgg gaa gga tgc tgt tct aat cct gcc tgt ctc gtg aat cat     96
Thr Val Trp Glu Gly Cys Cys Ser Asn Pro Ala Cys Leu Val Asn His
            20                  25                  30

ata cgc ttt tgt ggt gga aga cgc tgatgcccca ggaccctctg aaccacgacg    150
Ile Arg Phe Cys Gly Gly Arg Arg
        35                  40

t                                                                   151


<210> 398
<211> 40
<212> PRT
<213> Conus tulipa

<400> 398
Thr Asp Gly Arg Ser Ala Ala Ala Ile Ala Phe Ala Leu Ile Ala Pro
1               5                   10                  15

Thr Val Trp Glu Gly Cys Cys Ser Asn Pro Ala Cys Leu Val Asn His
            20                  25                  30

Ile Arg Phe Cys Gly Gly Arg Arg
        35                  40


<210> 399
<211> 157
<212> DNA
<213> Conus virgo

<220>
<221> CDS
<222> (1)..(117)

<400> 399
tct aat ggc atg aat gcc gca gcc atc agg aaa gcg tct gcc ctg gtg     48
Ser Asn Gly Met Asn Ala Ala Ala Ile Arg Lys Ala Ser Ala Leu Val
1               5                   10                  15

gct cag atc gcc cat cga gac tgc tgt gac gat cct gcc tgc acc gtg     96
Ala Gln Ile Ala His Arg Asp Cys Cys Asp Asp Pro Ala Cys Thr Val
            20                  25                  30

aat aat cca ggc ctt tgc act tgaagatgct gctgccccag gaccctctga        147
Asn Asn Pro Gly Leu Cys Thr
        35

accacgacgt                                                          157


<210> 400
<211> 39

<212> PRT
<213> Conus virgo

<400> 400
Ser Asn Gly Met Asn Ala Ala Ala Ile Arg Lys Ala Ser Ala Leu Val
1               5                   10                  15

Ala Gln Ile Ala His Arg Asp Cys Cys Asp Asp Pro Ala Cys Thr Val
            20                  25                  30

Asn Asn Pro Gly Leu Cys Thr
        35


<210> 401
<211> 154
<212> DNA
<213> Conus geographus

<220>
<221> CDS
<222> (1)..(114)

<400> 401
tct gat ggc ggg aat gcc gca gca aaa gag tct gac gtg atc gct ctg      48
Ser Asp Gly Gly Asn Ala Ala Ala Lys Glu Ser Asp Val Ile Ala Leu
1               5                   10                  15

acc gtc tgg aaa tgc tgt acc att cct tcc tgt tat gag aaa aaa aaa      96
Thr Val Trp Lys Cys Cys Thr Ile Pro Ser Cys Tyr Glu Lys Lys Lys
            20                  25                  30

att aaa gca tgt gtc ttt tgacgacgct gatgctccag gaccctctga             144
Ile Lys Ala Cys Val Phe
        35

accacgacgt                                                           154


<210> 402
<211> 38
<212> PRT
<213> Conus geographus

<400> 402
Ser Asp Gly Gly Asn Ala Ala Ala Lys Glu Ser Asp Val Ile Ala Leu
1               5                   10                  15

Thr Val Trp Lys Cys Cys Thr Ile Pro Ser Cys Tyr Glu Lys Lys Lys
            20                  25                  30

Ile Lys Ala Cys Val Phe
        35


<210> 403
<211> 154
<212> DNA
<213> Conus regius

<220>
<221> CDS
<222> (1)..(114)

<400> 403
tct gat ggc gca gtc gac gac aaa gcg ttg gat cga atc gct gaa atc      48
Ser Asp Gly Ala Val Asp Asp Lys Ala Leu Asp Arg Ile Ala Glu Ile
1               5                   10                  15

gtc agg aga gga tgc tgt ggc aat cct gcc tgt agc ggc tcc tcg aaa    96
Val Arg Arg Gly Cys Cys Gly Asn Pro Ala Cys Ser Gly Ser Ser Lys
            20                  25                  30

gat gca ccc tct tgt ggt tgaagacgct gctgctccag gaccctctga          144
Asp Ala Pro Ser Cys Gly
        35

accacgacgt                                                        154


<210> 404
<211> 38
<212> PRT
<213> Conus regius

<400> 404
Ser Asp Gly Ala Val Asp Asp Lys Ala Leu Asp Arg Ile Ala Glu Ile
1               5                   10                  15

Val Arg Arg Gly Cys Cys Gly Asn Pro Ala Cys Ser Gly Ser Ser Lys
            20                  25                  30

Asp Ala Pro Ser Cys Gly
        35

SEQUENCE LISTING

<110> University of Utah Research Foundation
      Cogentix, Inc.

<120> Alpha-Conotoxin Peptides

<130> E3805-EC/DIV

<140> EP 07102532.4
<141> 2007-02-16

<150> EP 00908382.5
<151> 2000-01-28

<150> PCT/US00/01979
<151> 2000-01-28

<150> US 60/118,381
<151> 1999-01-29

<160> 404

<170> WordPerfect/Word/PatentIn Ver. 2.0

<210> 1
<211> 17
<212> PRT
<213> Artificial Sequence

<220>
<223> Description of Artificial Sequence:Alpha-Conotoxin
      Peptide Generic Formula I

<220>
<221> PEPTIDE
<222> (1)..(17)
<223> Xaa is des-Xaa

<220>
<221> VARIANT
<222> (1)
<223> /replace = Ile
      /replace = Leu
      /replace = Val

<220>
<221> VARIANT
<222> (2)
<223> /replace = Ala
      /replace = Gly

<220>
<221> VARIANT
<222> (3)
<223> /replace = Gly
      /replace = Trp
      /replace = D-Trp
      /replace = neo-Trp

<220>
<221> VARIANT
<222> (3)
<223> /replace = halo-Trp
      /replace = any unnatural aromatic amino acid

<220>
<221> VARIANT
<222> (4)
<223> /replace = des-Asp
      /replace = Gly
      /replace = Trp
      /replace = D-Trp

<220>
<221> VARIANT
<222> (4)
<223> /replace = neo-Trp
      /replace = halo-Trp
      /replace = any unnatural aromatic amino acid

<220>
<221> VARIANT
<222> (5)
<223> /replace = Glu
      /replace = gamma-carboxy-Glu (Gla)
      /replace = Asp
      /replace = Ala

<220>
<221> VARIANT
<222> (5)
<223> /replace = Thr
      /replace = Ser
      /replace = Ile
      /replace = Tyr

<220>
<221> VARIANT
<222> (5)
<223> /replace = nor-Tyr
      /replace = mono-halo-Tyr
      /replace = di-halo-Tyr
      /replace = O-sulpho-Tyr

<220>
<221> VARIANT
<222> (5)
<223> /replace = O-phospho-Tyr
      /replace = nitro-Tyr
      /replace = any unnatural hydroxy containing amino acid

<220>
<221> VARIANT
<222> (8)
<223> /replace = Thr
      /replace = Arg
      /replace = ornithine
      /replace = homoarginine

<220>
<221> VARIANT
<222> (8)
<223> /replace = Lys
      /replace = N-methyl-Lys,
      /replace = N,N-dimethyl-Lys
      /replace = N,N,N-trimethyl-Lys

<220>
<221> VARIANT
<222> (8)
<223> /replace = any unnatural basic amino acid

<220>
<221> VARIANT
<222> (9)
<223> /replace = Glu
      /replace = Gla
      /replace = Arg
      /replace = ornithine

<220>
<221> VARIANT
<222> (9)
<223> /replace = homoarginine
      /replace = Lys
      /replace = N-methyl-Lys
      /replace = N,N-dimethyl-Lys

<220>
<221> VARIANT
<222> (9)
<223> /replace = N,N,N-trimethyl-Lys
      /replace = any unnatural basic amino acid

<220>
<221> VARIANT
<222> (10)
<223> /replace = Ser
      /replace = Thr
      /replace = Asn
      /replace = Ala

<220>
<221> VARIANT
<222> (10)
<223> /replace = Gly
      /replace = His
      /replace = halo-His
      /replace = hydroxy-Pro

<220>
<221> VARIANT
<222> (11)
<223> /replace = Thr
      /replace = Ser
      /replace = Ala
      /replace = Asp

<220>

<221> VARIANT
<222> (11)
<223> /replace = Asn
      /replace = Pro
      /replace = hydroxy-Pro
      /replace = ornithine

<220>
<221> VARIANT
<222> (11)
<223> /replace = homoarginine
      /replace = Lys
      /replace = N-methyl-Lys
      /replace = N,N-dimethyl-Lys

<220>
<221> VARIANT
<222> (11)
<223> /replace = N,N,N-trimethyl-Lys
      /replace = any unnatural basic amino acid

<220>
<221> VARIANT
<222> (13)
<223> /replace = Gly
      /replace = Ser
      /replace = Ala
      /replace = Asn

<220>
<221> VARIANT
<222> (13)
<223> /replace = Arg
      /replace = ornithine
      /replace = homoarginine
      /replace = Lys

<220>
<221> VARIANT
<222> (13)
<223> /replace = N-methyl-Lys
      /replace = N,N-dimethyl-Lys
      /replace = N,N,N-trimethyl-Lys
      /replace = any unnatural basic amino acid

<220>
<221> VARIANT
<222> (14)
<223> /replace = Gln
      /replace = His
      /replace = halo-His
      /replace = Trp

<220>
<221> VARIANT
<222> (14)
<223> /replace = D-Trp
      /replace = halo-Trp
      /replace = neo-Trp
      /replace = Tyr

<220>
<221> VARIANT
<222> (14)
<223> /replace = nor-Tyr
      /replace = mono-halo-Tyr
      /replace = di-halo-Tyr
      /replace = O-sulpho-Tyr

<220>
<221> VARIANT
<222> (14)
<223> /replace = O-phospho-Tyr
      /replace = nitro-Tyr
      /replace = Arg
      /replace = ornithine

<220>
<221> VARIANT
<222> (14)
<223> /replace = homoarginine
      /replace = Lys
      /replace = N-methyl-Lys
      /replace = N,N-dimethyl-Lys

<220>
<221> VARIANT
<222> (14)
<223> /replace = N,N,N-trimethyl-Lys
      /replace = any unnatural basic amino acid
      /replace = any unnatural aromatic amino acid

<220>
<221> VARIANT
<222> (15)
<223> /replace = Asn
      /replace = His
      /replace = halo-His
      /replace = Leu

<220>
<221> VARIANT
<222> (15)
<223> /replace = Val
      /replace = Gln
      /replace = Arg
      /replace = ornithine

<220>
<221> VARIANT
<222> (15)
<223> /replace = homoarginine
      /replace = Lys
      /replace = N-methyl-Lys
      /replace = N,N-dimethyl-Lys

<220>
<221> VARIANT
<222> (15)
<223> /replace = N,N,N-trimethyl-Lys

/replace = any unnatural basic amino acid

<220>
<221> VARIANT
<222> (17)
<223> /replace = Val
      /replace = Ile
      /replace = Leu
      /replace = Arg

<220>
<221> VARIANT
<222> (17)
<223> /replace = ornithine
      /replace = homoarginine
      /replace = Lys
      /replace = N-methyl-Lys

<220>
<221> VARIANT
<222> (17)
<223> /replace = N,N-dimethyl-Lys
      /replace = N,N,N-trimethyl-Lys
      /replace = or any unnatural basic amino acid

<400> 1
Xaa Xaa Xaa Xaa Gly Cys Cys Ser Asp Pro Arg Cys Thr Leu Ile Cys
 1               5                  10                  15

Xaa


<210> 2
<211> 21
<212> PRT
<213> Artificial Sequence

<220>
<223> Description of Artificial Sequence:Alpha-Conotoxin
      Peptide Generic Formula II.

<220>
<221> PEPTIDE
<222> (1)..(21)
<223> Xaa is des-Xaa

<220>
<221> VARIANT
<222> (1)
<223> /replace = Asp
      /replace = Glu
      /replace = gamma-carboxy-Glu (Gla)

<220>
<221> VARIANT
<222> (2)
<223> /replace = Gln
      /replace = Ala
      /replace = Asp
      /replace = Glu

<220>
<221> VARIANT
<222> (2)
<223> /replace = Gla

<220>
<221> VARIANT
<222> (3)
<223> /replace = Gly
      /replace = Ala
      /replace = Asp
      /replace = Glu

<220>
<221> VARIANT
<222> (3)
<223> /replace = Gla
      /replace = Pro
      /replace = hydroxy-Pro

<220>
<221> VARIANT
<222> (4)
<223> /replace = des-Xaa4
      /replace = Glu
      /replace = Gla
      /replace = Gln

<220>
<221> VARIANT
<222> (4)
<223> /replace = Asp
      /replace = Asn
      /replace = Pro
      /replace = hydroxy-Pro

<220>
<221> VARIANT
<222> (7)
<223> /replace = Thr
      /replace = Gly
      /replace = Glu
      /replace = Gla

<220>
<221> VARIANT
<222> (7)
<223> /replace = Asn
      /replace = Trp
      /replace = D-Trp
      /replace = neo-Trp

<220>
<221> VARIANT
<222> (7)
<223> /replace = halo-Trp
      /replace = Arg
      /replace = ornithine
      /replace = homoarginine

<220>
<221> VARIANT
<222> (7)
<223> /replace = Lys
      /replace = N-methyl-Lys
      /replace = N,N-dimethyl-Lys
      /replace = N,N,N-trimethyl-Lys

<220>
<221> VARIANT
<222> (7)
<223> /replace = any unnatural basic amino acid
      /replace = Tyr
      /replace = nor-Tyr
      /replace = mono-halo-Tyr

<220>
<221> VARIANT
<222> (7)
<223> /replace = di-halo-Tyr,
      /replace = O-sulpho-Tyr
      /replace = O-phospho-Tyr
      /replace = nitro-Tyr

<220>
<221> VARIANT
<222> (7)
<223> /replace = any unnatural hydroxy containing amino acid

<220>
<221> VARIANT
<222> (8)
<223> /replace = Asp
      /replace = His
      /replace = halo-His
      /replace = Thr

<220>
<221> VARIANT
<222> (8)
<223> /replace = Ser
      /replace = Tyr
      /replace = nor-Tyr
      /replace = mono-halo-Tyr

<220>
<221> VARIANT
<222> (8)
<223> /replace = di-halo-Tyr
      /replace = O-sulpho-Tyr
      /replace = O-phospho-Tyr
      /replace = nitro-Tyr

<220>
<221> VARIANT
<222> (8)
<223> /replace = any unnatural hydroxy containing amino acid

<220>

<221> VARIANT
<222> (9)
<223> /replace = hydroxy-Pro

<220>
<221> VARIANT
<222> (10)
<223> /replace = Ala
      /replace = Ser
      /replace = Thr
      /replace = Asp

<220>
<221> VARIANT
<222> (10)
<223> /replace = Val
      /replace = Ile
      /replace = hydroxy-Pro
      /replace = Tyr

<220>
<221> VARIANT
<222> (10)
<223> /replace = nor-Tyr
      /replace = mono-halo-Tyr
      /replace = di-halo-Tyr
      /replace = O-sulpho-Tyr

<220>
<221> VARIANT
<222> (10)
<223> /replace = O-phospho-Tyr
      /replace = nitro-Tyr
      /replace = any unnatural hydroxy containing amino acid

<220>
<221> VARIANT
<222> (12)
<223> /replace = Gly
      /replace = Ile
      /replace = Leu
      /replace = Val

<220>
<221> VARIANT
<222> (12)
<223> /replace = Ala
      /replace = Thr
      /replace = Ser
      /replace = Pro

<220>
<221> VARIANT
<222> (12)
<223> /replace = hydroxy-Pro
      /replace = Phe
      /replace = Trp
      /replace = D-Trp

<220>

<221> VARIANT
<222> (12)
<223> /replace = neo-Trp
      /replace = halo-Trp
      /replace = Arg
      /replace = ornithine

<220>
<221> VARIANT
<222> (12)
<223> /replace = homoarginine
      /replace = N-methyl-Lys
      /replace = N,N-dimethyl-Lys
      /replace = N,N,N-trimethyl-Lys

<220>
<221> VARIANT
<222> (12)
<223> /replace = any unnatural basic amino acid
      /replace = any unnatural aromatic amino acid

<220>
<221> VARIANT
<222> (13)
<223> /replace = Asn
      /replace = Phe
      /replace = Pro
      /replace = hydroxy-Pro

<220>
<221> VARIANT
<222> (13)
<223> /replace = Glu
      /replace = Gla
      /replace = Gln
      /replace = His

<220>
<221> VARIANT
<222> (13)
<223> /replace = halo-His
      /replace = Val
      /replace = Ser
      /replace = Thr

<220>
<221> VARIANT
<222> (13)
<223> /replace = Arg
      /replace = ornithine
      /replace = homoarginine
      /replace = Lys, N-methyl-Lys

<220>
<221> VARIANT
<222> (13)
<223> /replace = N,N-dimethyl-Lys
      /replace = N,N,N-trimethyl-Lys
      /replace = any unnatural basic amino acid

<220>
<221> VARIANT
<222> (14)
<223> /replace = Ser
      /replace = His
      /replace = halo-His
      /replace = Leu

<220>
<221> VARIANT
<222> (14)
<223> /replace = Ile
      /replace = Val
      /replace = Asn
      /replace = Met

<220>
<221> VARIANT
<222> (14)
<223> /replace = Pro
      /replace = hydroxy-Pro
      /replace = Arg
      /replace = ornithine

<220>
<221> VARIANT
<222> (14)
<223> /replace = homoarginine
      /replace = Lys
      /replace = N-methyl-Lys
      /replace = N,N-dimethyl-Lys

<220>
<221> VARIANT
<222> (14)
<223> /replace = N,N,N-trimethyl-Lys
      /replace = any unnatural basic amino acid
      /replace = Tyr
      /replace = nor-Tyr

<220>
<221> VARIANT
<222> (14)
<223> /replace = mono-halo-Tyr
      /replace = di-halo-Tyr
      /replace = O-sulpho-Tyr
      /replace = O-phospho-Tyr

<220>
<221> VARIANT
<222> (14)
<223> /replace = nitro-Tyr
      /replace = any unnatural hydroxy containing amino acid

<220>
<221> VARIANT
<222> (15)
<223> /replace = Pro
      /replace = hydroxy-Pro
      /replace = Gln

/replace = Ser

<220>
<221> VARIANT
<222> (15)
<223> /replace = Thr
      /replace = Arg
      /replace = ornithine
      /replace = homoarginine

<220>
<221> VARIANT
<222> (15)
<223> /replace = Lys
      /replace = N-methyl-Lys
      /replace = N,N-dimethyl-Lys
      /replace = N,N,N-trimethyl-Lys

<220>
<221> VARIANT
<222> (15)
<223> /replace = any unnatural basic amino acid
      /replace = Tyr
      /replace = nor-Tyr
      /replace = mono-halo-Tyr

<220>
<221> VARIANT
<222> (15)
<223> /replace = di-halo-Tyr
      /replace = O-sulpho-Tyr
      /replace = O-phospho-Tyr
      /replace = nitro-Tyr

<220>
<221> VARIANT
<222> (15)
<223> /replace = any unnatural hydroxy containing amino acid

<220>
<221> VARIANT
<222> (16)
<223> /replace = Gly
      /replace = Thr
      /replace = Ser
      /replace = Pro

<220>
<221> VARIANT
<222> (16)
<223> /replace = hydroxy-Pro
      /replace = Tyr
      /replace = nor-Tyr
      /replace = mono-halo-Tyr

<220>
<221> VARIANT
<222> (16)
<223> /replace = di-halo-Tyr
      /replace = O-sulpho-Tyr

/replace = O-phospho-Tyr
/replace = nitro-Tyr

<220>
<221> VARIANT
<222> (16)
<223> /replace = any unnatural hydroxy containing amino acid

<220>
<221> VARIANT
<222> (17)
<223> /replace = Ile
/replace = Val
/replace = Asp
/replace = Leu

<220>
<221> VARIANT
<222> (17)
<223> /replace = Phe
/replace = Arg
/replace = ornithine
/replace = homoarginine

<220>
<221> VARIANT
<222> (17)
<223> /replace = Lys
/replace = N-methyl-Lys
/replace = N,N-dimethyl-Lys
/replace = N,N,N-trimethyl-Lys

<220>
<221> VARIANT
<222> (17)
<223> /replace = any unnatural basic amino acid
/replace = Tyr
/replace = nor-Tyr
/replace = mono-halo-Tyr

<220>
<221> VARIANT
<222> (17)
<223> /replace = di-halo-Tyr
/replace = O-sulpho-Tyr
/replace = O-phospho-Tyr
/replace = nitro-Tyr

<220>
<221> VARIANT
<222> (17)
<223> /replace = any unnatural hydroxy containing amino acid

<220>
<221> VARIANT
<222> (19)
<223> /replace = Gly,
/replace = Ala
/replace = Met
/replace = Ser

<220>
<221> VARIANT
<222> (19)
<223> /replace = Thr
      /replace = Trp
      /replace = D-Trp
      /replace = neo-Trp

<220>
<221> VARIANT
<222> (19)
<223> /replace = halo-Trp
      /replace = any unnatural aromatic amino acid
      /replace = Arg
      /replace = ornithine

<220>
<221> VARIANT
<222> (19)
<223> /replace = homoarginine
      /replace = Lys
      /replace = N-methyl-Lys
      /replace = N,N-dimethyl-Lys

<220>
<221> VARIANT
<222> (19)
<223> /replace = N,N,N-trimethyl-Lys
      /replace = any unnatural basic amino acid

<220>
<221> VARIANT
<222> (20)
<223> /replace = Trp
      /replace = D-Trp
      /replace = neo-Trp
      /replace = halo-Trp

<220>
<221> VARIANT
<222> (20)
<223> /replace = any unnatural aromatic amino acid
      /replace = Arg
      /replace = ornithine
      /replace = homoarginine

<220>
<221> VARIANT
<222> (20)
<223> /replace = Lys
      /replace = N-methyl-Lys
      /replace = N,N-dimethyl-Lys
      /replace = N,N,N-trimethyl-Lys

<220>
<221> VARIANT
<222> (20)
<223> /replace = any unnatural basic amino acid

<220>
<221> VARIANT
<222> (21)
<223> /replace = Arg
      /replace = ornithine
      /replace = homoarginine
      /replace = Lys

<220>
<221> VARIANT
<222> (21)
<223> /replace = N-methyl-Lys
      /replace = N,N-dimethyl-Lys
      /replace = N,N,N-trimethyl-Lys
      /replace = any unnatural basic amino acid

<400> 2
Xaa Xaa Xaa Gly Cys Cys Ser Asn Pro Pro Cys Lys Ala Thr Asn Xaa
  1               5                  10                  15

Xaa Cys Xaa Xaa Xaa
            20


<210> 3
<211> 28
<212> PRT
<213> Artificial Sequence

<220>
<223> Description of Artificial Sequence:Alpha-Conotoxin
      Peptide Generic Formula III.

<220>
<221> PEPTIDE
<222> (1)..(28)
<223> Xaa is des-Xaa

<220>
<221> VARIANT
<222> (1)
<223> /replace = Ser
      /replace = Thr

<220>
<221> VARIANT
<222> (2)
<223> /replace = Asp
      /replace = Glu
      /replace = gamma-carboxy-Glu (Gla)
      /replace = Asn

<220>
<221> VARIANT
<222> (2)
<223> /replace = Ser
      /replace = Thr

<220>
<221> VARIANT

<222> (3)
<223> /replace = Ala
      /replace = Gly
      /replace = Asn
      /replace = Ser

<220>
<221> VARIANT
<222> (3)
<223> /replace = Thr
      /replace = Pro
      /replace = hydroxy-Pro
      /replace = Arg

<220>
<221> VARIANT
<222> (3)
<223> /replace = ornithine
      /replace = homoarginine
      /replace = Lys
      /replace = N-methyl-Lys

<220>
<221> VARIANT
<222> (3)
<223> /replace = N,N-dimethyl-Lys
      /replace = N,N,N-trimethyl-Lys
      /replace = any unnatural basic amino acid

<220>
<221> VARIANT
<222> (4)
<223> /replace = Ala
      /replace = Val
      /replace = Leu
      /replace = Ile

<220>
<221> VARIANT
<222> (4)
<223> /replace = Gly
      /replace = Glu
      /replace = Gla
      /replace = Gln

<220>
<221> VARIANT
<222> (4)
<223> /replace = Asp
      /replace = Asn
      /replace = Phe
      /replace = Pro

<220>
<221> VARIANT
<222> (4)
<223> /replace = hydroxy-Pro
      /replace = any unnatural aromatic amino acid

<220>

<221> VARIANT
<222> (5)
<223> /replace = des-Xaa
      /replace = Thr
      /replace = Ser
      /replace = Asp

<220>
<221> VARIANT
<222> (5)
<223> /replace = Glu
      /replace = Gla
      /replace = Gln
      /replace = Val

<220>
<221> VARIANT
<222> (5)
<223> /replace = Asp
      /replace = Asn
      /replace = Ala
      /replace = Pro

<220>
<221> VARIANT
<222> (5)
<223> /replace = hydroxy-Pro
      /replace = Arg
      /replace = ornithine
      /replace = homoarginine

<220>
<221> VARIANT
<222> (5)
<223> /replace = Lys
      /replace = N-methyl-Lys
      /replace = N,N-dimethyl-Lys
      /replace = N,N,N-trimethyl-Lys

<220>
<221> VARIANT
<222> (5)
<223> /replace = any unnatural basic amino acid

<220>
<221> VARIANT
<222> (8)
<223> /replace = Thr
      /replace = Asp
      /replace = Asn
      /replace = Met

<220>
<221> VARIANT
<222> (8)
<223> /replace = Val
      /replace = Ala
      /replace = Gly
      /replace = Leu

<220>
<221> VARIANT
<222> (8)
<223> /replace = Ile
      /replace = Phe
      /replace = any unnatural aromatic amino acid
      /replace = Pro

<220>
<221> VARIANT
<222> (8)
<223> /replace = hydroxy-Pro
      /replace = Tyr
      /replace = nor-Tyr
      /replace = mono-halo-Tyr

<220>
<221> VARIANT
<222> (8)
<223> /replace = di-halo-Tyr
      /replace = O-sulpho-Tyr
      /replace = O-phospho-Tyr
      /replace = nitro-Tyr

<220>
<221> VARIANT
<222> (8)
<223> /replace = any unnatural hydroxy containing amino acid

<220>
<221> VARIANT
<222> (9)
<223> /replace = Ile
      /replace = Leu
      /replace = Val
      /replace = Ser

<220>
<221> VARIANT
<222> (9)
<223> /replace = Thr
      /replace = Gln
      /replace = Asn
      /replace = Asp

<220>
<221> VARIANT
<222> (9)
<223> /replace = Arg
      /replace = halo-His
      /replace = Phe
      /replace = any unnatural aromatic amino acid

<220>
<221> VARIANT
<222> (9)
<223> /replace = homoarginine
      /replace = ornithine
      /replace = Lys
      /replace = N-methyl-Lys

<220>
<221> VARIANT
<222> (9)
<223> /replace = N,N-dimethyl-Lys
      /replace = N,N,N-trimethyl-Lys
      /replace = any unnatural basic amino acid
      /replace = Tyr

<220>
<221> VARIANT
<222> (9)
<223> /replace = nor-Tyr
      /replace = mono-halo-Tyr
      /replace = di-halo-Tyr
      /replace = O-sulpho-Tyr

<220>
<221> VARIANT
<222> (9)
<223> /replace = O-phospho-Tyr
      /replace = nitro-Tyr
      /replace = any unnatural hydroxy containing amino acid

<220>
<221> VARIANT
<222> (10)
<223> /replace = hyroxy-Pro
      /replace = Ser
      /replace = Thr
      /replace = Ile

<220>
<221> VARIANT
<222> (10)
<223> /replace = Asp
      /replace = Leu
      /replace = Val
      /replace = Gly

<220>
<221> VARIANT
<222> (10)
<223> /replace = Ala
      /replace = Phe
      /replace = any unnatural aromatic amino acid
      /replace = Arg

<220>
<221> VARIANT
<222> (10)
<223> /replace = ornithine
      /replace = homoarginine
      /replace = Lys
      /replace = N-methyl-Lys

<220>
<221> VARIANT
<222> (10)
<223> /replace = N,N-dimethyl-Lys

/replace = N,N,N-trimethyl-Lys
/replace = or any unnatural basic amino acid

<220>
<221> VARIANT
<222> (11)
<223> /replace = Val
/replace = Gly
/replace = Ile
/replace = Leu

<220>
<221> VARIANT
<222> (11)
<223> /replace = Asp
/replace = Ser
/replace = Thr
/replace = Pro

<220>
<221> VARIANT
<222> (11)
<223> /replace = hydroxy-Pro
/replace = Arg
/replace = ornithine
/replace = homoarginine

<220>
<221> VARIANT
<222> (11)
<223> /replace = Lys
/replace = N-methyl-Lys
/replace = N,N-dimethyl-Lys
/replace = N,N,N-trimethyl-Lys

<220>
<221> VARIANT
<222> (11)
<223> /replace = any unnatural basic amino acid

<220>
<221> VARIANT
<222> (13)
<223> /replace = His
/replace = halo-His
/replace = Arg
/replace = homoarginine

<220>
<221> VARIANT
<222> (13)
<223> /replace = ornithine
/replace = Lys
/replace = N-methyl-Lys
/replace = N,N-dimethyl-Lys

<220>
<221> VARIANT
<222> (13)
<223> /replace = N,N,N-trimethyl-Lys

/replace = any unnatural basic amino acid
/replace = Ala
/replace = Ser

<220>
<221> VARIANT
<222> (13)
<223> /replace = Thr
/replace = Phe
/replace = Ile
/replace = Leu

<220>
<221> VARIANT
<222> (13)
<223> /replace = Gly
/replace = Trp
/replace = D-Trp
/replace = neo-Trp

<220>
<221> VARIANT
<222> (13)
<223> /replace = halo-Trp
/replace = any unnatural aromatic amino acid
/replace = Tyr
/replace = nor-Tyr

<220>
<221> VARIANT
<222> (13)
<223> /replace = mono-halo-Tyr
/replace = di-halo-Tyr
/replace = O-sulpho-Tyr
/replace = O-phospho-Tyr

<220>
<221> VARIANT
<222> (13)
<223> /replace = nitro-Tyr
/replace = any unnatural hydroxy containing amino acid

<220>
<221> VARIANT
<222> (14)
<223> /replace = Leu,
/replace = Gln
/replace = Ile
/replace = Gly

<220>
<221> VARIANT
<222> (14)
<223> /replace = Met
/replace = Ala
/replace = Lys
/replace = N-methyl-Lys

<220>
<221> VARIANT

<222> (14)
<223> /replace = N,N-dimethyl-Lys
      /replace = N,N,N-trimethyl-Lys
      /replace = Ser
      /replace = Thr

<220>
<221> VARIANT
<222> (14)
<223> /replace = Arg
      /replace = homoarginine
      /replace = ornithine
      /replace = any unnatural basic amino acid

<220>
<221> VARIANT
<222> (14)
<223> /replace = Asn
      /replace = Glu
      /replace = Gla
      /replace = Gln

<220>
<221> VARIANT
<222> (14)
<223> /replace = Phe
      /replace = Trp
      /replace = D-Trp
      /replace = neo-Trp

<220>
<221> VARIANT
<222> (14)
<223> /replace = halo-Trp
      /replace = any unnatural aromatic amino acid

<220>
<221> VARIANT
<222> (15)
<223> /replace = Glu
      /replace = Gla
      /replace = Gln
      /replace = Asp

<220>
<221> VARIANT
<222> (15)
<223> /replace = Pro
      /replace = hydroxy-Pro
      /replace = Ser
      /replace = Gly

<220>
<221> VARIANT
<222> (15)
<223> /replace = Thr
      /replace = Lys
      /replace = N-methyl-Lys
      /replace = N,N-dimethyl-Lys

<220>
<221> VARIANT
<222> (15)
<223> /replace = N,N,N-trimethyl-Lys
      /replace = Arg
      /replace = homoarginine
      /replace = ornithine

<220>
<221> VARIANT
<222> (15)
<223> /replace = any unnatural basic amino acid
      /replace = Phe
      /replace = His
      /replace = halo-His

<220>
<221> VARIANT
<222> (15)
<223> /replace = any unnatural aromatic amino acid
      /replace = Leu
      /replace = Met
      /replace = Gly

<220>
<221> VARIANT
<222> (15)
<223> /replace = Ala
      /replace = Tyr
      /replace = nor-Tyr
      /replace = mono-halo-Tyr

<220>
<221> VARIANT
<222> (15)
<223> /replace = di-halo-Tyr
      /replace = O-sulpho-Tyr
      /replace = O-phospho-Tyr
      /replace = nitro-Tyr

<220>
<221> VARIANT
<222> (15)
<223> /replace = any unnatural hydroxy containing amino acid

<220>
<221> VARIANT
<222> (16)
<223> /replace = halo-His
      /replace = Asn
      /replace = Thr
      /replace = Ser

<220>
<221> VARIANT
<222> (16)
<223> /replace = Ile
      /replace = Val
      /replace = Leu
      /replace = Phe

<220>
<221> VARIANT
<222> (16)
<223> /replace = any unnatural aromatic amino acid
      /replace = Arg
      /replace = homoarginine
      /replace = ornithine

<220>
<221> VARIANT
<222> (16)
<223> /replace = Lys
      /replace = N-methyl-Lys
      /replace = N,N-dimethyl-Lys
      /replace = N,N,N-trimethyl-Lys

<220>
<221> VARIANT
<222> (16)
<223> /replace = any unnatural basic amino acid
      /replace = Tyr
      /replace = nor-Try
      /replace = mono-halo-Tyr

<220>
<221> VARIANT
<222> (16)
<223> /replace = di-halo-Tyr
      /replace = O-sulpho-Tyr
      /replace = O-phospho-Tyr
      /replace = nitro-Tyr

<220>
<221> VARIANT
<222> (16)
<223> /replace = any unnatural hydroxy containing amino acid

<220>
<221> VARIANT
<222> (17)
<223> /replace = Ser
      /replace = Thr
      /replace = Ala
      /replace = Gln

<220>
<221> VARIANT
<222> (17)
<223> /replace = hydroxy-Pro
      /replace = Gly
      /replace = Ile
      /replace = Leu

<220>
<221> VARIANT
<222> (17)
<223> /replace = Arg
      /replace = ornithine
      /replace = homoarginine

/replace = Lys

<220>
<221> VARIANT
<222> (17)
<223> /replace = N-methyl-Lys
      /replace = N,N-dimethyl-Lys
      /replace = N,N,N-trimethyl-Lys
      /replace = any unnatural basic amino acid

<220>
<221> VARIANT
<222> (18)
<223> /replace = Asn
      /replace = Gla
      /replace = Asp
      /replace = Gly

<220>
<221> VARIANT
<222> (18)
<223> /replace = His
      /replace = halo-His
      /replace = Ala
      /replace = Leu

<220>
<221> VARIANT
<222> (18)
<223> /replace = Gln
      /replace = Arg
      /replace = ornithine
      /replace = homoarginine

<220>
<221> VARIANT
<222> (18)
<223> /replace = Lys
      /replace = N-methyl-Lys
      /replace = N,N-dimethyl-Lys
      /replace = N,N,N-trimethyl-Lys

<220>
<221> VARIANT
<222> (18)
<223> /replace = any unnatural basic amino acid
      /replace = Tyr
      /replace = nor-Tyr
      /replace = mono-halo-Tyr

<220>
<221> VARIANT
<222> (18)
<223> /replace = di-halo-Tyr
      /replace = O-sulpho-Tyr
      /replace = O-phospho-Tyr
      /replace = nitro-Tyr

<220>
<221> VARIANT

<222> (18)
<223> /replace = any unnatural hydroxy containing amino acid

<220>
<221> VARIANT
<222> (19)
<223> /replace = Met,
      /replace = Ile
      /replace = Thr
      /replace = Ser

<220>
<221> VARIANT
<222> (19)
<223> /replace = Val
      /replace = Pro
      /replace = hydroxy-Pro
      /replace = Phe

<220>
<221> VARIANT
<222> (19)
<223> /replace = any unnatural aromatic amino acid
      /replace = Tyr
      /replace = nor-Tyr
      /replace = mono-halo-Tyr

<220>
<221> VARIANT
<222> (19)
<223> /replace = di-halo-Tyr
      /replace = O-sulpho-Tyr
      /replace = O-phospho-Tyr
      /replace = nitro-Tyr

<220>
<221> VARIANT
<222> (19)
<223> /replace = any unnatural hydroxy containing amino acid,
      /replace = Glu
      /replace = Gla
      /replace = Ala

<220>
<221> VARIANT
<222> (19)
<223> /replace = His
      /replace = halo-His
      /replace = Arg
      /replace = ornithine

<220>
<221> VARIANT
<222> (19)
<223> /replace = homoarginine
      /replace = Lys
      /replace = N-methyl-Lys
      /replace = N,N-dimethyl-Lys

<220>

<221> VARIANT
<222> (19)
<223> /replace = N,N,N-trimethyl-Lys
      /replace = any unnatural basic amino acid

<220>
<221> VARIANT
<222> (21)
<223> /replace = des-Xaa
      /replace = Gly
      /replace = Asp
      /replace = Asn

<220>
<221> VARIANT
<222> (21)
<223> /replace = Ala
      /replace = Ile
      /replace = Leu
      /replace = Ser

<220>
<221> VARIANT
<222> (21)
<223> /replace = Thr
      /replace = His
      /replace = halo-His
      /replace = ornithine

<220>
<221> VARIANT
<222> (21)
<223> /replace = homoarginine
      /replace = Lys
      /replace = N-methyl-Lys
      /replace = N,N-dimethyl-Lys

<220>
<221> VARIANT
<222> (21)
<223> /replace = N,N,N-trimethyl-Lys
      /replace = any unnatural basic amino acid

<220>
<221> VARIANT
<222> (22)
<223> /replace = Gly,
      /replace = Glu
      /replace = Gla
      /replace = Gln

<220>
<221> VARIANT
<222> (22)
<223> /replace = Trp
      /replace = D-Trp
      /replace = neo-Trp
      /replace = halo-Trp

<220>

<221> VARIANT
<222> (22)
<223> /replace = any unnatural aromatic amino acid
      /replace = Arg
      /replace = ornithine
      /replace = homoarginine

<220>
<221> VARIANT
<222> (22)
<223> /replace = Lys
      /replace = N-methyl-Lys
      /replace = N,N-dimethyl-Lys
      /replace = N,N,N-trimethyl-Lys

<220>
<221> VARIANT
<222> (22)
<223> /replace = any unnatural basic amino acid

<220>
<221> VARIANT
<222> (23)
<223> /replace = Ser
      /replace = Thr
      /replace = Val
      /replace = Ile

<220>
<221> VARIANT
<222> (23)
<223> /replace = Ala
      /replace = Arg
      /replace = ornithine
      /replace = homoarginine

<220>
<221> VARIANT
<222> (23)
<223> /replace = Lys
      /replace = N-methyl-Lys
      /replace = N,N-dimethyl-Lys
      /replace = N,N,N-trimethyl-Lys

<220>
<221> VARIANT
<222> (23)
<223> /replace = any unnatural basic amino acid

<220>
<221> VARIANT
<222> (24)
<223> /replace = Val
      /replace = Asp
      /replace = His
      /replace = halo-His

<220>
<221> VARIANT
<222> (24)

<223> /replace = Arg
      /replace = ornithine
      /replace = homoarginine
      /replace = Lys

<220>
<221> VARIANT
<222> (24)
<223> /replace = N-methyl-Lys
      /replace = N,N-dimethyl-Lys
      /replace = N,N,N-trimethyl-Lys
      /replace = any unnatural basic amino acid

<220>
<221> VARIANT
<222> (25)
<223> /replace = Asn
      /replace = Pro
      /replace = hydroxy-Pro

<220>
<221> VARIANT
<222> (26)
<223> /replace = Arg,
      /replace = ornithine
      /replace = homoarginine
      /replace = Lys

<220>
<221> VARIANT
<222> (26)
<223> /replace = N-methyl-Lys
      /replace = N,N-dimethyl-Lys
      /replace = N,N,N-trimethyl-Lys
      /replace = any unnatural basic amino acid

<220>
<221> VARIANT
<222> (27)
<223> /replace = Ser
      /replace = Thr

<220>
<221> VARIANT
<222> (28)
<223> /replace = Leu
      /replace = Ile
      /replace = Val

<400> 3
Xaa Xaa Xaa Xaa Gly Cys Cys Ser His Pro Ala Cys Asn Val Asn His
1               5                   10                  15

Pro Glu Leu Cys Arg Xaa Xaa Xaa Xaa Xaa Xaa Xaa
            20                  25


<210> 4
<211> 14
<212> PRT

<213> Conus imperialis

<220>
<221> VARIANT
<222> (2)
<223> /replace = gamma-carboxy-Glu

<220>
<221> VARIANT
<222> (11)
<223> /replace = N-methyl-Lys
      /replace = N,N-dimethyl-Lys
      /replace = N,N,N-trimethyl-Lys.

<400> 4
Asp Glu Cys Cys Ser Asp Ser Arg Cys Gly Lys Asn Cys Leu
1               5                   10

<210> 5
<211> 12
<212> PRT
<213> Conus imperialis

<220>
<221> VARIANT
<222> (10)
<223> /replace = D-Trp
      /replace = halo-Trp

<400> 5
Ala Cys Cys Ser Asp Arg Arg Cys Arg Trp Arg Cys
1               5                   10


<210> 6
<211> 13
<212> PRT
<213> Conus regius

<400> 6
Phe Thr Cys Cys Arg Arg Gly Thr Cys Ser Gln His Cys
1               5                   10


<210> 7
<211> 13
<212> PRT
<213> Conus regius

<220>
<221> VARIANT
<222> (2)
<223> /replace = nor-Tyr
      /replace = mono-halo-Tyr
      /replace = di-halo-Tyr
      /replace = O-sulpho-Tyr

<220>
<221> VARIANT
<222> (2)

<223> /replace = O-phospho-Tyr
      /replace = nitro-Tyr

<400> 7
Asp Tyr Cys Cys Arg Arg His Ala Cys Thr Leu Ile Cys
 1               5                   10


<210> 8
<211> 13
<212> PRT
<213> Conus regius

<220>
<221> VARIANT
<222> (2)
<223> /replace = nor-Tyr
      /replace = mono-halo-Tyr
      /replace = di-halo-Tyr
      /replace = O-sulpho-Tyr

<220>
<221> VARIANT
<222> (2)
<223> /replace = O-phospho-Tyr
      /replace = nitro-Tyr

<220>
<221> VARIANT
<222> (7)
<223> /replace = hydroxy-Pro

<220>
<221> VARIANT
<222> (8)
<223> /replace = hydroxy-Pro

<400> 8
Asp Tyr Cys Cys Arg Arg Pro Pro Cys Thr Leu Ile Cys
 1               5                   10


<210> 9
<211> 13
<212> PRT
<213> Conus regius

<220>
<221> VARIANT
<222> (6)
<223> /replace = hydroxy-Pro

<220>
<221> VARIANT
<222> (10)
<223> /replace = nor-Tyr
      /replace = mono-halo-Tyr
      /replace = di-halo-Tyr
      /replace = O-sulpho-Tyr

<220>
<221> VARIANT
<222> (10)
<223> /replace = O-phospho-Tyr
      /replace = nitro-Tyr

<400> 9
Gly Cys Cys Ser Asp Pro Arg Cys Arg Tyr Arg Cys Arg
 1               5                   10


<210> 10
<211> 13
<212> PRT
<213> Conus regius

<220>
<221> VARIANT
<222> (7)
<223> /replace = hydroxy-Pro

<220>
<221> VARIANT
<222> (11)
<223> /replace = D-Trp
      /replace = halo-Trp

<400> 10
Gly Gly Cys Cys Ser Asp Pro Arg Cys Ala Trp Arg Cys
 1               5                   10


<210> 11
<211> 17
<212> PRT
<213> Conus regius

<220>
<221> VARIANT
<222> (3)
<223> /replace = D-Trp
      /replace = halo-Trp

<220>
<221> VARIANT
<222> (9)
<223> /replace = gamma-carboxy-Glu

<220>
<221> VARIANT
<222> (10)
<223> /replace = hydroxy-Pro

<220>
<221> VARIANT
<222> (15)
<223> /replace = N-methyl-Lys
      /replace = N,N-dimethyl-Lys
      /replace = N,N,N-trimethyl-Lys

<400> 11
Ile Ala Trp Asp Ile Cys Cys Ser Glu Pro Asp Cys Asn His Lys Cys
1               5                   10                  15

Val


<210> 12
<211> 12
<212> PRT
<213> Conus regius

<220>
<221> VARIANT
<222> (6)
<223> /replace = hydroxy-Pro

<220>
<221> VARIANT
<222> (9)
<223> /replace = N-methyl-Lys
      /replace = N,N-dimethyl-Lys
      /replace = N,N,N-trimethyl-Lys

<400> 12
Gly Cys Cys Ser Asp Pro Arg Cys Lys His Gln Cys
1               5                   10


<210> 13
<211> 14
<212> PRT
<213> Conus sponsalis

<220>
<221> VARIANT
<222> (5)
<223> /replace = hydroxy-Pro

<220>
<221> VARIANT
<222> (8)
<223> /replace = N-methyl-Lys
      /replace = N,N-dimethyl-Lys
      /replace = N,N,N-trimethyl-Lys

<220>
<221> VARIANT
<222> (11)
<223> /replace = hydroxy-Pro

<400> 13
Cys Cys Ser Asp Xaa Ala Cys Lys Gln Thr Pro Gly Cys Arg
1               5                   10


<210> 14
<211> 13
<212> PRT
<213> Conus sponsalis

<220>
<221> VARIANT
<222> (3)
<223> /replace = gamma-carboxy-Glu

<220>
<221> VARIANT
<222> (5)
<223> /replace = hydroxy-Pro

<400> 14
Cys Cys Glu Asn Pro Ala Cys Arg His Thr Gln Gly Cys
1               5                   10


<210> 15
<211> 13
<212> PRT
<213> Conus sulcatus

<220>
<221> VARIANT
<222> (4)
<223> /replace = D-Trp
      /replace = halo-Trp

<220>
<221> VARIANT
<222> (6)
<223> /replace = hydroxy-Pro

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<400> 15
Gly Cys Cys Trp His Pro Ala Cys Gly Arg His Tyr Cys
1               5                   10


<210> 16
<211> 14
<212> PRT
<213> Conus achatinus

<220>
<221> VARIANT
<222> (2)
<223> /replace = hydroxy-Pro

<220>
<221> VARIANT
<222> (7)
<223> /replace = hydroxy-Pro

<220>
<221> VARIANT
<222> (11)
<223> /replace = N-methyl-Lys
      /replace = N,N-dimethyl-Lys
      /replace = N,N,N-trimethyl-Lys

<400> 16
Ala Pro Cys Cys Asn Asn Pro Ala Cys Val Lys His Arg Cys
1               5                   10


<210> 17
<211> 15
<212> PRT
<213> Conus bullatus

<220>
<221> VARIANT
<222> (2)
<223> /replace = hydroxy-Pro

<220>
<221> VARIANT
<222> (8)
<223> /replace = hydroxy-Pro

<220>
<221> VARIANT
<222> (12)
<223> /replace = N-methyl-Lys
      /replace = N,N-dimethyl-Lys
      /replace = N,N,N-trimethyl-Lys

<400> 17
Ala Pro Gly Cys Cys Asn Asn Pro Ala Cys Val Lys His Arg Cys
1               5                   10                  15


<210> 18
<211> 14
<212> PRT
<213> Conus bullatus

<220>
<221> VARIANT
<222> (1)
<223> /replace = hydroxy-Pro

<220>
<221> VARIANT
<222> (2)
<223> /replace = hydroxy-Pro

<220>
<221> VARIANT
<222> (7)
<223> /replace = hydroxy-Pro

<220>
<221> VARIANT
<222> (11)
<223> /replace = N-methyl-Lys
      /replace = N,N-dimethyl-Lys
      /replace = N,N,N-trimethyl-Lys

<400> 18
Pro Pro Cys Cys Asn Asn Pro Ala Cys Val Lys His Arg Cys
1               5                   10


<210> 19
<211> 16
<212> PRT
<213> Conus bullatus

<220>
<221> VARIANT
<222> (2)
<223> /replace = gamma-carboxy-Glu

<220>
<221> VARIANT
<222> (6)
<223> /replace = D-Trp.
      /replace = halo-Trp

<220>
<221> VARIANT
<222> (8)
<223> /replace = hydroxy-Pro

<220>
<221> VARIANT
<222> (11)
<223> /replace = hydroxy-Pro

<220>
<221> VARIANT
<222> (13)
<223> /replace = hydroxy-Pro

<400> 19
Asp Glu Asn Cys Cys Trp Asn Pro Ser Cys Pro Arg Pro Arg Cys Thr
1               5                   10                  15


<210> 20
<211> 13
<212> PRT
<213> Conus bullatus

<220>
<221> VARIANT

<222> (6)
<223> /replace = hydroxy-Pro

<220>
<221> VARIANT
<222> (7)
<223> /replace = hydroxy-Pro

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<400> 20
Gly Cys Cys Ser Arg Pro Pro Cys Ala Val Leu Tyr Cys
1               5                   10


<210> 21
<211> 13
<212> PRT
<213> Conus circumcisus

<220>
<221> VARIANT
<222> (6)
<223> /replace = hydroxy-Pro

<400> 21
Gly Cys Cys Gly Asn Pro Asp Cys Thr Ser His Ser Cys
1               5                   10


<210> 22
<211> 16
<212> PRT
<213> Conus stercusmuscarum

<220>
<221> VARIANT
<222> (6)
<223> /replace = hydroxy-Pro

<220>
<221> VARIANT
<222> (11)
<223> /replace = gamma-carboxy-Glu

<400> 22
Gly Cys Cys Ser Asn Pro Val Cys His Leu Glu His Ser Asn Met Cys
1               5                   10                  15

<210> 23
<211> 17
<212> PRT
<213> Conus obscurus

<220>
<221> VARIANT
<222> (6)
<223> /replace = hydroxy-Pro

<220>
<221> VARIANT
<222> (14)
<223> /replace = gamma-carboxy-Glu

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 23
Gly Cys Cys Ser Asn Pro Val Cys Arg Gln Asn Asn Ala Glu Tyr Cys
1               5                   10                  15

Arg


<210> 24
<211> 18
<212> PRT
<213> Conus textile

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro

<220>
<221> VARIANT

<222> (15)
<223> /replace = gamma-carboxy-Glu

<400> 24
Pro Gln Cys Cys Ser His Pro Ala Cys Asn Val Asp His Pro Glu Ile
1               5                   10                  15

Cys Arg

<210> 25
<211> 18
<212> PRT
<213> Conus radiatus

<220>
<221> VARIANT
<222> (1)
<223> /replace = hydroxy-Pro

<220>
<221> VARIANT
<222> (2)
<223> /replace = gamma-carboxy-Glu

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 25
Pro Glu Cys Cys Ser His Pro Ala Cys Asn Val Asp His Pro Glu Ile
1               5                   10                  15

Cys Arg

<210> 26
<211> 18
<212> PRT
<213> Conus radiatus

<220>
<221> VARIANT
<222> (1)
<223> /replace = hydroxy-Pro

<220>

<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 26
Pro Gln Cys Cys Ser His Pro Ala Cys Asn Val Asp His Pro Glu Ile
1               5                   10                  15

Cys Asp

<210> 27
<211> 18
<212> PRT
<213> Conus omaria

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 27
Pro Arg Cys Cys Ser His Pro Ala Cys Asn Val Asp His Pro Glu Ile
1               5                   10                  15

Cys Arg

<210> 28
<211> 18
<212> PRT
<213> Conus omaria

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro

<400> 28
Pro Gln Cys Cys Ser His Pro Ala Cys Asn Val Asp His Pro Gly Ile
1               5                   10                  15

Cys Arg

<210> 29
<211> 18
<212> PRT
<213> Conus omaria

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 29
Pro Gln Cys Cys Ser His Pro Ala Cys Asn Val Asp His Pro Glu Thr
1               5                   10                  15

Cys Arg

<210> 30
<211> 18
<212> PRT
<213> Conus omaria

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 30
Pro Gln Cys Cys Ser His Pro Ala Cys Asn Val Asp His Pro Glu Val
1               5                   10                  15

Cys Arg



<210> 31
<211> 18
<212> PRT
<213> Conus omaria

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 31
Pro Gln Cys Cys Ser His Pro Ala Cys Asn Ile Asp His Pro Glu Ile
1               5                   10                  15

Cys Arg

<210> 32
<211> 21
<212> PRT
<213> Conus omaria

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro
<220>
<221> VARIANT
<222> (15)
<223> /replace = gamma-carboxy-Glu

<400> 32
Pro Gln Cys Cys Ser His Pro Ala Cys Asn Val Asp His Pro Glu Ile
1               5                   10                  15

Cys Arg Arg Arg Arg
            20


<210> 33
<211> 17
<212> PRT
<213> Conus betulinus

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 33
Gly Gly Cys Cys Ser His Pro Ala Cys Ala Val Asn His Pro Glu Leu
1               5                   10                  15

Cys

<210> 34
<211> 16
<212> PRT
<213> Conus betulinus

<220>
<221> VARIANT
<222> (6)
<223> /replace = hydroxy-Pro

<220>
<221> VARIANT
<222> (13)
<223> /replace = hydroxy-Pro

<220>
<221> VARIANT
<222> (14)
<223> /replace = gamma-carboxy-Glu

<400> 34
Gly Cys Cys Ser His Pro Ala Cys Ser Val Asn His Pro Glu Leu Cys
1               5                   10                  15


<210> 35
<211> 16
<212> PRT
<213> Conus dalli

<220>
<221> VARIANT
<222> (6)
<223> /replace = hydroxy-Pro

<220>
<221> VARIANT
<222> (13)
<223> /replace = hydroxy-Pro

<220>
<221> VARIANT
<222> (14)
<223> /replace = gamma-carboxy-Glu

<400> 35
Gly Cys Cys Ser His Pro Ala Cys Asn Val Asp His Pro Glu Ile Cys
1               5                   10                  15


<210> 36
<211> 19
<212> PRT
<213> Conus obscurus

<220>
<221> VARIANT
<222> (6)
<223> /replace = hydroxy-Pro

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (14)
<223> /replace = gamma-carboxy-Glu

<220>
<221> VARIANT
<222> (15)
<223> /replace = hydroxy-Pro

<220>
<221> VARIANT
<222> (18)
<223> /replace = gamma-carboxy-Glu

<400> 36
Gly Cys Cys Ser His Pro Ala Cys Ser Gly Lys Thr Gln Glu Pro Cys
1               5                   10                  15

Arg Glu Ser



<210> 37
<211> 18
<212> PRT
<213> Conus tulipa

<220>
<221> VARIANT
<222> (1)
<223> /replace = hydroxy-Pro

<220>
<221> VARIANT
<222> (6)
<223> /replace = hydroxy-Pro

<220>
<221> VARIANT
<222> (13)
<223> /replace = hydroxy-Pro

<220>
<221> VARIANT
<222> (14)
<223> /replace = gamma-carboxy-Glu

<400> 37
Pro Cys Cys Ser His Pro Ala Cys Ser Gly Asn Asn Pro Glu Phe Cys
1               5                   10                  15

Arg Gln

<210> 38
<211> 18
<212> PRT
<213> Conus tulipa

<220>
<221> VARIANT
<222> (6)
<223> /replace = hydroxy-Pro

<220>
<221> VARIANT
<222> (13)
<223> /replace = hydroxy-Pro

<220>
<221> VARIANT
<222> (14)
<223> /replace = gamma-carboxy-Glu

<400> 38
Gly Cys Cys Ser His Pro Ala Cys Ser Gly Asn Asn Pro Glu Phe Cys
1               5                   10                  15

Arg Gln

<210> 39
<211> 16
<212> PRT
<213> Conus pennaceus

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (13)
<223> /replace = hydroxy-Pro

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 39
Gly Cys Cys Ser His Pro Pro Cys Ala Met Asn Asn Pro Asp Tyr Cys
1               5                   10                  15


<210> 40
<211> 16
<212> PRT
<213> Conus pennaceus

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (13)
<223> /replace = hydroxy-Pro

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 40
Gly Cys Cys Ser His Pro Pro Cys Phe Leu Asn Asn Pro Asp Tyr Cys
1               5                   10                  15


<210> 41
<211> 17
<212> PRT
<213> Conus textile

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (13)
<223> /replace = hydroxy-Pro

<400> 41
Gly Cys Cys Ser Asn Pro Pro Cys Ile Ala Lys Asn Pro His Met Cys
1               5                   10                  15

Gly


<210> 42
<211> 16
<212> PRT
<213> Conus distans

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (13)
<223> /replace = hydroxy-Pro

<400> 42
Gly Cys Cys Ser Asn Pro Pro Cys Ala His Asn Asn Pro Asp Cys Arg
1               5                   10                  15

<210> 43
<211> 17
<212> PRT
<213> Conus tulipa

<220>
<221> VARIANT
<222> (6)
<223> /replace = hydroxy-Pro

<220>
<221> VARIANT

<222> (13)
<223> /replace = hydroxy-Pro

<400> 43
Gly Cys Cys Ser Asn Pro Ala Cys Ala Gly Asn Asn Pro His Val Cys
1               5                   10                  15

Arg


<210> 44
<211> 16
<212> PRT
<213> Conus dalli

<220>
<221> VARIANT
<222> (6)
<223> /replace = hydroxy-Pro

<220>
<221> VARIANT
<222> (13)
<223> /replace = hydroxy-Pro

<400> 44
Gly Cys Cys Ser Arg Pro Ala Cys Ile Ala Asn Asn Pro Asp Leu Cys
1               5                   10                  15


<210> 45
<211> 20
<212> PRT
<213> Conus circumcisus

<220>
<221> VARIANT
<222> (6)
<223> /replace = hydroxy-Pro

<220>
<221> VARIANT
<222> (11)
<223> /replace = gamma-carboxy-Glu

<220>
<221> VARIANT
<222> (13)
<223> /replace = hydroxy-Pro

<220>
<221> VARIANT
<222> (14)
<223> /replace = gamma-carboxy-Glu

<400> 45
Gly Cys Cys Ser Asn Pro Val Cys His Val Glu His Pro Glu Leu Cys
1               5                   10                  15

Arg Arg Arg Arg

            20

<210> 46
<211> 18
<212> PRT
<213> Conus sulcatus

<220>
<221> VARIANT
<222> (7)
<223> /replace = hydroxy-Pro

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (12)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 46
Gly Gly Cys Cys Ser Phe Pro Ala Cys Arg Lys Pro Arg Pro Glu Met
1               5                   10                  15

Cys Gly

<210> 47
<211> 18
<212> PRT
<213> Conus textile

<220>
<221> VARIANT
<222> (1)
<223> /replace = hydroxy-Pro

<220>
<221> VARIANT
<222> (2)
<223> /replace = gamma-carboxy-Glu

<220>
<221> VARIANT

<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 47
Pro Glu Cys Cys Ser Asp Pro Arg Cys Asn Ser Ser His Pro Glu Leu
1               5                   10                  15

Cys Arg

<210> 48
<211> 18
<212> PRT
<213> Conus dalli

<220>
<221> VARIANT
<222> (1)
<223> /replace = hydroxy-Pro

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 48
Pro Gln Cys Cys Ser Asp Pro Arg Cys Asn Val Gly His Pro Glu Leu
1               5                   10                  15

Cys Gly

<210> 49
<211> 18
<212> PRT
<213> Conus dalli

<220>

<221> VARIANT
<222> (1)
<223> /replace = pyro-Glu

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 49
Gln Val Cys Cys Ser Asp Pro Arg Cys Asn Val Gly His Pro Glu Ile
1               5                   10                  15

Cys Gly

<210> 50
<211> 16
<212> PRT
<213> Conus textile

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (13)
<223> /replace = hydroxy-Pro

<400> 50
Gly Cys Cys Ser Arg Pro Pro Cys Ile Ala Asn Asn Pro Asp Leu Cys
1               5                   10                  15

<210> 51
<211> 18
<212> PRT
<213> Conus omaria

<220>
<221> VARIANT
<222> (1)

<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 51
Pro Gln Cys Cys Ser His Leu Ala Cys Asn Val Asp His Pro Glu Ile
1               5                   10                  15

Cys Arg



<210> 52
<211> 19
<212> PRT
<213> Conus sulcatus

<220>
<221> VARIANT
<222> (5)
<223> /replace = nor-Tyr
      /replace = mono-halo-Tyr
      /replace = di-halo-Tyr
      /replace = O-sulpho-Tyr

<220>
<221> VARIANT
<222> (5)
<223> /replace = O-phospho-Tyr
      /replace = nitro-Tyr

<220>
<221> VARIANT
<222> (13)
<223> /replace = hydroxy-Pro

<220>
<221> VARIANT
<222> (14)
<223> /replace = gamma-carboxy-Glu

<220>
<221> VARIANT
<222> (18)
<223> /replace = D-Trp
      /replace = halo-Trp

<400> 52
Gly Cys Cys Ser Tyr Phe Asp Cys Arg Met Met Phe Pro Glu Met Cys
1               5                   10                  15

Gly Trp Arg

<210> 53
<211> 18
<212> PRT
<213> Conus sulcatus

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<220>
<221> VARIANT
<222> (14)
<223> /replace = hydroxy-Pro

<220>
<221> VARIANT
<222> (15)
<223> /replace = gamma-carboxy-Glu

<400> 53
Gly Gly Cys Cys Ser Phe Ala Ala Cys Arg Lys Tyr Arg Pro Glu Met
 1               5                   10                  15

Cys Gly

<210> 54
<211> 20
<212> PRT
<213> Conus sulcatus

<220>
<221> VARIANT
<222> (7)
<223> /replace = hydroxy-Pro

<220>
<221> VARIANT
<222> (10)

<223> /replace = nor-Tyr
      /replace = mono-halo-Tyr
      /replace = di-halo-Tyr
      /replace = O-sulpho-Tyr

<220>
<221> VARIANT
<222> (10)
<223> /replace = O-phospho-Tyr
      /replace = nitro-Tyr

<220>
<221> VARIANT
<222> (15)
<223> /replace = gamma-carboxy-Glu

<400> 54
Gly Gly Cys Cys Phe His Pro Val Cys Tyr Ile Asn Leu Leu Glu Met
 1               5                   10                  15

Cys Arg Gln Arg
            20


<210> 55
<211> 19
<212> PRT
<213> Conus betulinus

<220>
<221> VARIANT
<222> (7)
<223> /replace = nor-Tyr
      /replace = mono-halo-Tyr
      /replace = di-halo-Tyr
      /replace = O-sulpho-Tyr

<220>
<221> VARIANT
<222> (7)
<223> /replace = O-phospho-Tyr
      /replace = nitro-Tyr

<220>
<221> VARIANT
<222> (8)
<223> /replace = hydroxy-Pro

<220>
<221> VARIANT
<222> (9)
<223> /replace = hydroxy-Pro

<220>
<221> VARIANT
<222> (11)
<223> /replace = nor-Tyr
      /replace = mono-halo-Tyr
      /replace = di-halo-Tyr
      /replace = O-sulpho-Tyr

<220>
<221> VARIANT
<222> (11)
<223> /replace = O-phospho-Tyr
      /replace = nitro-Tyr

<220>
<221> VARIANT
<222> (12)
<223> /replace = gamma-carboxy-Glu

<220>
<221> VARIANT
<222> (14)
<223> /replace = nor-Tyr
      /replace = mono-halo-Tyr
      /replace = di-halo-Tyr
      /replace = O-sulpho-Tyr

<220>
<221> VARIANT
<222> (14)
<223> /replace = O-phospho-Tyr
      /replace = nitro-Tyr

<220>
<221> VARIANT
<222> (15)
<223> /replace = hydroxy-Pro

<220>
<221> VARIANT
<222> (16)
<223> /replace = gamma-carboxy-Glu

<400> 55
Ser Ala Thr Cys Cys Asn Tyr Pro Pro Cys Tyr Glu Thr Tyr Pro Glu
1               5                   10                  15

Ser Cys Leu

<210> 56
<211> 17
<212> PRT
<213> Conus betulinus

<220>
<221> VARIANT
<222> (5)
<223> /replace = nor-Tyr
      /replace = mono-halo-Tyr
      /replace = di-halo-Tyr
      /replace = O-sulpho-Tyr

<220>
<221> VARIANT
<222> (5)

<223> /replace = O-phospho-Tyr
      /replace = nitro-Tyr

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (10)
<223> /replace = gamma-carboxy-Glu

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<220>
<221> VARIANT
<222> (13)
<223> /replace = hydroxy-Pro

<220>
<221> VARIANT
<222> (14)
<223> /replace = gamma-carboxy-Glu

<400> 56
Ala Cys Cys Ala Tyr Pro Pro Cys Phe Glu Ala Tyr Pro Glu Arg Cys
 1               5                   10                  15

Leu


<210> 57
<211> 19
<212> PRT
<213> Conus betulinus

<220>
<221> VARIANT
<222> (3)
<223> /replace = gamma-carboxy-Glu

<220>

<221> VARIANT
<222> (6)
<223> /replace = nor-Tyr
      /replace = mono-halo-Tyr
      /replace = di-halo-Tyr
      /replace = O-sulpho-Tyr

<220>
<221> VARIANT
<222> (6)
<223> /replace = nor-Tyr
      /replace = O-phospho-Tyr
      /replace = nitro-Tyr

<220>
<221> VARIANT
<222> (7)
<223> /replace = nor-Tyr
      /replace = mono-halo-Tyr
      /replace = di-halo-Tyr
      /replace = O-sulpho-Tyr

<220>
<221> VARIANT
<222> (7)
<223> /replace = O-phospho-Tyr
      /replace = nitro-Tyr

<220>
<221> VARIANT
<222> (8)
<223> /replace = hydroxy-Pro

<220>
<221> VARIANT
<222> (9)
<223> /replace = hydroxy-Pro

<220>
<221> VARIANT
<222> (11)
<223> /replace = nor-Tyr
      /replace = mono-halo-Tyr
      /replace = di-halo-Tyr
      /replace = O-sulpho-Tyr

<220>
<221> VARIANT
<222> (11)
<223> /replace = O-phospho-Tyr
      /replace = nitro-Tyr

<220>
<221> VARIANT
<222> (12)
<223> /replace = gamma-carboxy-Glu

<220>
<221> VARIANT
<222> (14)

<223> /replace = nor-Tyr
      /replace = mono-halo-Tyr
      /replace = di-halo-Tyr
      /replace = O-sulpho-Tyr

<220>
<221> VARIANT
<222> (14)
<223> /replace = O-phospho-Tyr
      /replace = nitro-Tyr

<220>
<221> VARIANT
<222> (15)
<223> /replace = hydroxy-Pro

<220>
<221> VARIANT
<222> (16)
<223> /replace = gamma-carboxy-Glu

<400> 57
Asn Ala Glu Cys Cys Tyr Tyr Pro Pro Cys Tyr Glu Ala Tyr Pro Glu
 1               5                  10                  15

Ile Cys Leu

<210> 58
<211> 227
<212> DNA
<213> Conus magus

<220>
<221> CDS
<222> (1)..(189)

<400> 58
atg ttc acc gtg ttt ctg ttg gtt gtc ttg gca acc act gtc gtt tcc     48
Met Phe Thr Val Phe Leu Leu Val Val Leu Ala Thr Thr Val Val Ser
 1               5                  10                  15

ttc cct tca gat cgt gca tct gat ggc agg aat gcc gca gcc aac gac     96
Phe Pro Ser Asp Arg Ala Ser Asp Gly Arg Asn Ala Ala Ala Asn Asp
            20                  25                  30

aaa gcg tct gac gtg atc acg ctg gcc ctc aag gga tgc tgt tcc aac     144
Lys Ala Ser Asp Val Ile Thr Leu Ala Leu Lys Gly Cys Cys Ser Asn
        35                  40                  45

cct gtc tgt cac ttg gag cat tca aac ctt tgt ggt aga aga cgc         189
Pro Val Cys His Leu Glu His Ser Asn Leu Cys Gly Arg Arg Arg
    50                  55                  60

tgatgctcca ggaccctctg aaccacgacg ttcgagca                           227

<210> 59
<211> 63

<212> PRT
<213> Conus magus

<400> 59
Met Phe Thr Val Phe Leu Leu Val Val Leu Ala Thr Thr Val Val Ser
1               5                   10                  15

Phe Pro Ser Asp Arg Ala Ser Asp Gly Arg Asn Ala Ala Ala Asn Asp
            20                  25                  30

Lys Ala Ser Asp Val Ile Thr Leu Ala Leu Lys Gly Cys Cys Ser Asn
        35                  40                  45

Pro Val Cys His Leu Glu His Ser Asn Leu Cys Gly Arg Arg Arg
    50                  55                  60


<210> 60
<211> 208
<212> DNA
<213> Conus aulicus

<220>
<221> CDS
<222> (1)..(168)

<400> 60
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc act tca gat cgt gca tct gat ggc agg aag gac gca gcg tct ggc      96
Phe Thr Ser Asp Arg Ala Ser Asp Gly Arg Lys Asp Ala Ala Ser Gly
            20                  25                  30

ctg atc gct ctg acc atc aag gga tgc tgt tct tat cct ccc tgt ttc      144
Leu Ile Ala Leu Thr Ile Lys Gly Cys Cys Ser Tyr Pro Pro Cys Phe
        35                  40                  45

gcg act aat tca gac tat tgt ggt tgacgacgct gatgctccag gaccctctga     198
Ala Thr Asn Ser Asp Tyr Cys Gly
    50                  55

accacgacgt                                                           208


<210> 61
<211> 56
<212> PRT
<213> Conus aulicus

<400> 61
Met Phe Thr Val Phe Leu Leu Val Val Leu Ala Thr Thr Val Val Ser
1               5                   10                  15

Phe Thr Ser Asp Arg Ala Ser Asp Gly Arg Lys Asp Ala Ala Ser Gly
            20                  25                  30

Leu Ile Ala Leu Thr Ile Lys Gly Cys Cys Ser Tyr Pro Pro Cys Phe
        35                  40                  45

Ala Thr Asn Ser Asp Tyr Cys Gly
     50                  55

<210> 62
<211> 205
<212> DNA
<213> Conus aulicus

<220>
<221> CDS
<222> (1)..(174)

<400> 62
atg ttc acc gtg ttt ctg ttg gtc gtc ttg gca acc acc gtc gtt tcc     48
Met Phe Thr Val Phe Leu Leu Val Val Leu Ala Thr Thr Val Val Ser
  1               5                  10                  15

ttc act tca gat cgt gca tct gat ggc agg aag gac gca gcg tct ggc     96
Phe Thr Ser Asp Arg Ala Ser Asp Gly Arg Lys Asp Ala Ala Ser Gly
             20                  25                  30

ctg att gct ctg acc atg aag gga tgc tgt tct tat cct ccc tgt ttc     144
Leu Ile Ala Leu Thr Met Lys Gly Cys Cys Ser Tyr Pro Pro Cys Phe
         35                  40                  45

gcg act aat cca gac tgt ggt cga cga cgc tgatgctcca ggaccctctg      194
Ala Thr Asn Pro Asp Cys Gly Arg Arg Arg
     50                  55

aaccacgacg t                                                        205

<210> 63
<211> 58
<212> PRT
<213> Conus aulicus

<400> 63
Met Phe Thr Val Phe Leu Leu Val Val Leu Ala Thr Thr Val Val Ser
  1               5                  10                  15

Phe Thr Ser Asp Arg Ala Ser Asp Gly Arg Lys Asp Ala Ala Ser Gly
             20                  25                  30

Leu Ile Ala Leu Thr Met Lys Gly Cys Cys Ser Tyr Pro Pro Cys Phe
         35                  40                  45

Ala Thr Asn Pro Asp Cys Gly Arg Arg Arg
     50                  55

<210> 64
<211> 223
<212> DNA
<213> Conus textile

<220>
<221> CDS

<222> (1)..(192)

<400> 64
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc tct tca ggt cgt agt aca ttt cgt ggc agg aat gcc gca gcc aaa     96
Phe Ser Ser Gly Arg Ser Thr Phe Arg Gly Arg Asn Ala Ala Ala Lys
            20                  25                  30

gcg tct ggc ctg gtc agt ctg act gac agg aga cca gaa tgc tgt agt     144
Ala Ser Gly Leu Val Ser Leu Thr Asp Arg Arg Pro Glu Cys Cys Ser
        35                  40                  45

gat cct cgc tgt aac tcg agt cat cca gaa ctt tgt ggt gga aga cgc     192
Asp Pro Arg Cys Asn Ser Ser His Pro Glu Leu Cys Gly Gly Arg Arg
    50                  55                  60

tgatgctcca ggaccctctg aaccacgacg t                                  223


<210> 65
<211> 64
<212> PRT
<213> Conus textile

<400> 65
Met Phe Thr Val Phe Leu Leu Val Val Leu Ala Thr Thr Val Val Ser
1               5                   10                  15

Phe Ser Ser Gly Arg Ser Thr Phe Arg Gly Arg Asn Ala Ala Ala Lys
            20                  25                  30

Ala Ser Gly Leu Val Ser Leu Thr Asp Arg Arg Pro Glu Cys Cys Ser
        35                  40                  45

Asp Pro Arg Cys Asn Ser Ser His Pro Glu Leu Cys Gly Gly Arg Arg
    50                  55                  60


<210> 66
<211> 244
<212> DNA
<213> Conus textile

<220>
<221> CDS
<222> (1)..(168)

<400> 66
atg ttc acc gtg ttt ctg ttg gtt gtc ttg gca acc gcc gtc gtt tcc     48
Met Phe Thr Val Phe Leu Leu Val Val Leu Ala Thr Ala Val Val Ser
1               5                   10                  15

ttc act tca gat cgt gca tct gat gac ggg aaa gcc gct gcg tct gac     96
Phe Thr Ser Asp Arg Ala Ser Asp Asp Gly Lys Ala Ala Ala Ser Asp
            20                  25                  30
ctg atc act ctg acc atc aag gga tgc tgt tct cgt cct ccc tgt atc     144
Leu Ile Thr Leu Thr Ile Lys Gly Cys Cys Ser Arg Pro Pro Cys Ile

        35                  40                  45

gcg aat aat cca gac ttg tgt ggt tgacgacgct gatgctccag aacggtctga 198
Ala Asn Asn Pro Asp Leu Cys Gly
    50                  55

accacgacgt tcgagcaatg ttcaccgtgt ttctgttggt tgtctt 244

<210> 67
<211> 56
<212> PRT
<213> Conus textile

<400> 67
Met Phe Thr Val Phe Leu Leu Val Val Leu Ala Thr Ala Val Val Ser
1               5                   10                  15

Phe Thr Ser Asp Arg Ala Ser Asp Asp Gly Lys Ala Ala Ala Ser Asp
            20                  25                  30

Leu Ile Thr Leu Thr Ile Lys Gly Cys Cys Ser Arg Pro Pro Cys Ile
        35                  40                  45

Ala Asn Asn Pro Asp Leu Cys Gly
    50                  55

<210> 68
<211> 223
<212> DNA
<213> Conus textile

<220>
<221> CDS
<222> (1)..(183)

<400> 68
atg ttc acc gtg ttt ctg ttg gtt gtc ttg gca acc acc gtc gtt tcc 48
Met Phe Thr Val Phe Leu Leu Val Val Leu Ala Thr Thr Val Val Ser
1               5                   10                  15

ttc act tca ggt cgt agt aca ttt cgt ggc agg aat gcc gca gcc aaa 96
Phe Thr Ser Gly Arg Ser Thr Phe Arg Gly Arg Asn Ala Ala Ala Lys
            20                  25                  30

gcg tct ggc ctg gtc agt ctg act gac agg aga cca caa tgc tgt tct 144
Ala Ser Gly Leu Val Ser Leu Thr Asp Arg Arg Pro Gln Cys Cys Ser
        35                  40                  45

cat cct gcc tgt aac gta gat cat cca gaa att tgt cgt tgaagacgct 193
His Pro Ala Cys Asn Val Asp His Pro Glu Ile Cys Arg
    50                  55                  60

gatgctccag gaccctctga accacgacgt 223

<210> 69
<211> 61
<212> PRT

<213> Conus textile

<400> 69
Met Phe Thr Val Phe Leu Leu Val Val Leu Ala Thr Thr Val Val Ser
1               5                   10                  15

Phe Thr Ser Gly Arg Ser Thr Phe Arg Gly Arg Asn Ala Ala Ala Lys
            20                  25                  30

Ala Ser Gly Leu Val Ser Leu Thr Asp Arg Arg Pro Gln Cys Cys Ser
        35                  40                  45

His Pro Ala Cys Asn Val Asp His Pro Glu Ile Cys Arg
    50                  55                  60


<210> 70
<211> 223
<212> DNA
<213> Conus radiatus

<220>
<221> CDS
<222> (1)..(183)

<400> 70
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca ggt cgt cgt aca ttt cat ggc agg aat gcc gca gcc aaa     96
Phe Thr Ser Gly Arg Arg Thr Phe His Gly Arg Asn Ala Ala Ala Lys
            20                  25                  30

gcg tct ggc ctg gtc agt ctg act gac agg aga cca gaa tgc tgt tct     144
Ala Ser Gly Leu Val Ser Leu Thr Asp Arg Arg Pro Glu Cys Cys Ser
        35                  40                  45

cat cct gcc tgt aac gta gat cat cca gaa att tgt cgt tgaagacgct     193
His Pro Ala Cys Asn Val Asp His Pro Glu Ile Cys Arg
    50                  55                  60

gatgctccag gaccctctga accacgacgt                                   223


<210> 71
<211> 61
<212> PRT
<213> Conus radiatus

<400> 71
Met Phe Thr Val Phe Leu Leu Val Val Leu Ala Thr Thr Val Val Ser
1               5                   10                  15

Phe Thr Ser Gly Arg Arg Thr Phe His Gly Arg Asn Ala Ala Ala Lys
            20                  25                  30

Ala Ser Gly Leu Val Ser Leu Thr Asp Arg Arg Pro Glu Cys Cys Ser
        35                  40                  45

His Pro Ala Cys Asn Val Asp His Pro Glu Ile Cys Arg
     50                  55                  60

<210> 72
<211> 223
<212> DNA
<213> Conus radiatus

<220>
<221> CDS
<222> (1)..(183)

<400> 72
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
  1               5                  10                  15

ttc act tca ggt cgt agt aca ttt cgt ggc agg aat gcc gca gcc aaa      96
Phe Thr Ser Gly Arg Ser Thr Phe Arg Gly Arg Asn Ala Ala Ala Lys
             20                  25                  30

gcg tct ggc ctg gtc agt ctg act gac agg aga cca caa tgc tgt tct     144
Ala Ser Gly Leu Val Ser Leu Thr Asp Arg Arg Pro Gln Cys Cys Ser
         35                  40                  45

cat cct gcc tgt aac gta gat cat cca gaa att tgc gat tgaagacgct      193
His Pro Ala Cys Asn Val Asp His Pro Glu Ile Cys Asp
     50                  55                  60

gatgctccag gaccctctga accacgacgt                                    223

<210> 73
<211> 61
<212> PRT
<213> Conus radiatus

<400> 73
Met Phe Thr Val Phe Leu Leu Val Val Leu Ala Thr Thr Val Val Ser
  1               5                  10                  15

Phe Thr Ser Gly Arg Ser Thr Phe Arg Gly Arg Asn Ala Ala Ala Lys
             20                  25                  30

Ala Ser Gly Leu Val Ser Leu Thr Asp Arg Arg Pro Gln Cys Cys Ser
         35                  40                  45

His Pro Ala Cys Asn Val Asp His Pro Glu Ile Cys Asp
     50                  55                  60

<210> 74
<211> 218
<212> DNA
<213> Conus striatus

<220>
<221> CDS
<222> (1)..(171)

<400> 74
atg ttc act gtg ttt ctg ttg gtt gtc ttg gca atc act gtc gtt tcc     48
Met Phe Thr Val Phe Leu Leu Val Val Leu Ala Ile Thr Val Val Ser
1               5                   10                  15

ttc cct tta gat cgt gaa tct gat ggc gcg aat gcc gaa gcc cgc acc     96
Phe Pro Leu Asp Arg Glu Ser Asp Gly Ala Asn Ala Glu Ala Arg Thr
            20                  25                  30

cac gat cat gag aag cac gca ctg gac cgg aat gga tgc tgt agg aat     144
His Asp His Glu Lys His Ala Leu Asp Arg Asn Gly Cys Cys Arg Asn
        35                  40                  45

cct gcc tgt gag agc cac aga tgt ggt tgacgacgct gatgctccag           191
Pro Ala Cys Glu Ser His Arg Cys Gly
    50                  55

gaccctctga accacgacgt tcgagca                                       218

<210> 75
<211> 57
<212> PRT
<213> Conus striatus

<400> 75
Met Phe Thr Val Phe Leu Leu Val Val Leu Ala Ile Thr Val Val Ser
1               5                   10                  15

Phe Pro Leu Asp Arg Glu Ser Asp Gly Ala Asn Ala Glu Ala Arg Thr
            20                  25                  30

His Asp His Glu Lys His Ala Leu Asp Arg Asn Gly Cys Cys Arg Asn
        35                  40                  45

Pro Ala Cys Glu Ser His Arg Cys Gly
    50                  55

<210> 76
<211> 227
<212> DNA
<213> Conus bandanus

<220>
<221> CDS
<222> (1)..(180)

<400> 76
atg ttc acc atg ttt ctg ttg gtt gtc ttg gca acc act gtc gtt tcc     48
Met Phe Thr Met Phe Leu Leu Val Val Leu Ala Thr Thr Val Val Ser
1               5                   10                  15

ttc gct tca gat cgt gca tct gat ggc agg aat gcc gca gcc aag gac     96
Phe Ala Ser Asp Arg Ala Ser Asp Gly Arg Asn Ala Ala Ala Lys Asp
            20                  25                  30

aaa gcg tct gac ctg gtc gct ctg acc gtc aag gga tgc tgt tct cat     144
Lys Ala Ser Asp Leu Val Ala Leu Thr Val Lys Gly Cys Cys Ser His

        35                  40                  45

cct gcc tgt agc gtg aat aat cca gac att tgt ggt tgaagacgct 190
Pro Ala Cys Ser Val Asn Asn Pro Asp Ile Cys Gly
    50                  55                  60

gatgctccag gaccctctga accacgacgt tcgagca 227

<210> 77
<211> 60
<212> PRT
<213> Conus bandanus

<400> 77
Met Phe Thr Met Phe Leu Leu Val Val Leu Ala Thr Thr Val Val Ser
1               5                   10                  15

Phe Ala Ser Asp Arg Ala Ser Asp Gly Arg Asn Ala Ala Ala Lys Asp
            20                  25                  30

Lys Ala Ser Asp Leu Val Ala Leu Thr Val Lys Gly Cys Cys Ser His
        35                  40                  45

Pro Ala Cys Ser Val Asn Asn Pro Asp Ile Cys Gly
    50                  55                  60

<210> 78
<211> 104
<212> DNA
<213> Conus bandanus

<220>
<221> CDS
<222> (1)..(54)

<400> 78
aaa gaa tgc tgt act cat cct gcc tgt cac gtg agt cat cca gaa ctc 48
Lys Glu Cys Cys Thr His Pro Ala Cys His Val Ser His Pro Glu Leu
1               5                   10                  15

tgt ggt tgaaaagcga cgtgacgctc caggaccctc tgaaccacga cgttcgagca 104
Cys Gly

<210> 79
<211> 18
<212> PRT
<213> Conus bandanus

<400> 79
Lys Glu Cys Cys Thr His Pro Ala Cys His Val Ser His Pro Glu Leu
1               5                   10                  15

Cys Gly

<210> 80

<211> 206
<212> DNA
<213> Conus bandanus

<220>
<221> CDS
<222> (1)..(171)

<400> 80
atg ttc acc gtg ttt ctg ttg gtt gtc ttg gca act gct gtt ctt cca     48
Met Phe Thr Val Phe Leu Leu Val Val Leu Ala Thr Ala Val Leu Pro
1               5                   10                  15

gtc act tta gat cgt gca tct gat gga agg aat gca gca gcc aac gcc     96
Val Thr Leu Asp Arg Ala Ser Asp Gly Arg Asn Ala Ala Ala Asn Ala
            20                  25                  30

aaa acg cct cgc ctg atc gcg cca ttc atc agg gat tat tgc tgt cat     144
Lys Thr Pro Arg Leu Ile Ala Pro Phe Ile Arg Asp Tyr Cys Cys His
        35                  40                  45

aga ggt ccc tgt atg gta tgg tgt ggt tgaagccgct gctgctccag           191
Arg Gly Pro Cys Met Val Trp Cys Gly
    50                  55
gaccctctga accac                                                    206


<210> 81
<211> 57
<212> PRT
<213> Conus bandanus

<400> 81
Met Phe Thr Val Phe Leu Leu Val Val Leu Ala Thr Ala Val Leu Pro
1               5                   10                  15

Val Thr Leu Asp Arg Ala Ser Asp Gly Arg Asn Ala Ala Ala Asn Ala
            20                  25                  30

Lys Thr Pro Arg Leu Ile Ala Pro Phe Ile Arg Asp Tyr Cys Cys His
        35                  40                  45

Arg Gly Pro Cys Met Val Trp Cys Gly
    50                  55


<210> 82
<211> 174
<212> DNA
<213> Conus caracteristicus

<220>
<221> CDS
<222> (1)..(171)

<400> 82
atg ttc acc gtg ttt ctg ttg gtt gtc ttg gca acc act gtg gtt tcc     48
Met Phe Thr Val Phe Leu Leu Val Val Leu Ala Thr Thr Val Val Ser
1               5                   10                  15

ttc act tca gat cgt gct tct gat ggc agg aat gcc gca gcc aac gcg     96
Phe Thr Ser Asp Arg Ala Ser Asp Gly Arg Asn Ala Ala Ala Asn Ala
            20                  25                  30

ttt gac ctg atc gct ctg atc gcc agg caa aat tgc tgt agc att ccc     144
Phe Asp Leu Ile Ala Leu Ile Ala Arg Gln Asn Cys Cys Ser Ile Pro
        35                  40                  45

agc tgt tgg gag aaa tat aaa tgt agt taa                             174
Ser Cys Trp Glu Lys Tyr Lys Cys Ser
    50                  55


<210> 83
<211> 57
<212> PRT
<213> Conus caracteristicus

<400> 83
Met Phe Thr Val Phe Leu Leu Val Val Leu Ala Thr Thr Val Val Ser
1               5                   10                  15

Phe Thr Ser Asp Arg Ala Ser Asp Gly Arg Asn Ala Ala Ala Asn Ala
            20                  25                  30

Phe Asp Leu Ile Ala Leu Ile Ala Arg Gln Asn Cys Cys Ser Ile Pro
        35                  40                  45

Ser Cys Trp Glu Lys Tyr Lys Cys Ser
    50                  55


<210> 84
<211> 219
<212> DNA
<213> Conus caracteristicus

<220>
<221> CDS
<222> (1)..(189)

<400> 84
atg ttc acc gtg ttt ctg ttg gtt gtc ttg gca acc act gtg gtt tcc     48
Met Phe Thr Val Phe Leu Leu Val Val Leu Ala Thr Thr Val Val Ser
1               5                   10                  15

ttc act tca gat cgt gcg tct gaa ggc agg aat gct gca gcc aag gac     96
Phe Thr Ser Asp Arg Ala Ser Glu Gly Arg Asn Ala Ala Ala Lys Asp
            20                  25                  30

aaa gcg tct gac ctg gtg gct ctg aca gtc agg gga tgc tgt gcc att     144
Lys Ala Ser Asp Leu Val Ala Leu Thr Val Arg Gly Cys Cys Ala Ile
        35                  40                  45

cgt gaa tgt cgc ttg cag aat gca gcg tat tgt ggt gga ata tac         189
Arg Glu Cys Arg Leu Gln Asn Ala Ala Tyr Cys Gly Gly Ile Tyr
    50                  55                  60

tgatgctcca ggaccctctg aaccacgacg                                    219

<210> 85
<211> 63
<212> PRT
<213> Conus caracteristicus

<400> 85
Met Phe Thr Val Phe Leu Leu Val Val Leu Ala Thr Thr Val Val Ser
1               5                   10                  15

Phe Thr Ser Asp Arg Ala Ser Glu Gly Arg Asn Ala Ala Ala Lys Asp
            20                  25                  30

Lys Ala Ser Asp Leu Val Ala Leu Thr Val Arg Gly Cys Cys Ala Ile
        35                  40                  45

Arg Glu Cys Arg Leu Gln Asn Ala Ala Tyr Cys Gly Gly Ile Tyr
    50                  55                  60


<210> 86
<211> 217
<212> DNA
<213> Conus tulipa

<220>
<221> CDS
<222> (1)..(186)

<400> 86
atg ttc acc gtg ttt ctg ttg gtt gtc ttg gca acc act gtc gtt tcc      48
Met Phe Thr Val Phe Leu Leu Val Val Leu Ala Thr Thr Val Val Ser
1               5                   10                  15

ttc cct tca gat att gca act gag ggc agg aat gcc gca gcc aaa gcg      96
Phe Pro Ser Asp Ile Ala Thr Glu Gly Arg Asn Ala Ala Ala Lys Ala
            20                  25                  30

ttt gac ctg ata tct tcg atc gtc aag aaa gga tgc tgt tcc cat cct      144
Phe Asp Leu Ile Ser Ser Ile Val Lys Lys Gly Cys Cys Ser His Pro
        35                  40                  45

gcc tgt tcg ggg aat aat cca gaa ttt tgt cgt caa ggt cgc              186
Ala Cys Ser Gly Asn Asn Pro Glu Phe Cys Arg Gln Gly Arg
    50                  55                  60

tgatgctcca ggaccctctg aaccacgacg t                                   217


<210> 87
<211> 62
<212> PRT
<213> Conus tulipa

<400> 87
Met Phe Thr Val Phe Leu Leu Val Val Leu Ala Thr Thr Val Val Ser
1               5                   10                  15

Phe Pro Ser Asp Ile Ala Thr Glu Gly Arg Asn Ala Ala Ala Lys Ala
            20                  25                  30

Phe Asp Leu Ile Ser Ser Ile Val Lys Lys Gly Cys Cys Ser His Pro
        35                  40                  45

Ala Cys Ser Gly Asn Asn Pro Glu Phe Cys Arg Gln Gly Arg
    50                  55                  60


<210> 88
<211> 217
<212> DNA
<213> Conus tulipa

<220>
<221> CDS
<222> (1)..(186)

<400> 88
atg ttc acc gtg ttt ctg ttg gtt gtc ttg gca acc act gtc gtt tcc      48
Met Phe Thr Val Phe Leu Leu Val Val Leu Ala Thr Thr Val Val Ser
1               5                   10                  15

ttc cct tca gat ata gca act gag ggc agg aat gcc gca gcc aaa gcg      96
Phe Pro Ser Asp Ile Ala Thr Glu Gly Arg Asn Ala Ala Ala Lys Ala
            20                  25                  30

ttt gac ctg ata tct tcg atc gtc agg aaa gga tgc tgt tcc aat ccc      144
Phe Asp Leu Ile Ser Ser Ile Val Arg Lys Gly Cys Cys Ser Asn Pro
        35                  40                  45

gcc tgt gcg ggg aat aat cca cat gtt tgt cgt caa ggt cgc              186
Ala Cys Ala Gly Asn Asn Pro His Val Cys Arg Gln Gly Arg
    50                  55                  60

tgatgctcca ggaccctctg aaccacgacg t                                   217


<210> 89
<211> 62
<212> PRT
<213> Conus tulipa

<400> 89
Met Phe Thr Val Phe Leu Leu Val Val Leu Ala Thr Thr Val Val Ser
1               5                   10                  15

Phe Pro Ser Asp Ile Ala Thr Glu Gly Arg Asn Ala Ala Ala Lys Ala
            20                  25                  30

Phe Asp Leu Ile Ser Ser Ile Val Arg Lys Gly Cys Cys Ser Asn Pro
        35                  40                  45

Ala Cys Ala Gly Asn Asn Pro His Val Cys Arg Gln Gly Arg
    50                  55                  60


<210> 90
<211> 226
<212> DNA
<213> Conus sulcatus

<220>
<221> CDS
<222> (1)..(195)

<400> 90
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc aat tca gat cgt gat cca gca tta ggt ggc agg aat gct gca gcc      96
Phe Asn Ser Asp Arg Asp Pro Ala Leu Gly Gly Arg Asn Ala Ala Ala
            20                  25                  30

aaa gcg tct gac aag atc gct tcg acc ctc aag aga aga gga tgc tgt      144
Lys Ala Ser Asp Lys Ile Ala Ser Thr Leu Lys Arg Arg Gly Cys Cys
        35                  40                  45

tcg tat ttt gac tgt aga atg atg ttt cca gaa atg tgt ggt tgg cga      192
Ser Tyr Phe Asp Cys Arg Met Met Phe Pro Glu Met Cys Gly Trp Arg
    50                  55                  60

ggc tgatgctcca ggaccctctg aaccacgacg t                               226
Gly
65


<210> 91
<211> 65
<212> PRT
<213> Conus sulcatus

<400> 91
Met Phe Thr Val Phe Leu Leu Val Val Leu Ala Thr Thr Val Val Ser
1               5                   10                  15

Phe Asn Ser Asp Arg Asp Pro Ala Leu Gly Gly Arg Asn Ala Ala Ala
            20                  25                  30

Lys Ala Ser Asp Lys Ile Ala Ser Thr Leu Lys Arg Arg Gly Cys Cys
        35                  40                  45

Ser Tyr Phe Asp Cys Arg Met Met Phe Pro Glu Met Cys Gly Trp Arg
    50                  55                  60
Gly
65


<210> 92
<211> 226
<212> DNA
<213> Conus sulcatus

<220>
<221> CDS
<222> (1)..(195)

<400> 92
atg ttc acc gtg ttt ctg ttg gtt gtc ttg gca acc acc gtc gtt tcc      48
Met Phe Thr Val Phe Leu Leu Val Val Leu Ala Thr Thr Val Val Ser

1                   5                       10                      15

ttc aat tca gat cgt gat cca gca tta ggt ggc agg aat gct gca gcc     96
Phe Asn Ser Asp Arg Asp Pro Ala Leu Gly Gly Arg Asn Ala Ala Ala
                20                      25                      30

ata gcg tct gac aag atc gct tcg acc ctc agg aga gga gga tgc tgt     144
Ile Ala Ser Asp Lys Ile Ala Ser Thr Leu Arg Arg Gly Gly Cys Cys
            35                      40                      45

tct ttt cct gcc tgt aga aag tat cgt cca gaa atg tgt ggt gga cga     192
Ser Phe Pro Ala Cys Arg Lys Tyr Arg Pro Glu Met Cys Gly Gly Arg
    50                      55                      60

cgc tgatgctcca ggaccctctg aaccacgacg t                              226
Arg
65

<210> 93
<211> 65
<212> PRT
<213> Conus sulcatus

<400> 93
Met Phe Thr Val Phe Leu Leu Val Val Leu Ala Thr Thr Val Val Ser
1               5                   10                  15

Phe Asn Ser Asp Arg Asp Pro Ala Leu Gly Gly Arg Asn Ala Ala Ala
            20                  25                  30

Ile Ala Ser Asp Lys Ile Ala Ser Thr Leu Arg Arg Gly Gly Cys Cys
        35                  40                  45

Ser Phe Pro Ala Cys Arg Lys Tyr Arg Pro Glu Met Cys Gly Gly Arg
    50                  55                  60

Arg
65

<210> 94
<211> 211
<212> DNA
<213> Conus sulcatus

<220>
<221> CDS
<222> (1)..(180)
<400> 94
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca gat cat gaa tct gat cgc ggt gat gcc caa acc atc caa     96
Phe Thr Ser Asp His Glu Ser Asp Arg Gly Asp Ala Gln Thr Ile Gln
            20                  25                  30

gaa gtg ttt gag atg ttc gct ctg gac agc gat gga tgc tgt tgg cat     144
Glu Val Phe Glu Met Phe Ala Leu Asp Ser Asp Gly Cys Cys Trp His

35                  40                  45

cct gct tgt ggc aga cac tat tgt ggt cga aga cgc tgatgctcca          190
Pro Ala Cys Gly Arg His Tyr Cys Gly Arg Arg Arg
    50                  55                  60

ggaccctctg aaccacgacg t                                             211


<210> 95
<211> 60
<212> PRT
<213> Conus sulcatus

<400> 95
Met Phe Thr Val Phe Leu Leu Val Val Leu Ala Thr Thr Val Val Ser
1               5                   10                  15

Phe Thr Ser Asp His Glu Ser Asp Arg Gly Asp Ala Gln Thr Ile Gln
            20                  25                  30

Glu Val Phe Glu Met Phe Ala Leu Asp Ser Asp Gly Cys Cys Trp His
        35                  40                  45

Pro Ala Cys Gly Arg His Tyr Cys Gly Arg Arg Arg
    50                  55                  60


<210> 96
<211> 202
<212> DNA
<213> Conus sulcatus

<220>
<221> CDS
<222> (1)..(195)

<400> 96
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc aat tca gat cgt gat cca gca tta ggt ggc agg aat gct gca gcc      96
Phe Asn Ser Asp Arg Asp Pro Ala Leu Gly Gly Arg Asn Ala Ala Ala
            20                  25                  30

ata gcg tct gac aag atc gct tcg acc ctc agg aga gga gga tgc tgt     144
Ile Ala Ser Asp Lys Ile Ala Ser Thr Leu Arg Arg Gly Gly Cys Cys
        35                  40                  45

tct ttt gct gcc tgt aga aag tat cgt cca gaa atg tgt ggt gga cga     192
Ser Phe Ala Ala Cys Arg Lys Tyr Arg Pro Glu Met Cys Gly Gly Arg
    50                  55                  60

cgc tgatgct                                                         202
Arg
65


<210> 97

<211> 65
<212> PRT
<213> Conus sulcatus

<400> 97
Met Phe Thr Val Phe Leu Leu Val Val Leu Ala Thr Thr Val Val Ser
1               5                   10                  15

Phe Asn Ser Asp Arg Asp Pro Ala Leu Gly Gly Arg Asn Ala Ala Ala
            20                  25                  30

Ile Ala Ser Asp Lys Ile Ala Ser Thr Leu Arg Arg Gly Gly Cys Cys
        35                  40                  45

Ser Phe Ala Ala Cys Arg Lys Tyr Arg Pro Glu Met Cys Gly Gly Arg
    50                  55                  60

Arg
65


<210> 98
<211> 220
<212> DNA
<213> Conus sulcatus

<220>
<221> CDS
<222> (1)..(189)

<400> 98
atg ttc acc gtg ttt ctg ttg gtt ctc ttg gca acc acc gtc gtt tcc      48
Met Phe Thr Val Phe Leu Leu Val Leu Leu Ala Thr Thr Val Val Ser
1               5                   10                  15

ttc aat tca gat cgt gca tta ggt ggc agg aat gct gca gcc aaa gcg      96
Phe Asn Ser Asp Arg Ala Leu Gly Gly Arg Asn Ala Ala Ala Lys Ala
            20                  25                  30

tct gac aag atc ctt tcg aac ctc agg aga gga gga tgc tgt ttt cat      144
Ser Asp Lys Ile Leu Ser Asn Leu Arg Arg Gly Gly Cys Cys Phe His
        35                  40                  45

cct gtc tgt tac atc aat ctt cta gaa atg tgt cgt caa cga ggc          189
Pro Val Cys Tyr Ile Asn Leu Leu Glu Met Cys Arg Gln Arg Gly
    50                  55                  60

tgatcgtcca ggaccctctg aaccacgacg t                                   220


<210> 99
<211> 63
<212> PRT
<213> Conus sulcatus

<400> 99
Met Phe Thr Val Phe Leu Leu Val Leu Leu Ala Thr Thr Val Val Ser
1               5                   10                  15

Phe Asn Ser Asp Arg Ala Leu Gly Gly Arg Asn Ala Ala Ala Lys Ala

                20                  25                  30

Ser Asp Lys Ile Leu Ser Asn Leu Arg Arg Gly Gly Cys Cys Phe His
        35                  40                  45

Pro Val Cys Tyr Ile Asn Leu Leu Glu Met Cys Arg Gln Arg Gly
    50                  55                  60


<210> 100
<211> 208
<212> DNA
<213> Conus consors

<220>
<221> CDS
<222> (1)..(177)

<400> 100
atg ttc acc gtg ttt ctg ttg gtt gtc ttg aca acc act gtc gtt tcc    48
Met Phe Thr Val Phe Leu Leu Val Val Leu Thr Thr Thr Val Val Ser
1               5                   10                  15

ttc cct tca gat agt gca tct gat gtc agg gat gac gaa gcc aaa gac    96
Phe Pro Ser Asp Ser Ala Ser Asp Val Arg Asp Asp Glu Ala Lys Asp
            20                  25                  30

gaa agg tct gac atg tac aaa tcg aaa cgg aat gga cgc tgt tgc cat    144
Glu Arg Ser Asp Met Tyr Lys Ser Lys Arg Asn Gly Arg Cys Cys His
        35                  40                  45

cct gcc tgt ggc aaa cac ttt agt tgt gga cgc tgatgctcca ggaccctctg 197
Pro Ala Cys Gly Lys His Phe Ser Cys Gly Arg
    50                  55

aaccacgacg t                                                       208


<210> 101
<211> 59
<212> PRT
<213> Conus consors

<400> 101
Met Phe Thr Val Phe Leu Leu Val Val Leu Thr Thr Thr Val Val Ser
1               5                   10                  15

Phe Pro Ser Asp Ser Ala Ser Asp Val Arg Asp Asp Glu Ala Lys Asp
            20                  25                  30

Glu Arg Ser Asp Met Tyr Lys Ser Lys Arg Asn Gly Arg Cys Cys His
        35                  40                  45

Pro Ala Cys Gly Lys His Phe Ser Cys Gly Arg
    50                  55


<210> 102
<211> 219
<212> DNA

<213> Conus stercusmuscarum

<220>
<221> CDS
<222> (1)..(189)

<400> 102
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

tcc cct tca gat cgt gca tct gat ggc agg aat gcc gca gcc aac gag     96
Ser Pro Ser Asp Arg Ala Ser Asp Gly Arg Asn Ala Ala Ala Asn Glu
            20                  25                  30

aaa gcg tct gac gtg atc gcg ctg gcc ctc aag gga tgc tgt tcc aac     144
Lys Ala Ser Asp Val Ile Ala Leu Ala Leu Lys Gly Cys Cys Ser Asn
        35                  40                  45

cct gtc tgt cac ctg gag cat tca aac atg tgt ggt aga aga cgc         189
Pro Val Cys His Leu Glu His Ser Asn Met Cys Gly Arg Arg Arg
    50                  55                  60

tgatgctcca ggaccctctg aaccacgacg                                    219


<210> 103
<211> 63
<212> PRT
<213> Conus stercusmuscarum

<400> 103
Met Phe Thr Val Phe Leu Leu Val Val Leu Ala Thr Thr Val Val Ser
1               5                   10                  15

Ser Pro Ser Asp Arg Ala Ser Asp Gly Arg Asn Ala Ala Ala Asn Glu
            20                  25                  30

Lys Ala Ser Asp Val Ile Ala Leu Ala Leu Lys Gly Cys Cys Ser Asn
        35                  40                  45

Pro Val Cys His Leu Glu His Ser Asn Met Cys Gly Arg Arg Arg
    50                  55                  60


<210> 104
<211> 248
<212> DNA
<213> Conus betulinus

<220>
<221> CDS
<222> (1)..(180)

<400> 104
atg ttc tcc gtg ttt ctg ttg gtt gtc ttg gca acc act gtc gtt tcc     48
Met Phe Ser Val Phe Leu Leu Val Val Leu Ala Thr Thr Val Val Ser
1               5                   10                  15

tcc act tca ggt ggt gca tct ggt ggc agg aag gct gca gcc aaa gcg     96

Ser Thr Ser Gly Gly Ala Ser Gly Gly Arg Lys Ala Ala Ala Lys Ala
            20                  25                  30

tct aac cgg atc gct ctg acc gtc agg agt gca aca tgc tgt aat tat    144
Ser Asn Arg Ile Ala Leu Thr Val Arg Ser Ala Thr Cys Cys Asn Tyr
        35                  40                  45

cct ccc tgt tac gag act tat cca gaa agt tgt ctg taacgtgaat         190
Pro Pro Cys Tyr Glu Thr Tyr Pro Glu Ser Cys Leu
    50                  55                  60

catccagagc tttgtggctg aagacactga tgctccagga ccctctgaac cacgacgt    248


<210> 105
<211> 60
<212> PRT
<213> Conus betulinus

<400> 105
Met Phe Ser Val Phe Leu Leu Val Val Leu Ala Thr Thr Val Val Ser
1               5                   10                  15

Ser Thr Ser Gly Gly Ala Ser Gly Gly Arg Lys Ala Ala Ala Lys Ala
            20                  25                  30

Ser Asn Arg Ile Ala Leu Thr Val Arg Ser Ala Thr Cys Cys Asn Tyr
        35                  40                  45

Pro Pro Cys Tyr Glu Thr Tyr Pro Glu Ser Cys Leu
    50                  55                  60


<210> 106
<211> 223
<212> DNA
<213> Conus betulinus

<220>
<221> CDS
<222> (1)..(183)

<400> 106
atg ttc acc gtg ttt ctg ttg gtt gtc ttg gca acc act gtg gtt tcc    48
Met Phe Thr Val Phe Leu Leu Val Val Leu Ala Thr Thr Val Val Ser
1               5                   10                  15

ttc act tca ggt cgt gca ttt cgt ggc agg aat cgc gca gcc gac gac    96
Phe Thr Ser Gly Arg Ala Phe Arg Gly Arg Asn Arg Ala Ala Asp Asp
            20                  25                  30

aaa agg tct gac ctg gcc gct ctg agc gtc agg gga gga tgc tgt tcc    144
Lys Arg Ser Asp Leu Ala Ala Leu Ser Val Arg Gly Gly Cys Cys Ser
        35                  40                  45

cat cct gcc tgt gcg gtg aat cat cca gag ctt tgt ggc tgaagacgct     193
His Pro Ala Cys Ala Val Asn His Pro Glu Leu Cys Gly
    50                  55                  60

gatgccccag gaccctctga accacgacgt                                   223

<210> 107
<211> 61
<212> PRT
<213> Conus betulinus

<400> 107
Met Phe Thr Val Phe Leu Leu Val Val Leu Ala Thr Thr Val Val Ser
1               5                   10                  15

Phe Thr Ser Gly Arg Ala Phe Arg Gly Arg Asn Arg Ala Ala Asp Asp
            20                  25                  30

Lys Arg Ser Asp Leu Ala Ala Leu Ser Val Arg Gly Gly Cys Cys Ser
        35                  40                  45

His Pro Ala Cys Ala Val Asn His Pro Glu Leu Cys Gly
    50                  55                  60


<210> 108
<211> 248
<212> DNA
<213> Conus betulinus

<220>
<221> CDS
<222> (1)..(180)

<400> 108
atg ttc acc gtg ttt ctg ttg gtt gtc ttg gca acc act gtc gtt tcc    48
Met Phe Thr Val Phe Leu Leu Val Val Leu Ala Thr Thr Val Val Ser
1               5                   10                  15

ttc act tca ggt cgt gca tct ggt ggc agg aat gct gca gcc aaa gcg    96
Phe Thr Ser Gly Arg Ala Ser Gly Gly Arg Asn Ala Ala Ala Lys Ala
            20                  25                  30

tct aac cgg atc gct atg gcc atc agc agt gga gca tgc tgt gca tat    144
Ser Asn Arg Ile Ala Met Ala Ile Ser Ser Gly Ala Cys Cys Ala Tyr
        35                  40                  45

cct ccc tgt ttc gag gct tat cca gaa aga tgt ctg taacgtgaat         190
Pro Pro Cys Phe Glu Ala Tyr Pro Glu Arg Cys Leu
    50                  55                  60

catccagacc tttgtggctg aagacgctga tgccccagga ccctctgaac cacgacgt    248


<210> 109
<211> 60
<212> PRT
<213> Conus betulinus

<400> 109
Met Phe Thr Val Phe Leu Leu Val Val Leu Ala Thr Thr Val Val Ser
1               5                   10                  15

Phe Thr Ser Gly Arg Ala Ser Gly Gly Arg Asn Ala Ala Ala Lys Ala

20                  25                  30

Ser Asn Arg Ile Ala Met Ala Ile Ser Ser Gly Ala Cys Cys Ala Tyr
        35                  40                  45

Pro Pro Cys Phe Glu Ala Tyr Pro Glu Arg Cys Leu
    50                  55                  60


<210> 110
<211> 223
<212> DNA
<213> Conus betulinus

<220>
<221> CDS
<222> (1)..(192)

<400> 110
atg ttc acc gtg ttt ctg ttg gtt gtc ttg gca acc act gtc gtt tcc      48
Met Phe Thr Val Phe Leu Leu Val Val Leu Ala Thr Thr Val Val Ser
1               5                   10                  15

ttc act tca gat cgt gca ttt cgt ggc agg aat tcc gca gcc aac gac      96
Phe Thr Ser Asp Arg Ala Phe Arg Gly Arg Asn Ser Ala Ala Asn Asp
            20                  25                  30

aaa agg tct gac ctg gcc gct ctg agc gtc agg aga gga tgc tgc tcc      144
Lys Arg Ser Asp Leu Ala Ala Leu Ser Val Arg Arg Gly Cys Cys Ser
        35                  40                  45

cat ccc gcc tgt agc gtg aat cat cca gag ctt tgt ggt aga aga cgc      192
His Pro Ala Cys Ser Val Asn His Pro Glu Leu Cys Gly Arg Arg Arg
    50                  55                  60

tgatgcccca ggaccctctg aaccacgacg t                                   223


<210> 111
<211> 64
<212> PRT
<213> Conus betulinus

<400> 111
Met Phe Thr Val Phe Leu Leu Val Val Leu Ala Thr Thr Val Val Ser
1               5                   10                  15

Phe Thr Ser Asp Arg Ala Phe Arg Gly Arg Asn Ser Ala Ala Asn Asp
            20                  25                  30

Lys Arg Ser Asp Leu Ala Ala Leu Ser Val Arg Arg Gly Cys Cys Ser
        35                  40                  45

His Pro Ala Cys Ser Val Asn His Pro Glu Leu Cys Gly Arg Arg Arg
    50                  55                  60


<210> 112
<211> 248
<212> DNA

<213> Conus betulinus

<220>
<221> CDS
<222> (1)..(180)

<400> 112
atg ttc acc gtg ttt ctg ttg gtt gtc ttg gca acc act gtc gtt tcc    48
Met Phe Thr Val Phe Leu Leu Val Val Leu Ala Thr Thr Val Val Ser
1               5                   10                  15

ttc act tca ggt cgt gca tct ggt ggc agg aat gct gca gcc aaa gcg    96
Phe Thr Ser Gly Arg Ala Ser Gly Gly Arg Asn Ala Ala Ala Lys Ala
            20                  25                  30

tct aac cgg atc gct ctg atc gtc agg aat gca gaa tgc tgt tat tat    144
Ser Asn Arg Ile Ala Leu Ile Val Arg Asn Ala Glu Cys Cys Tyr Tyr
        35                  40                  45

cct ccc tgt tac gag gct tat cca gaa att tgt ctg taacgtgaat         190
Pro Pro Cys Tyr Glu Ala Tyr Pro Glu Ile Cys Leu
    50                  55                  60

catccagacc tttgtggctg aagaccctga tgctccagga ccctctgaac cacgacgt    248

<210> 113
<211> 60
<212> PRT
<213> Conus betulinus

<400> 113
Met Phe Thr Val Phe Leu Leu Val Val Leu Ala Thr Thr Val Val Ser
1               5                   10                  15

Phe Thr Ser Gly Arg Ala Ser Gly Gly Arg Asn Ala Ala Ala Lys Ala
            20                  25                  30

Ser Asn Arg Ile Ala Leu Ile Val Arg Asn Ala Glu Cys Cys Tyr Tyr
        35                  40                  45

Pro Pro Cys Tyr Glu Ala Tyr Pro Glu Ile Cys Leu
    50                  55                  60


<210> 114
<211> 207
<212> DNA
<213> Conus pennaceus

<220>
<221> CDS
<222> (1)..(168)

<400> 114
atg ttc acc gtg ttt ctg ttg gtt gtc ttg gca acc acc gtc att tcc    48
Met Phe Thr Val Phe Leu Leu Val Val Leu Ala Thr Thr Val Ile Ser
1               5                   10                  15

ttc act tca gat cgt gca tct gat ggc ggg aat gcc gca gcg tct gac    96
Phe Thr Ser Asp Arg Ala Ser Asp Gly Gly Asn Ala Ala Ala Ser Asp

20                          25                          30

ctg atc gct ctg acc atc aag gga tgc tgt tct cat cct ccc tgt gcc    144
Leu Ile Ala Leu Thr Ile Lys Gly Cys Cys Ser His Pro Pro Cys Ala
            35                  40                  45

atg aat aat cca gac tat tgt ggt tgacgacgct gatgctccag gaccctctga    198
Met Asn Asn Pro Asp Tyr Cys Gly
    50                  55

accacgacg                                                           207


<210> 115
<211> 56
<212> PRT
<213> Conus pennaceus

<400> 115
Met Phe Thr Val Phe Leu Leu Val Val Leu Ala Thr Thr Val Ile Ser
  1               5                  10                  15

Phe Thr Ser Asp Arg Ala Ser Asp Gly Gly Asn Ala Ala Ala Ser Asp
                20                  25                  30

Leu Ile Ala Leu Thr Ile Lys Gly Cys Cys Ser His Pro Pro Cys Ala
            35                  40                  45

Met Asn Asn Pro Asp Tyr Cys Gly
    50                  55


<210> 116
<211> 207
<212> DNA
<213> Conus pennaceus

<220>
<221> CDS
<222> (1)..(168)

<400> 116
atg ttc acc gtg ttt ctg ttg gtt gtc ttg gca acc acc gtc gtt tcc    48
Met Phe Thr Val Phe Leu Leu Val Val Leu Ala Thr Thr Val Val Ser
  1               5                  10                  15

ttc act tca gat cgt gca tct gat ggc ggg aat gcc gca atg tct gac    96
Phe Thr Ser Asp Arg Ala Ser Asp Gly Gly Asn Ala Ala Met Ser Asp
                20                  25                  30

ctg atc gct ctg acc atc aag gga tgc tgt tct cat cct ccc tgt ttc    144
Leu Ile Ala Leu Thr Ile Lys Gly Cys Cys Ser His Pro Pro Cys Phe
            35                  40                  45

ctg aat aat cca gac tat tgt ggt tgacgacgct gatgctccag gaccctctga    198
Leu Asn Asn Pro Asp Tyr Cys Gly
    50                  55

accacgacg                                                           207

<210> 117
<211> 56
<212> PRT
<213> Conus pennaceus

<400> 117
Met Phe Thr Val Phe Leu Leu Val Val Leu Ala Thr Thr Val Val Ser
1               5                   10                  15

Phe Thr Ser Asp Arg Ala Ser Asp Gly Gly Asn Ala Ala Met Ser Asp
            20                  25                  30

Leu Ile Ala Leu Thr Ile Lys Gly Cys Cys Ser His Pro Pro Cys Phe
        35                  40                  45

Leu Asn Asn Pro Asp Tyr Cys Gly
    50                  55


<210> 118
<211> 210
<212> DNA
<213> Conus stercusmuscarum

<220>
<221> CDS
<222> (1)..(171)

<400> 118
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca gat cgt gaa tct gat ggc gcg aat gac gaa gcc cgc acc     96
Phe Pro Ser Asp Arg Glu Ser Asp Gly Ala Asn Asp Glu Ala Arg Thr
            20                  25                  30

gac gag cct gag gag cac gga ccg gac agg aat gga tgc tgt agg aat     144
Asp Glu Pro Glu Glu His Gly Pro Asp Arg Asn Gly Cys Cys Arg Asn
        35                  40                  45

cct gcc tgt gag agc cac aga tgt ggt tgacgacgct gatgctccag           191
Pro Ala Cys Glu Ser His Arg Cys Gly
    50                  55

gaccctctga accacgacg                                                210


<210> 119
<211> 57
<212> PRT
<213> Conus stercusmuscarum

<400> 119
Met Phe Thr Val Phe Leu Leu Val Val Leu Ala Thr Thr Val Val Ser
1               5                   10                  15

Phe Pro Ser Asp Arg Glu Ser Asp Gly Ala Asn Asp Glu Ala Arg Thr
            20                  25                  30

Asp Glu Pro Glu Glu His Gly Pro Asp Arg Asn Gly Cys Cys Arg Asn
        35                  40                  45

Pro Ala Cys Glu Ser His Arg Cys Gly
    50                  55


<210> 120
<211> 210
<212> DNA
<213> Conus circumcisus

<220>
<221> CDS
<222> (1)..(180)

<400> 120
atg ttc acc gtg ttt ctg ttg gtt gtc ttg gca acc act gtc gtt tcc      48
Met Phe Thr Val Phe Leu Leu Val Val Leu Ala Thr Thr Val Val Ser
1               5                   10                  15

ttc cct tca gat cgt gca tct gat ggc agg aat gcc gca gcc agc gac      96
Phe Pro Ser Asp Arg Ala Ser Asp Gly Arg Asn Ala Ala Ala Ser Asp
            20                  25                  30

aga gcg tct gac gcg gcc cac cag gga tgc tgt tcc aac cct gtc tgt      144
Arg Ala Ser Asp Ala Ala His Gln Gly Cys Cys Ser Asn Pro Val Cys
        35                  40                  45

cac gtg gaa cat cca gaa ctt tgt cgt aga aga cgc tgatgctcca           190
His Val Glu His Pro Glu Leu Cys Arg Arg Arg Arg
    50                  55                  60

ggaccctctg aaccacgacg                                                210


<210> 121
<211> 60
<212> PRT
<213> Conus circumcisus

<400> 121
Met Phe Thr Val Phe Leu Leu Val Val Leu Ala Thr Thr Val Val Ser
1               5                   10                  15

Phe Pro Ser Asp Arg Ala Ser Asp Gly Arg Asn Ala Ala Ala Ser Asp
            20                  25                  30

Arg Ala Ser Asp Ala Ala His Gln Gly Cys Cys Ser Asn Pro Val Cys
        35                  40                  45

His Val Glu His Pro Glu Leu Cys Arg Arg Arg Arg
    50                  55                  60


<210> 122
<211> 213
<212> DNA
<213> Conus circumcisus

<220>
<221> CDS
<222> (1)..(174)

<400> 122
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca aat cgt gaa tct gat ggc gcg aat gcc gaa gtc cgc acc     96
Phe Pro Ser Asn Arg Glu Ser Asp Gly Ala Asn Ala Glu Val Arg Thr
            20                  25                  30

gac gag cct gag gag cac gac gaa ctg ggc ggg aat gga tgc tgt ggg     144
Asp Glu Pro Glu Glu His Asp Glu Leu Gly Gly Asn Gly Cys Cys Gly
        35                  40                  45

aat cct gac tgt acg agc cac agt tgt gat tgacgacgct gatgctccag     194
Asn Pro Asp Cys Thr Ser His Ser Cys Asp
    50                  55

gaccctctga accacgacg                                              213


<210> 123
<211> 58
<212> PRT
<213> Conus circumcisus

<400> 123
Met Phe Thr Val Phe Leu Leu Val Val Leu Ala Thr Thr Val Val Ser
1               5                   10                  15

Phe Pro Ser Asn Arg Glu Ser Asp Gly Ala Asn Ala Glu Val Arg Thr
            20                  25                  30

Asp Glu Pro Glu Glu His Asp Glu Leu Gly Gly Asn Gly Cys Cys Gly
        35                  40                  45

Asn Pro Asp Cys Thr Ser His Ser Cys Asp
    50                  55


<210> 124
<211> 207
<212> DNA
<213> Conus episcopatus

<220>
<221> CDS
<222> (1)..(168)

<400> 124
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca gat cgt gca tct gat agc agg aag gac gca gcg tct ggc     96
Phe Thr Ser Asp Arg Ala Ser Asp Ser Arg Lys Asp Ala Ala Ser Gly

            20                  25                  30

ctg atc gct ctg acc atc aag gga tgc tgt tct gat cct cgc tgt aac 144
Leu Ile Ala Leu Thr Ile Lys Gly Cys Cys Ser Asp Pro Arg Cys Asn
        35                  40                  45

atg aat aat cca gac tat tgt ggt tgacgacgct gatgctccag gaccctctga 198
Met Asn Asn Pro Asp Tyr Cys Gly
    50                  55

accacgacg 207

<210> 125
<211> 56
<212> PRT
<213> Conus episcopatus

<400> 125
Met Phe Thr Val Phe Leu Leu Val Val Leu Ala Thr Thr Val Val Ser
1               5                   10                  15

Phe Thr Ser Asp Arg Ala Ser Asp Ser Arg Lys Asp Ala Ala Ser Gly
            20                  25                  30

Leu Ile Ala Leu Thr Ile Lys Gly Cys Cys Ser Asp Pro Arg Cys Asn
        35                  40                  45

Met Asn Asn Pro Asp Tyr Cys Gly
    50                  55

<210> 126
<211> 213
<212> DNA
<213> Conus sponsalis

<220>
<221> CDS
<222> (1)..(174)

<400> 126
atg tcc acc gtg ttt ctg ttg gtt gtc ctc gca acc acc gtc gtt tcc 48
Met Ser Thr Val Phe Leu Leu Val Val Leu Ala Thr Thr Val Val Ser
1               5                   10                  15

ttc act gta gat cgt gca tct gat ggc agg gat gtc gca atc gac gac 96
Phe Thr Val Asp Arg Ala Ser Asp Gly Arg Asp Val Ala Ile Asp Asp
            20                  25                  30
aga ttg gtg tct ctc cct cag atc gcc cat gct gac tgt tgt tcc gat 144
Arg Leu Val Ser Leu Pro Gln Ile Ala His Ala Asp Cys Cys Ser Asp
        35                  40                  45

cct gcc tgc aag cag acg ccc ggt tgt cgt taaagacgct gctgctccag 194
Pro Ala Cys Lys Gln Thr Pro Gly Cys Arg
    50                  55

gaccctctga accacgacg 213

<210> 127
<211> 58
<212> PRT
<213> Conus sponsalis

<400> 127
Met Ser Thr Val Phe Leu Leu Val Val Leu Ala Thr Thr Val Val Ser
1               5                   10                  15

Phe Thr Val Asp Arg Ala Ser Asp Gly Arg Asp Val Ala Ile Asp Asp
            20                  25                  30

Arg Leu Val Ser Leu Pro Gln Ile Ala His Ala Asp Cys Cys Ser Asp
        35                  40                  45

Pro Ala Cys Lys Gln Thr Pro Gly Cys Arg
    50                  55


<210> 128
<211> 221
<212> DNA
<213> Conus sponsalis

<220>
<221> CDS
<222> (1)..(168)

<400> 128
atg ttc acc gtg ttt ctg ttg gtt gtc ttg gca acc acc gtc gct tcc      48
Met Phe Thr Val Phe Leu Leu Val Val Leu Ala Thr Thr Val Ala Ser
1               5                   10                  15

ttc att atc gat gat cca tct gat ggc agg aat att gca gtc gac gac      96
Phe Ile Ile Asp Asp Pro Ser Asp Gly Arg Asn Ile Ala Val Asp Asp
            20                  25                  30

aga ggg ctt ttc tct acg ctc ttc cat gct gat tgc tgt gaa aat cct      144
Arg Gly Leu Phe Ser Thr Leu Phe His Ala Asp Cys Cys Glu Asn Pro
        35                  40                  45

gcc tgt aga cac acg cag ggt tgt tgatctttgt tcttcaaaga cactgctggc     198
Ala Cys Arg His Thr Gln Gly Cys
    50                  55

ccaggaccct ctgaaccacg acg                                            221


<210> 129
<211> 56
<212> PRT
<213> Conus sponsalis

<400> 129
Met Phe Thr Val Phe Leu Leu Val Val Leu Ala Thr Thr Val Ala Ser
1               5                   10                  15

Phe Ile Ile Asp Asp Pro Ser Asp Gly Arg Asn Ile Ala Val Asp Asp
            20                  25                  30

Arg Gly Leu Phe Ser Thr Leu Phe His Ala Asp Cys Cys Glu Asn Pro
        35                  40                  45

Ala Cys Arg His Thr Gln Gly Cys
    50                  55


<210> 130
<211> 220
<212> DNA
<213> Conus dalli

<220>
<221> CDS
<222> (1)..(180)

<400> 130
atg ttc acc gtg ttt ctg ttg gtt gtc ttg gca acc acc gtc gtt tcc      48
Met Phe Thr Val Phe Leu Leu Val Val Leu Ala Thr Thr Val Val Ser
1               5                   10                  15

ttc act tca gat cgt gca ttt cgt ggc agg aat gcc gca gcc aaa gag      96
Phe Thr Ser Asp Arg Ala Phe Arg Gly Arg Asn Ala Ala Ala Lys Glu
            20                  25                  30

tct ggc ctg gtc ggt ctg acc gac aag acg cga gga tgc tgt tct cat      144
Ser Gly Leu Val Gly Leu Thr Asp Lys Thr Arg Gly Cys Cys Ser His
        35                  40                  45

cct gcc tgt aac gta gat cat cca gaa att tgt ggt tgaagacgct           190
Pro Ala Cys Asn Val Asp His Pro Glu Ile Cys Gly
    50                  55                  60

gatgctccag gaccctctga accacgacgt                                     220


<210> 131
<211> 60
<212> PRT
<213> Conus dalli

<400> 131
Met Phe Thr Val Phe Leu Leu Val Val Leu Ala Thr Thr Val Val Ser
1               5                   10                  15

Phe Thr Ser Asp Arg Ala Phe Arg Gly Arg Asn Ala Ala Ala Lys Glu
            20                  25                  30

Ser Gly Leu Val Gly Leu Thr Asp Lys Thr Arg Gly Cys Cys Ser His
        35                  40                  45

Pro Ala Cys Asn Val Asp His Pro Glu Ile Cys Gly
    50                  55                  60


<210> 132
<211> 208
<212> DNA
<213> Conus dalli

<220>
<221> CDS
<222> (1)..(177)

<400> 132
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca gat ggt gca tct gat gac agg aaa gcc gct gcg tct gac     96
Phe Thr Ser Asp Gly Ala Ser Asp Asp Arg Lys Ala Ala Ala Ser Asp
            20                  25                  30

ctg atc act ctg acc atc aag gga tgc tgt tct cgt cct ccc tgt atc     144
Leu Ile Thr Leu Thr Ile Lys Gly Cys Cys Ser Arg Pro Pro Cys Ile
        35                  40                  45

gcg aat aat cca gac ttg tgt ggt cga cga cgc tgatgctcca ggaccctctg 197
Ala Asn Asn Pro Asp Leu Cys Gly Arg Arg Arg
    50                  55

aaccacgacg t                                                        208


<210> 133
<211> 59
<212> PRT
<213> Conus dalli

<400> 133
Met Phe Thr Val Phe Leu Leu Val Val Leu Ala Thr Thr Val Val Ser
1               5                   10                  15

Phe Thr Ser Asp Gly Ala Ser Asp Asp Arg Lys Ala Ala Ala Ser Asp
            20                  25                  30

Leu Ile Thr Leu Thr Ile Lys Gly Cys Cys Ser Arg Pro Pro Cys Ile
        35                  40                  45

Ala Asn Asn Pro Asp Leu Cys Gly Arg Arg Arg
    50                  55


<210> 134
<211> 223
<212> DNA
<213> Conus dalli

<220>
<221> CDS
<222> (1)..(192)

<400> 134
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

tcc act tca ggt cgt cgt gca ttt cat ggc agg aat gcc gca gcc aaa     96
Ser Thr Ser Gly Arg Arg Ala Phe His Gly Arg Asn Ala Ala Ala Lys
            20                  25                  30

gcg tct gga ctg gtc ggt ctg act gac agg aga cca caa tgc tgt agt     144
Ala Ser Gly Leu Val Gly Leu Thr Asp Arg Arg Pro Gln Cys Cys Ser
        35                  40                  45

gat cct cgc tgt aac gta ggt cat cca gaa ctt tgt ggt gga aga cgc     192
Asp Pro Arg Cys Asn Val Gly His Pro Glu Leu Cys Gly Gly Arg Arg
    50                  55                  60

tgatgctcca ggaccctctg aaccacaacg t                                  223


<210> 135
<211> 64
<212> PRT
<213> Conus dalli

<400> 135
Met Phe Thr Val Phe Leu Leu Val Val Leu Ala Thr Thr Val Val Ser
1               5                   10                  15

Ser Thr Ser Gly Arg Arg Ala Phe His Gly Arg Asn Ala Ala Ala Lys
            20                  25                  30

Ala Ser Gly Leu Val Gly Leu Thr Asp Arg Arg Pro Gln Cys Cys Ser
        35                  40                  45

Asp Pro Arg Cys Asn Val Gly His Pro Glu Leu Cys Gly Gly Arg Arg
    50                  55                  60


<210> 136
<211> 220
<212> DNA
<213> Conus dalli

<220>
<221> CDS
<222> (1)..(189)

<400> 136
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

tcc act tca ggt cgt gca ttt cat ggc agg aat gcc gca gcc aaa gcg     96
Ser Thr Ser Gly Arg Ala Phe His Gly Arg Asn Ala Ala Ala Lys Ala
            20                  25                  30

tct ggc ctg gtc ggt ctg acc gac aag agg caa gta tgc tgt agt gat     144
Ser Gly Leu Val Gly Leu Thr Asp Lys Arg Gln Val Cys Cys Ser Asp
        35                  40                  45

cct cgc tgt aac gta ggt cat cca gaa att tgt ggt gga aga cgc         189
Pro Arg Cys Asn Val Gly His Pro Glu Ile Cys Gly Gly Arg Arg
    50                  55                  60

tgatgctcca ggaccctctg aaccacgacg t                                  220

<210> 137
<211> 63
<212> PRT
<213> Conus dalli

<400> 137
Met Phe Thr Val Phe Leu Leu Val Val Leu Ala Thr Thr Val Val Ser
1               5                   10                  15

Ser Thr Ser Gly Arg Ala Phe His Gly Arg Asn Ala Ala Ala Lys Ala
            20                  25                  30

Ser Gly Leu Val Gly Leu Thr Asp Lys Arg Gln Val Cys Cys Ser Asp
        35                  40                  45

Pro Arg Cys Asn Val Gly His Pro Glu Ile Cys Gly Gly Arg Arg
    50                  55                  60


<210> 138
<211> 208
<212> DNA
<213> Conus achatinus

<220>
<221> CDS
<222> (1)..(180)

<400> 138
atg ttc acc gtg ttt ctg ttg gtt gtc ttg aca acc act gtc gtt tcc      48
Met Phe Thr Val Phe Leu Leu Val Val Leu Thr Thr Thr Val Val Ser
1               5                   10                  15

ttc cct tca gat agt gca tct ggt ggc agg gat gac gag gcc aaa gac      96
Phe Pro Ser Asp Ser Ala Ser Gly Gly Arg Asp Asp Glu Ala Lys Asp
            20                  25                  30

gaa agg tct gac atg tac gaa ttg aaa cgg aat gga cgc tgt tgc cat      144
Glu Arg Ser Asp Met Tyr Glu Leu Lys Arg Asn Gly Arg Cys Cys His
        35                  40                  45

cct gcc tgt ggt ggc aaa tac gtt aaa tgt gga cgc tgatgctcca           190
Pro Ala Cys Gly Gly Lys Tyr Val Lys Cys Gly Arg
    50                  55                  60

ggaccctctc gaaccacg                                                  208


<210> 139
<211> 60
<212> PRT
<213> Conus achatinus

<400> 139
Met Phe Thr Val Phe Leu Leu Val Val Leu Thr Thr Thr Val Val Ser
1               5                   10                  15

Phe Pro Ser Asp Ser Ala Ser Gly Gly Arg Asp Asp Glu Ala Lys Asp
            20                  25                  30

Glu Arg Ser Asp Met Tyr Glu Leu Lys Arg Asn Gly Arg Cys Cys His
        35                  40                  45

Pro Ala Cys Gly Gly Lys Tyr Val Lys Cys Gly Arg
    50                  55                  60


<210> 140
<211> 211
<212> DNA
<213> Conus bullatus

<220>
<221> CDS
<222> (1)..(174)
<400> 140
atg ttc acc gtg ttt ctg ttg gtt gtc ttg gca acc act gtc gtt tcc      48
Met Phe Thr Val Phe Leu Leu Val Val Leu Ala Thr Thr Val Val Ser
  1               5                  10                  15

ttc tct aca gat gat gaa tct gat ggc tcg aat gaa gaa ccc agc gcc      96
Phe Ser Thr Asp Asp Glu Ser Asp Gly Ser Asn Glu Glu Pro Ser Ala
                20                  25                  30

gac cag act gcc agg tcc tca atg aac agg gcg cct gga tgc tgt aac     144
Asp Gln Thr Ala Arg Ser Ser Met Asn Arg Ala Pro Gly Cys Cys Asn
        35                  40                  45

aat cct gcc tgt gtg aag cac aga tgt gga tgacgctgat gctccaggac       194
Asn Pro Ala Cys Val Lys His Arg Cys Gly
    50                  55

cctctgaacc acgacgt                                                  211


<210> 141
<211> 58
<212> PRT
<213> Conus bullatus

<400> 141
Met Phe Thr Val Phe Leu Leu Val Val Leu Ala Thr Thr Val Val Ser
  1               5                  10                  15

Phe Ser Thr Asp Asp Glu Ser Asp Gly Ser Asn Glu Glu Pro Ser Ala
                20                  25                  30

Asp Gln Thr Ala Arg Ser Ser Met Asn Arg Ala Pro Gly Cys Cys Asn
        35                  40                  45

Asn Pro Ala Cys Val Lys His Arg Cys Gly
    50                  55


<210> 142
<211> 214
<212> DNA
<213> Conus bullatus

<220>

<221> CDS
<222> (1)..(177)

<400> 142
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc tct aca gat gat gaa tct gat ggc tcg aat gaa gaa ccc agc gcc     96
Phe Ser Thr Asp Asp Glu Ser Asp Gly Ser Asn Glu Glu Pro Ser Ala
            20                  25                  30

gac cag gct gcc agg tcc gca atg aac agg ccg cct gga tgc tgt aac     144
Asp Gln Ala Ala Arg Ser Ala Met Asn Arg Pro Pro Gly Cys Cys Asn
        35                  40                  45

aat cct gcc tgt gtg aag cac aga tgt ggt gga tgacgctgat gctccaggac 197
Asn Pro Ala Cys Val Lys His Arg Cys Gly Gly
    50                  55

cctctgaacc acgacgt                                                214


<210> 143
<211> 59
<212> PRT
<213> Conus bullatus

<400> 143
Met Phe Thr Val Phe Leu Leu Val Val Leu Ala Thr Thr Val Val Ser
1               5                   10                  15

Phe Ser Thr Asp Asp Glu Ser Asp Gly Ser Asn Glu Glu Pro Ser Ala
            20                  25                  30

Asp Gln Ala Ala Arg Ser Ala Met Asn Arg Pro Pro Gly Cys Cys Asn
        35                  40                  45

Asn Pro Ala Cys Val Lys His Arg Cys Gly Gly
    50                  55


<210> 144
<211> 208
<212> DNA
<213> Conus bullatus

<220>
<221> CDS
<222> (1)..(177)

<400> 144
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

ttc cct tca gat cgt gac tct gat ggc gcg gat gcc gaa gcc agt gac     96
Phe Pro Ser Asp Arg Asp Ser Asp Gly Ala Asp Ala Glu Ala Ser Asp
            20                  25                  30

gag cct gtt gag ttc gaa agg gac gag aat gga tgc tgt tgg aat cct    144
Glu Pro Val Glu Phe Glu Arg Asp Glu Asn Gly Cys Cys Trp Asn Pro
        35                  40                  45

tcc tgt ccg agg ccc aga tgt aca gga cga cgc taatgctcca ggaccctctg 197
Ser Cys Pro Arg Pro Arg Cys Thr Gly Arg Arg
    50                  55

aaccacgacg t                                                      208


<210> 145
<211> 59
<212> PRT
<213> Conus bullatus

<400> 145
Met Phe Thr Val Phe Leu Leu Val Val Leu Ala Thr Thr Val Val Ser
1               5                   10                  15

Phe Pro Ser Asp Arg Asp Ser Asp Gly Ala Asp Ala Glu Ala Ser Asp
            20                  25                  30

Glu Pro Val Glu Phe Glu Arg Asp Glu Asn Gly Cys Cys Trp Asn Pro
        35                  40                  45
Ser Cys Pro Arg Pro Arg Cys Thr Gly Arg Arg
    50                  55


<210> 146
<211> 211
<212> DNA
<213> Conus bullatus

<220>
<221> CDS
<222> (1)..(180)

<400> 146
atg ttc acc gtg ttt ctg ttg gtt gtc ttg aca acc act gtc gtt tcc    48
Met Phe Thr Val Phe Leu Leu Val Val Leu Thr Thr Thr Val Val Ser
1               5                   10                  15

ttc cct tca gat cgt gca tct gat ggc agg aat gcc gca gcc aac gac    96
Phe Pro Ser Asp Arg Ala Ser Asp Gly Arg Asn Ala Ala Ala Asn Asp
            20                  25                  30

aaa gcg tct gac gtg gtc acg ctg gtc ctc aag gga tgc tgt tcc acc    144
Lys Ala Ser Asp Val Val Thr Leu Val Leu Lys Gly Cys Cys Ser Thr
        35                  40                  45

cct ccc tgt gct gtg ctg tat tgt ggt aga aga cgc tgatgctcca         190
Pro Pro Cys Ala Val Leu Tyr Cys Gly Arg Arg Arg
    50                  55                  60

ggaccctctg aaccacgacg t                                            211


<210> 147
<211> 60

<212> PRT
<213> Conus bullatus

<400> 147
Met Phe Thr Val Phe Leu Leu Val Val Leu Thr Thr Thr Val Val Ser
1               5                   10                  15

Phe Pro Ser Asp Arg Ala Ser Asp Gly Arg Asn Ala Ala Ala Asn Asp
            20                  25                  30

Lys Ala Ser Asp Val Val Thr Leu Val Leu Lys Gly Cys Cys Ser Thr
        35                  40                  45

Pro Pro Cys Ala Val Leu Tyr Cys Gly Arg Arg Arg
    50                  55                  60


<210> 148
<211> 212
<212> DNA
<213> Conus distans

<220>
<221> CDS
<222> (1)..(171)

<400> 148
atg ttc acc gtg ttt ctg ttg gtt gtc ttc gca tcc tct gtc acc tta    48
Met Phe Thr Val Phe Leu Leu Val Val Phe Ala Ser Ser Val Thr Leu
1               5                   10                  15
gat cgt gca tct tat ggc agg tat gcc tca ccc gtc gac aga gcg tct    96
Asp Arg Ala Ser Tyr Gly Arg Tyr Ala Ser Pro Val Asp Arg Ala Ser
            20                  25                  30

gcc ctg atc gct cag gcc atc ctt cga gat tgc tgc tcc aat cct cct    144
Ala Leu Ile Ala Gln Ala Ile Leu Arg Asp Cys Cys Ser Asn Pro Pro
        35                  40                  45

tgt gcc cat aat aat cca gac tgt cgt taaagacgct gcttgctcca          191
Cys Ala His Asn Asn Pro Asp Cys Arg
    50                  55

ggaccctctg aaccacgacg t                                            212


<210> 149
<211> 57
<212> PRT
<213> Conus distans

<400> 149
Met Phe Thr Val Phe Leu Leu Val Val Phe Ala Ser Ser Val Thr Leu
1               5                   10                  15

Asp Arg Ala Ser Tyr Gly Arg Tyr Ala Ser Pro Val Asp Arg Ala Ser
            20                  25                  30

Ala Leu Ile Ala Gln Ala Ile Leu Arg Asp Cys Cys Ser Asn Pro Pro
        35                  40                  45

Cys Ala His Asn Asn Pro Asp Cys Arg
    50                  55

<210> 150
<211> 63
<212> DNA
<213> Conus textile

<220>
<221> CDS
<222> (1)..(60)

<400> 150
gga tgc tgt tct aat cct ccc tgt atc gcg aag aat cca cac atg tgt     48
Gly Cys Cys Ser Asn Pro Pro Cys Ile Ala Lys Asn Pro His Met Cys
 1               5                  10                  15

ggt gga aga cgc tga                                                 63
Gly Gly Arg Arg
            20

<210> 151
<211> 20
<212> PRT
<213> Conus textile

<400> 151
Gly Cys Cys Ser Asn Pro Pro Cys Ile Ala Lys Asn Pro His Met Cys
 1               5                  10                  15

Gly Gly Arg Arg
            20

<210> 152
<211> 220
<212> DNA
<213> Conus consors

<220>
<221> CDS
<222> (1)..(189)

<400> 152
atg ttc acc gtg ttt ctg ttg gtt gtc ttg gca acc act gtc gtt tcc     48
Met Phe Thr Val Phe Leu Leu Val Val Leu Ala Thr Thr Val Val Ser
 1               5                  10                  15

ttc cct tca gat cgt gca tct gat ggc agg aat gcc gca gcc aac gac     96
Phe Pro Ser Asp Arg Ala Ser Asp Gly Arg Asn Ala Ala Ala Asn Asp
            20                  25                  30

aaa gcg tct gac gtg atc acg ctg gcc ctc aag gga tgc tgt tcc aac    144
Lys Ala Ser Asp Val Ile Thr Leu Ala Leu Lys Gly Cys Cys Ser Asn
        35                  40                  45

cct gtc tgt cac ttg gag cat tca aac ctt tgt ggt aga aga cgc        189
Pro Val Cys His Leu Glu His Ser Asn Leu Cys Gly Arg Arg Arg
    50                  55                  60

tgatgctcca ggaccctctg aaccacgacg t                                    220

<210> 153
<211> 63
<212> PRT
<213> Conus consors

<400> 153
Met Phe Thr Val Phe Leu Leu Val Val Leu Ala Thr Thr Val Val Ser
1               5                   10                  15

Phe Pro Ser Asp Arg Ala Ser Asp Gly Arg Asn Ala Ala Ala Asn Asp
            20                  25                  30

Lys Ala Ser Asp Val Ile Thr Leu Ala Leu Lys Gly Cys Cys Ser Asn
        35                  40                  45

Pro Val Cys His Leu Glu His Ser Asn Leu Cys Gly Arg Arg Arg
    50                  55                  60


<210> 154
<211> 15
<212> PRT
<213> Conus musicus

<220>
<221> VARIANT
<222> (4)
<223> /replace = nor-Tyr
      /replace = mono-halo-Tyr
      /replace = di-halo-Tyr
      /replace = O-sulpho-Tyr

<220>
<221> VARIANT
<222> (4)
<223> /replace = O-phospho-Tyr
      /replace = nitro-Tyr

<220>
<221> VARIANT
<222> (6)
<223> /replace = hydroxy-Pro

<220>
<221> VARIANT
<222> (9)
<223> /replace = N-methyl-Lys
      /replace = N,N-dimethyl-Lys
      /replace = N,N,N-trimethyl-Lys

<220>
<221> VARIANT
<222> (10)
<223> /replace = N-methyl-Lys
      /replace = N,N-dimethyl-Lys
      /replace = N,N,N-trimethyl-Lys

<220>
<221> VARIANT
<222> (11)
<223> /replace = nor-Tyr
      /replace = mono-halo-Tyr
      /replace = di-halo-Tyr
      /replace = O-sulpho-Tyr

<220>
<221> VARIANT
<222> (11)
<223> /replace = O-phospho-Tyr
      /replace = nitro-Tyr

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<220>
<221> VARIANT
<222> (15)
<223> /replace = N-methyl-Lys
      /replace = N,N-dimethyl-Lys
      /replace = N,N,N-trimethyl-Lys

<220>
<221> VARIANT
<222> (10)
<223> /replace = D-Trp
      /replace = halo-Trp

<400> 154
Gly Cys Cys Tyr Asn Pro Val Cys Lys Lys Tyr Tyr Cys Trp Lys
1               5                   10                  15

<210> 155
<211> 16
<212> PRT
<213> Conus purpurascens

<220>
<221> VARIANT
<222> (1)
<223> /replace = pyro-Glu

<220>
<221> VARIANT
<222> (2)
<223> /replace = gamma-carboxy-Glu

<220>
<221> VARIANT
<222> (3)
<223> /replace = hydroxy-Pro

<220>
<221> VARIANT
<222> (9)
<223> /replace = hydroxy-Pro

<220>
<221> VARIANT
<222> (13)
<223> /replace = N-methyl-Lys
      /replace = N,N-dimethyl-Lys
      /replace = N,N,N-trimethyl-Lys

<400> 155
Gln Glu Pro Gly Cys Cys Arg His Pro Ala Cys Gly Lys Asn Arg Cys
1               5                   10                  15


<210> 156
<211> 13
<212> PRT
<213> Conus musicus

<220>
<221> VARIANT
<222> (5)
<223> /replace = hydroxy-Pro

<220>
<221> VARIANT
<222> (14)
<223> /replace = hydroxy-Pro


<400> 156
Cys Cys Ala Asp Pro Asp Cys Arg Phe Arg Pro Gly Cys
1               5                   10


<210> 157
<211> 17
<212> PRT
<213> Conus musicus

<220>
<221> VARIANT
<222> (4)
<223> /replace = nor-Tyr
      /replace = mono-halo-Tyr
      /replace = di-halo-Tyr
      /replace = O-sulpho-Tyr

<220>
<221> VARIANT
<222> (4)

<223> /replace = O-phospho-Tyr
      /replace = nitro-Tyr

<220>
<221> VARIANT
<222> (6)
<223> /replace = hydroxy-Pro

<220>
<221> VARIANT
<222> (9)
<223> /replace = D-Trp
      /replace = halo-Trp

<220>
<221> VARIANT
<222> (10)
<223> /replace = hydroxy-Pro

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (13)
<223> /replace = nor-Tyr
      /replace = mono-halo-Tyr
      /replace = di-halo-Tyr
      /replace = O-sulpho-Tyr

<220>
<221> VARIANT
<222> (13)
<223> /replace = O-phospho-Tyr
      /replace = nitro-Tyr

<220>
<221> VARIANT
<222> (16)
<223> /replace = D-Trp
      /replace = halo-Trp

<220>
<221> VARIANT
<222> (11)
<223> /replace = N-methyl-Lys
      /replace = N,N-dimethyl-Lys
      /replace = N,N,N-trimethyl-Lys

<400> 157
Gly Cys Cys Tyr Asn Pro Ser Cys Trp Pro Lys Thr Tyr Cys Ser Trp
 1               5                  10                  15

Lys

<210> 158
<211> 13
<212> PRT
<213> Conus musicus

<220>
<221> VARIANT
<222> (5)
<223> /replace = hydroxy-Pro

<220>
<221> VARIANT
<222> (8)
<223> /replace = N-methyl-Lys
      /replace = N,N-dimethyl-Lys
      /replace = N,N,N-trimethyl-Lys

<220>
<221> VARIANT
<222> (9)
<223> /replace = gamma-carboxy-Glu

<220>
<221> VARIANT
<222> (11)
<223> /replace = nor-Tyr
      /replace = mono-halo-Tyr
      /replace = di-halo-Tyr
      /replace = O-sulpho-Tyr

<220>
<221> VARIANT
<222> (11)
<223> /replace = O-phospho-Tyr
      /replace = nitro-Tyr

<400> 158
Cys Cys Ser Asn Pro Thr Cys Lys Glu Thr Tyr Gly Cys
 1               5                   10


<210> 159
<211> 13
<212> PRT
<213> Conus musicus

<220>
<221> VARIANT
<222> (5)
<223> /replace = hydroxy-Pro


<220>
<221> VARIANT
<222> (8)
<223> /replace = N-methyl-Lys
      /replace = N,N-dimethyl-Lys
      /replace = N,N,N-trimethyl-Lys

<220>

<221> VARIANT
<222> (11)
<223> /replace = hydroxy-Pro

<400> 159
Cys Cys Ala Asn Pro Ile Cys Lys Asn Thr Pro Gly Cys
 1               5                   10


<210> 160
<211> 13
<212> PRT
<213> Conus musicus

<220>
<221> VARIANT
<222> (5)
<223> /replace = hydroxy-Pro

<220>
<221> VARIANT
<222> (8)
<223> /replace = N-methyl-Lys
      /replace = N,N-dimethyl-Lys
      /replace = N,N,N-trimethyl-Lys

<220>
<221> VARIANT
<222> (9)
<223> /replace = gamma-carboxy-Glu

<220>
<221> VARIANT
<222> (11)
<223> /replace = nor-Tyr
      /replace = mono-halo-Tyr
      /replace = di-halo-Tyr
      /replace = O-sulpho-Tyr

<220>
<221> VARIANT
<222> (11)
<223> /replace = O-phospho-Tyr
      /replace = nitro-Tyr

<400> 160
Cys Cys Asn Asn Pro Thr Cys Lys Glu Thr Tyr Gly Cys
 1               5                   10


<210> 161
<211> 13
<212> PRT
<213> Conus musicus

<220>
<221> VARIANT
<222> (5)
<223> /replace = hydroxy-Pro

<220>
<221> VARIANT
<222> (8)
<223> /replace = N-methyl-Lys
      /replace = N,N-dimethyl-Lys
      /replace = N,N,N-trimethyl-Lys

<220>
<221> VARIANT
<222> (9)
<223> /replace = gamma-carboxy-Glu

<220>
<221> VARIANT
<222> (11)
<223> /replace = nor-Tyr
      /replace = mono-halo-Tyr
      /replace = di-halo-Tyr
      /replace = O-sulpho-Tyr

<220>
<221> VARIANT
<222> (11)
<223> /replace = O-phospho-Tyr
      /replace = nitro-Tyr

<400> 161
Cys Cys Ser Asn Pro Val Cys Lys Glu Thr Tyr Gly Cys
1               5                   10


<210> 162
<211> 17
<212> PRT
<213> Conus betulinus

<220>
<221> VARIANT
<222> (6)
<223> /replace = nor-Tyr
      /replace = mono-halo-Tyr
      /replace = di-halo-Tyr
      /replace = O-sulpho-Tyr

<220>
<221> VARIANT
<222> (6)
<223> /replace = O-phospho-Tyr
      /replace = nitro-Tyr

<220>
<221> VARIANT
<222> (7)
<223> /replace = hydroxy-Pro

<220>
<221> VARIANT
<222> (8)
<223> /replace = hydroxy-Pro

<220>
<221> VARIANT
<222> (14)
<223> /replace = hydroxy-Pro

<220>
<221> VARIANT
<222> (15)
<223> /replace = N-methyl-Lys
      /replace = N,N-dimethyl-Lys
      /replace = N,N,N-trimethyl-Lys

<400> 162
Gly Gly Cys Cys Ser Tyr Pro Pro Cys Ile Ala Ser Asn Pro Lys Cys
1               5                   10                  15

Gly


<210> 163
<211> 15
<212> PRT
<213> Conus lividus

<220>
<221> VARIANT
<222> (7)
<223> /replace = hydroxy-Pro

<220>
<221> VARIANT
<222> (13)
<223> /replace = hydroxy-Pro

<400> 163
Gly Cys Cys Ser His Pro Val Cys Ser Ala Met Ser Pro Ile Cys
1               5                   10                  15


<210> 164
<211> 15
<212> PRT
<213> Conus musicus

<220>
<221> VARIANT
<222> (4)
<223> /replace = N-methyl-Lys
      /replace = N,N-dimethyl-Lys
      /replace = N,N,N-trimethyl-Lys

<220>
<221> VARIANT
<222> (6)
<223> /replace = hydroxy-Pro

<220>
<221> VARIANT
<222> (7)
<223> /replace = nor-Tyr

/replace = mono-halo-Tyr
      /replace = di-halo-Tyr
      /replace = O-sulpho-Tyr

<220>
<221> VARIANT
<222> (7)
<223> /replace = O-phospho-Tyr
      /replace = nitro-Tyr

<220>
<221> VARIANT
<222> (12)
<223> /replace = N-methyl-Lys
      /replace = N,N-dimethyl-Lys
      /replace = N,N,N-trimethyl-Lys

<220>
<221> VARIANT
<222> (14)
<223> /replace = nor-Tyr
      /replace = mono-halo-Tyr
      /replace = di-halo-Tyr
      /replace = O-sulpho-Tyr

<220>
<221> VARIANT
<222> (14)
<223> /replace = O-phospho-Tyr
      /replace = nitro-Tyr

<400> 164
Gly Cys Cys Lys Asn Pro Tyr Cys Gly Ala Ser Lys Thr Tyr Cys
1               5                   10                  15


<210> 165
<211> 15
<212> PRT
<213> Conus omaria

<220>
<221> VARIANT
<222> (5)
<223> /replace = nor-Tyr
      /replace = mono-halo-Tyr
      /replace = di-halo-Tyr
      /replace = O-sulpho-Tyr

<220>
<221> VARIANT
<222> (5)
<223> /replace = O-phospho-Tyr
      /replace = nitro-Tyr

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (13)
<223> /replace = hydroxy-Pro

<400> 165
Gly Cys Cys Ser Tyr Pro Pro Cys Phe Ala Thr Asn Pro Asp Cys
1               5                   10                  15


<210> 166
<211> 17
<212> PRT
<213> Conus radiatus

<220>
<221> VARIANT
<222> (6)
<223> /replace = nor-Tyr
      /replace = mono-halo-Tyr
      /replace = di-halo-Tyr
      /replace = O-sulpho-Tyr

<220>
<221> VARIANT
<222> (6)
<223> /replace = O-phospho-Tyr
      /replace = nitro-Tyr

<220>
<221> VARIANT
<222> (7)
<223> /replace = hydroxy-Pro

<220>
<221> VARIANT
<222> (8)
<223> /replace = hydroxy-Pro

<220>
<221> VARIANT
<222> (14)
<223> /replace = hydroxy-Pro

<400> 166
Gly Gly Cys Cys Ser Tyr Pro Pro Cys Ile Ala Asn Asn Pro Leu Cys
1               5                   10                  15

Ala


<210> 167
<211> 17
<212> PRT
<213> Conus radiatus

<220>
<221> VARIANT
<222> (6)
<223> /replace = nor-Tyr
      /replace = mono-halo-Tyr
      /replace = di-halo-Tyr
      /replace = O-sulpho-Tyr

<220>
<221> VARIANT
<222> (6)
<223> /replace = O-phospho-Tyr
      /replace = nitro-Tyr

<220>
<221> VARIANT
<222> (7)
<223> /replace = hydroxy-Pro

<220>
<221> VARIANT
<222> (8)
<223> /replace = hydroxy-Pro

<220>
<221> VARIANT
<222> (14)
<223> /replace = hydroxy-Pro

<400> 167
Gly Gly Cys Cys Ser Tyr Pro Pro Cys Ile Ala Asn Asn Pro Phe Cys
 1               5                   10                  15

Ala


<210> 168
<211> 16
<212> PRT
<213> Conus virgo

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (13)
<223> /replace = hydroxy-Pro

<400> 168
Asp Cys Cys Ser Asn Pro Pro Cys Ser Gln Asn Asn Pro Asp Cys Met
 1               5                   10                  15

<210> 169
<211> 16
<212> PRT
<213> Conus virgo

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (13)
<223> /replace = hydroxy-Pro

<400> 169
Asp Cys Cys Ser Asn Pro Pro Cys Ala His Asn Asn Pro Asp Cys Arg
1               5                   10                  15


<210> 170
<211> 20
<212> PRT
<213> Conus achatinus

<220>
<221> VARIANT
<222> (1)
<223> /replace = gamma-carboxy-Glu

<220>
<221> VARIANT
<222> (6)
<223> /replace = hydroxy-Pro

<220>
<221> VARIANT
<222> (11)
<223> /replace = gamma-carboxy-Glu

<220>
<221> VARIANT
<222> (14)
<223> /replace = gamma-carboxy-Glu

<400> 170
Glu Cys Cys Thr Asn Pro Val Cys His Ala Glu His Gln Glu Leu Cys
1               5                   10                  15

Ala Arg Arg Arg
            20

<210> 171
<211> 16
<212> PRT
<213> Conus achatinus

<220>
<221> VARIANT
<222> (6)
<223> /replace = hydroxy-Pro

<220>
<221> VARIANT
<222> (11)
<223> /replace = gamma-carboxy-Glu

<400> 171
Gly Cys Cys Ser Asn Pro Val Cys His Leu Glu His Ser Asn Leu Cys
1               5                   10                  15


<210> 172
<211> 20
<212> PRT
<213> Conus achatinus

<220>
<221> VARIANT
<222> (1)
<223> /replace = gamma-carboxy-Glu

<220>
<221> VARIANT
<222> (6)
<223> /replace = hydroxy-Pro

<220>
<221> VARIANT
<222> (11)
<223> /replace = gamma-carboxy-Glu

<220>
<221> VARIANT
<222> (14)
<223> /replace = gamma-carboxy-Glu

<400> 172
Glu Cys Cys Thr Asn Pro Val Cys His Val Glu His Gln Glu Leu Cys
1               5                   10                  15

Ala Arg Arg Arg
            20


<210> 173
<211> 17
<212> PRT
<213> Conus ammiralis

<220>
<221> VARIANT

<222> (1)
<223> /replace = pyro-Glu

<220>
<221> VARIANT
<222> (2)
<223> /replace = gamma-carboxy-Glu

<220>
<221> VARIANT
<222> (6)
<223> /replace = nor-Tyr
      /replace = mono-halo-Tyr
      /replace = di-halo-Tyr
      /replace = O-sulpho-Tyr

<220>
<221> VARIANT
<222> (6)
<223> /replace = O-phospho-Tyr
      /replace = nitro-Tyr

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 173
Gln Glu Cys Cys Ser Tyr Pro Ala Cys Asn Leu Asp His Pro Glu Leu
1               5                   10                  15
Cys


<210> 174
<211> 18
<212> PRT
<213> Conus ammiralis

<220>
<221> VARIANT
<222> (1)
<223> /replace = hydroxy-Pro

<220>
<221> VARIANT
<222> (2)
<223> /replace = gamma-carboxy-Glu

<220>
<221> VARIANT

<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 174
Pro Glu Cys Cys Ser Asp Pro Arg Cys Asn Ser Thr His Pro Glu Leu
1               5                   10                  15

Cys Gly

<210> 175
<211> 21
<212> PRT
<213> Conus arenatus

<220>
<221> VARIANT
<222> (7)
<223> /replace = hydroxy-Pro

<220>
<221> VARIANT
<222> (8)
<223> /replace = hydroxy-Pro

<220>
<221> VARIANT
<222> (10)
<223> /replace = D-Trp
      /replace = halo-Trp

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (12)
<223> /replace = N-methyl-Lys
      /replace = N,N-dimethyl-Lys
      /replace = N,N,N-trimethyl-Lys

<220>
<221> VARIANT
<222> (13)
<223> /replace = nor-Tyr

/replace = mono-halo-Tyr
      /replace = di-halo-Tyr
      /replace = O-sulpho-Tyr
<220>
<221> VARIANT
<222> (13)
<223> /replace = O-phospho-Tyr
      /replace = nitro-Tyr

<220>
<221> VARIANT
<222> (15)
<223> /replace = gamma-carboxy-Glu

<400> 175
Leu Asn Cys Cys Met Ile Pro Pro Cys Trp Lys Lys Tyr Gly Asp Arg
1               5                   10                  15

Cys Ser Glu Val Arg
            20


<210> 176
<211> 22
<212> PRT
<213> Conus arenatus

<220>
<221> VARIANT
<222> (9)
<223> /replace = hydroxy-Pro

<220>
<221> VARIANT
<222> (12)
<223> /replace = gamma-carboxy-Glu

<220>
<221> VARIANT
<222> (14)
<223> /replace = nor-Tyr
      /replace = mono-halo-Tyr
      /replace = di-halo-Tyr
      /replace = O-sulpho-Tyr

<220>
<221> VARIANT
<222> (14)
<223> /replace = O-phospho-Tyr
      /replace = nitro-Tyr

<220>
<221> VARIANT
<222> (20)
<223> /replace = gamma-carboxy-Glu

<400> 176
Ala Phe Gly Cys Cys Asp Leu Ile Pro Cys Leu Glu Arg Tyr Gly Asn
1               5                   10                  15

Arg Cys Asn Glu Val His
            20

<210> 177
<211> 21
<212> PRT
<213> Conus arenatus

<220>
<221> VARIANT
<222> (8)
<223> /replace = hydroxy-Pro

<220>
<221> VARIANT
<222> (10)
<223> /replace = D-Trp
      /replace = halo-Trp

<220>
<221> VARIANT
<222> (11)
<223> /replace = gamma-carboxy-Glu

<220>
<221> VARIANT
<222> (12)
<223> /replace = N-methyl-Lys
      /replace = N,N-dimethyl-Lys
      /replace = N,N,N-trimethyl-Lys

<220>
<221> VARIANT
<222> (13)
<223> /replace = nor-Tyr
      /replace = mono-halo-Tyr
      /replace = di-halo-Tyr
      /replace = O-sulpho-Tyr

<220>
<221> VARIANT
<222> (13)
<223> /replace = O-phospho-Tyr
      /replace = nitro-Tyr

<220>
<221> VARIANT
<222> (16)
<223> /replace = N-methyl-Lys
      /replace = N,N-dimethyl-Lys
      /replace = N,N,N-trimethyl-Lys

<220>
<221> VARIANT
<222> (19)
<223> /replace = gamma-carboxy-Glu

<400> 177
Leu Gly Cys Cys Asn Val Thr Pro Cys Trp Glu Lys Tyr Gly Asp Lys

1                 5                   10                    15

Cys Asn Glu Val Arg
              20


<210> 178
<211> 20
<212> PRT
<213> Conus arenatus

<220>
<221> VARIANT
<222> (2)
<223> /replace = gamma-carboxy-Glu

<220>
<221> VARIANT
<222> (7)
<223> /replace = hydroxy-Pro

<220>
<221> VARIANT
<222> (14)
<223> /replace = hydroxy-Pro

<400> 178
Asp Glu Cys Cys Ser Asn Pro Ala Cys Arg Val Asn Asn Pro His Val
1                 5                   10                    15

Cys Arg Arg Arg
              20


<210> 179
<211> 21
<212> PRT
<213> Conus arenatus

<220>
<221> VARIANT
<222> (7)
<223> /replace = hydroxy-Pro

<220>
<221> VARIANT
<222> (10)
<223> /replace = D-Trp
      /replace = halo-Trp

<220>
<221> VARIANT
<222> (12)
<223> /replace = gamma-carboxy-Glu

<220>
<221> VARIANT
<222> (13)
<223> /replace = nor-Tyr
      /replace = mono-halo-Tyr

/replace = di-halo-Tyr
/replace = O-sulpho-Tyr

<220>
<221> VARIANT
<222> (13)
<223> /replace = O-phospho-Tyr
/replace = nitro-Tyr

<220>
<221> VARIANT
<222> (14)
<223> /replace = N-methyl-Lys
/replace = N,N-dimethyl-Lys
/replace = N,N,N-trimethyl-Lys

<220>
<221> VARIANT
<222> (19)
<223> /replace = N-methyl-Lys
/replace = N,N-dimethyl-Lys
/replace = N,N,N-trimethyl-Lys

<400> 179
Leu Asn Cys Cys Ser Ile Pro Gly Cys Trp Asn Glu Tyr Lys Asp Arg
1               5                   10                  15

Cys Ser Lys Val Arg
            20

<210> 180
<211> 18
<212> PRT
<213> Conus aurisiacus

<220>
<221> VARIANT
<222> (7)
<223> /replace = hydroxy-Pro

<220>
<221> VARIANT
<222> (10)
<223> /replace = nor-Tyr
/replace = mono-halo-Tyr
/replace = di-halo-Tyr
/replace = O-sulpho-Tyr

<220>
<221> VARIANT
<222> (10)
<223> /replace = O-phospho-Tyr
/replace = nitro-Tyr

<220>
<221> VARIANT
<222> (14)
<223> /replace = hydroxy-Pro

<400> 180
Gly Gly Cys Cys Ser His Pro Val Cys Tyr Phe Asn Asn Pro Gln Met
1               5                   10                  15

Cys Arg


<210> 181
<211> 18
<212> PRT
<213> Conus aurisiacus

<220>
<221> VARIANT
<222> (7)
<223> /replace = hydroxy-Pro

<220>
<221> VARIANT
<222> (14)
<223> /replace = hydroxy-Pro

<400> 181
Gly Gly Cys Cys Ser His Pro Val Cys Asn Leu Asn Asn Pro Gln Met
1               5                   10                  15

Cys Arg



<210> 182
<211> 17
<212> PRT
<213> Conus bandanus

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (9)
<223> /replace = nor-Tyr
      /replace = mono-halo-Tyr
      /replace = di-halo-Tyr
      /replace = O-sulpho-Tyr

<220>
<221> VARIANT
<222> (9)
<223> /replace = O-phospho-Tyr
      /replace = nitro-Tyr

<220>

<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 182
Gly Cys Cys Ser His Pro Pro Cys Tyr Ala Asn Asn Gln Ala Tyr Cys
 1               5                   10                  15

Asn


<210> 183
<211> 17
<212> PRT
<213> Conus betulinus

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 183
Gly Gly Cys Cys Ser His Pro Ala Cys Ser Val Thr His Pro Glu Leu
 1               5                   10                  15

Cys


<210> 184
<211> 18
<212> PRT
<213> Conus betulinus

<220>
<221> VARIANT
<222> (6)
<223> /replace = nor-Tyr
      /replace = mono-halo-Tyr
      /replace = di-halo-Tyr
      /replace = O-sulpho-Tyr

<220>
<221> VARIANT
<222> (6)
<223> /replace = O-phospho-Tyr
      /replace = nitro-Tyr

<220>
<221> VARIANT
<222> (7)
<223> /replace = hydroxy-Pro

<220>
<221> VARIANT
<222> (12)
<223> /replace = gamma-carboxy-Glu

<400> 184
Gly Gly Cys Cys Ser Tyr Pro Ala Cys Ser Val Glu His Gln Asp Leu
1               5                   10                  15

Cys Asp

<210> 185
<211> 25
<212> PRT
<213> Conus caracteristicus

<220>
<221> VARIANT
<222> (8)
<223> /replace = hydroxy-Pro

<220>
<221> VARIANT
<222> (10)
<223> /replace = D-Trp
      /replace = halo-Trp

<220>
<221> VARIANT
<222> (13)
<223> /replace = nor-Tyr
      /replace = mono-halo-Tyr
      /replace = di-halo-Tyr
      /replace = O-sulpho-Tyr

<220>
<221> VARIANT
<222> (13)
<223> /replace = O-phospho-Tyr
      /replace = nitro-Tyr

<220>
<221> VARIANT
<222> (15)
<223> /replace = gamma-carboxy-Glu

<220>

<221> VARIANT
<222> (16)
<223> /replace = gamma-carboxy-Glu

<220>
<221> VARIANT
<222> (19)
<223> /replace = gamma-carboxy-Glu

<220>
<221> VARIANT
<222> (22)
<223> /replace = hydroxy-Pro

<400> 185
Val Ser Cys Cys Val Val Arg Pro Cys Trp Ile Arg Tyr Gln Glu Glu
1               5                   10                  15

Cys Leu Glu Ala Asp Pro Arg Thr Leu
            20                  25

<210> 186
<211> 21
<212> PRT
<213> Conus caracteristicus

<220>
<221> VARIANT
<222> (1)
<223> /replace = pyro-Glu

<220>
<221> VARIANT
<222> (7)
<223> /replace = hydroxy-Pro

<220>
<221> VARIANT
<222> (10)
<223> /replace = D-Trp
      /replace = halo-Trp

<220>
<221> VARIANT
<222> (11)
<223> /replace = gamma-carboxy-Glu

<220>
<221> VARIANT
<222> (12)
<223> /replace = N-methyl-Lys
      /replace = N,N-dimethyl-Lys
      /replace = N,N,N-trimethyl-Lys

<220>
<221> VARIANT
<222> (13)
<223> /replace = nor-Tyr
      /replace = mono-halo-Tyr
      /replace = di-halo-Tyr

/replace = O-sulpho-Tyr

<220>
<221> VARIANT
<222> (13)
<223> /replace = O-phospho-Tyr
      /replace = nitro-Tyr

<220>
<221> VARIANT
<222> (16)
<223> /replace = N-methyl-Lys
      /replace = N,N-dimethyl-Lys
      /replace = N,N,N-trimethyl-Lys

<220>
<221> VARIANT
<222> (19)
<223> /replace = gamma-carboxy-Glu

<400> 186
Gln Asn Cys Cys Ser Ile Pro Gly Cys Trp Glu Lys Tyr Gly Asp Lys
1               5                   10                  15

Cys Ser Glu Val Arg
            20

<210> 187
<211> 16
<212> PRT
<213> Conus catus

<220>
<221> VARIANT
<222> (6)
<223> /replace = hydroxy-Pro

<220>
<221> VARIANT
<222> (15)
<223> /replace = gamma-carboxy-Glu

<220>
<221> VARIANT
<222> (13)
<223> /replace = hydroxy-Pro

<400> 187
Gly Cys Cys Ser Asn Pro Val Cys His Leu Glu His Pro Asn Ala Cys
1               5                   10                  15


<210> 188
<211> 17
<212> PRT
<213> Conus catus

<220>
<221> VARIANT
<222> (6)

<223> /replace = hydroxy-Pro

<220>
<221> VARIANT
<222> (9)
<223> /replace = nor-Tyr
      /replace = mono-halo-Tyr
      /replace = di-halo-Tyr
      /replace = O-sulpho-Tyr

<220>
<221> VARIANT
<222> (9)
<223> /replace = O-phospho-Tyr
      /replace = nitro-Tyr

<220>
<221> VARIANT
<222> (13)
<223> /replace = hydroxy-Pro

<400> 188
Gly Cys Cys Ser Asn Pro Ile Cys Tyr Phe Asn Asn Pro Arg Ile Cys
1               5                   10                  15

Arg


<210> 189
<211> 17
<212> PRT
<213> Conus episcopatus

<220>
<221> VARIANT
<222> (1)
<223> /replace = gamma-carboxy-Glu

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (10)
<223> /replace = D-Trp
      /replace = halo-Trp

<220>
<221> VARIANT
<222> (13)
<223> /replace = hydroxy-Pro

<220>

<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (14)
<223> /replace = gamma-carboxy-Glu

<400> 189
Glu Cys Cys Ser Gln Pro Pro Cys Arg Trp Lys His Pro Glu Leu Cys
1               5                   10                  15

Ser


<210> 190
<211> 16
<212> PRT
<213> Conus geographus

<220>
<221> VARIANT
<222> (6)
<223> /replace = hydroxy-Pro.

<400> 190
Gly Cys Cys Ser His Pro Ala Cys Ala Gly Asn Asn Gln His Ile Cys
1               5                   10                  15


<210> 191
<211> 18
<212> PRT
<213> Conus geographus

<220>
<221> VARIANT
<222> (6)
<223> /replace = hydroxy-Pro

<220>
<221> VARIANT
<222> (13)
<223> /replace = hydroxy-Pro

<400> 191
Gly Cys Cys Ala Val Pro Ser Cys Arg Leu Arg Asn Pro Asp Leu Cys
1               5                   10                  15

Gly Gly


<210> 192
<211> 16
<212> PRT
<213> Conus imperialis

<220>
<221> VARIANT
<222> (6)
<223> /replace = hydroxy-Pro

<220>
<221> VARIANT
<222> (13)
<223> /replace = hydroxy-Pro

<400> 192
Gly Cys Cys Ser His Pro Ala Cys Asn Val Asn Asn Pro His Ile Cys
1               5                   10                  15

<210> 193
<211> 20
<212> PRT
<213> Conus lividus

<220>
<221> VARIANT
<222> (2)
<223> /replace = hydroxy-Pro

<220>
<221> VARIANT
<222> (3)
<223> /replace = gamma-carboxy-Glu

<220>
<221> VARIANT
<222> (4)
<223> /replace = gamma-carboxy-Glu

<220>
<221> VARIANT
<222> (7)
<223> /replace = hydroxy-Pro

<220>
<221> VARIANT
<222> (9)
<223> /replace = hydroxy-Pro

<220>
<221> VARIANT
<222> (10)
<223> /replace = hydroxy-Pro

<400> 193
Thr Pro Glu Glu Cys Cys Pro Asn Pro Pro Cys Phe Ala Thr Asn Ser
1               5                   10                  15

Asp Ile Cys Gly
            20

<210> 194

<211> 17
<212> PRT
<213> Conus lividus

<220>
<221> VARIANT
<222> (7)
<223> /replace = hydroxy-Pro

<220>
<221> VARIANT
<222> (12)
<223> /replace = N-methyl-Lys
      /replace = N,N-dimethyl-Lys
      /replace = N,N,N-trimethyl-Lys

<400> 194
Asp Ala Cys Cys Ser Asp Pro Arg Cys Ser Gly Lys His Gln Asp Leu
1               5                   10                  15

Cys


<210> 195
<211> 17
<212> PRT
<213> Conus lividus

<220>
<221> VARIANT
<222> (1)
<223> /replace = gamma-carboxy-Glu

<220>
<221> VARIANT
<222> (7)
<223> /replace = hydroxy-Pro

<400> 195
Glu Asp Cys Cys Ser Asp Pro Arg Cys Ser Val Gly His Gln Asp Leu
1               5                   10                  15

Cys


<210> 196
<211> 16
<212> PRT
<213> Conus lividus

<220>
<221> VARIANT
<222> (6)
<223> /replace = hydroxy-Pro

<400> 196
Gly Cys Cys Ser His Pro Ala Cys Ala Gly Ser Asn Ala His Ile Cys
1               5                   10                  15

<210> 197
<211> 17
<212> PRT
<213> Conus lividus

<220>
<221> VARIANT
<222> (1)
<223> /replace = gamma-carboxy-Glu

<220>
<221> VARIANT
<222> (7)
<223> /replace = hydroxy-Pro

<400> 197
Glu Asp Cys Cys Ser Asp Pro Arg Cys Ser Val Gly His Gln Asp Met
1               5                   10                  15

Cys


<210> 198
<211> 16
<212> PRT
<213> Conus lividus

<220>
<221> VARIANT
<222> (6)
<223> /replace = hydroxy-Pro

<220>
<221> VARIANT
<222> (13)
<223> /replace = hydroxy-Pro

<400> 198
Gly Cys Cys Ser His Pro Ala Cys Ala Gly Asn Asn Pro His Ile Cys
1               5                   10                  15


<210> 199
<211> 17
<212> PRT
<213> Conus lividus

<220>
<221> VARIANT
<222> (6)
<223> /replace = hydroxy-Pro

<220>
<221> VARIANT
<222> (13)
<223> /replace = hydroxy-Pro

<220>
<221> VARIANT
<222> (14)

<223> /replace = nor-Tyr
      /replace = mono-halo-Tyr
      /replace = di-halo-Tyr
      /replace = O-sulpho-Tyr

<220>
<221> VARIANT
<222> (14)
<223> /replace = O-phospho-Tyr
      /replace = nitro-Tyr

<400> 199
Gly Cys Cys Gly Asn Pro Ser Cys Ser Ile His Ile Pro Tyr Val Cys
 1               5                  10                  15

Asn


<210> 200
<211> 21
<212> PRT
<213> Conus lividus

<220>
<221> VARIANT
<222> (4)
<223> /replace = gamma-carboxy-Glu

<220>
<221> VARIANT
<222> (5)
<223> /replace = gamma-carboxy-Glu

<400> 200
Thr Asp Ser Glu Glu Cys Cys Leu Asp Ser Arg Cys Ala Gly Gln His
 1               5                  10                  15

Gln Asp Leu Cys Gly
            20


<210> 201
<211> 17
<212> PRT
<213> Conus marmoreus

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (9)
<223> /replace = nor-Tyr

/replace = mono-halo-Tyr
      /replace = di-halo-Tyr
      /replace = O-sulpho-Tyr

<220>
<221> VARIANT
<222> (9)
<223> /replace = O-phospho-Tyr
      /replace = nitro-Tyr

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223>/replace = O-phospho-Tyr
      /replace = nitro-Tyr

<400> 201
Gly Cys Cys Ser Asn Pro Pro Cys Tyr Ala Asn Asn Gln Ala Tyr Cys
1               5                   10                  15

Asn


<210> 202
<211> 16
<212> PRT
<213> Conus marmoreus

<220>
<221> VARIANT
<222> (6)
<223> /replace = hydroxy-Pro

<220>
<221> VARIANT
<222> (13)
<223> /replace = hydroxy-Pro

<400> 202
Gly Cys Cys Ser His Pro Ala Cys Ser Val Asn Asn Pro Asp Ile Cys
1               5                   10                  15


<210> 203
<211> 18
<212> PRT
<213> Conus musicus

<220>
<221> VARIANT
<222> (2)
<223> /replace = N-methyl-Lys

/replace = N,N-dimethyl-Lys
      /replace = N,N,N-trimethyl-Lys

<220>
<221> VARIANT
<222> (12)
<223> /replace = N-methyl-Lys
      /replace = N,N-dimethyl-Lys
      /replace = N,N,N-trimethyl-Lys

<220>
<221> VARIANT
<222> (14)
<223> /replace = hydroxy-Pro

<220>
<221> VARIANT
<222> (16)
<223> /replace = nor-Tyr
      /replace = mono-halo-Tyr
      /replace = di-halo-Tyr
      /replace = O-sulpho-Tyr

<220>
<221> VARIANT
<222> (16)
<223> /replace = O-phospho-Tyr
      /replace = nitro-Tyr

<400> 203
Gly Lys Cys Cys Ile Asn Asp Ala Cys Arg Ser Lys His Pro Gln Tyr
 1               5                   10                  15

Cys Ser

<210> 204
<211> 17
<212> PRT
<213> Conus musicus

<220>
<221> VARIANT
<222> (4)
<223> /replace = nor-Tyr
      /replace = mono-halo-Tyr
      /replace = di-halo-Tyr
      /replace = O-sulpho-Tyr

<220>
<221> VARIANT
<222> (4)
<223> /replace = O-phospho-Tyr
      /replace = nitro-Tyr

<220>
<221> VARIANT
<222> (13)
<223> /replace = hydroxy-Pro.

<220>
<221> VARIANT
<222> (15)
<223> /replace = nor-Tyr
      /replace = mono-halo-Tyr
      /replace = di-halo-Tyr
      /replace = O-sulpho-Tyr
<220>
<221> VARIANT
<222> (15)
<223> /replace = O-phospho-Tyr
      /replace = nitro-Tyr

<400> 204
Gly Cys Cys Tyr Asn Ile Ala Cys Arg Ile Asn Asn Pro Arg Tyr Cys
1               5                   10                  15

Arg


<210> 205
<211> 17
<212> PRT
<213> Conus obscurus

<220>
<221> VARIANT
<222> (6)
<223> /replace = hydroxy-Pro

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<220>
<221> VARIANT
<222> (13)
<223> /replace = hydroxy-Pro

<220>
<221> VARIANT
<222> (14)
<223> /replace = N-methyl-Lys
      /replace = N,N-dimethyl-Lys
      /replace = N,N,N-trimethyl-Lys

<220>
<221> VARIANT
<222> (15)

<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 205
Gly Cys Cys Ser His Pro Val Cys Arg Phe Asn Tyr Pro Lys Tyr Cys
1               5                   10                  15

Gly


<210> 206
<211> 18
<212> PRT
<213> Conus obscurus

<220>
<221> VARIANT
<222> (2)
<223> /replace = gamma-carboxy-Glu

<220>
<221> VARIANT
<222> (7)
<223> /replace = hydroxy-Pro

<220>
<221> VARIANT
<222> (8)
<223> /replace = hydroxy-Pro

<220>
<221> VARIANT
<222> (14)
<223> /replace = hydroxy-Pro

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 206
Asp Glu Cys Cys Ala Ser Pro Pro Cys Arg Leu Asn Asn Pro Tyr Val
1               5                   10                  15

Cys His

<210> 207
<211> 19
<212> PRT
<213> Conus obscurus

<220>
<221> VARIANT
<222> (6)
<223> /replace = hydroxy-Pro

<220>
<221> VARIANT
<222> (9)
<223> /replace = D-Trp
      /replace = halo-Trp

<220>
<221> VARIANT
<222> (14)
<223> /replace = gamma-carboxy-Glu

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<220>
<221> VARIANT
<222> (18)
<223> /replace = gamma-carboxy-Glu

<400> 207
Gly Cys Cys Ser Asn Pro Val Cys Trp Gln Asn Asn Ala Glu Tyr Cys
1               5                   10                  15

Arg Glu Ser

<210> 208
<211> 16
<212> PRT
<213> Conus obscurus

<220>
<221> VARIANT

<222> (6)
<223> /replace = hydroxy-Pro

<220>
<221> VARIANT
<222> (7)
<223> /replace = hydroxy-Pro

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 208
Gly Cys Cys Ser His Pro Pro Cys Ala Gln Asn Asn Gln Asp Tyr Cys
1               5                   10                  15


<210> 209
<211> 19
<212> PRT
<213> Conus obscurus

<220>
<221> VARIANT
<222> (6)
<223> /replace = hydroxy-Pro

<220>
<221> VARIANT
<222> (14)
<223> /replace = gamma-carboxy-Glu

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<220>
<221> VARIANT
<222> (18)
<223> /replace = gamma-carboxy-Glu

<400> 209
Gly Cys Cys Ser His Pro Ala Cys Ser Gly Asn Asn Arg Glu Tyr Cys
1               5                   10                  15

Arg Glu Ser

<210> 210
<211> 18
<212> PRT
<213> Conus omaria

<220>
<221> VARIANT
<222> (2)
<223> /replace = hydroxy-Pro

<220>
<221> VARIANT
<222> (6)
<223> /replace = nor-Tyr
      /replace = mono-halo-Tyr
      /replace = di-halo-Tyr
      /replace = O-sulpho-Tyr

<220>
<221> VARIANT
<222> (6)
<223> /replace = O-phospho-Tyr
      /replace = nitro-Tyr

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 210
Asp Pro Cys Cys Ser Tyr Pro Asp Cys Gly Ala Asn His Pro Glu Ile
1               5                   10                  15

Cys Gly

<210> 211
<211> 17
<212> PRT
<213> Conus omaria

<220>
<221> VARIANT
<222> (1)
<223> /replace = gamma-carboxy-Glu

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (10)
<223> /replace = D-Trp
      /replace = halo=Trp

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (13)
<223> /replace = hydroxy-Pro

<220>
<221> VARIANT
<222> (14)
<223> /replace = gamma-carboxy-Glu

<400> 211
Glu Cys Cys Ser Gln Pro Pro Cys Arg Trp Lys His Pro Glu Leu Cys
 1               5                  10                  15

Ser


<210> 212
<211> 16
<212> PRT
<213> Conus omaria

<220>
<221> VARIANT
<222> (6)
<223> /replace = hydroxy-Pro

<220>
<221> VARIANT
<222> (13)
<223> /replace = hydroxy-Pro

<400> 212
Gly Cys Cys Ser His Pro Ala Cys Ala Gly Asn Asn Pro His Ile Cys
1               5                   10                  15

<210> 213
<211> 16
<212> PRT
<213> Conus omaria

<220>
<221> VARIANT
<222> (6)
<223> /replace = hydroxy-Pro

<220>
<221> VARIANT
<222> (13)
<223> /replace = hydroxy-Pro

<220>
<221> VARIANT
<222> (15)
<223> /replace = nor-Tyr
      /replace = mono-halo-Tyr
      /replace = di-halo-Tyr
      /replace = O-sulpho-Tyr

<220>
<221> VARIANT
<222> (15)
<223> /replace = O-phospho-Tyr
      /replace = nitro-Tyr

<400> 213
Gly Cys Cys Ser Asp Pro Ser Cys Asn Val Asn Asn Pro Asp Tyr Cys
1               5                   10                  15

<210> 214
<211> 18
<212> PRT
<213> Conus omaria

<220>
<221> VARIANT
<222> (1)
<223> /replace = gamma-carboxy-Glu

<220>
<221> VARIANT
<222> (2)
<223> /replace = gamma-carboxy-Glu

<220>
<221> VARIANT
<222> (7)
<223> /replace = hydroxy-Pro

<400> 214
Glu Glu Cys Cys Ser Asp Pro Arg Cys Ser Val Gly His Gln Asp Met
1               5                   10                  15

Cys Arg

<210> 215
<211> 17
<212> PRT
<213> Conus purpurascens

<220>
<221> VARIANT
<222> (7)
<223> /replace = hydroxy-Pro

<220>
<221> VARIANT
<222> (15)
<223> /replace = gamma-carboxy-Glu

<400> 215
Gly Gly Cys Cys Ser Asn Pro Ala Cys Leu Val Asn His Leu Glu Met
1               5                   10                  15

Cys

<210> 216
<211> 18
<212> PRT
<213> Conus purpurascens

<220>
<221> VARIANT
<222> (3)
<223> /replace = hydroxy-Pro

<220>
<221> VARIANT
<222> (8)
<223> /replace = hydroxy-Pro

<220>
<221> VARIANT
<222> (15)
<223> /replace = hydroxy-Pro

<400> 216
Arg Asp Pro Cys Cys Phe Asn Pro Ala Cys Asn Val Asn Asn Pro Gln
1               5                   10                  15

Ile Cys

<210> 217
<211> 21

<212> PRT
<213> Conus purpurascens

<220>
<221> VARIANT
<222> (53)
<223> /replace = hydroxy-Pro

<220>
<221> VARIANT
<222> (8)
<223> /replace = D-Trp
      /replace = halo-Trp

<400> 217
Cys Cys Ser Asp Pro Ser Cys Trp Arg Leu His Ser Leu Ala Cys Thr
 1               5                  10                  15

Gly Ile Val Asn Arg
            20


<210> 218
<211> 16
<212> PRT
<213> Conus purpurascens

<220>
<221> VARIANT
<222> (5)
<223> /replace = hydroxy-Pro

<400> 218
Cys Cys Thr Asn Pro Ala Cys Leu Val Asn Asn Ile Arg Phe Cys Gly
 1               5                  10                  15


<210> 219
<211> 18
<212> PRT
<213> Conus regius

<220>
<221> VARIANT
<222> (2)
<223> /replace = gamma-carboxy-Glu

<220>
<221> VARIANT
<222> (7)
<223> /replace = hydroxy-Pro

<400> 219
Asp Glu Cys Cys Ser Asp Pro Arg Cys His Gly Asn Asn Arg Asp His
 1               5                  10                  15

Cys Ala

<210> 220
<211> 17
<212> PRT
<213> Conus regius

<220>
<221> VARIANT
<222> (6)
<223> /replace = hydroxy-Pro

<220>
<221> VARIANT
<222> (13)
<223> /replace = hydroxy-Pro

<400> 220
Asp Cys Cys Ser His Pro Leu Cys Arg Leu Phe Val Pro Gly Leu Cys
1               5                   10                  15

Ile


<210> 221
<211> 17
<212> PRT
<213> Conus regius

<220>
<221> VARIANT
<222> (6)
<223> /replace = hydroxy-Pro

<220>
<221> VARIANT
<222> (9)
<223> /replace = N-methyl-Lys
      /replace = N,N-dimethyl-Lys
      /replace = N,N,N-trimethyl-Lys

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<220>
<221> VARIANT
<222> (13)
<223> /replace = hydroxy-Pro

<400> 221
Gly Cys Cys Ser His Pro Val Cys Lys Val Arg Tyr Pro Asp Leu Cys

1                   5                   10                  15

Arg


<210> 222
<211> 16
<212> PRT
<213> Conus regius

<220>
<221> VARIANT
<222> (6)
<223> /replace = hydroxy-Pro

<220>
<221> VARIANT
<222> (13)
<223> /replace = hydroxy-Pro

<400> 222
Gly Cys Cys Ser His Pro Ala Cys Asn Val Asn Asn Pro His Ile Cys
1               5                   10                  15


<210> 223
<211> 16
<212> PRT
<213> Conus regius

<220>
<221> VARIANT
<222> (6)
<223> /replace = hydroxy-Pro

<220>
<221> VARIANT
<222> (9)
<223> /replace = N-methyl-Lys
      /replace = N,N-dimethyl-Lys
      /replace = N,N,N-trimethyl-Lys

<220>
<221> VARIANT
<222> (12)
<223> /replace = nor-Tyr
      /replace = mono-halo-Tyr
      /replace = di-halo-Tyr
      /replace = O-sulpho-Tyr

<220>
<221> VARIANT
<222> (12)
<223> /replace = O-phospho-Tyr
      /replace = nitro-Tyr

<400> 223
Gly Cys Cys Ser His Pro Val Cys Lys Val Arg Tyr Ser Asp Met Cys
1               5                   10                  15

<210> 224
<211> 17
<212> PRT
<213> Conus stercusmuscarum

<220>
<221> VARIANT
<222> (7)
<223> /replace = hydroxy-Pro

<220>
<221> VARIANT
<222> (10)
<223> /replace = N-methyl-Lys
      /replace = N,N-dimethyl-Lys
      /replace = N,N,N-trimethyl-Lys

<400> 224
Gly Gly Cys Cys Ser His Pro Ala Cys Lys Val His Phe Pro His Ser
1               5                   10                  15

Cys


<210> 225
<211> 20
<212> PRT
<213> Conus stercusmuscarum

220>
<221> VARIANT
<222> (6)
<223> /replace = hydroxy-Pro

<220>
<221> VARIANT
<222> (13)
<223> /replace = hydroxy-Pro

<220>
<221> VARIANT
<222> (14)
<223> /replace = gamma-carboxy-Glu

<400> 225
Val Cys Cys Ser Asn Pro Val Cys His Val Asp His Pro Glu Leu Cys
1               5                   10                  15

Arg Arg Arg Arg
            20


<210> 226
<211> 17
<212> PRT
<213> Conus striatus

<220>
<221> VARIANT
<222> (6)

<223> /replace = hydroxy-Pro

<220>
<221> VARIANT
<222> (13)
<223> /replace = hydroxy-Pro

<400> 226
Gly Cys Cys Ser His Pro Val Cys Asn Leu Ser Asn Pro Gln Ile Cys
1               5                   10                  15

Arg


<210> 227
<211> 18
<212> PRT
<213> Conus textile

<220>
<221> VARIANT
<222> (1)
<223> /replace = pyro-Glu

<220>
<221> VARIANT
<222> (2)
<223> /replace = gamma-carboxy-Glu

<220>
<221> VARIANT
<222> (7)
<223> /replace = hydroxy-Pro

<220>
<221> VARIANT
<222> (14)
<223> /replace = hydroxy-Pro

<220>
<221> VARIANT
<222> (15)
<223> /replace = gamma-carboxy-Glu

<400> 227
Gln Glu Cys Cys Ser His Pro Ala Cys Asn Val Asp His Pro Glu Ile
1               5                   10                  15

Cys Arg


<210> 228
<211> 17
<212> PRT
<213> Conus tulipa

<220>
<221> VARIANT
<222> (6)

<223> /replace = hydroxy-Pro

<400> 228
Gly Cys Cys Ser Asn Pro Ala Cys Leu Val Asn His Ile Arg Phe Cys
1               5                   10                  15

Gly


<210> 229
<211> 17
<212> PRT
<213> Conus virgo

<220>
<221> VARIANT
<222> (6)
<223> /replace = hydroxy-Pro

<220>
<221> VARIANT
<222> (13)
<223> /replace = hydroxy-Pro

<400> 229
Asp Cys Cys Asp Asp Pro Ala Cys Thr Val Asn Asn Pro Gly Leu Cys
1               5                   10                  15

Thr


<210> 230
<211> 20
<212> PRT
<213> Conus textile

<220>
<221> VARIANT
<222> (6)
<223> /replace = hydroxy-Pro

<220>
<221> VARIANT
<222> (7)
<223> /replace = hydroxy-Pro

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (13)
<223> /replace = hydroxy-Pro

<400> 230
Gly Cys Cys Ser Asn Pro Pro Cys Ile Ala Lys Asn Pro His Met Cys

1                   5                   10                  15

Gly Gly Arg Arg
            20

<210> 231
<211> 18
<212> PRT
<213> Conus geographus

<220>
<221> VARIANT
<222> (5)
<223> /replace = hydroxy-Pro

<220>
<221> VARIANT
<222> (8)
<223> /replace = nor-Tyr
      /replace = mono-halo-Tyr
      /replace = di-halo-Tyr
      /replace = O-sulpho-Tyr

<220>
<221> VARIANT
<222> (8)
<223> /replace = O-phospho-Tyr
      /replace = nitro-Tyr

<220>
<221> VARIANT
<222> (9)
<223> /replace = gamma-carboxy-Glu

<220>
<221> VARIANT
<222> (10)
<223> /replace = N-methyl-Lys
      /replace = N,N-dimethyl-Lys
      /replace = N,N,N-trimethyl-Lys

<220>
<221> VARIANT
<222> (11)
<223> /replace = N-methyl-Lys
      /replace = N,N-dimethyl-Lys
      /replace = N,N,N-trimethyl-Lys

<220>
<221> VARIANT
<222> (12)
<223> /replace = N-methyl-Lys
      /replace = N,N-dimethyl-Lys
      /replace = N,N,N-trimethyl-Lys

<220>
<221> VARIANT
<222> (14)
<223> /replace = N-methyl-Lys

/replace = N,N-dimethyl-Lys
      /replace = N,N,N-trimethyl-Lys

<400> 231
Cys Cys Thr Ile Pro Ser Cys Tyr Glu Lys Lys Lys Ile Lys Ala Cys
 1               5                  10                  15

Val Phe

<210> 232
<211> 18
<212> PRT
<213> Conus regius

<220>
<221> VARIANT
<222> (6)
<223> /replace = hydroxy-Pro

<220>
<221> VARIANT
<222> (13)
<223> /replace = N-methyl-Lys
      /replace = N,N-dimethyl-Lys
      /replace = N,N,N-trimethyl-Lys

<220>
<221> VARIANT
<222> (16)
<223> /replace = hydroxy-Pro

<400> 232
Gly Cys Cys Gly Asn Pro Ala Cys Ser Gly Ser Ser Lys Asp Ala Pro
 1               5                  10                  15

Ser Cys

<210> 233
<211> 108
<212> DNA
<213> Conus imperialis

<220>
<221> CDS
<222> (1)..(105)

<400> 233
tct gat gga aag agt gcc gcg gcc aaa gcc aaa ccg tct cac ctg acg    48
Ser Asp Gly Lys Ser Ala Ala Ala Lys Ala Lys Pro Ser His Leu Thr
 1               5                  10                  15

gct cca ttc atc agg gac gaa tgc tgt tcc gat tct cgc tgt ggc aag    96
Ala Pro Phe Ile Arg Asp Glu Cys Cys Ser Asp Ser Arg Cys Gly Lys
            20                  25                  30

aac tgt ctt tga                                                    108

Asn Cys Leu
        35

<210> 234
<211> 35
<212> PRT
<213> Conus imperialis

<400> 234
Ser Asp Gly Lys Ser Ala Ala Ala Lys Ala Lys Pro Ser His Leu Thr
1               5                   10                  15

Ala Pro Phe Ile Arg Asp Glu Cys Cys Ser Asp Ser Arg Cys Gly Lys
            20                  25                  30

Asn Cys Leu
        35

<210> 235
<211> 108
<212> DNA
<213> Conus imperialis

<220>
<221> CDS
<222> (1)..(105)

<400> 235
ttt gat gga agg aat gcc cca gcc gac gac aaa gcg tct gac ctg atc      48
Phe Asp Gly Arg Asn Ala Pro Ala Asp Asp Lys Ala Ser Asp Leu Ile
1               5                   10                  15

gct caa atc gtc agg aga gca tgc tgt tcc gat cgt cgc tgt aga tgg      96
Ala Gln Ile Val Arg Arg Ala Cys Cys Ser Asp Arg Arg Cys Arg Trp
            20                  25                  30

agg tgt ggt tga                                                      108
Arg Cys Gly
        35

<210> 236
<211> 35
<212> PRT
<213> Conus imperialis

<400> 236
Phe Asp Gly Arg Asn Ala Pro Ala Asp Asp Lys Ala Ser Asp Leu Ile
1               5                   10                  15

Ala Gln Ile Val Arg Arg Ala Cys Cys Ser Asp Arg Arg Cys Arg Trp
            20                  25                  30

Arg Cys Gly
        35

<210> 237

<211> 145
<212> DNA
<213> Conus regius

<220>
<221> CDS
<222> (1)..(105)

<400> 237
tct gat gga agg aat gcc gca gcc gac gcc aga gcg tct ccc cgg atc     48
Ser Asp Gly Arg Asn Ala Ala Ala Asp Ala Arg Ala Ser Pro Arg Ile
1               5                   10                  15

gct ctt ttc ctc agg ttc aca tgc tgt agg aga ggt acc tgt tcc cag     96
Ala Leu Phe Leu Arg Phe Thr Cys Cys Arg Arg Gly Thr Cys Ser Gln
            20                  25                  30

cac tgt ggt tgaagacact gctgctccag gaccctctga accacgacgt             145
His Cys Gly
        35


<210> 238
<211> 35
<212> PRT
<213> Conus regius

<400> 238
Ser Asp Gly Arg Asn Ala Ala Ala Asp Ala Arg Ala Ser Pro Arg Ile
1               5                   10                  15

Ala Leu Phe Leu Arg Phe Thr Cys Cys Arg Arg Gly Thr Cys Ser Gln
            20                  25                  30

His Cys Gly
        35


<210> 239
<211> 145
<212> DNA
<213> Conus regius

<220>
<221> CDS
<222> (1)..(105)

<400> 239
tct aat gga agg aat gcc gca gcc gac gcc aaa gcg tct caa cgg atc     48
Ser Asn Gly Arg Asn Ala Ala Ala Asp Ala Lys Ala Ser Gln Arg Ile
1               5                   10                  15

gct cca ttc ctc agg gac tat tgc tgt agg aga cat gcc tgt acg ttg     96
Ala Pro Phe Leu Arg Asp Tyr Cys Cys Arg Arg His Ala Cys Thr Leu
            20                  25                  30

att tgt ggt tgaagacgct gctgctccag gaccctctga accacgacgt             145
Ile Cys Gly
        35

<210> 240
<211> 35
<212> PRT
<213> Conus regius

<400> 240
Ser Asn Gly Arg Asn Ala Ala Ala Asp Ala Lys Ala Ser Gln Arg Ile
1               5                   10                  15

Ala Pro Phe Leu Arg Asp Tyr Cys Cys Arg Arg His Ala Cys Thr Leu
            20                  25                  30

Ile Cys Gly
        35


<210> 241
<211> 145
<212> DNA
<213> Conus regius

<220>
<221> CDS
<222> (1)..(105)

<400> 241
tct aat gga agg aat gcc gca gcc gac gcc aaa gcg tct caa cgg atc      48
Ser Asn Gly Arg Asn Ala Ala Ala Asp Ala Lys Ala Ser Gln Arg Ile
1               5                   10                  15

gct cca ttc ctc agg gac tat tgc tgt agg aga cct ccc tgt acg ttg      96
Ala Pro Phe Leu Arg Asp Tyr Cys Cys Arg Arg Pro Pro Cys Thr Leu
            20                  25                  30

att tgt ggt tgaagacgct gctgctccag gaccctctga accacgacgt              145
Ile Cys Gly
        35


<210> 242
<211> 35
<212> PRT
<213> Conus regius

<400> 242
Ser Asn Gly Arg Asn Ala Ala Ala Asp Ala Lys Ala Ser Gln Arg Ile
1               5                   10                  15

Ala Pro Phe Leu Arg Asp Tyr Cys Cys Arg Arg Pro Pro Cys Thr Leu
            20                  25                  30

Ile Cys Gly
        35


<210> 243
<211> 136
<212> DNA
<213> Conus regius

<220>
<221> CDS
<222> (1)..(96)

<400> 243
tct aat aaa agg aag aat gcc gca atg ctt gac atg atc gct caa cac     48
Ser Asn Lys Arg Lys Asn Ala Ala Met Leu Asp Met Ile Ala Gln His
1               5                   10                  15

gcc ata agg ggt tgc tgt tcc gat cct cgc tgt aga tat aga tgt cgt     96
Ala Ile Arg Gly Cys Cys Ser Asp Pro Arg Cys Arg Tyr Arg Cys Arg
            20                  25                  30

tgaagacgct gctgctccag gaccctctga accacgacgt                         136


<210> 244
<211> 32
<212> PRT
<213> Conus regius

<400> 244
Ser Asn Lys Arg Lys Asn Ala Ala Met Leu Asp Met Ile Ala Gln His
1               5                   10                  15

Ala Ile Arg Gly Cys Cys Ser Asp Pro Arg Cys Arg Tyr Arg Cys Arg
            20                  25                  30


<210> 245
<211> 145
<212> DNA
<213> Conus regius

<220>
<221> CDS
<222> (1)..(105)

<400> 245
ttt aat gga agg agt gcc gca gcc gac caa aat gcg cct ggc ctg atc     48
Phe Asn Gly Arg Ser Ala Ala Ala Asp Gln Asn Ala Pro Gly Leu Ile
1               5                   10                  15

gct caa gtc gtc aga gga ggg tgc tgt tcc gat ccc cgc tgc gcc tgg     96
Ala Gln Val Val Arg Gly Gly Cys Cys Ser Asp Pro Arg Cys Ala Trp
            20                  25                  30

aga tgt ggt tgaagacgtt gctgctccag gaccctctga accacgacgt             145
Arg Cys Gly
        35


<210> 246
<211> 35
<212> PRT
<213> Conus regius

<400> 246
Phe Asn Gly Arg Ser Ala Ala Ala Asp Gln Asn Ala Pro Gly Leu Ile

1                   5                   10                  15

Ala Gln Val Val Arg Gly Gly Cys Cys Ser Asp Pro Arg Cys Ala Trp
            20                  25                  30

Arg Cys Gly
        35


<210> 247
<211> 145
<212> DNA
<213> Conus regius

<220>
<221> CDS
<222> (1)..(105)

<400> 247
ttt gat gga agg aat gcc gca gcc gac gcc aaa gtg att aac acg gtc      48
Phe Asp Gly Arg Asn Ala Ala Ala Asp Ala Lys Val Ile Asn Thr Val
1               5                   10                  15

gct cga atc gcc tgg gat ata tgc tgt tcc gaa cct gac tgt aac cat      96
Ala Arg Ile Ala Trp Asp Ile Cys Cys Ser Glu Pro Asp Cys Asn His
            20                  25                  30

aaa tgt gtt tgaagacgct tctgctccag gaccctctga accacgacgt              145
Lys Cys Val
        35


<210> 248
<211> 35
<212> PRT
<213> Conus regius

<400> 248
Phe Asp Gly Arg Asn Ala Ala Ala Asp Ala Lys Val Ile Asn Thr Val
1               5                   10                  15

Ala Arg Ile Ala Trp Asp Ile Cys Cys Ser Glu Pro Asp Cys Asn His
            20                  25                  30

Lys Cys Val
        35

<210> 249
<211> 136
<212> DNA
<213> Conus regius

<220>
<221> CDS
<222> (1)..(96)

<400> 249
tct aat aaa agg aag aat gcc gca atg ctt gac atg atc gct caa cac      48
Ser Asn Lys Arg Lys Asn Ala Ala Met Leu Asp Met Ile Ala Gln His
1               5                   10                  15

gcc ata agg ggt tgc tgt tcc gat cct cgc tgt aaa cat cag tgt ggt    96
Ala Ile Arg Gly Cys Cys Ser Asp Pro Arg Cys Lys His Gln Cys Gly
            20                  25                  30

tgaagacgct gctgctccag gaccctctga accacgacgt                        136


<210> 250
<211> 32
<212> PRT
<213> Conus regius

<400> 250
Ser Asn Lys Arg Lys Asn Ala Ala Met Leu Asp Met Ile Ala Gln His
1               5                   10                  15

Ala Ile Arg Gly Cys Cys Ser Asp Pro Arg Cys Lys His Gln Cys Gly
            20                  25                  30


<210> 251
<211> 136
<212> DNA
<213> Conus musicus

<220>
<221> CDS
<222> (1)..(105)

<400> 251
atc aag aat aca gca gcc agc aac aaa gcg tct agc ctg gtg gct ctt    48
Ile Lys Asn Thr Ala Ala Ser Asn Lys Ala Ser Ser Leu Val Ala Leu
1               5                   10                  15

gtt gtc agg gga tgc tgt tac aat cct gtc tgc aag aaa tat tat tgt    96
Val Val Arg Gly Cys Cys Tyr Asn Pro Val Cys Lys Lys Tyr Tyr Cys
            20                  25                  30

tgg aaa ggc tgatgctcca ggaccctctg aaccacgacg t                     136
Trp Lys Gly
        35


<210> 252
<211> 35
<212> PRT
<213> Conus musicus

<400> 252
Ile Lys Asn Thr Ala Ala Ser Asn Lys Ala Ser Ser Leu Val Ala Leu
1               5                   10                  15

Val Val Arg Gly Cys Cys Tyr Asn Pro Val Cys Lys Lys Tyr Tyr Cys
            20                  25                  30

Trp Lys Gly
        35

<210> 253
<211> 148
<212> DNA
<213> Conus purpurascens

<220>
<221> CDS
<222> (1)..(117)

<400> 253
tct gaa ggc agg aat gct gaa gcc atc gac aac gcc tta gac cag agg 48
Ser Glu Gly Arg Asn Ala Glu Ala Ile Asp Asn Ala Leu Asp Gln Arg
1               5                   10                  15

gat cca aag cga cag gag ccg ggg tgc tgt agg cat cct gcc tgt ggg 96
Asp Pro Lys Arg Gln Glu Pro Gly Cys Cys Arg His Pro Ala Cys Gly
            20                  25                  30

aag aac aga tgt gga aga cgc tgatgctcca ggaccctctg aaccacgacg t 148
Lys Asn Arg Cys Gly Arg Arg
        35


<210> 254
<211> 39
<212> PRT
<213> Conus purpurascens

<400> 254
Ser Glu Gly Arg Asn Ala Glu Ala Ile Asp Asn Ala Leu Asp Gln Arg
1               5                   10                  15

Asp Pro Lys Arg Gln Glu Pro Gly Cys Cys Arg His Pro Ala Cys Gly
            20                  25                  30

Lys Asn Arg Cys Gly Arg Arg
        35


<210> 255
<211> 156
<212> DNA
<213> Conus musicus

<220>
<221> CDS
<222> (1)..(102)

<400> 255
tct gat ggc agg aat att gca gtc gac gac aga tgg tct ttc tat acg 48
Ser Asp Gly Arg Asn Ile Ala Val Asp Asp Arg Trp Ser Phe Tyr Thr
1               5                   10                  15

ctc ttc cat gct act tgc tgt gcc gat cct gac tgt aga ttc cgg ccc 96
Leu Phe His Ala Thr Cys Cys Ala Asp Pro Asp Cys Arg Phe Arg Pro
            20                  25                  30
ggt tgt tgatctttgt tcttcaaaga cgctgctggc ccaggaccct ctgaaccacg 152
Gly Cys

acgt 156

<210> 256
<211> 34
<212> PRT
<213> Conus musicus

<400> 256
Ser Asp Gly Arg Asn Ile Ala Val Asp Asp Arg Trp Ser Phe Tyr Thr
1               5                   10                  15

Leu Phe His Ala Thr Cys Cys Ala Asp Pro Asp Cys Arg Phe Arg Pro
            20                  25                  30

Gly Cys

<210> 257
<211> 142
<212> DNA
<213> Conus musicus

<220>
<221> CDS
<222> (1)..(102)

<400> 257
atc aag aat act gca gcc agc aac aaa gcg cct agc ctg gtg gct att     48
Ile Lys Asn Thr Ala Ala Ser Asn Lys Ala Pro Ser Leu Val Ala Ile
1               5                   10                  15

gcc gtc agg gga tgc tgt tac aat cct tcc tgt tgg ccg aaa aca tat     96
Ala Val Arg Gly Cys Cys Tyr Asn Pro Ser Cys Trp Pro Lys Thr Tyr
            20                  25                  30

tgt agt tggaaaggct gatgctccag gaccctctga accacgacgt                 142
Cys Ser

<210> 258
<211> 34
<212> PRT
<213> Conus musicus

<400> 258
Ile Lys Asn Thr Ala Ala Ser Asn Lys Ala Pro Ser Leu Val Ala Ile
1               5                   10                  15

Ala Val Arg Gly Cys Cys Tyr Asn Pro Ser Cys Trp Pro Lys Thr Tyr
            20                  25                  30

Cys Ser

<210> 259
<211> 161
<212> DNA
<213> Conus musicus

<220>
<221> CDS
<222> (1)..(108)

<400> 259
tct gat agc agg aat gtc gca atc gag gac aga gtg tct gac ctg cac     48
Ser Asp Ser Arg Asn Val Ala Ile Glu Asp Arg Val Ser Asp Leu His
1               5                   10                  15

tct atg ttc ttc gat gtt tct tgc tgt agc aat cct acc tgt aaa gaa     96
Ser Met Phe Phe Asp Val Ser Cys Cys Ser Asn Pro Thr Cys Lys Glu
            20                  25                  30

acg tat ggt tgt tgatcgttgg ttttgaagac gctgatgctc caggaccctc        148
Thr Tyr Gly Cys
        35

tgaaccacga cgt                                                     161


<210> 260
<211> 36
<212> PRT
<213> Conus musicus

<400> 260
Ser Asp Ser Arg Asn Val Ala Ile Glu Asp Arg Val Ser Asp Leu His
1               5                   10                  15

Ser Met Phe Phe Asp Val Ser Cys Cys Ser Asn Pro Thr Cys Lys Glu
            20                  25                  30

Thr Tyr Gly Cys
        35


<210> 261
<211> 156
<212> DNA
<213> Conus musicus

<220>
<221> CDS
<222> (1)..(102)

<400> 261
tct gtt ggc agg aat att gca gtc gac gac aga ggg att ttc tct acg     48
Ser Val Gly Arg Asn Ile Ala Val Asp Asp Arg Gly Ile Phe Ser Thr
1               5                   10                  15

ctc ttc cat gct cat tgc tgt gcc aat ccc atc tgt aaa aac acg ccc     96
Leu Phe His Ala His Cys Cys Ala Asn Pro Ile Cys Lys Asn Thr Pro
            20                  25                  30

ggt tgt tgatctttgt tcttcaaaga cgctgctggc ccaggaccct ctgaaccacg     152
Gly Cys

acgt                                                               156

<210> 262
<211> 34
<212> PRT
<213> Conus musicus

<400> 262
Ser Val Gly Arg Asn Ile Ala Val Asp Asp Arg Gly Ile Phe Ser Thr
1               5                   10                  15

Leu Phe His Ala His Cys Cys Ala Asn Pro Ile Cys Lys Asn Thr Pro
            20                  25                  30

Gly Cys



<210> 263
<211> 161
<212> DNA
<213> Conus musicus

<220>
<221> CDS
<222> (1)..(108)

<400> 263
tcc gat ggc agg aat gtc gca atc gac gac aga gtg tct gac ctg cac     48
Ser Asp Gly Arg Asn Val Ala Ile Asp Asp Arg Val Ser Asp Leu His
1               5                   10                  15

tct atg ttc ttc gat att gct tgc tgt aac aat cct acc tgt aaa gaa     96
Ser Met Phe Phe Asp Ile Ala Cys Cys Asn Asn Pro Thr Cys Lys Glu
            20                  25                  30

acg tat ggt tgt tgatcgttgg ttttgaagac gctgatgctc caggaccctc        148
Thr Tyr Gly Cys
        35

tgaaccacga cgt                                                      161


<210> 264
<211> 36
<212> PRT
<213> Conus musicus

<400> 264
Ser Asp Gly Arg Asn Val Ala Ile Asp Asp Arg Val Ser Asp Leu His
1               5                   10                  15

Ser Met Phe Phe Asp Ile Ala Cys Cys Asn Asn Pro Thr Cys Lys Glu
            20                  25                  30

Thr Tyr Gly Cys
        35


<210> 265
<211> 161

<212> DNA
<213> Conus musicus

<220>
<221> CDS
<222> (1)..(108)

<400> 265
tct gat ggc agg aat gtc gca atc gag gac aga gtg tct gac ctg ctc     48
Ser Asp Gly Arg Asn Val Ala Ile Glu Asp Arg Val Ser Asp Leu Leu
1               5                   10                  15
tct atg ctc ttc gat gtt gct tgc tgt agc aat cct gtc tgt aaa gaa     96
Ser Met Leu Phe Asp Val Ala Cys Cys Ser Asn Pro Val Cys Lys Glu
            20                  25                  30

acg tat ggt tgt tgatcgttgg ttttgaagac gctgatgctc caggaccctc     148
Thr Tyr Gly Cys
        35

tgaaccacga cgt                                                  161


<210> 266
<211> 36
<212> PRT
<213> Conus musicus

<400> 266
Ser Asp Gly Arg Asn Val Ala Ile Glu Asp Arg Val Ser Asp Leu Leu
1               5                   10                  15

Ser Met Leu Phe Asp Val Ala Cys Cys Ser Asn Pro Val Cys Lys Glu
            20                  25                  30

Thr Tyr Gly Cys
        35


<210> 267
<211> 154
<212> DNA
<213> Conus betulinus

<220>
<221> CDS
<222> (1)..(123)

<400> 267
tat gat ggc agg aat gct gcc gcc gac gac aaa gct ttt gac ctg ctg     48
Tyr Asp Gly Arg Asn Ala Ala Ala Asp Asp Lys Ala Phe Asp Leu Leu
1               5                   10                  15

gct atg acc ata agg gga gga tgc tgt tcc tat cct ccc tgt atc gcg     96
Ala Met Thr Ile Arg Gly Gly Cys Cys Ser Tyr Pro Pro Cys Ile Ala
            20                  25                  30

agt aat cct aaa tgt ggt gga aga cgc tgatgctcca ggaccctctg     143
Ser Asn Pro Lys Cys Gly Gly Arg Arg
        35                  40

aaccacaacg t                                                          154


<210> 268
<211> 41
<212> PRT
<213> Conus betulinus

<400> 268
Tyr Asp Gly Arg Asn Ala Ala Ala Asp Asp Lys Ala Phe Asp Leu Leu
1               5                   10                  15

Ala Met Thr Ile Arg Gly Gly Cys Cys Ser Tyr Pro Pro Cys Ile Ala
            20                  25                  30

Ser Asn Pro Lys Cys Gly Gly Arg Arg
        35                  40


<210> 269
<211> 151
<212> DNA
<213> Conus lividus

<220>
<221> CDS
<222> (1)..(111)

<400> 269
ttt gat ggc agg aat gct gca ggc aac gcc aaa atg tcc gcc ctg atg    48
Phe Asp Gly Arg Asn Ala Ala Gly Asn Ala Lys Met Ser Ala Leu Met
1               5                   10                  15

gcc ctg acc atc agg gga tgc tgt tcc cat cct gtc tgt agc gcg atg    96
Ala Leu Thr Ile Arg Gly Cys Cys Ser His Pro Val Cys Ser Ala Met
            20                  25                  30

agt cca atc tgt ggc tgaagacgct gatgccccag gaccctctga accacgacgt    151
Ser Pro Ile Cys Gly
        35


<210> 270
<211> 37
<212> PRT
<213> Conus lividus

<400> 270
Phe Asp Gly Arg Asn Ala Ala Gly Asn Ala Lys Met Ser Ala Leu Met
1               5                   10                  15

Ala Leu Thr Ile Arg Gly Cys Cys Ser His Pro Val Cys Ser Ala Met
            20                  25                  30

Ser Pro Ile Cys Gly
        35


<210> 271
<211> 196

<212> DNA
<213> Conus musicus

<220>
<221> CDS
<222> (1)..(165)

<400> 271
atc aag aat gct gca gct gac gac aaa gca tct gac ctg ctc tct cag    48
Ile Lys Asn Ala Ala Ala Asp Asp Lys Ala Ser Asp Leu Leu Ser Gln
1               5                   10                  15

atc gtc agg aat gct gca tcc aat gac aaa ggg tct gac ctg atg act    96
Ile Val Arg Asn Ala Ala Ser Asn Asp Lys Gly Ser Asp Leu Met Thr
            20                  25                  30

ctt gcc ctc agg gga tgc tgt aaa aat cct tac tgt ggt gcg tcg aaa    144
Leu Ala Leu Arg Gly Cys Cys Lys Asn Pro Tyr Cys Gly Ala Ser Lys
        35                  40                  45

aca tat tgt ggt aga aga cgc tgatgctcca ggaccctctg aaccacgacg t    196
Thr Tyr Cys Gly Arg Arg Arg
    50                  55


<210> 272
<211> 55
<212> PRT
<213> Conus musicus

<400> 272
Ile Lys Asn Ala Ala Ala Asp Asp Lys Ala Ser Asp Leu Leu Ser Gln
1               5                   10                  15

Ile Val Arg Asn Ala Ala Ser Asn Asp Lys Gly Ser Asp Leu Met Thr
            20                  25                  30

Leu Ala Leu Arg Gly Cys Cys Lys Asn Pro Tyr Cys Gly Ala Ser Lys
        35                  40                  45

Thr Tyr Cys Gly Arg Arg Arg
    50                  55


<210> 273
<211> 139
<212> DNA
<213> Conus omaria

<220>
<221> CDS
<222> (40)..(108)

<400> 273
tctgatggca ggaatgccgc agcgtctgac ctgatggat ctg acc atc aag gga    54
                                           Leu Thr Ile Lys Gly
                                           1               5

tgc tgt tct tat cct ccc tgt ttc gcg act aat cca gac tgt ggt cga    102
Cys Cys Ser Tyr Pro Pro Cys Phe Ala Thr Asn Pro Asp Cys Gly Arg

10                  15                  20

cga cgc tgatgctcca ggaccctctg aaccacgacg t                    139
Arg Arg


<210> 274
<211> 23
<212> PRT
<213> Conus omaria

<400> 274
Leu Thr Ile Lys Gly Cys Cys Ser Tyr Pro Pro Cys Phe Ala Thr Asn
1               5                   10                  15

Pro Asp Cys Gly Arg Arg Arg
            20


<210> 275
<211> 126
<212> DNA
<213> Conus radiatus

<220>
<221> CDS
<222> (1)..(123)

<400> 275
ttt gat ggc agg aat gcc gca gcc gac tac aaa ggg tct gaa ttg ctc    48
Phe Asp Gly Arg Asn Ala Ala Ala Asp Tyr Lys Gly Ser Glu Leu Leu
1               5                   10                  15

gct atg acc gtc agg gga gga tgc tgt tcc tat cct ccc tgt atc gca    96
Ala Met Thr Val Arg Gly Gly Cys Cys Ser Tyr Pro Pro Cys Ile Ala
            20                  25                  30

aat aat cct ctt tgt gct gga aga cgc tga                            126
Asn Asn Pro Leu Cys Ala Gly Arg Arg
        35                  40


<210> 276
<211> 41
<212> PRT
<213> Conus radiatus

<400> 276
Phe Asp Gly Arg Asn Ala Ala Ala Asp Tyr Lys Gly Ser Glu Leu Leu
1               5                   10                  15

Ala Met Thr Val Arg Gly Gly Cys Cys Ser Tyr Pro Pro Cys Ile Ala
            20                  25                  30

Asn Asn Pro Leu Cys Ala Gly Arg Arg
        35                  40


<210> 277
<211> 126

<212> DNA
<213> Conus radiatus

<220>
<221> CDS
<222> (1)..(123)

<400> 277
ttt gat ggc agg aat gcc gca gcc gac tac aaa ggg tct gaa ttg ctc     48
Phe Asp Gly Arg Asn Ala Ala Ala Asp Tyr Lys Gly Ser Glu Leu Leu
1               5                   10                  15

gct atg acc gtc agg gga gga tgc tgt tcc tat cct ccc tgt atc gca     96
Ala Met Thr Val Arg Gly Gly Cys Cys Ser Tyr Pro Pro Cys Ile Ala
            20                  25                  30

aat aat cct ttt tgt gct gga aga cgc tga                             126
Asn Asn Pro Phe Cys Ala Gly Arg Arg
        35                  40


<210> 278
<211> 41
<212> PRT
<213> Conus radiatus

<400> 278
Phe Asp Gly Arg Asn Ala Ala Ala Asp Tyr Lys Gly Ser Glu Leu Leu
1               5                   10                  15

Ala Met Thr Val Arg Gly Gly Cys Cys Ser Tyr Pro Pro Cys Ile Ala
            20                  25                  30

Asn Asn Pro Phe Cys Ala Gly Arg Arg
        35                  40


<210> 279
<211> 155
<212> DNA
<213> Conus virgo

<220>
<221> CDS
<222> (1)..(114)

<400> 279
tct tat gac agg tat gcc tcg ccc gtc gac aga gcg tct gcc ctg atc     48
Ser Tyr Asp Arg Tyr Ala Ser Pro Val Asp Arg Ala Ser Ala Leu Ile
1               5                   10                  15

gct cag gcc atc ctt cga gat tgc tgt tcc aat cct ccc tgt tcc caa     96
Ala Gln Ala Ile Leu Arg Asp Cys Cys Ser Asn Pro Pro Cys Ser Gln
            20                  25                  30

aat aat cca gac tgt atg taaagacgct gcttgctcca ggaccctctg            144
Asn Asn Pro Asp Cys Met
        35

aaccacgacg t                                                        155

<210> 280
<211> 38
<212> PRT
<213> Conus virgo

<400> 280
Ser Tyr Asp Arg Tyr Ala Ser Pro Val Asp Arg Ala Ser Ala Leu Ile
1               5                   10                  15

Ala Gln Ala Ile Leu Arg Asp Cys Cys Ser Asn Pro Pro Cys Ser Gln
            20                  25                  30

Asn Asn Pro Asp Cys Met
        35


<210> 281
<211> 155
<212> DNA
<213> Conus virgo

<220>
<221> CDS
<222> (1)..(114)

<400> 281
tct tat ggc agg tat gcc tca ccc gtc gac aga gcg tct gcc ctg atc      48
Ser Tyr Gly Arg Tyr Ala Ser Pro Val Asp Arg Ala Ser Ala Leu Ile
1               5                   10                  15

gct cag gcc atc ctt cga gat tgc tgc tcc aat cct cct tgt gcc cat      96
Ala Gln Ala Ile Leu Arg Asp Cys Cys Ser Asn Pro Pro Cys Ala His
            20                  25                  30

aat aat cca gac tgt cgt taaagacgct gcttgctcca ggaccctctg             144
Asn Asn Pro Asp Cys Arg
        35

aaccacgacg t                                                         155


<210> 282
<211> 38
<212> PRT
<213> Conus virgo

<400> 282
Ser Tyr Gly Arg Tyr Ala Ser Pro Val Asp Arg Ala Ser Ala Leu Ile
1               5                   10                  15

Ala Gln Ala Ile Leu Arg Asp Cys Cys Ser Asn Pro Pro Cys Ala His
            20                  25                  30

Asn Asn Pro Asp Cys Arg
        35


<210> 283

<211> 126
<212> DNA
<213> Conus achatinus

<220>
<221> CDS
<222> (1)..(123)

<400> 283
tct gat ggc agg aat gcc gca gcc aac gac aaa gcg tct ggc atg agc     48
Ser Asp Gly Arg Asn Ala Ala Ala Asn Asp Lys Ala Ser Gly Met Ser
1               5                   10                  15

gcg ctg gcc gtc aat gaa tgc tgt acc aac cct gtc tgt cac gcg gaa     96
Ala Leu Ala Val Asn Glu Cys Cys Thr Asn Pro Val Cys His Ala Glu
            20                  25                  30

cat caa gaa ctt tgt gct aga aga cgc tga                             126
His Gln Glu Leu Cys Ala Arg Arg Arg
        35                  40


<210> 284
<211> 41
<212> PRT
<213> Conus achatinus

<400> 284
Ser Asp Gly Arg Asn Ala Ala Ala Asn Asp Lys Ala Ser Gly Met Ser
1               5                   10                  15

Ala Leu Ala Val Asn Glu Cys Cys Thr Asn Pro Val Cys His Ala Glu
            20                  25                  30

His Gln Glu Leu Cys Ala Arg Arg Arg
        35                  40


<210> 285
<211> 126
<212> DNA
<213> Conus achatinus

<220>
<221> CDS
<222> (1)..(123)

<400> 285
tct gat ggc agg aat gcc gca gcc aac gac aaa gcg tct gac gtg atc     48
Ser Asp Gly Arg Asn Ala Ala Ala Asn Asp Lys Ala Ser Asp Val Ile
1               5                   10                  15

acg ctg gcc ctc aag gga tgc tgt tcc aac cct gtc tgt cac ttg gag     96
Thr Leu Ala Leu Lys Gly Cys Cys Ser Asn Pro Val Cys His Leu Glu
            20                  25                  30

cat tca aac ctt tgt ggt aga aga cgc tga                             126
His Ser Asn Leu Cys Gly Arg Arg Arg
        35                  40

<210> 286
<211> 41
<212> PRT
<213> Conus achatinus

<400> 286
Ser Asp Gly Arg Asn Ala Ala Ala Asn Asp Lys Ala Ser Asp Val Ile
1               5                   10                  15

Thr Leu Ala Leu Lys Gly Cys Cys Ser Asn Pro Val Cys His Leu Glu
            20                  25                  30

His Ser Asn Leu Cys Gly Arg Arg Arg
        35                  40


<210> 287
<211> 126
<212> DNA
<213> Conus achatinus

<220>
<221> CDS
<222> (1)..(123)

<400> 287
tct gat ggc agg aat gcc gca gcc aac gac aaa gcg tct ggc atg agc      48
Ser Asp Gly Arg Asn Ala Ala Ala Asn Asp Lys Ala Ser Gly Met Ser
1               5                   10                  15

gcg ctg gcc gtc aat gaa tgc tgt acc aac cct gtc tgt cac gtg gaa      96
Ala Leu Ala Val Asn Glu Cys Cys Thr Asn Pro Val Cys His Val Glu
            20                  25                  30

cat caa gaa ctt tgt gct aga aga cgc tga                              126
His Gln Glu Leu Cys Ala Arg Arg Arg
        35                  40


<210> 288
<211> 41
<212> PRT
<213> Conus achatinus

<400> 288
Ser Asp Gly Arg Asn Ala Ala Ala Asn Asp Lys Ala Ser Gly Met Ser
1               5                   10                  15

Ala Leu Ala Val Asn Glu Cys Cys Thr Asn Pro Val Cys His Val Glu
            20                  25                  30

His Gln Glu Leu Cys Ala Arg Arg Arg
        35                  40


<210> 289
<211> 220
<212> DNA
<213> Conus ammiralis

<220>
<221> CDS
<222> (1)..(180)

<400> 289
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca gat cgt gca ttt cgt ggc agg aat gcc gca gcc aaa gcg     96
Phe Thr Ser Asp Arg Ala Phe Arg Gly Arg Asn Ala Ala Ala Lys Ala
            20                  25                  30

tct ggc ctg gtc ggt ctg acc gac aag agg caa gaa tgc tgt tct tat     144
Ser Gly Leu Val Gly Leu Thr Asp Lys Arg Gln Glu Cys Cys Ser Tyr
        35                  40                  45

cct gcc tgt aac cta gat cat cca gaa ctt tgt ggt tgaagacgct          190
Pro Ala Cys Asn Leu Asp His Pro Glu Leu Cys Gly
    50                  55                  60

gatgctccag gaccctctga accacgacgt                                    220


<210> 290
<211> 60
<212> PRT
<213> Conus ammiralis

<400> 290
Met Phe Thr Val Phe Leu Leu Val Val Leu Ala Thr Thr Val Val Ser
1               5                   10                  15

Phe Thr Ser Asp Arg Ala Phe Arg Gly Arg Asn Ala Ala Ala Lys Ala
            20                  25                  30

Ser Gly Leu Val Gly Leu Thr Asp Lys Arg Gln Glu Cys Cys Ser Tyr
        35                  40                  45

Pro Ala Cys Asn Leu Asp His Pro Glu Leu Cys Gly
    50                  55                  60


<210> 291
<211> 223
<212> DNA
<213> Conus ammiralis

<220>
<221> CDS
<222> (1)..(192)

<400> 291
atg ttc acc gtg ttt ctg ttg gtt gtc ttg gca acc act gtc gtt tcc     48
Met Phe Thr Val Phe Leu Leu Val Val Leu Ala Thr Thr Val Val Ser
1               5                   10                  15

tcc act tca ggt cgt cgt gca ttt cgt ggc agg aat gcc gca gcc aaa     96
Ser Thr Ser Gly Arg Arg Ala Phe Arg Gly Arg Asn Ala Ala Ala Lys

20                  25                  30

gcg tct gga ctg gtc ggt ctg act gac agg aga cca gaa tgc tgt agt     144
Ala Ser Gly Leu Val Gly Leu Thr Asp Arg Arg Pro Glu Cys Cys Ser
        35                  40                  45

gat cct cgc tgt aac tcg act cat cca gaa ctt tgt ggt gga aga cgc     192
Asp Pro Arg Cys Asn Ser Thr His Pro Glu Leu Cys Gly Gly Arg Arg
    50                  55                  60

tgatgctcca ggaccctctg aaccacgacg t                                  223

<210> 292
<211> 64
<212> PRT
<213> Conus ammiralis

<400> 292
Met Phe Thr Val Phe Leu Leu Val Val Leu Ala Thr Thr Val Val Ser
 1               5                  10                  15

Ser Thr Ser Gly Arg Arg Ala Phe Arg Gly Arg Asn Ala Ala Ala Lys
            20                  25                  30

Ala Ser Gly Leu Val Gly Leu Thr Asp Arg Arg Pro Glu Cys Cys Ser
        35                  40                  45

Asp Pro Arg Cys Asn Ser Thr His Pro Glu Leu Cys Gly Gly Arg Arg
    50                  55                  60

<210> 293
<211> 151
<212> DNA
<213> Conus arenatus

<220>
<221> CDS
<222> (1)..(120)

<400> 293
tct gat ggc agg aat gcc gca gcc aac gcg ttt gac ctg atc gat ctg     48
Ser Asp Gly Arg Asn Ala Ala Ala Asn Ala Phe Asp Leu Ile Asp Leu
 1               5                  10                  15

acc gcc agg cta aat tgc tgt atg att ccc ccc tgt tgg aag aaa tat     96
Thr Ala Arg Leu Asn Cys Cys Met Ile Pro Pro Cys Trp Lys Lys Tyr
            20                  25                  30

gga gac aga tgt agt gaa gta cgc tgatgctcca ggaccctctg aaccacgacg   150
Gly Asp Arg Cys Ser Glu Val Arg
        35                  40

t                                                                   151

<210> 294
<211> 40
<212> PRT
<213> Conus arenatus

<400> 294
Ser Asp Gly Arg Asn Ala Ala Ala Asn Ala Phe Asp Leu Ile Asp Leu
1               5                   10                  15

Thr Ala Arg Leu Asn Cys Cys Met Ile Pro Pro Cys Trp Lys Lys Tyr
            20                  25                  30

Gly Asp Arg Cys Ser Glu Val Arg
        35                  40


<210> 295
<211> 126
<212> DNA
<213> Conus arenatus

<220>
<221> CDS
<222> (1)..(93)

<400> 295
tct gat ggc agg aat gcc gca cgc aaa gcg ttt ggc tgc tgc gac tta    48
Ser Asp Gly Arg Asn Ala Ala Arg Lys Ala Phe Gly Cys Cys Asp Leu
1               5                   10                  15

ata ccc tgt ttg gag aga tat ggt aac aga tgt aat gaa gtg cac        93
Ile Pro Cys Leu Glu Arg Tyr Gly Asn Arg Cys Asn Glu Val His
            20                  25                  30

tgatgctcca ggaccctctg aaccacgcga cgt                               126


<210> 296
<211> 31
<212> PRT
<213> Conus arenatus

<400> 296
Ser Asp Gly Arg Asn Ala Ala Arg Lys Ala Phe Gly Cys Cys Asp Leu
1               5                   10                  15

Ile Pro Cys Leu Glu Arg Tyr Gly Asn Arg Cys Asn Glu Val His
            20                  25                  30


<210> 297
<211> 151
<212> DNA
<213> Conus arenatus

<220>
<221> CDS
<222> (1)..(120)

<400> 297
tct gat ggc agc aat gcc gca gcc aac gag ttt gac ctg atc gct ctg    48
Ser Asp Gly Ser Asn Ala Ala Ala Asn Glu Phe Asp Leu Ile Ala Leu
1               5                   10                  15

acc gcc agg cta ggt tgc tgt aac gtt aca ccc tgt tgg gag aaa tat    96
Thr Ala Arg Leu Gly Cys Cys Asn Val Thr Pro Cys Trp Glu Lys Tyr
            20                  25                  30

gga gac aaa tgt aat gaa gta cgc tgatgcttca ggaccctctg aaccacgacg   150
Gly Asp Lys Cys Asn Glu Val Arg
        35                  40

t                                                                  151


<210> 298
<211> 40
<212> PRT
<213> Conus arenatus

<400> 298
Ser Asp Gly Ser Asn Ala Ala Ala Asn Glu Phe Asp Leu Ile Ala Leu
  1               5                  10                  15

Thr Ala Arg Leu Gly Cys Cys Asn Val Thr Pro Cys Trp Glu Lys Tyr
            20                  25                  30

Gly Asp Lys Cys Asn Glu Val Arg
        35                  40


<210> 299
<211> 148
<212> DNA
<213> Conus arenatus

<220>
<221> CDS
<222> (1)..(117)

<400> 299
tct gat ggc agg aat gtc gca gca aaa gcg ttt cac cgg atc ggc cgg    48
Ser Asp Gly Arg Asn Val Ala Ala Lys Ala Phe His Arg Ile Gly Arg
  1               5                  10                  15

acc atc agg gat gaa tgc tgt tcc aat cct gcc tgt agg gtg aat aat    96
Thr Ile Arg Asp Glu Cys Cys Ser Asn Pro Ala Cys Arg Val Asn Asn
            20                  25                  30

cca cac gtt tgt aga cga cgc tgatgctcca ggaccctctg aaccacgacg t    148
Pro His Val Cys Arg Arg Arg
        35


<210> 300
<211> 39
<212> PRT
<213> Conus arenatus

<400> 300
Ser Asp Gly Arg Asn Val Ala Ala Lys Ala Phe His Arg Ile Gly Arg
  1               5                  10                  15

Thr Ile Arg Asp Glu Cys Cys Ser Asn Pro Ala Cys Arg Val Asn Asn

            20                  25                  30

Pro His Val Cys Arg Arg Arg
        35

<210> 301
<211> 151
<212> DNA
<213> Conus arenatus

<220>
<221> CDS
<222> (1)..(120)

<400> 301
tct gat ggc agg aat gcc gca gcc aac gcg ttt gac ctg atg cct ctg 48
Ser Asp Gly Arg Asn Ala Ala Ala Asn Ala Phe Asp Leu Met Pro Leu
1               5                   10                  15

acc gcc agg cta aat tgc tgt agc att ccc ggc tgt tgg aac gaa tat 96
Thr Ala Arg Leu Asn Cys Cys Ser Ile Pro Gly Cys Trp Asn Glu Tyr
            20                  25                  30

aaa gac aga tgt agt aaa gta cgc tgatgctcca ggaccctctg aaccacgacg 150
Lys Asp Arg Cys Ser Lys Val Arg
        35                  40

t 151

<210> 302
<211> 40
<212> PRT
<213> Conus arenatus

<400> 302
Ser Asp Gly Arg Asn Ala Ala Ala Asn Ala Phe Asp Leu Met Pro Leu
1               5                   10                  15

Thr Ala Arg Leu Asn Cys Cys Ser Ile Pro Gly Cys Trp Asn Glu Tyr
            20                  25                  30

Lys Asp Arg Cys Ser Lys Val Arg
        35                  40

<210> 303
<211> 157
<212> DNA
<213> Conus aurisiacus

<220>
<221> CDS
<222> (52)..(126)

<400> 303
tctgatggca ggaatgccgc agccgacgac aaagcgtctg acctggtcgc t ctg gtc 57
Leu Val
1

gtc agg gga gga tgc tgt tcc cac cct gtc tgt tac ttt aat aat cca    105
Val Arg Gly Gly Cys Cys Ser His Pro Val Cys Tyr Phe Asn Asn Pro
        5                   10                  15

caa atg tgt cgt gga aga cgc tgatgctcca ggaccctctg aaccacgacg t    157
Gln Met Cys Arg Gly Arg Arg
    20                  25

<210> 304
<211> 25
<212> PRT
<213> Conus aurisiacus

<400> 304
Leu Val Val Arg Gly Gly Cys Cys Ser His Pro Val Cys Tyr Phe Asn
1               5                   10                  15

Asn Pro Gln Met Cys Arg Gly Arg Arg
            20                  25

<210> 305
<211> 157
<212> DNA
<213> Conus aurisiacus

<220>
<221> CDS
<222> (52)..(126)

<400> 305
tctgatggca ggaatgccgc agccgacgac aaagcgtctg acctggtcgc t ctg gcc    57
                                                         Leu Ala
                                                         1

gtc agg gga gga tgc tgt tcc cac cct gtc tgt aac ttg aat aat cca    105
Val Arg Gly Gly Cys Cys Ser His Pro Val Cys Asn Leu Asn Asn Pro
        5                   10                  15

caa atg tgt cgt gga aga cgc tgatgctcca ggaccctctg aaccacgacg t    157
Gln Met Cys Arg Gly Arg Arg
    20                  25

<210> 306
<211> 25
<212> PRT
<213> Conus aurisiacus

<400> 306
Leu Ala Val Arg Gly Gly Cys Cys Ser His Pro Val Cys Asn Leu Asn
1               5                   10                  15

Asn Pro Gln Met Cys Arg Gly Arg Arg
            20                  25

<210> 307

<211> 157
<212> DNA
<213> Conus betulinus

<220>
<221> CDS
<222> (1)..(117)

<400> 307
ttt cgt ggc agg aat ccc gca gcc aac gac aaa agg tct gac ctg gcc     48
Phe Arg Gly Arg Asn Pro Ala Ala Asn Asp Lys Arg Ser Asp Leu Ala
1               5                   10                  15

gct ctg agc gtc agg gga gga tgc tgt tcc cat cct gcc tgt agc gtg     96
Ala Leu Ser Val Arg Gly Gly Cys Cys Ser His Pro Ala Cys Ser Val
            20                  25                  30
act cat cca gag ctt tgt ggc tgaagacgct gatgccccag gaccctctga       147
Thr His Pro Glu Leu Cys Gly
        35

accacgacgt                                                          157


<210> 308
<211> 39
<212> PRT
<213> Conus betulinus

<400> 308
Phe Arg Gly Arg Asn Pro Ala Ala Asn Asp Lys Arg Ser Asp Leu Ala
1               5                   10                  15

Ala Leu Ser Val Arg Gly Gly Cys Cys Ser His Pro Ala Cys Ser Val
            20                  25                  30

Thr His Pro Glu Leu Cys Gly
        35


<210> 309
<211> 151
<212> DNA
<213> Conus betulinus

<220>
<221> CDS
<222> (1)..(120)

<400> 309
tct gat ggc ggg aat gcc gca gcc aaa gcg tct gac ctg atc gct cag     48
Ser Asp Gly Gly Asn Ala Ala Ala Lys Ala Ser Asp Leu Ile Ala Gln
1               5                   10                  15

acc atc agg gga gga tgc tgt tcc tat cct gcc tgt agc gtg gaa cat     96
Thr Ile Arg Gly Gly Cys Cys Ser Tyr Pro Ala Cys Ser Val Glu His
            20                  25                  30

caa gac ctt tgt gat gga aga cgc tgatgctcca ggaccctctg aaccacgacg   150
Gln Asp Leu Cys Asp Gly Arg Arg
        35                  40

t 151

<210> 310
<211> 40
<212> PRT
<213> Conus betulinus

<400> 310
Ser Asp Gly Gly Asn Ala Ala Ala Lys Ala Ser Asp Leu Ile Ala Gln
1               5                   10                  15

Thr Ile Arg Gly Gly Cys Cys Ser Tyr Pro Ala Cys Ser Val Glu His
            20                  25                  30

Gln Asp Leu Cys Asp Gly Arg Arg
        35                  40

<210> 311
<211> 114
<212> DNA
<213> Conus caracteristicus

<220>
<221> CDS
<222> (1)..(111)

<400> 311
tct tat ggc agg aat gcc gca gcc aaa gcg ttt gaa gtg agt tgc tgt 48
Ser Tyr Gly Arg Asn Ala Ala Ala Lys Ala Phe Glu Val Ser Cys Cys
1               5                   10                  15

gtc gtt cgc ccc tgt tgg att cgc tat caa gag gaa tgt ctt gaa gca 96
Val Val Arg Pro Cys Trp Ile Arg Tyr Gln Glu Glu Cys Leu Glu Ala
            20                  25                  30

gat ccc agg acc ctc tga 114
Asp Pro Arg Thr Leu
        35

<210> 312
<211> 37
<212> PRT
<213> Conus caracteristicus

<400> 312
Ser Tyr Gly Arg Asn Ala Ala Ala Lys Ala Phe Glu Val Ser Cys Cys
1               5                   10                  15

Val Val Arg Pro Cys Trp Ile Arg Tyr Gln Glu Glu Cys Leu Glu Ala
            20                  25                  30

Asp Pro Arg Thr Leu
        35

<210> 313

<211> 123
<212> DNA
<213> Conus caracteristicus

<220>
<221> CDS
<222> (1)..(120)

<400> 313
tct gat ggc agg aat gcc gca gcc aac gcc ctt gac ctg atc act ctg 48
Ser Asp Gly Arg Asn Ala Ala Ala Asn Ala Leu Asp Leu Ile Thr Leu
1               5                   10                  15

atc gcc agg caa aat tgc tgt agc att ccc ggc tgt tgg gag aaa tat 96
Ile Ala Arg Gln Asn Cys Cys Ser Ile Pro Gly Cys Trp Glu Lys Tyr
            20                  25                  30

gga gac aaa tgt agt gaa gta cgc tga 123
Gly Asp Lys Cys Ser Glu Val Arg
        35                  40


<210> 314
<211> 40
<212> PRT
<213> Conus caracteristicus

<400> 314
Ser Asp Gly Arg Asn Ala Ala Ala Asn Ala Leu Asp Leu Ile Thr Leu
1               5                   10                  15

Ile Ala Arg Gln Asn Cys Cys Ser Ile Pro Gly Cys Trp Glu Lys Tyr
            20                  25                  30

Gly Asp Lys Cys Ser Glu Val Arg
        35                  40


<210> 315
<211> 154
<212> DNA
<213> Conus catus

<220>
<221> CDS
<222> (1)..(123)

<400> 315
tct gat ggc agg aat gaa gca gcc aac gac gaa gcg tct gac gtg atc 48
Ser Asp Gly Arg Asn Glu Ala Ala Asn Asp Glu Ala Ser Asp Val Ile
1               5                   10                  15

gag ctg gcc ctc aag gga tgc tgt tcc aac cct gtc tgt cac ttg gag 96
Glu Leu Ala Leu Lys Gly Cys Cys Ser Asn Pro Val Cys His Leu Glu
            20                  25                  30

cat cca aac gct tgt ggt aga aga cgc tgatgctcca ggaccctctg 143
His Pro Asn Ala Cys Gly Arg Arg Arg
        35                  40

aaccacgacg t                                                          154


<210> 316
<211> 41
<212> PRT
<213> Conus catus

<400> 316
Ser Asp Gly Arg Asn Glu Ala Ala Asn Asp Glu Ala Ser Asp Val Ile
1               5                   10                  15

Glu Leu Ala Leu Lys Gly Cys Cys Ser Asn Pro Val Cys His Leu Glu
            20                  25                  30

His Pro Asn Ala Cys Gly Arg Arg Arg
        35                  40


<210> 317
<211> 154
<212> DNA
<213> Conus catus

<220>
<221> CDS
<222> (1)..(123)

<400> 317
tct gat ggc agg aat gcc gca gcc aac gac aaa gcg tct gac ctg gtc      48
Ser Asp Gly Arg Asn Ala Ala Ala Asn Asp Lys Ala Ser Asp Leu Val
1               5                   10                  15
gct ctg gcc gtc agg gga tgc tgt tcc aac cct atc tgt tac ttt aat      96
Ala Leu Ala Val Arg Gly Cys Cys Ser Asn Pro Ile Cys Tyr Phe Asn
            20                  25                  30

aat cca cga att tgt cgt gga aga cgc tgatgctcca ggaccctctg           143
Asn Pro Arg Ile Cys Arg Gly Arg Arg
        35                  40

aaccacgacg t                                                          154


<210> 318
<211> 41
<212> PRT
<213> Conus catus

<400> 318
Ser Asp Gly Arg Asn Ala Ala Ala Asn Asp Lys Ala Ser Asp Leu Val
1               5                   10                  15

Ala Leu Ala Val Arg Gly Cys Cys Ser Asn Pro Ile Cys Tyr Phe Asn
            20                  25                  30

Asn Pro Arg Ile Cys Arg Gly Arg Arg
        35                  40


<210> 319

<211> 111
<212> DNA
<213> Conus episcopatus

<220>
<221> CDS
<222> (1)..(108)

<400> 319
tct cat ggc agg aat gcc gca cgc aaa gcg tct gac ctg atc gct ctg     48
Ser His Gly Arg Asn Ala Ala Arg Lys Ala Ser Asp Leu Ile Ala Leu
1               5                   10                  15

acc gtc agg gaa tgc tgt tct cag cct ccc tgt cgc tgg aaa cat cca     96
Thr Val Arg Glu Cys Cys Ser Gln Pro Pro Cys Arg Trp Lys His Pro
            20                  25                  30

gaa ctt tgt agt tga                                                 111
Glu Leu Cys Ser
        35


<210> 320
<211> 36
<212> PRT
<213> Conus episcopatus

<400> 320
Ser His Gly Arg Asn Ala Ala Arg Lys Ala Ser Asp Leu Ile Ala Leu
1               5                   10                  15

Thr Val Arg Glu Cys Cys Ser Gln Pro Pro Cys Arg Trp Lys His Pro
            20                  25                  30

Glu Leu Cys Ser
        35

<210> 321
<211> 151
<212> DNA
<213> Conus geographus

<220>
<221> CDS
<222> (1)..(120)

<400> 321
tct gat ggc agg aat gac gca gcc aaa gcg ttt gac ctg ata tct tcg     48
Ser Asp Gly Arg Asn Asp Ala Ala Lys Ala Phe Asp Leu Ile Ser Ser
1               5                   10                  15

acc gtc aag aaa gga tgc tgt tcc cat cct gcc tgt gcg ggg aat aat     96
Thr Val Lys Lys Gly Cys Cys Ser His Pro Ala Cys Ala Gly Asn Asn
            20                  25                  30

caa cat att tgt ggc cga aga cgc tgatgctcca ggaccctctg aaccacgacg   150
Gln His Ile Cys Gly Arg Arg Arg
        35                  40

t                                                                   151

<210> 322
<211> 40
<212> PRT
<213> Conus geographus

<400> 322
Ser Asp Gly Arg Asn Asp Ala Ala Lys Ala Phe Asp Leu Ile Ser Ser
1               5                   10                  15

Thr Val Lys Lys Gly Cys Cys Ser His Pro Ala Cys Ala Gly Asn Asn
            20                  25                  30

Gln His Ile Cys Gly Arg Arg Arg
        35                  40


<210> 323
<211> 154
<212> DNA
<213> Conus geographus

<220>
<221> CDS
<222> (1)..(123)

<400> 323
tct gat ggc agg aat gcc gca gcc aac gac caa gcg tct gac ctg atg      48
Ser Asp Gly Arg Asn Ala Ala Ala Asn Asp Gln Ala Ser Asp Leu Met
1               5                   10                  15

gct gcg acc gtc agg gga tgc tgt gcc gtt cct tcc tgt cgc ctc cgt      96
Ala Ala Thr Val Arg Gly Cys Cys Ala Val Pro Ser Cys Arg Leu Arg
            20                  25                  30

aat cca gac ctt tgt ggt gga gga cgc tgatgctcca ggaccctctg           143
Asn Pro Asp Leu Cys Gly Gly Gly Arg
        35                  40

aaccacgacg t                                                         154

<210> 324
<211> 41
<212> PRT
<213> Conus geographus

<400> 324
Ser Asp Gly Arg Asn Ala Ala Ala Asn Asp Gln Ala Ser Asp Leu Met
1               5                   10                  15

Ala Ala Thr Val Arg Gly Cys Cys Ala Val Pro Ser Cys Arg Leu Arg
            20                  25                  30

Asn Pro Asp Leu Cys Gly Gly Gly Arg
        35                  40


<210> 325
<211> 120

<212> DNA
<213> Conus imperialis

<220>
<221> CDS
<222> (1)..(117)

<400> 325
ctt gat gaa agg aat gcc gca gcc gac gac aaa gcg tct gac ctg atc 48
Leu Asp Glu Arg Asn Ala Ala Ala Asp Asp Lys Ala Ser Asp Leu Ile
1               5                   10                  15

gct caa atc gtc agg aga gga tgc tgt tcc cat cct gcc tgt aac gtg 96
Ala Gln Ile Val Arg Arg Gly Cys Cys Ser His Pro Ala Cys Asn Val
            20                  25                  30

aat aat cca cac att tgt ggt tga 120
Asn Asn Pro His Ile Cys Gly
        35


<210> 326
<211> 39
<212> PRT
<213> Conus imperialis

<400> 326
Leu Asp Glu Arg Asn Ala Ala Ala Asp Asp Lys Ala Ser Asp Leu Ile
1               5                   10                  15

Ala Gln Ile Val Arg Arg Gly Cys Cys Ser His Pro Ala Cys Asn Val
            20                  25                  30

Asn Asn Pro His Ile Cys Gly
        35


<210> 327
<211> 142
<212> DNA
<213> Conus lividus

<220>
<221> CDS
<222> (1)..(111)

<400> 327
tct gat ggc agg aat act gca gcc aaa gtc aaa tat tct aag acg ccg 48
Ser Asp Gly Arg Asn Thr Ala Ala Lys Val Lys Tyr Ser Lys Thr Pro
1               5                   10                  15

gag gaa tgc tgt ccc aat cct ccc tgt ttc gcg aca aat tcg gat att 96
Glu Glu Cys Cys Pro Asn Pro Pro Cys Phe Ala Thr Asn Ser Asp Ile
            20                  25                  30

tgt ggc gga aga cgc tgatgctcca ggaccctctg aaccacgacg t 142
Cys Gly Gly Arg Arg
        35

<210> 328
<211> 37
<212> PRT
<213> Conus lividus

<400> 328
Ser Asp Gly Arg Asn Thr Ala Ala Lys Val Lys Tyr Ser Lys Thr Pro
1               5                   10                  15

Glu Glu Cys Cys Pro Asn Pro Pro Cys Phe Ala Thr Asn Ser Asp Ile
            20                  25                  30

Cys Gly Gly Arg Arg
        35


<210> 329
<211> 157
<212> DNA
<213> Conus lividus

<220>
<221> CDS
<222> (1)..(117)

<400> 329
tct aat ggc agg aat gcc gca gcc aaa ttc aaa gcg cct gcc ctg atg    48
Ser Asn Gly Arg Asn Ala Ala Ala Lys Phe Lys Ala Pro Ala Leu Met
1               5                   10                  15

aag cgg acc gtc agg gat gct tgc tgt tca gac cct cgc tgt tcc ggg    96
Lys Arg Thr Val Arg Asp Ala Cys Cys Ser Asp Pro Arg Cys Ser Gly
            20                  25                  30

aaa cat caa gac ctg tgt ggc tgaagacgct gatgctccag gaccctctga      147
Lys His Gln Asp Leu Cys Gly
        35

accacgacgt                                                         157


<210> 330
<211> 39
<212> PRT
<213> Conus lividus

<400> 330
Ser Asn Gly Arg Asn Ala Ala Ala Lys Phe Lys Ala Pro Ala Leu Met
1               5                   10                  15

Lys Arg Thr Val Arg Asp Ala Cys Cys Ser Asp Pro Arg Cys Ser Gly
            20                  25                  30
Lys His Gln Asp Leu Cys Gly
        35


<210> 331
<211> 157
<212> DNA
<213> Conus lividus

<220>
<221> CDS
<222> (1)..(117)

<400> 331
tct aat ggc agg aat gcc gca gcc aaa ttc aaa gcg cct gcc ctg atg     48
Ser Asn Gly Arg Asn Ala Ala Ala Lys Phe Lys Ala Pro Ala Leu Met
1               5                   10                  15

gag ctg acc gtc agg gaa gat tgc tgt tca gac cct cgc tgt tcc gtg     96
Glu Leu Thr Val Arg Glu Asp Cys Cys Ser Asp Pro Arg Cys Ser Val
            20                  25                  30

gga cat caa gac ctg tgt ggc tgaagacgct gatgctccag gaccctctga       147
Gly His Gln Asp Leu Cys Gly
        35

accacgacgt                                                          157


<210> 332
<211> 39
<212> PRT
<213> Conus lividus

<400> 332
Ser Asn Gly Arg Asn Ala Ala Ala Lys Phe Lys Ala Pro Ala Leu Met
1               5                   10                  15

Glu Leu Thr Val Arg Glu Asp Cys Cys Ser Asp Pro Arg Cys Ser Val
            20                  25                  30

Gly His Gln Asp Leu Cys Gly
        35


<210> 333
<211> 157
<212> DNA
<213> Conus lividus

<220>
<221> CDS
<222> (1)..(126)

<400> 333
gca ttt gat ggc agg aat gct gca gcc agc gac aaa gcg tcc gag ctg     48
Ala Phe Asp Gly Arg Asn Ala Ala Ala Ser Asp Lys Ala Ser Glu Leu
1               5                   10                  15

atg gct ctg gcc gtc agg gga tgc tgt tcc cat cct gcc tgt gct ggg     96
Met Ala Leu Ala Val Arg Gly Cys Cys Ser His Pro Ala Cys Ala Gly
            20                  25                  30

agt aat gca cat atc tgt ggc aga aga cgc tgatgctcca ggaccctctg      146
Ser Asn Ala His Ile Cys Gly Arg Arg Arg
        35                  40

aaccacgacg t                                                        157

<210> 334
<211> 42
<212> PRT
<213> Conus lividus

<400> 334
Ala Phe Asp Gly Arg Asn Ala Ala Ala Ser Asp Lys Ala Ser Glu Leu
1               5                   10                  15

Met Ala Leu Ala Val Arg Gly Cys Cys Ser His Pro Ala Cys Ala Gly
            20                  25                  30

Ser Asn Ala His Ile Cys Gly Arg Arg Arg
        35                  40


<210> 335
<211> 157
<212> DNA
<213> Conus lividus

<220>
<221> CDS
<222> (1)..(117)

<400> 335
tct aat ggc agg aat gcc gca gcc aaa ttc aaa gcg cct gcc ctg atg      48
Ser Asn Gly Arg Asn Ala Ala Ala Lys Phe Lys Ala Pro Ala Leu Met
1               5                   10                  15

aag ctg acc gtc agg gag gat tgc tgt tca gac cct cgc tgt tcc gtg      96
Lys Leu Thr Val Arg Glu Asp Cys Cys Ser Asp Pro Arg Cys Ser Val
            20                  25                  30

gga cat caa gac atg tgt ggc tgaagacgct gatgctccag gaccctctga      147
Gly His Gln Asp Met Cys Gly
        35

atcacgacgt                                                            157


<210> 336
<211> 39
<212> PRT
<213> Conus lividus

<400> 336
Ser Asn Gly Arg Asn Ala Ala Ala Lys Phe Lys Ala Pro Ala Leu Met
1               5                   10                  15

Lys Leu Thr Val Arg Glu Asp Cys Cys Ser Asp Pro Arg Cys Ser Val
            20                  25                  30

Gly His Gln Asp Met Cys Gly
        35


<210> 337

<211> 154
<212> DNA
<213> Conus lividus

<220>
<221> CDS
<222> (1)..(114)

<400> 337
ttt gaa tgc agg aat gct gca ggc aac gac aaa gcg act gac ctg atg     48
Phe Glu Cys Arg Asn Ala Ala Gly Asn Asp Lys Ala Thr Asp Leu Met
1               5                   10                  15

gct ctg act gtc agg gga tgc tgt tcc cat cct gcc tgt gct ggg aat     96
Ala Leu Thr Val Arg Gly Cys Cys Ser His Pro Ala Cys Ala Gly Asn
            20                  25                  30

aat cca cat atc tgc ggc tgaagacgct gatgctccag gaccctctga           144
Asn Pro His Ile Cys Gly
        35

accacgacgt                                                         154


<210> 338
<211> 38
<212> PRT
<213> Conus lividus

<400> 338
Phe Glu Cys Arg Asn Ala Ala Gly Asn Asp Lys Ala Thr Asp Leu Met
1               5                   10                  15

Ala Leu Thr Val Arg Gly Cys Cys Ser His Pro Ala Cys Ala Gly Asn
            20                  25                  30

Asn Pro His Ile Cys Gly
        35


<210> 339
<211> 154
<212> DNA
<213> Conus lividus

<220>
<221> CDS
<222> (1)..(114)

<400> 339
ttt gat ggc agg aac gcc gca gcc aac aac aaa gcg act gat ctg atg     48
Phe Asp Gly Arg Asn Ala Ala Ala Asn Asn Lys Ala Thr Asp Leu Met
1               5                   10                  15

gct ctg act gtc aga gga tgc tgt ggc aat cct tca tgt agc atc cat     96
Ala Leu Thr Val Arg Gly Cys Cys Gly Asn Pro Ser Cys Ser Ile His
            20                  25                  30

att cct tac gtt tgt aat tagagacact gatgctccag gaccctctga           144
Ile Pro Tyr Val Cys Asn

        35

accacgacgt                                                          154


<210> 340
<211> 38
<212> PRT
<213> Conus lividus

<400> 340
Phe Asp Gly Arg Asn Ala Ala Ala Asn Asn Lys Ala Thr Asp Leu Met
1               5                   10                  15

Ala Leu Thr Val Arg Gly Cys Cys Gly Asn Pro Ser Cys Ser Ile His
            20                  25                  30

Ile Pro Tyr Val Cys Asn
        35


<210> 341
<211> 157
<212> DNA
<213> Conus lividus

<220>
<221> CDS
<222> (1)..(126)

<400> 341
tct aat ggc agg aat gcc gca gcc aaa ttc aaa gcg cct gcc ctg atg    48
Ser Asn Gly Arg Asn Ala Ala Ala Lys Phe Lys Ala Pro Ala Leu Met
1               5                   10                  15

aag cgg acc gac agc gaa gaa tgc tgt tta gac tct cgc tgt gcc ggg    96
Lys Arg Thr Asp Ser Glu Glu Cys Cys Leu Asp Ser Arg Cys Ala Gly
            20                  25                  30

caa cat caa gac ctg tgt ggc gga aga cgc tgatgctcca ggaccctctg      146
Gln His Gln Asp Leu Cys Gly Gly Arg Arg
        35                  40

aaccacgacg t                                                       157


<210> 342
<211> 42
<212> PRT
<213> Conus lividus

<400> 342
Ser Asn Gly Arg Asn Ala Ala Ala Lys Phe Lys Ala Pro Ala Leu Met
1               5                   10                  15

Lys Arg Thr Asp Ser Glu Glu Cys Cys Leu Asp Ser Arg Cys Ala Gly
            20                  25                  30

Gln His Gln Asp Leu Cys Gly Gly Arg Arg
        35                  40

<210> 343
<211> 126
<212> DNA
<213> Conus marmoreus

<220>
<221> CDS
<222> (1)..(123)

<400> 343
tct gat ggc agg aat gcc gca gcc aag gac aaa gcg tct gac ctg gtc     48
Ser Asp Gly Arg Asn Ala Ala Ala Lys Asp Lys Ala Ser Asp Leu Val
1               5                   10                  15

gct ctg acc gtc aag gga tgc tgt tct aat cct ccc tgt tac gcg aat     96
Ala Leu Thr Val Lys Gly Cys Cys Ser Asn Pro Pro Cys Tyr Ala Asn
            20                  25                  30

aat caa gcc tat tgt aat gga aga cgc tga                             126
Asn Gln Ala Tyr Cys Asn Gly Arg Arg
        35                  40


<210> 344
<211> 41
<212> PRT
<213> Conus marmoreus

<400> 344
Ser Asp Gly Arg Asn Ala Ala Ala Lys Asp Lys Ala Ser Asp Leu Val
1               5                   10                  15

Ala Leu Thr Val Lys Gly Cys Cys Ser Asn Pro Pro Cys Tyr Ala Asn
            20                  25                  30

Asn Gln Ala Tyr Cys Asn Gly Arg Arg
        35                  40


<210> 345
<211> 117
<212> DNA
<213> Conus marmoreus

<220>
<221> CDS
<222> (1)..(114)

<400> 345
tct gat ggc agg aat gcc gca gcc aag gac aaa gcg tct gac ctg gtc     48
Ser Asp Gly Arg Asn Ala Ala Ala Lys Asp Lys Ala Ser Asp Leu Val
1               5                   10                  15

gct ctg acc gtc aag gga tgc tgt tct cat cct gcc tgt agc gtg aat     96
Ala Leu Thr Val Lys Gly Cys Cys Ser His Pro Ala Cys Ser Val Asn
            20                  25                  30

aat cca gac att tgt ggt tga                                         117

Asn Pro Asp Ile Cys Gly
        35

<210> 346
<211> 38
<212> PRT
<213> Conus marmoreus

<400> 346
Ser Asp Gly Arg Asn Ala Ala Ala Lys Asp Lys Ala Ser Asp Leu Val
 1               5                   10                  15

Ala Leu Thr Val Lys Gly Cys Cys Ser His Pro Ala Cys Ser Val Asn
            20                  25                  30

Asn Pro Asp Ile Cys Gly
        35

<210> 347
<211> 145
<212> DNA
<213> Conus musicus

<220>
<221> CDS
<222> (1)..(114)

<400> 347
tct gat ggc agg aat gct gca gcc aac aac aaa gtg gct ttg acc atg    48
Ser Asp Gly Arg Asn Ala Ala Ala Asn Asn Lys Val Ala Leu Thr Met
 1               5                   10                  15

agg gga aaa tgc tgt atc aat gat gcg tgt cgc tcg aaa cat cca cag    96
Arg Gly Lys Cys Cys Ile Asn Asp Ala Cys Arg Ser Lys His Pro Gln
            20                  25                  30

tac tgt tct gga aga cgc tgatactcca ggaccctctg aaccacgacg t         145
Tyr Cys Ser Gly Arg Arg
        35

<210> 348
<211> 38
<212> PRT
<213> Conus musicus

<400> 348
Ser Asp Gly Arg Asn Ala Ala Ala Asn Asn Lys Val Ala Leu Thr Met
 1               5                   10                  15

Arg Gly Lys Cys Cys Ile Asn Asp Ala Cys Arg Ser Lys His Pro Gln
            20                  25                  30

Tyr Cys Ser Gly Arg Arg
        35

<210> 349

<211> 154
<212> DNA
<213> Conus musicus

<220>
<221> CDS
<222> (1)..(123)

<400> 349
tct gat ggc agg aat gct gca gcc aac gac aaa gtg tct gac cag atg     48
Ser Asp Gly Arg Asn Ala Ala Ala Asn Asp Lys Val Ser Asp Gln Met
1               5                   10                  15

gct ctg gtt gtc agg gga tgc tgt tac aat att gcc tgt aga att aat     96
Ala Leu Val Val Arg Gly Cys Cys Tyr Asn Ile Ala Cys Arg Ile Asn
            20                  25                  30

aat cca cgg tac tgt cgt gga aaa cgc tgatgttcca ggaccctctg          143
Asn Pro Arg Tyr Cys Arg Gly Lys Arg
        35                  40

aaccacgacg t                                                        154

<210> 350
<211> 41
<212> PRT
<213> Conus musicus

<400> 350
Ser Asp Gly Arg Asn Ala Ala Ala Asn Asp Lys Val Ser Asp Gln Met
1               5                   10                  15

Ala Leu Val Val Arg Gly Cys Cys Tyr Asn Ile Ala Cys Arg Ile Asn
            20                  25                  30

Asn Pro Arg Tyr Cys Arg Gly Lys Arg
        35                  40


<210> 351
<211> 154
<212> DNA
<213> Conus obscurus

<220>
<221> CDS
<222> (52)..(123)

<400> 351
tctgaaggca ggaatgccgc agccaacgac aaagcgtctg acctgatggc t ctg aac    57
                                                         Leu Asn
                                                         1

gtc agg gga tgc tgt tcc cat cct gtc tgt cgc ttc aat tat cca aaa    105
Val Arg Gly Cys Cys Ser His Pro Val Cys Arg Phe Asn Tyr Pro Lys
        5                   10                  15

tat tgt ggt gga aga cgc tgatggtcca ggaccctctg aaccacgacg t         154
Tyr Cys Gly Gly Arg Arg
    20

<210> 352
<211> 24
<212> PRT
<213> Conus obscurus

<400> 352
Leu Asn Val Arg Gly Cys Cys Ser His Pro Val Cys Arg Phe Asn Tyr
1               5                   10                  15

Pro Lys Tyr Cys Gly Gly Arg Arg
            20


<210> 353
<211> 151
<212> DNA
<213> Conus obscurus

<220>
<221> CDS
<222> (46)..(111)

<400> 353
tctgatggcg ggaatgccgc agcaaaagcg tttgatctaa tcact ctg gcc ctc agg 57
                                                  Leu Ala Leu Arg
                                                  1

gat gaa tgc tgt gcc agt cct ccc tgt cgt ttg aat aat cca tac gta   105
Asp Glu Cys Cys Ala Ser Pro Pro Cys Arg Leu Asn Asn Pro Tyr Val
5                   10                  15                  20

tgt cat tgacgacgct gatgctccag gaccctctga accacgacgt               151
Cys His


<210> 354
<211> 22
<212> PRT
<213> Conus obscurus

<400> 354
Leu Ala Leu Arg Asp Glu Cys Cys Ala Ser Pro Pro Cys Arg Leu Asn
1               5                   10                  15

Asn Pro Tyr Val Cys His
            20


<210> 355
<211> 217
<212> DNA
<213> Conus obscurus

<220>
<221> CDS
<222> (1)..(186)

<400> 355

atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ccc act tca gat cgt gca tct gat agg agg aat gcc gca gcc aaa gcg     96
Pro Thr Ser Asp Arg Ala Ser Asp Arg Arg Asn Ala Ala Ala Lys Ala
            20                  25                  30

ttt gac ctg aga tat tcg acc gcc aag aga gga tgc tgt tcc aat cct     144
Phe Asp Leu Arg Tyr Ser Thr Ala Lys Arg Gly Cys Cys Ser Asn Pro
        35                  40                  45

gtc tgt tgg cag aat aat gca gaa tac tgt cgt gaa agt ggc             186
Val Cys Trp Gln Asn Asn Ala Glu Tyr Cys Arg Glu Ser Gly
    50                  55                  60

taatgctcca ggaccctctg aaccacgacg t                                  217


<210> 356
<211> 62
<212> PRT
<213> Conus obscurus

<400> 356
Met Phe Thr Val Phe Leu Leu Val Val Leu Ala Thr Thr Val Val Ser
1               5                   10                  15

Pro Thr Ser Asp Arg Ala Ser Asp Arg Arg Asn Ala Ala Ala Lys Ala
            20                  25                  30

Phe Asp Leu Arg Tyr Ser Thr Ala Lys Arg Gly Cys Cys Ser Asn Pro
        35                  40                  45

Val Cys Trp Gln Asn Asn Ala Glu Tyr Cys Arg Glu Ser Gly
    50                  55                  60


<210> 357
<211> 208
<212> DNA
<213> Conus obscurus

<220>
<221> CDS
<222> (1)..(168)

<400> 357
atg ttc acc gtg ttt ctg ttg gtt gtc ttg gca acc acc gtc gtt tcc     48
Met Phe Thr Val Phe Leu Leu Val Val Leu Ala Thr Thr Val Val Ser
1               5                   10                  15

ttc act tca gat cgt gca tct gat ggc ggg aat gtc gca gcg tct cac     96
Phe Thr Ser Asp Arg Ala Ser Asp Gly Gly Asn Val Ala Ala Ser His
            20                  25                  30

ctg atc gct ctg acc atc aag gga tgc tgt tct cac cct ccc tgt gcc     144
Leu Ile Ala Leu Thr Ile Lys Gly Cys Cys Ser His Pro Pro Cys Ala
        35                  40                  45

cag aat aat caa gac tat tgt ggt tgacgacgct gatgctccag gaccctctga 198
Gln Asn Asn Gln Asp Tyr Cys Gly
    50                  55

accacgacgt 208

<210> 358
<211> 56
<212> PRT
<213> Conus obscurus

<400> 358
Met Phe Thr Val Phe Leu Leu Val Val Leu Ala Thr Thr Val Val Ser
1               5                   10                  15

Phe Thr Ser Asp Arg Ala Ser Asp Gly Gly Asn Val Ala Ala Ser His
            20                  25                  30

Leu Ile Ala Leu Thr Ile Lys Gly Cys Cys Ser His Pro Pro Cys Ala
        35                  40                  45

Gln Asn Asn Gln Asp Tyr Cys Gly
    50                  55

<210> 359
<211> 217
<212> DNA
<213> Conus obscurus

<220>
<221> CDS
<222> (1)..(186)

<400> 359
atg ttc acc gtg ttt ctg ttg gtt gtc tta tca acc acc gtc gtt tcc 48
Met Phe Thr Val Phe Leu Leu Val Val Leu Ser Thr Thr Val Val Ser
1               5                   10                  15

tcc act tca gat cgt gca tct gat agg agg aat gcc gca gcc aaa gcg 96
Ser Thr Ser Asp Arg Ala Ser Asp Arg Arg Asn Ala Ala Ala Lys Ala
            20                  25                  30

tct gac ctg atg tat tcg acc gtc aag aaa gga tgt tgt tcc cat cct 144
Ser Asp Leu Met Tyr Ser Thr Val Lys Lys Gly Cys Cys Ser His Pro
        35                  40                  45

gcc tgt tcg ggg aat aat cga gaa tat tgt cgt gaa agt ggc 186
Ala Cys Ser Gly Asn Asn Arg Glu Tyr Cys Arg Glu Ser Gly
    50                  55                  60

taatgctcca ggaccctctg aaccacgacg t 217

<210> 360
<211> 62
<212> PRT
<213> Conus obscurus

<400> 360
Met Phe Thr Val Phe Leu Leu Val Val Leu Ser Thr Thr Val Val Ser
1               5                   10                  15

Ser Thr Ser Asp Arg Ala Ser Asp Arg Arg Asn Ala Ala Ala Lys Ala
            20                  25                  30

Ser Asp Leu Met Tyr Ser Thr Val Lys Lys Gly Cys Cys Ser His Pro
        35                  40                  45

Ala Cys Ser Gly Asn Asn Arg Glu Tyr Cys Arg Glu Ser Gly
    50                  55                  60


<210> 361
<211> 157
<212> DNA
<213> Conus omaria

<220>
<221> CDS
<222> (52)..(126)

<400> 361
tttgatggca ggaatgcctc agccgacagc aaagtggctg cccggatcgc t cag atc   57
                                                         Gln Ile
                                                         1

gac agg gat cca tgc tgt tcc tat cct gac tgt ggc gcg aat cat cca   105
Asp Arg Asp Pro Cys Cys Ser Tyr Pro Asp Cys Gly Ala Asn His Pro
        5                   10                  15

gag att tgt ggt gga aaa cgc tgatgctcca ggaccctctg aaccacgacg t    157
Glu Ile Cys Gly Gly Lys Arg
    20                  25


<210> 362
<211> 25
<212> PRT
<213> Conus omaria

<400> 362
Gln Ile Asp Arg Asp Pro Cys Cys Ser Tyr Pro Asp Cys Gly Ala Asn
1               5                   10                  15

His Pro Glu Ile Cys Gly Gly Lys Arg
            20                  25


<210> 363
<211> 128
<212> DNA
<213> Conus omaria

<220>
<221> CDS
<222> (26)..(88)

<400> 363

tctcatggca ggaatgccgc acgct ctg acc gtc agg gaa tgc tgt tct cag    52
                              Leu Thr Val Arg Glu Cys Cys Ser Gln
                               1               5

cct cct tgt cgc tgg aaa cat cca gaa ctt tgt agt tgaagacgct         98
Pro Pro Cys Arg Trp Lys His Pro Glu Leu Cys Ser
 10                  15                  20

gatgctccag gaccctctga accacgacgt                                   128

<210> 364
<211> 21
<212> PRT
<213> Conus omaria

<400> 364
Leu Thr Val Arg Glu Cys Cys Ser Gln Pro Pro Cys Arg Trp Lys His
 1               5                  10                  15

Pro Glu Leu Cys Ser
            20

<210> 365
<211> 154
<212> DNA
<213> Conus omaria

<220>
<221> CDS
<222> (52)..(123)

<400> 365
tttgatggca ggaatgctgc agccagcgac aaagcgtctg agctgatggc t ctg gcc   57
                                                         Leu Ala
                                                          1

gtc agg gga tgc tgt tcc cat cct gcc tgt gct ggg aat aat cca cat    105
Val Arg Gly Cys Cys Ser His Pro Ala Cys Ala Gly Asn Asn Pro His
        5                  10                  15

atc tgt ggc aga aga cgc tgatgctcca ggaccctctg aaccacgacg t         154
Ile Cys Gly Arg Arg Arg
    20

<210> 366
<211> 24
<212> PRT
<213> Conus omaria

<400> 366
Leu Ala Val Arg Gly Cys Cys Ser His Pro Ala Cys Ala Gly Asn Asn
 1               5                  10                  15

Pro His Ile Cys Gly Arg Arg Arg
            20

<210> 367
<211> 142
<212> DNA
<213> Conus omaria

<220>
<221> CDS
<222> (40)..(102)

<400> 367
tctggtgtca ggaaagacgc agcgcctggc ctgatcgct ctg acc atc aag gga 54
Leu Thr Ile Lys Gly
1               5

tgc tgt tct gat cct agc tgt aac gtg aat aat cca gac tat tgt ggt 102
Cys Cys Ser Asp Pro Ser Cys Asn Val Asn Asn Pro Asp Tyr Cys Gly
                10                  15                  20

tgacgacgct gatgctccag gaccctctga accacgacgt 142


<210> 368
<211> 21
<212> PRT
<213> Conus omaria

<400> 368
Leu Thr Ile Lys Gly Cys Cys Ser Asp Pro Ser Cys Asn Val Asn Asn
1               5                   10                  15

Pro Asp Tyr Cys Gly
            20


<210> 369
<211> 157
<212> DNA
<213> Conus omaria

<220>
<221> CDS
<222> (52)..(117)

<400> 369
tctaatggca ggaatgccgc agccaaattc aaagcgcctg ccctgatgga g ctg acc 57
Leu Thr
1

gtc agg gaa gaa tgc tgt tca gac cct cgc tgt tcc gtg gga cat caa 105
Val Arg Glu Glu Cys Cys Ser Asp Pro Arg Cys Ser Val Gly His Gln
        5                   10                  15

gat atg tgt cgg tgaagcacgt gatgctccag gaccctctga accacgacgt 157
Asp Met Cys Arg
    20


<210> 370
<211> 22
<212> PRT

<213> Conus omaria

<400> 370
Leu Thr Val Arg Glu Glu Cys Cys Ser Asp Pro Arg Cys Ser Val Gly
1               5                   10                  15

His Gln Asp Met Cys Arg
            20


<210> 371
<211> 151
<212> DNA
<213> Conus purpurascens

<220>
<221> CDS
<222> (1)..(120)

<400> 371
act gat ggc agg aat gct gca gcc ata gcg ctt gac ctg atc gct ccg     48
Thr Asp Gly Arg Asn Ala Ala Ala Ile Ala Leu Asp Leu Ile Ala Pro
1               5                   10                  15

gcc gtc agg gga gga tgc tgt tcc aat cct gcc tgt tta gtg aat cat     96
Ala Val Arg Gly Gly Cys Cys Ser Asn Pro Ala Cys Leu Val Asn His
            20                  25                  30

cta gaa atg tgt ggt aaa aga cgc tgatgcccca ggaccctctg aaccacgacg   150
Leu Glu Met Cys Gly Lys Arg Arg
        35                  40

t                                                                   151


<210> 372
<211> 40
<212> PRT
<213> Conus purpurascens

<400> 372
Thr Asp Gly Arg Asn Ala Ala Ala Ile Ala Leu Asp Leu Ile Ala Pro
1               5                   10                  15

Ala Val Arg Gly Gly Cys Cys Ser Asn Pro Ala Cys Leu Val Asn His
            20                  25                  30

Leu Glu Met Cys Gly Lys Arg Arg
        35                  40


<210> 373
<211> 160
<212> DNA
<213> Conus purpurascens

<220>
<221> CDS
<222> (1)..(120)

<400> 373
tct gat ggc agg gat gcc gca gcc aac gac aaa gcg tct gac ctg atc    48
Ser Asp Gly Arg Asp Ala Ala Ala Asn Asp Lys Ala Ser Asp Leu Ile
1               5                   10                  15

gct ctg acc gcc agg aga gat cca tgc tgt ttc aat cct gcc tgt aac    96
Ala Leu Thr Ala Arg Arg Asp Pro Cys Cys Phe Asn Pro Ala Cys Asn
            20                  25                  30

gtg aat aat cca cag att tgt ggt tgaagacgct gatgctccag gaccctctga   150
Val Asn Asn Pro Gln Ile Cys Gly
        35                  40

accacgacgt                                                         160


<210> 374
<211> 40
<212> PRT
<213> Conus purpurascens

<400> 374
Ser Asp Gly Arg Asp Ala Ala Ala Asn Asp Lys Ala Ser Asp Leu Ile
1               5                   10                  15

Ala Leu Thr Ala Arg Arg Asp Pro Cys Cys Phe Asn Pro Ala Cys Asn
            20                  25                  30

Val Asn Asn Pro Gln Ile Cys Gly
        35                  40


<210> 375
<211> 151
<212> DNA
<213> Conus purpurascens

<220>
<221> CDS
<222> (1)..(120)

<400> 375
tct gat ggc agg gat gct gag aaa aca ggc ttt gac acg acc att gtg    48
Ser Asp Gly Arg Asp Ala Glu Lys Thr Gly Phe Asp Thr Thr Ile Val
1               5                   10                  15

ccg gaa gac tgc tgt tcg gat cct tcc tgt tgg agg ctg cat agt tta    96
Pro Glu Asp Cys Cys Ser Asp Pro Ser Cys Trp Arg Leu His Ser Leu
            20                  25                  30

gct tgt act gga att gta aac cgc tgatgctcca ggaccctctg aaccacgacg   150
Ala Cys Thr Gly Ile Val Asn Arg
        35                  40

t                                                                  151


<210> 376
<211> 40
<212> PRT

<213> Conus purpurascens

<400> 376
Ser Asp Gly Arg Asp Ala Glu Lys Thr Gly Phe Asp Thr Thr Ile Val
1               5                   10                  15

Pro Glu Asp Cys Cys Ser Asp Pro Ser Cys Trp Arg Leu His Ser Leu
            20                  25                  30

Ala Cys Thr Gly Ile Val Asn Arg
        35                  40


<210> 377
<211> 142
<212> DNA
<213> Conus purpurascens

<220>
<221> CDS
<222> (1)..(111)

<400> 377
act gat ggc agg agt gct gca gcc ata gcg ttt gcc ctg atc gct ccg      48
Thr Asp Gly Arg Ser Ala Ala Ala Ile Ala Phe Ala Leu Ile Ala Pro
1               5                   10                  15

acc gtc tgc tgt act aat cct gcc tgt ctc gtg aat aat ata cgc ttt      96
Thr Val Cys Cys Thr Asn Pro Ala Cys Leu Val Asn Asn Ile Arg Phe
            20                  25                  30

tgt ggt gga aga cgc tgatgcccca ggaccctctg aaccacgacg t              142
Cys Gly Gly Arg Arg
        35


<210> 378
<211> 37
<212> PRT
<213> Conus purpurascens

<400> 378
Thr Asp Gly Arg Ser Ala Ala Ala Ile Ala Phe Ala Leu Ile Ala Pro
1               5                   10                  15

Thr Val Cys Cys Thr Asn Pro Ala Cys Leu Val Asn Asn Ile Arg Phe
            20                  25                  30

Cys Gly Gly Arg Arg
        35


<210> 379
<211> 157
<212> DNA
<213> Conus regius

<220>
<221> CDS
<222> (1)..(117)

<400> 379
tct gat gga aga aat gcc gca agc gac gcc aaa gcg ttt ccc cgg atc     48
Ser Asp Gly Arg Asn Ala Ala Ser Asp Ala Lys Ala Phe Pro Arg Ile
1               5                   10                  15

gct cca atc gtc agg gac gaa tgc tgt agc gat cct agg tgt cac ggg     96
Ala Pro Ile Val Arg Asp Glu Cys Cys Ser Asp Pro Arg Cys His Gly
            20                  25                  30

aat aat cgg gac cac tgt gct tgaagacgct gctgctccag gaccctctga       147
Asn Asn Arg Asp His Cys Ala
        35

accacgacgt                                                          157


<210> 380
<211> 39
<212> PRT
<213> Conus regius

<400> 380
Ser Asp Gly Arg Asn Ala Ala Ser Asp Ala Lys Ala Phe Pro Arg Ile
1               5                   10                  15

Ala Pro Ile Val Arg Asp Glu Cys Cys Ser Asp Pro Arg Cys His Gly
            20                  25                  30

Asn Asn Arg Asp His Cys Ala
        35


<210> 381
<211> 156
<212> DNA
<213> Conus regius

<220>
<221> CDS
<222> (1)..(117)

<400> 381
tct gat ggc agg aat acc gcg gcc gac gaa aaa gcg tcc gac ctg atc     48
Ser Asp Gly Arg Asn Thr Ala Ala Asp Glu Lys Ala Ser Asp Leu Ile
1               5                   10                  15

tct caa act gtc aag aga gat tgc tgt tcc cat cct ctc tgt aga tta     96
Ser Gln Thr Val Lys Arg Asp Cys Cys Ser His Pro Leu Cys Arg Leu
            20                  25                  30

ttt gtt cca gga ctt tgt att tgaagacgct gctgctccag gaccctctga       147
Phe Val Pro Gly Leu Cys Ile
        35

accacgact                                                           156


<210> 382
<211> 39

<212> PRT
<213> Conus regius

<400> 382
Ser Asp Gly Arg Asn Thr Ala Ala Asp Glu Lys Ala Ser Asp Leu Ile
1               5                   10                  15

Ser Gln Thr Val Lys Arg Asp Cys Cys Ser His Pro Leu Cys Arg Leu
            20                  25                  30

Phe Val Pro Gly Leu Cys Ile
        35

<210> 383
<211> 157
<212> DNA
<213> Conus regius

<220>
<221> CDS
<222> (1)..(117)

<400> 383
tct gat ggc agg aat gcc gca gcc gac aac aaa gcg tct gac cta atc      48
Ser Asp Gly Arg Asn Ala Ala Ala Asp Asn Lys Ala Ser Asp Leu Ile
1               5                   10                  15

gct caa atc gtc agg aga gga tgc tgt tcc cat cct gtc tgt aaa gtg      96
Ala Gln Ile Val Arg Arg Gly Cys Cys Ser His Pro Val Cys Lys Val
            20                  25                  30

agg tat cca gac ctg tgt cgt tgaagacgct gctgctccag gaccctctga        147
Arg Tyr Pro Asp Leu Cys Arg
        35

accacgacgt                                                           157


<210> 384
<211> 39
<212> PRT
<213> Conus regius

<400> 384
Ser Asp Gly Arg Asn Ala Ala Ala Asp Asn Lys Ala Ser Asp Leu Ile
1               5                   10                  15

Ala Gln Ile Val Arg Arg Gly Cys Cys Ser His Pro Val Cys Lys Val
            20                  25                  30

Arg Tyr Pro Asp Leu Cys Arg
        35


<210> 385
<211> 157
<212> DNA
<213> Conus regius

<220>

<221> CDS
<222> (1)..(117)

<400> 385
tct gat ggc agg aat gcc gca gcc gac aac aga gcg tct gac cta atc     48
Ser Asp Gly Arg Asn Ala Ala Ala Asp Asn Arg Ala Ser Asp Leu Ile
1               5                   10                  15

gct caa atc gtc agg aga gga tgc tgt tcc cat cct gcc tgt aat gtg     96
Ala Gln Ile Val Arg Arg Gly Cys Cys Ser His Pro Ala Cys Asn Val
            20                  25                  30

aat aat cca cac att tgt ggt tgaagacgct gctgctccag gaccctctga       147
Asn Asn Pro His Ile Cys Gly
        35

accacgacgt                                                         157

<210> 386
<211> 39
<212> PRT
<213> Conus regius

<400> 386
Ser Asp Gly Arg Asn Ala Ala Ala Asp Asn Arg Ala Ser Asp Leu Ile
1               5                   10                  15

Ala Gln Ile Val Arg Arg Gly Cys Cys Ser His Pro Ala Cys Asn Val
            20                  25                  30

Asn Asn Pro His Ile Cys Gly
        35


<210> 387
<211> 157
<212> DNA
<213> Conus regius

<220>
<221> CDS
<222> (1)..(117)

<400> 387
tct gat ggc agg aat gcc gca gcc gac aac aaa ccg tct gac cta atc     48
Ser Asp Gly Arg Asn Ala Ala Ala Asp Asn Lys Pro Ser Asp Leu Ile
1               5                   10                  15

gct caa atc gtc agg aga gga tgc tgt tcg cat cct gtc tgt aaa gtg     96
Ala Gln Ile Val Arg Arg Gly Cys Cys Ser His Pro Val Cys Lys Val
            20                  25                  30

agg tat tca gac atg tgt ggt tgaagacgct gctgctccag gaccctctga       147
Arg Tyr Ser Asp Met Cys Gly
        35

accacgacgt                                                         157


<210> 388

<211> 39
<212> PRT
<213> Conus regius

<400> 388
Ser Asp Gly Arg Asn Ala Ala Ala Asp Asn Lys Pro Ser Asp Leu Ile
1               5                   10                  15

Ala Gln Ile Val Arg Arg Gly Cys Cys Ser His Pro Val Cys Lys Val
            20                  25                  30

Arg Tyr Ser Asp Met Cys Gly
        35


<210> 389
<211> 154
<212> DNA
<213> Conus stercusmuscarum

<220>
<221> CDS
<222> (1)..(114)

<400> 389
tct gat ggc agg aat gca gag cga cga caa agc gtc tgt cct ggt cgc     48
Ser Asp Gly Arg Asn Ala Glu Arg Arg Gln Ser Val Cys Pro Gly Arg
1               5                   10                  15

tct ggc ccc agg gga gga tgt tgt tcc cac cct gcc tgt aag gtg cat     96
Ser Gly Pro Arg Gly Gly Cys Cys Ser His Pro Ala Cys Lys Val His
            20                  25                  30

ttt cca cac agt tgt ggt tgacgacgct gatgctccag gaccctctga           144
Phe Pro His Ser Cys Gly
        35

accacgacgt                                                          154


<210> 390
<211> 38
<212> PRT
<213> Conus stercusmuscarum

<400> 390
Ser Asp Gly Arg Asn Ala Glu Arg Arg Gln Ser Val Cys Pro Gly Arg
1               5                   10                  15

Ser Gly Pro Arg Gly Gly Cys Cys Ser His Pro Ala Cys Lys Val His
            20                  25                  30

Phe Pro His Ser Cys Gly
        35


<210> 391
<211> 145
<212> DNA
<213> Conus stercusmuscarum

<220>
<221> CDS
<222> (1)..(114)

<400> 391
tct gat ggc agg aat gcc gca gcc agc gac aga gcg tct gac gcg gcc     48
Ser Asp Gly Arg Asn Ala Ala Ala Ser Asp Arg Ala Ser Asp Ala Ala
1               5                   10                  15

cac cag gta tgc tgt tcc aac cct gtc tgt cac gtg gat cat cca gaa     96
His Gln Val Cys Cys Ser Asn Pro Val Cys His Val Asp His Pro Glu
            20                  25                  30

ctt tgt cgt aga aga cgc tgatgctcca ggaccctctg aaccacgacg t          145
Leu Cys Arg Arg Arg Arg
        35


<210> 392
<211> 38
<212> PRT
<213> Conus stercusmuscarum

<400> 392
Ser Asp Gly Arg Asn Ala Ala Ala Ser Asp Arg Ala Ser Asp Ala Ala
1               5                   10                  15
His Gln Val Cys Cys Ser Asn Pro Val Cys His Val Asp His Pro Glu
            20                  25                  30

Leu Cys Arg Arg Arg Arg
        35


<210> 393
<211> 154
<212> DNA
<213> Conus striatus

<220>
<221> CDS
<222> (1)..(123)

<400> 393
tct gat ggc agg aat gcc gcg gcc aac gac aaa gcg tct gac ctg gtc     48
Ser Asp Gly Arg Asn Ala Ala Ala Asn Asp Lys Ala Ser Asp Leu Val
1               5                   10                  15

gct ccg gcc atc agg gga tgc tgt tcc cac cct gtc tgt aac ttg agt     96
Ala Pro Ala Ile Arg Gly Cys Cys Ser His Pro Val Cys Asn Leu Ser
            20                  25                  30

aat cca caa att tgt cgt gga aga cgc tgatgctcca ggaccctctg          143
Asn Pro Gln Ile Cys Arg Gly Arg Arg
        35                  40

aaccacgacg t                                                        154


<210> 394

<211> 41
<212> PRT
<213> Conus striatus

<400> 394
Ser Asp Gly Arg Asn Ala Ala Ala Asn Asp Lys Ala Ser Asp Leu Val
1               5                   10                  15

Ala Pro Ala Ile Arg Gly Cys Cys Ser His Pro Val Cys Asn Leu Ser
            20                  25                  30

Asn Pro Gln Ile Cys Arg Gly Arg Arg
        35                  40


<210> 395
<211> 117
<212> DNA
<213> Conus textile

<220>
<221> CDS
<222> (1)..(114)

<400> 395
ttt cat ggc agg aat gcc gca gcc aaa gcg tct ggc ctg gtc ggt ctg     48
Phe His Gly Arg Asn Ala Ala Ala Lys Ala Ser Gly Leu Val Gly Leu
1               5                   10                  15

acc gac aag agg caa gaa tgc tgt tct cat cct gcc tgt aac gta gat     96
Thr Asp Lys Arg Gln Glu Cys Cys Ser His Pro Ala Cys Asn Val Asp
            20                  25                  30
cat cca gaa att tgt cgt tga                                         117
His Pro Glu Ile Cys Arg
        35


<210> 396
<211> 38
<212> PRT
<213> Conus textile

<400> 396
Phe His Gly Arg Asn Ala Ala Ala Lys Ala Ser Gly Leu Val Gly Leu
1               5                   10                  15

Thr Asp Lys Arg Gln Glu Cys Cys Ser His Pro Ala Cys Asn Val Asp
            20                  25                  30

His Pro Glu Ile Cys Arg
        35


<210> 397
<211> 151
<212> DNA
<213> Conus tulipa

<220>
<221> CDS

<222> (1)..(120)

<400> 397
act gat ggc agg agt gct gca gcc ata gcg ttt gcc ctg atc gct ccg    48
Thr Asp Gly Arg Ser Ala Ala Ala Ile Ala Phe Ala Leu Ile Ala Pro
1               5                   10                  15

acc gtc tgg gaa gga tgc tgt tct aat cct gcc tgt ctc gtg aat cat    96
Thr Val Trp Glu Gly Cys Cys Ser Asn Pro Ala Cys Leu Val Asn His
            20                  25                  30

ata cgc ttt tgt ggt gga aga cgc tgatgcccca ggaccctctg aaccacgacg   150
Ile Arg Phe Cys Gly Gly Arg Arg
        35                  40

t                                                                  151


<210> 398
<211> 40
<212> PRT
<213> Conus tulipa

<400> 398
Thr Asp Gly Arg Ser Ala Ala Ala Ile Ala Phe Ala Leu Ile Ala Pro
1               5                   10                  15

Thr Val Trp Glu Gly Cys Cys Ser Asn Pro Ala Cys Leu Val Asn His
            20                  25                  30

Ile Arg Phe Cys Gly Gly Arg Arg
        35                  40


<210> 399
<211> 157
<212> DNA
<213> Conus virgo

<220>
<221> CDS
<222> (1)..(117)

<400> 399
tct aat ggc atg aat gcc gca gcc atc agg aaa gcg tct gcc ctg gtg    48
Ser Asn Gly Met Asn Ala Ala Ala Ile Arg Lys Ala Ser Ala Leu Val
1               5                   10                  15

gct cag atc gcc cat cga gac tgc tgt gac gat cct gcc tgc acc gtg    96
Ala Gln Ile Ala His Arg Asp Cys Cys Asp Asp Pro Ala Cys Thr Val
            20                  25                  30

aat aat cca ggc ctt tgc act tgaagatgct gctgccccag gaccctctga       147
Asn Asn Pro Gly Leu Cys Thr
        35

accacgacgt                                                         157


<210> 400

<211> 39
<212> PRT
<213> Conus virgo

<400> 400
Ser Asn Gly Met Asn Ala Ala Ala Ile Arg Lys Ala Ser Ala Leu Val
1               5                   10                  15

Ala Gln Ile Ala His Arg Asp Cys Cys Asp Asp Pro Ala Cys Thr Val
            20                  25                  30

Asn Asn Pro Gly Leu Cys Thr
        35


<210> 401
<211> 154
<212> DNA
<213> Conus geographus

<220>
<221> CDS
<222> (1)..(114)

<400> 401
tct gat ggc ggg aat gcc gca gca aaa gag tct gac gtg atc gct ctg     48
Ser Asp Gly Gly Asn Ala Ala Ala Lys Glu Ser Asp Val Ile Ala Leu
1               5                   10                  15

acc gtc tgg aaa tgc tgt acc att cct tcc tgt tat gag aaa aaa aaa     96
Thr Val Trp Lys Cys Cys Thr Ile Pro Ser Cys Tyr Glu Lys Lys Lys
            20                  25                  30

att aaa gca tgt gtc ttt tgacgacgct gatgctccag gaccctctga           144
Ile Lys Ala Cys Val Phe
        35

accacgacgt                                                         154


<210> 402
<211> 38
<212> PRT
<213> Conus geographus

<400> 402
Ser Asp Gly Gly Asn Ala Ala Ala Lys Glu Ser Asp Val Ile Ala Leu
1               5                   10                  15

Thr Val Trp Lys Cys Cys Thr Ile Pro Ser Cys Tyr Glu Lys Lys Lys
            20                  25                  30

Ile Lys Ala Cys Val Phe
        35


<210> 403
<211> 154
<212> DNA
<213> Conus regius

<220>
<221> CDS
<222> (1)..(114)

<400> 403
tct gat ggc gca gtc gac gac aaa gcg ttg gat cga atc gct gaa atc     48
Ser Asp Gly Ala Val Asp Asp Lys Ala Leu Asp Arg Ile Ala Glu Ile
1               5                   10                  15

gtc agg aga gga tgc tgt ggc aat cct gcc tgt agc ggc tcc tcg aaa     96
Val Arg Arg Gly Cys Cys Gly Asn Pro Ala Cys Ser Gly Ser Ser Lys
            20                  25                  30

gat gca ccc tct tgt ggt tgaagacgct gctgctccag gaccctctga            144
Asp Ala Pro Ser Cys Gly
        35

accacgacgt                                                          154


<210> 404
<211> 38
<212> PRT
<213> Conus regius

<400> 404
Ser Asp Gly Ala Val Asp Asp Lys Ala Leu Asp Arg Ile Ala Glu Ile
1               5                   10                  15

Val Arg Arg Gly Cys Cys Gly Asn Pro Ala Cys Ser Gly Ser Ser Lys
            20                  25                  30

Asp Ala Pro Ser Cys Gly
        35

## Claims

1. An isolated α-conotoxin peptide selected from the group consisting of:
(i) a peptide having the amino acid sequence Gly-Cys-Cys-Ser-Asp-Xaa₅-Arg-Cys-Arg-Xaa₄-Arg-Cys-Arg (SEQ ID NO:9) and
(ii) a derivative of (i),
wherein Xaa₄ is Tyr, nor-Tyr, mono-halo-Tyr, di-halo-Tyr, O-sulpho-Tyr, O-phospho-Tyr or nitro-Tyr, and Xaa₅ is Pro or hydroxy-Pro, and the C-terminus contains a carboxyl or amide group.

2. The isolated α-conotoxin peptide of claim 1, wherein Xaa₄ is Tyr.

3. The isolated α-conotoxin peptide of claim 1, wherein Xaa₄ is mono-iodo-Tyr or di-iodo-Tyr.

4. The isolated α-conotoxin peptide of claim 1, wherein Xaa₅ is Pro.

5. The isolated α-conotoxin peptide of claim 1, wherein Xaa₅ is hydroxy-Pro.

6. The isolated α-conotoxin peptide of claim 1, wherein Xaa₄ is Tyr and Xaa₅ is Pro.

7. The isolated α-conotoxin peptide of claim 6, wherein the C-terminus contains an amide group.

8. The isolated α-conotoxin peptide of any one of claims 1-7, wherein the peptide is the derivative of (i) and wherein the derivative is a peptide in which an Arg residue may be substituted by Lys, ornithine, homoargine, N-methyl-Lys, N,N-dimethyl-Lys, N,N,N-trimethyl-Lys or any unnatural basic amino acid; a Tyr residue may be substituted with any unnatural hydroxy containing amino acid or with the 3-hydroxyl or 2-hydroxyl isomers and corresponding O-sulpho- and O-phospho-derivatives; a Ser residue may be substituted with Thr; the Cys residues may be in D or L configuration and may optionally be substituted with homocysteine (D or L); or an acidic amino acid residue may be substituted with any synthetic acidic bioisoteric amino acid surrogate, e.g., tetrazolyl derivatives of Gly and Ala.

9. The isolated α-conotoxin peptide of any one of claims 1-8 which is modified to contain an O-glycan, an S-glycan or an N-glycan.

10. An isolated precursor of the α-conotoxin peptide of claim 1, wherein the precursor has the amino acid sequence SNKRKNAAMLDMIAQHAIRGCCSDPRCRYRCR (SEQ ID NO:244).
